(19) Europäisches Patentamt
European Patent Office
Office européen des brevets

(11) EP 0 839 806 B1

(12) EUROPEAN PATENT SPECIFICATION

(45) Date of publication and mention of the grant of the patent:
**09.07.2003 Bulletin 2003/28**

(21) Application number: **97308645.7**

(22) Date of filing: **29.10.1997**

(51) Int Cl.[7]: **C07D 209/88**, A61K 31/40, C07F 9/38, C07D 471/04 // (C07D471/04, 221:00, 209:00)

# (54) Substituted tricyclics

Substituierte tricyclische Verbindungen

Composés tricycliques substitués

(84) Designated Contracting States:
**AT BE CH DE DK ES FI FR GB GR IE IT LI LU NL PT SE**
Designated Extension States:
**AL LT LV RO SI**

(30) Priority: **30.10.1996 US 29849 P**

(43) Date of publication of application:
**06.05.1998 Bulletin 1998/19**

(73) Proprietor: **ELI LILLY AND COMPANY**
**Indianapolis, Indiana 46285 (US)**

(72) Inventors:
- **Bach, Nicholas James**
  **Indianapolis, Indiana 46217 (US)**
- **Dillard, Robert Delane**
  **Zionsville, Indiana 46077 (US)**
- **Draheim, Susan Elizabeth**
  **Indianapolis, Indiana 46220 (US)**
- **Morin, John Michael, Jr.**
  **Brownsburg, Indiana 46112 (US)**

(74) Representative: **Pritchard, Judith et al**
**Eli Lilly and Company Limited**
**Lilly Research Centre**
**Erl Wood Manor**
**Windlesham, Surrey GU20 6PH (GB)**

(56) References cited:
**EP-A- 0 620 214** **EP-A- 0 620 215**
**US-A- 3 939 177** **US-A- 5 420 289**

- **GARRATT ET AL.: "Mapping the melatonin receptor.2. Synthesis and biological activity of indole derived melatonin analogs with restricted conformations of the C-3 amidoethane side chain" BIOORGANIC & MEDICINAL CHEMISTRY LETTERS, vol. 4, no. 13, 1994, pages 1559-64, XP002054522**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).



Printed by Jouve, 75001 PARIS (FR)

## Description

BACKGROUND OF THE INVENTION

Field of the Invention

This invention relates to novel substituted tricyclic organic compounds useful for inhibiting $sPLA_2$ mediated release of fatty acids for conditions such as septic shock.

Background Information

The structure and physical properties of human non-pancreatic secretory phospholipase $A_2$ (hereinafter called, "$sPLA_2$") has been thoroughly described in two articles, namely, "Cloning and Recombinant Expression of Phospholipase $A_2$ Present in Rheumatoid Arthritic Synovial Fluid" by Seilhamer, Jeffrey J.; Pruzanski, Waldemar; Vadas Peter; Plant, Shelley; Miller, Judy A.; Kloss, Jean; and Johnson, Lorin K.; The Journal of Biological Chemistry, Vol. 264, No. 10, Issue of April 5, pp. 5335-5338, 1989; and "Structure and Properties of a Human Non-pancreatic Phospholipase $A_2$" by Kramer, Ruth M.; Hession, Catherine; Johansen, Berit; Hayes, Gretchen; McGray, Paula; Chow, E. Pingchang; Tizard, Richard; and Pepinsky, R. Blake; The Journal of Biological Chemistry, Vol. 264, No. 10, Issue of April 5, pp. 5768-5775, 1989; the disclosures of which are incorporated herein by reference.

It is believed that $sPLA_2$ is a rate limiting enzyme in the arachidonic acid cascade which hydrolyzes membrane phospholipids. Thus, it is important to develop compounds which inhibit $sPLA_2$ mediated release of fatty acids (e.g., arachidonic acid). Such compounds would be of value in general treatment of conditions induced and/or maintained by overproduction of $sPLA_2$ such as septic shock, adult respiratory distress syndrome, pancreatitis, trauma-induced shock, bronchial asthma, allergic rhinitis, rheumatoid arthritis, etc.

It is desirable to develop new compounds and treatments for $sPLA_2$ induced diseases.

Alexander, et al., United States Patent Nos. 3,939,177 and 3,979,391, disclose 1,2,3,4-tetrahydrocarbazoles useful as antibacterial agents.

Summary of the Invention

This invention provides tricyclic compounds as depicted in the general formula (III) below:

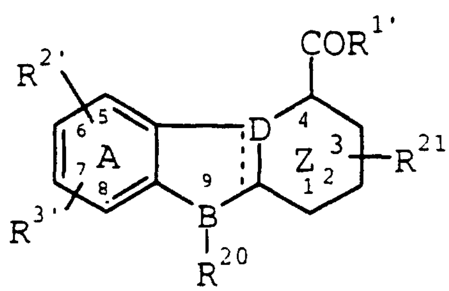

(III)

wherein ;

A is phenyl or pyridyl wherein the nitrogen is at the 5-, 6-, 7- or 8-position;
one of B or D is nitrogen and the other is carbon;
Z is cyclohexenyl, phenyl, pyridyl, wherein the nitrogen is at the 1-, 2-, or 3-position, or a 6-membered heterocyclic ring having one heteroatom selected from the group consisting of sulfur or oxygen at the 1-, 2- or 3-position, and nitrogen at the 1-, 2-, 3- or 4-position;
---- is a double or single bond;
$R^{20}$ is selected from groups (a), (b) and (c) where;

(a) is -($C_5$-$C_{20}$)alkyl, -($C_5$-$C_{20}$)alkenyl, -($C_5$-$C_{20}$)alkynyl, carbocyclic radicals, or heterocyclic radicals, or
(b) is a member of (a) substituted with one or more independently selected non-interfering substituents; or
(c) is the group -(L)-$R^{80}$; where, -(L)- is a divalent linking group of 1 to 12 atoms selected from carbon, hydrogen, oxygen, nitrogen, and sulfur; wherein the combination of atoms in -(L)- are selected from the group consisting

of (i) carbon and hydrogen only, (ii) one sulfur only, (iii) one oxygen only, (iv) one or two nitrogen and hydrogen only, (v) carbon, hydrogen, and one sulfur only, and (vi) and carbon, hydrogen, and oxygen only; and where $R^{80}$ is a group selected from (a) or (b);

$R^{21}$ is a non-interfering substituent;
$R^{1'}$ is $-NHNH_2$ or $-NH_2$;
$R^{2'}$ is selected from the group -OH, $-O(CH_2)_tR^5$ where $R^5$ is CN or phenyl, or $-(L_a)$-(acidic group); wherein $-(L_a)$- is an acid linker having an acid linker length of 1 to 7 and t is 1-5;
$R^{3'}$ is selected from non-interfering substituent, carbocyclic radicals, carbocyclic radicals substituted with non-interfering substituents, heterocyclic radicals, and heterocyclic radicals substituted with non-interfering substituents;
or a pharmaceutically acceptable salt, racemate, solvate, tautomer, optical isomer or prodrug derivative thereof; provided that when D is nitrogen, the heteroatom of Z is selected from the group consisting of sulfur or oxygen at the 1-, 2- or 3-position and nitrogen at the 1-, 2-, 3- or 4-position.

These substituted tricyclics are effective in inhibiting human $sPLA_2$ mediated release of fatty acids.
This invention is also a pharmaceutical formulation comprising a compound of formula III in association with one or more pharmaceutically acceptable diluents, carriers and excipients.
This invention is also a method of inhibiting $sPLA_2$ comprising administering to a mammal in need of such treatment a therapeutically effective amount of a compound of formula III.
According to a further aspect of the present invention, there is provided a method of selectively inhibiting $sPLA_2$ in a mammal in need of such treatment comprising administering to said mammal a therapeutically effective amount of a compound of formula III.
This invention also provides a method of alleviating the pathological effects of septic shock, adult respiratory distress syndrome, pancreatitis, trauma-induced shock, bronchial asthma, allergic rhinitis, rheumatoid arthritis, cystic fibrosis, stroke, acute bronchitis, chronic bronchitis, acute bronchiolitis, chronic bronchiolitis, osteoarthritis, gout, spondylarthropathris, ankylosing spondylitis, Reiter's syndrome, psoriatic arthropathy, enterapathric spondylitis, Juvenile arthropathy or juvenile ankylosing spondylitis, Reactive arthropathy, infectious or post-infectious arthritis, gonoccocal arthritis, tuberculous arthritis, viral arthritis, fungal arthritis, syphilitic arthritis, Lyme disease, arthritis associated with "vasculitic syndromes", polyarteritis nodosa, hypersensitivity vasculitis, Luegenec's granulomatosis, polymyalgin rheumatica, joint cell arteritis, calcium crystal deposition arthropathris, pseudo gout, non-articular rheumatism, bursitis, tenosynomitis, epicondylitis (tennis elbow), carpal tunnel syndrome, repetitive use injury (typing), miscellaneous forms of arthritis, neuropathic joint disease (charco and joint), hemarthrosis (hemarthrosic), Henoch-Schonlein Purpura, hypertrophic osteoarthropathy, multicentric reticulohistiocytosis, arthritis associated with certain diseases, surcoilosis, hemochromatosis, sickle cell disease and other hemoglobinopathries, hyperlipoproteineimia, hypogammaglobulinemia, hyperparathyroidism, acromegaly, familial Mediterranean fever, Behat's Disease, systemic lupus erythrematosis, or relapsing polychondritis and related diseases which comprises administering to a mammal in need of such treatment a therapeutically effective amount of the compound of formula III in an amount sufficient to inhibit $sPLA_2$ mediated release of fatty acid and to thereby inhibit or prevent the arachidonic acid cascade and its deleterious products.
This invention provides, in addition, a process of preparing compounds of formula

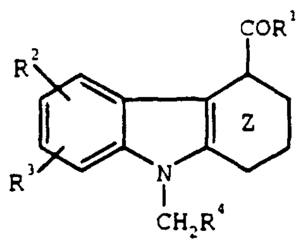

wherein

$R^1$ is $-NHNH_2$, or $-NH_2$;
$R^2$ is -OH, or $-O(CH_2)_mR^5$ where
$R^5$ is H, $-CO_2H$, $-CO_2(C_1-C_4$ alkyl),

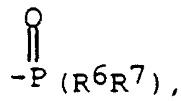

phenyl, or phenyl substituted with $-CO_2H$ or $-CO_2(C_1-C_4$ alkyl) where $R^6$ and $R^7$ are each independently -OH or $-O(C_1-C_4$ alkyl) and m is 1-3;
$R^3$ is H, $-O(C_1-C_4$ alkyl), or $-(CH_2)_nR^8$ where
$R^8$ is H, $-NR^9R^{10}$,

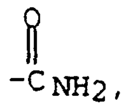

-CN, or phenyl where $R^9$ and $R^{10}$ are independently $-(C_1-C_4)$alkyl, or -phenyl$(C_1-C_4)$alkyl and n is 1-8;
$R^4$ is H, $-(C_5-C_{14})$alkyl, $-(C_3-C_{14})$cycloalkyl, phenyl, or phenyl substituted with 1 or 2 substituents selected from the group consisting of $-(C_1-C_4)$alkyl, $-(C_1-C_4)$alkoxy, -phenyl$(C_1-C_4)$alkyl, $-(C_1-C_4)$alkylthio, halo or phenyl; and
Z is phenyl comprising dehydrogenating a compound of the formula

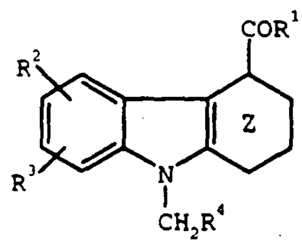

wherein

$R^1$ is $-NHNH_2$, or $-NH_2$;
$R^2$ is -OH, or $-O(CH_2)_mR^5$ where
$R^5$ is H, $-CO_2H$, $-CO_2(C_1-C_4$ alkyl),

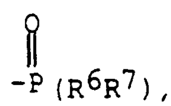

phenyl, or phenyl substituted with $-CO_2H$ or $-CO_2(C_1-C_4$ alkyl) where $R^6$ and $R^7$ are each independently -OH or $-O(C_1-C_4$ alkyl) and m is 1-3;
$R^3$ is H, $-O(C_1-C_4$ alkyl), or $-(CH_2)_nR^8$ where $R^8$ is H, $-NR^9R^{10}$,

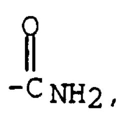

-CN, or phenyl where $R^9$ and $R^{10}$ are independently $-(C_1-C_4)$alkyl, or -phenyl$(C_1-C_4)$alkyl and n is 1-8;
$R^4$ is H, $-(C_5-C_{14})$alkyl, $-(C_3-C_{14})$cycloalkyl, phenyl, or phenyl substituted with 1 or 2 substituents selected from the group consisting of $-(C_1-C_4)$alkyl, $-(C_1-C_4)$alkoxy, -phenyl$(C_1-C_4)$alkyl, $-(C_1-C_4)$alkylthio, halo or phenyl; and
Z is cyclohexxenyl.

Other objects, features and advantages of the present invention will become apparent from the subsequent

description and the appended claims.

Detailed Description of the Invention

Definitions:

As used herein, the term, "alkyl" by itself or as part of another substituent means, unless otherwise defined, a straight or branched chain monovalent hydrocarbon radical such as methyl, ethyl, n-propyl, isopropyl, n-butyl, tertiary butyl, isobutyl, sec-butyl tert butyl, n-pentyl, isopentyl, neopentyl, heptyl, hexyl, octyl, nonyl, decyl, undecyl, dodecyl, tridecyl, tetradecyl and the like. The term "alkyl" includes -($C_1$-$C_2$)alkyl, -($C_1$-$C_4$)alkyl, -($C_1$-$C_6$)alkyl, -($C_5$-$C_{14}$)alkyl, and -($C_1$-$C_{10}$)alkyl.

The term "alkenyl" as used herein represents an olefinically unsaturated branched or linear group having at least one double bond. Examples of such groups include radicals such as vinyl, allyl, 2-butenyl, 3-butenyl, 2-pentenyl, 3-pentenyl, 4-pentenyl, 2-hexenyl, 3-hexenyl, 4-hexenyl, 5-hexenyl, 2-heptenyl, 3-heptenyl, 4-heptenyl, 5-heptenyl, 6-heptenyl as well as dienes and trienes of straight and branched chains.

The term "alkynyl" denotes such radicals as ethynyl, propynyl, butynyl, pentynyl, hexynyl, heptynyl as well as di- and tri-ynes.

The term "halo" means chloro, fluoro, bromo or iodo.

The term "-($C_1$-$C_4$)alkoxy", as used herein, denotes a group such as methoxy, ethoxy, n-propoxy, isopropoxy, n-butoxy, t-butoxy and like groups, attached to the remainder of the molecule by the oxygen atom.

The term "phenyl($C_1$-$C_4$)alkyl" refers to a straight or branched chain alkyl group having from one to four carbon atoms attached to a phenyl ring which chain is attached to the remainder of the molecule. Typical phenylalkyl groups include benzyl, phenylethyl, phenylpropyl, phenylisopropyl, and phenylbutyl.

The term "-($C_1$-$C_4$)alkylthio" defines a straight or branched alkyl chain having one to four carbon atoms attached to the remainder of the molecule by a sulfur atom. Typical -($C_1$-$C_4$)alkylthio groups include methylthio, ethylthio, propylthio, butylthio and the like.

The term "-($C_3$-$C_{14}$)cycloalkyl" includes groups such as cyclopropyl, cyclobutyl, cyclopentyl, cyclohexyl, cycloheptyl, cyclooctyl, cyclononyl, cyclodecyl, cycloundecyl, cyclododecyl, cyclotridecyl, cyclotetradecyl and the like. The term "-($C_3$-$C_{14}$)cycloalkyl" includes and - ($C_3$-$C_7$)cycloalkyl.

The term, "heterocyclic radical", refers to radicals derived from monocyclic or polycyclic, saturated or unsaturated, substituted or unsubstituted heterocyclic nuclei having 5 to 14 ring atoms and containing from 1 to 3 hetero atoms selected from the group consisting of nitrogen, oxygen or sulfur. Typical heterocyclic radicals are pyrrolyl, furanyl, thiophenyl, pyrazolyl, imidazolyl, phenylimidazolyl, triazolyl, isoxazolyl, oxazolyl, thiazolyl, thiadiazolyl, indolyl, carbazolyl, norharmanyl, azaindolyl, benzofuranyl, dibenzofuranyl, thianaphtheneyl, dibenzothiophenyl, indazolyl, imidazo (1.2-A)pyridinyl, benzotriazolyl, anthranilyl, 1,2-benzisoxazolyl, benzoxazolyl, benzothiazolyl, purinyl, pryidinyl, dipyridylyl, phenylpyridinyl, benzylpyridinyl, pyrimidinyl, phenylpyrimidinyl, pyrazinyl, 1,3,5-triazinyl, quinolinyl, phthalazinyl, quinazolinyl, and quinoxalinyl.

The term "carbocyclic radical" refers to radicals derived from a saturated or unsaturated, substituted or unsubstituted 5 to 14 membered organic nucleus whose ring forming atoms (other than hydrogen) are solely carbon atoms. Typical carbocyclic radicals are cycloalkyl, cycloalkenyl, phenyl, naphthyl, norbornanyl, bicycloheptadienyl, tolulyl, xylenyl, indenyl, stilbenyl, terphenylyl, diphenylethylenyl, phenylcyclohexeyl, acenaphthylenyl, and anthracenyl, biphenyl, bibenzylyl and related bibenzylyl homologues represented by the formula (bb),

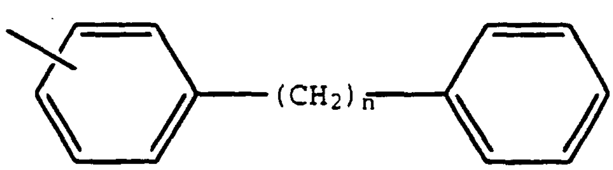

(bb)

where n is an integer from 1 to 8.

The term; "non-interfering substituent", refers to radicals suitable for substitution at positions 1, 2, 3, 7 and/ or 8 on the tricyclic nucleus (as depicted in Formula III) and radical(s) suitable for substitution on the heterocyclic radical and carbocyclic radical as defined above. Illustrative non-interfering radicals are hydrogen, -($C_1$-$C_{12}$)alkyl, -($C_2$-$C_6$) alkenyl, -($C_2$-$C_6$)alkynyl, -($C_7$-$C_{12}$)aralkyl, -($C_7$-$C_{12}$)alkaryl, -($C_3$-$C_8$)cycloalkyl, -($C_3$-$C_8$)cycloalkenyl, phenyl, tolulyl, xylenyl, biphenyl, -($C_1$-$C_6$)alkoxy, -($C_2$-$C_6$)alkenyloxy, -($C_2$-$C_6$)alkynyloxy, -($C_1$-$C_{12}$)alkoxyalkyl, -($C_1$-$C_{12}$)alkoxyalkyloxy, -($C_1$-$C_{12}$)alkylcarbonyl, -($C_1$-$C_{12}$)alkylcarbonylamino, - ($C_1$-$C_{12}$)alkoxyamino, -($C_1$-$C_{12}$)alkoxyaminocarbonyl,

-($C_1$-$C_{12}$)alkylamino, -($C_1$-$C_6$)alkylthio, - ($C_1$-$C_{12}$)alkylthiocarbonyl, -($C_1$-$C_6$)alkylsulfinyl, - ($C_1$-$C_6$)alkylsulfonyl, -($C_1$-$C_6$)haloalkoxy, -($C_1$-$C_6$)haloalkylsulfonyl, -($C_1$-$C_6$)haloalkyl, -($C_1$-$C_6$)hydroxyalkyl, -$(CH_2)_n$CN, -$(CH_2)_n$$NR^9R^{10}$, -C (O)O($C_1$-$C_6$ alkyl), -$(CH_2)_n$O($C_1$-$C_6$ alkyl), benzyloxy, phenoxy, phenylthio, -($CONHSO_2R$), -CHO, amino, amidino, halo, carbamyl, carboxyl, carbalkoxy, -$(CH_2)_nCO_2H$, cyano, cyanoguanidinyl, guanidino, hydrazide, hydrazino, hydrazido, hydroxy, hydroxyamino, nitro, phosphono, -$SO_3H$, thioacetal, thiocarbonyl, and ($C_1$-$C_6$)alkylcarbonyl; where n is from 1 to 8 and $R^9$ and $R^{10}$ are independently -($C_1$-$C_4$)alkyl or phenyl($C_1$-$C_4$)alkyl. A preferred group of non-interfering substituents include hydrogen, halo, -($C_1$-$C_3$)alkyl, - ($C_3$-$C_4$)cycloalkyl, -($C_3$-$C_4$)cycloalkenyl, -O($C_1$-$C_2$)alkyl or -S ($C_1$-$C_2$)alkyl.

The term, "acidic group" means an organic group which when attached to a tricyclic nucleus, through suitable linking atoms (hereinafter defined as the "acid linker"), acts as a proton donor capable of hydrogen bonding. Illustrative of an acidic group are the following:

-$CO_2H$,

-5-tetrazolyl,

-$SO_3H$,

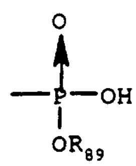
,

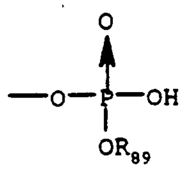
,

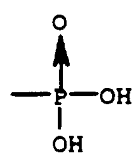
,

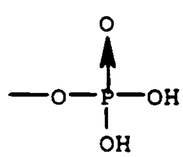
,

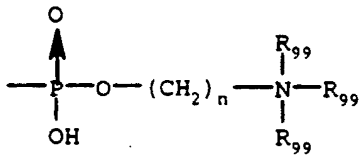

,

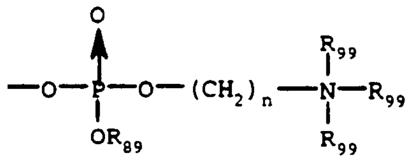

,

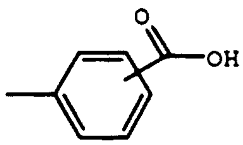

,

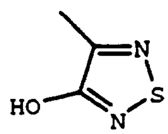

;

where n is 1 to 8, $R_{89}$ is a metal or -($C_1$-$C_{10}$)alkyl, and $R_{99}$ is hydrogen or -($C_1$-$C_{10}$)alkyl.

The words, "acid linker" refer to a divalent linking group symbolized as, -($L_a$)-, which has the function of joining the 5 or 6 position of the tricyclic nucleus to an acidic group in the general relationship:

(tricyclic nucleus) -($L_a$)- Acidic Group

The words, "acid linker length", refer to the number of atoms (excluding hydrogen) in the shortest chain of the linking group -($L_a$)- that connects the 5 or 6 position of the tricyclic nucleus with the acidic group. The presence of a carbocyclic ring in -($L_a$)- counts as the number of atoms approximately equivalent to the calculated diameter of the carbocyclic ring. Thus, a benzene or cyclohexane ring in the acid linker counts as 2 atoms in calculating the length of -($L_a$)-. Illustrative acid linker groups are;

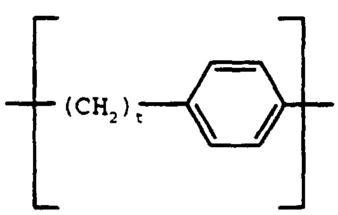

(a)

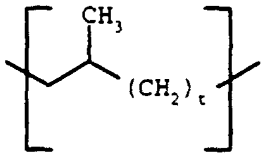

(b)

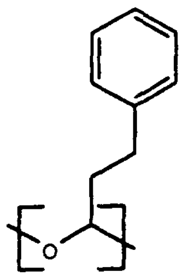

(c)

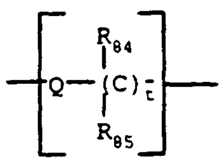

(d)

where t is 1 to 5, Q is selected from the group $-(CH_2)-$, -O-, -NH-, and -S-, and $R_{84}$ and $R_{85}$ are each independently selected from hydrogen, $-(C_1-C_{10})$alkyl, aryl, $-(C_1-C_{10})$alkaryl, $-(C_1-C_{10})$aralkyl, carboxy, carbalkoxy, and halo, when t is one (1), groups (a), (b), (c) and (d) have acid linker lengths of 3, 3, 2, and 2, respectively.

The salts of the above tricyclics are an additional aspect of the invention. In those instances where the compounds of the invention possess acidic functional groups various salts may be formed which are more water soluble and physiologically suitable than the parent compound. Representative pharmaceutically acceptable salts include but are not limited to the alkali and alkaline earth salts such as lithium, sodium, potassium, calcium, magnesium, aluminum and the like. Salts are conveniently prepared from the free acid by treating the acid in solution with a base or by exposing the acid to an ion exchange resin.

Included within the definition of pharmaceutically acceptable salts are the relatively non-toxic, inorganic and organic base addition salts of compounds of the present invention, for example, ammonium, quaternary ammonium, and amine cations, derived from nitrogenous bases of sufficient basicity to form salts with the compounds of this invention (see, for example, S. M. Serge, *et al.*, "Pharmaceutical salts," J. Phar. Sci., 66: 1-19 (1977)).

Compounds of the invention may have chiral centers and exist in optically active forms. R- and S-isomers and racemic mixtures are contemplated by this invention. A particular stereoisomer may be prepared by known methods using stereospecific reactions with starting materials containing asymmetric centers already resolved or, alternatively, by subsequent resolution of mixtures of stereoisomers using known methods.

Prodrugs are derivatives of the compounds of the invention which have chemically or metabolically cleavable groups and become by solvolysis or under physiological conditions the compounds of the invention which are pharmaceutically active in vivo. Derivatives of the compounds of this invention have activity in both their acid and base derivative forms, but the acid derivative form often offers advantages of solubility, tissue compatibility, or delayed release in a mammalian organism (see, Bundgard, H., Design of Prodrugs, pp. 7-9, 21-24, Elsevier, Amsterdam 1985). Prodrugs include acid derivatives, such as, esters prepared by reaction of the parent acidic compound with a suitable alcohol, or amides prepared by reaction of the parent acid compound with a suitable amine. Simple aliphatic esters (e.g., methyl, ethyl, propyl, isopropyl, butyl, sec-butyl, tert-butyl) or aromatic esters derived from acidic groups pendent on the compounds of this invention are preferred prodrugs. In some cases it is desirable to prepare double ester type prodrugs such as (acyloxy) alkyl esters or ((alkoxycarbonyl)oxy)alkyl esters. Preferred esters include morpholinoethyloxy and

diethylaminocarbonylmethoxy.

Preferred Compounds of the Invention

Preferred Subgroups of Compounds of Formula (III):

Another preferred subclass of compounds of formula (III) are those wherein $R^{21}$ is selected from the group hydrogen, halo, $-(C_1-C_3)$alkyl, $-(C_3-C_4)$cycloalkyl, $-(C_3-C_4)$cycloalkenyl, $-O(C_1-C_2)$alkyl and $-S(C_1-C_2)$alkyl.

Another preferred subclass of compounds of formula (III) are those wherein for $R^{2'}$, -(L)- is an alkyl chain of 1 or 2 carbon atoms.

Another preferred subclass of compounds of formula (III) are those wherein for $R^{20}$, group $R_{80}$ is selected from the group consisting of cycloalkyl, cycloalkenyl, phenyl, naphthyl, norbornanyl, bicycloheptadienyl, tolulyl, xylenyl, indenyl, stilbenyl, terphenylyl, diphenylethylenyl, phenyl-cyclohexenyl, acenaphthylenyl, and anthracenyl, biphenyl, bibenzylyl and related bibenzylyl homologues represented by the formula (bb),

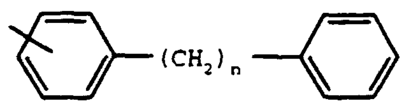

(bb)

,

where n is a number from 1 to 8. Particularly preferred are compounds wherein $R^{20}$ is selected from the group consisting of

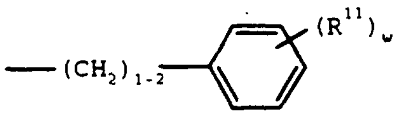

and

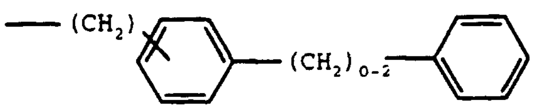

where $R^{11}$ is a radical independently selected from halo, $-(C_1-C_{10})$alkyl, $-(C_1-C_{10})$alkoxy, $-S-(C_1-C_{10}$ alkyl), and $-(C_1-C_{10})$haloalkyl, and w is a number from 0 to 5.

Another preferred subclass of compounds of formula (III) are those wherein $R^{2'}$ is a substituent having an acid linker with an acid linker length of 2 or 3. Most preferred are compounds where the acidic group is selected from

$-CO_2H$

-5-tetrazolyl,

$-SO_3H$,

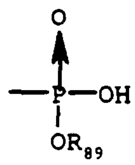

,

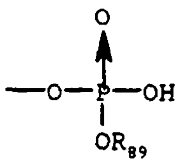

,

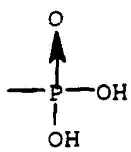

,

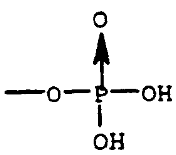

,

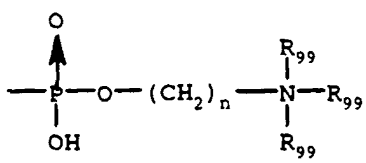

,

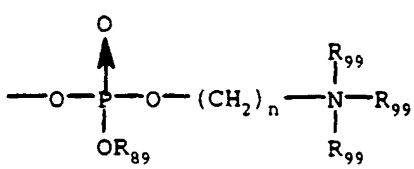

,

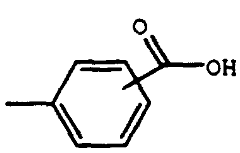

,

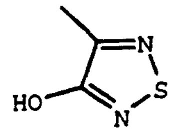 ;

where n is 1 to 8, $R_{89}$ is a metal or -($C_1$-$C_{10}$)alkyl, and $R_{99}$ is hydrogen or -($C_1$-$C_{10}$)alkyl. Particularly preferred are compounds wherein the acidic group of $R^{2'}$ is selected from;

$$—CO_2H\ ,$$

$$—SO_3H\ ,$$

$$—P(O)(OH)_2\ ,$$

or salt, and prodrug (e.g., ester) derivatives thereof.

Another preferred subclass of compounds of formula (III) are those wherein $R^{2'}$ is a substituent having an acid linker with an acid linker length of 2 or 3 and the acid linker group, -($L_a$)-, for $R^{2'}$ is selected from a group represented by the formula;

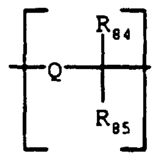

where Q is selected from the group -($CH_2$)-, -O-, -NH-, and -S-, and $R_{84}$ and $R_{85}$ are each independently selected from hydrogen,-($C_1$-$C_{10}$)alkyl, aryl, -($C_1$-$C_{10}$)alkylaryl, -aryl($C_1$-$C_{10}$)alkyl, carboxy, carbalkoxy, and halo. Most preferred are compounds where the acid linker, -($L_a$)-, for $R^{2'}$ is selected from the specific groups;

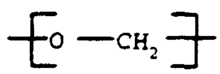 ,

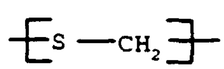 ,

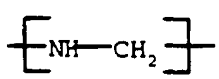 ,

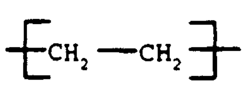 ,

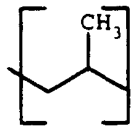

and

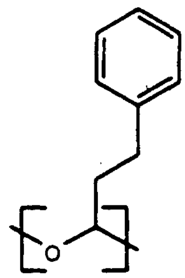 ;

Another preferred subclass of compounds of formula (III) are those wherein $R^{2'}$ is a substituent having an acid linker with an acid linker length of 3 to 8 atoms and the acid linker group, $-(L_a)-$, for $R^{2'}$ is selected from;

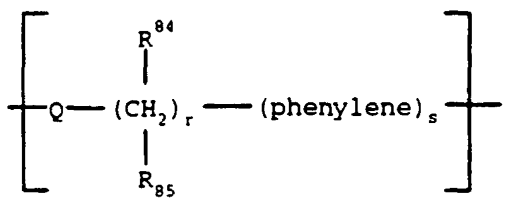

where r is a number from 2 to 7, s is 0 or 1, and Q is selected from the group $-(CH_2)-$, -O-, -NH-, and -S-, and $R_{84}$ and $R_{85}$ are each independently selected from hydrogen, $-(C_1-C_{10})$alkyl, aryl, $-(C_1-C_{10})$alkylaryl, -aryl$(C_1-C_{10})$alkyl, carboxy, carbalkoxy, and halo.

Most preferred are compounds where the acid linker, $-(L_a)-$, for $R^{2'}$ is selected from the specific groups;

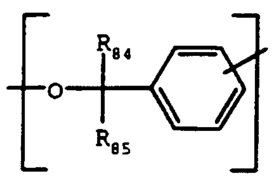 ,

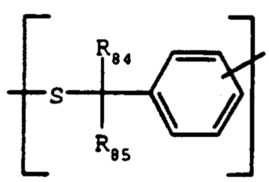 ,

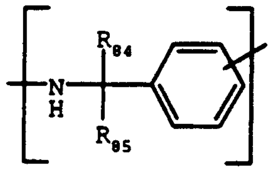,

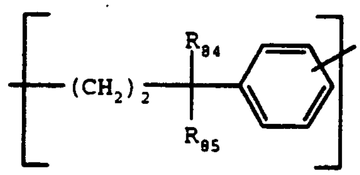,

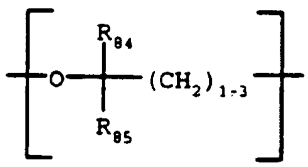,

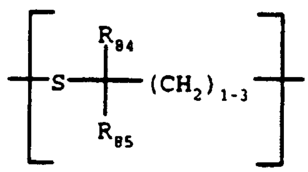,

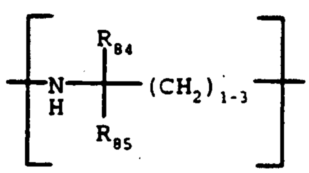

and

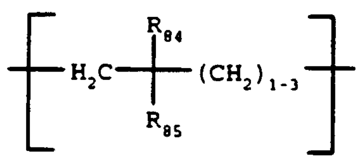;

wherein $R_{84}$ and $R_{85}$ are each independently selected from hydrogen, -($C_1$-$C_{10}$)alkyl, aryl, -($C_1$-$C_{10}$)alkaryl, -($C_1$-$C_{10}$)aralkyl, carboxy, carbalkoxy, and halo.

Another preferred subclass of compounds of formula (III) are those wherein $R^{3'}$ is selected from hydrogen and non-interfering substituents, with the non-interfering substituents being selected from the group consisting of hydrogen, -($C_1$-$C_6$)alkyl, -($C_2$-$C_6$)alkenyl, -($C_2$-$C_6$)alkynyl, -($C_7$-$C_{12}$)aralkyl, -($C_7$-$C_{12}$)alkaryl, -($C_3$-$C_8$)cycloalkyl, -($C_3$-$C_8$)cycloalkenyl, phenyl, tolulyl, xylenyl, biphenyl, -($C_1$-$C_6$)alkoxy, -($C_2$-$C_6$)alkenyloxy -($C_2$-$C_6$)alkynyloxy, -($C_1$-$C_{12}$)alkoxy-

alkyl, -($C_1$-$C_{12}$)alkoxyalkyloxy, -($C_1$-$C_{12}$)alkylcarbonyl, -($C_1$-$C_{12}$)alkylcarbonylamino, - ($C_1$-$C_{12}$)alkoxyamino, -($C_1$-$C_{12}$) alkoxyaminocarbonyl, - ($C_1$-$C_{12}$)alkylamino, -($C_1$-$C_6$)alkylthio, -($C_1$-$C_{12}$)alkylthiocarbonyl, -($C_1$-$C_6$)alkylsulfinyl, -($C_1$-$C_6$)alkylsulfonyl, -($C_1$-$C_6$)haloalkoxy, -($C_1$-$C_6$)haloalkylsulfonyl, -($C_1$-$C_6$)haloalkyl, -($C_1$-$C_6$)hydroxyalkyl, -C(O)O ($C_1$-$C_6$ alkyl), -$(CH_2)_n$O($C_1$-$C_6$ alkyl), benzyloxy, halo, phenylthio, -$(CH_2)_n$CN, -$(CH_2)_n$$NR^9R^{10}$, where $R^9$ and $R^{10}$ are independently -($C_1$-$C_4$)alkyl or -phenyl($C_1$-$C_4$)alkyl, -($CONHSO_2R$), -CHO, amino, amidino, carbamyl, carboxyl, carbalkoxy, -$(CH_2)_nCO_2H$, cyano, cyanoguanidinyl, guanidino, hydrazide, hydrazino, hydrazido, hydroxy, hydroxyamino, nitro, phosphono, -$SO_3H$, thioacetal, thiocarbonyl, and -($C_1$-$C_6$)alkylcarbonyl; where n is from 1 to 8.

Preferred compounds of the invention are those having the general formula (II)

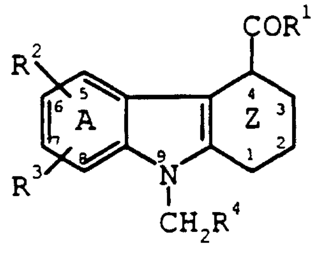

(II)

wherein:

$R^1$ is -$NHNH_2$, or -$NH_2$;
$R^2$ is -OH, or -$O(CH_2)_mR^5$ where
$R^5$ is H, -$CO_2H$, -$CO_2$($C_1$-$C_4$ alkyl),

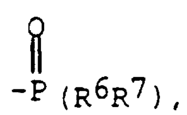

-$NHSO_2$($C_1$-$C_6$)alkyl, -$CONHSO_2$($C_1$-$C_6$)alkyl, -CN, tetrazolyl, phenyl, or phenyl substituted with -$CO_2H$ or -$CO_2$ ($C_1$-$C_4$ alkyl) where $R^6$ and $R^7$ are each independently -OH or -O($C_1$-$C_4$ alkyl) and m is 1-3;
$R^3$ is H, -O($C_1$-$C_4$ alkyl), or -$(CH_2)_nR^8$ where
$R^8$ is H, -$NR^9R^{10}$,

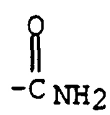

-CN, or phenyl where $R^9$ and $R^{10}$ are independently -($C_1$-$C_4$)alkyl, or -phenyl($C_1$-$C_4$)alkyl and n is 1-8;
$R^4$ is H, -($C_5$-$C_{14}$)alkyl, -($C_3$-$C_{14}$)cycloalkyl, phenyl, or phenyl substituted with 1 or 2 substituents selected from the group consisting of -($C_1$-$C_4$)alkyl, -($C_1$-$C_4$)alkoxy, -phenyl($C_1$-$C_4$)alkyl,-($C_1$-$C_4$)alkylthio, halo or phenyl; and
Z is cyclohexenyl, phenyl, pyridyl wherein the nitrogen is at the 1-, 2- or 3-position or a 6-membered heterocyclic ring having one heteroatom selected from the group consisting of sulfur oroxygen at the 1-, 2- or 3-position and nitrogen at the 1-, 2-, 3- or 4-position, or wherein one carbon on the heterocyclic ring is optionally substituted with =O
A is phenyl or pyridyl wherein the nitrogen is at the 5-, 6-, 7- or 8-position;

or a pharmaceutically acceptable salt, racemate, solvate, tautomer, optical isomer or prodrug derivative thereof;

provided that when $R^3$ is H, $R^4$ is phenyl, m is 1 or 2 and $R^2$ is substituted at the 6 position, $R^5$ cannot be H; and when $R^1$ is $NHNH_2$, $R^8$ cannot be

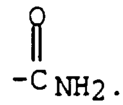

Another preferred group are compounds of the formula (I)

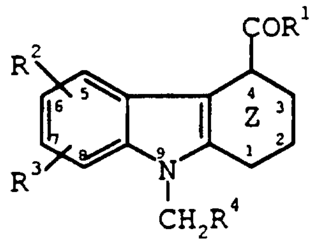

(I)

wherein:

$R^1$ is $-NHNH_2$, or $-NH_2$;
$R^2$ is -OH, or $-O(CH_2)_mR^5$ where
$R^5$ is H, $-CO_2H$, $-CO_2(C_1-C_4\ alkyl)$,

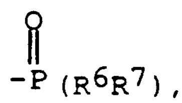

phenyl, or phenyl substituted with $-CO_2H$ or $-CO_2(C_1-C_4\ alkyl)$ where $R^6$ and $R^7$ are each independently -OH or $-O(C_1-C_4\ alkyl)$ and m is 1-3;
$R^3$ is H, $-O(C_1-C_4\ alkyl)$, or $-(CH_2)_nR^8$ where
$R^8$ is H, $-NR^9R^{10}$,

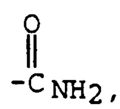

-CN, or phenyl where $R^9$ and $R^{10}$ are independently $-(C_1-C_4)$alkyl, or -phenyl$(C_1-C_4)$alkyl and n is 1-8;
$R^4$ is H, $-(C_5-C_{14})$alkyl, $-(C_3-C_{14})$cycloalkyl, phenyl, or phenyl substituted with 1 or 2 substituents selected from the group consisting of $-(C_1-C_4)$alkyl, $-(C_1-C_4)$alkoxy, -phenyl$(C_1-C_4)$alkyl, $-(C_1-C_4)$alkylthio, halo or phenyl; and
Z is cyclohexenyl or phenyl;

or a pharmaceutically acceptable salt, racemate, optical isomer, solvate, tautomer or prodrug derivative thereof; provided that when $R^3$ is H, $R^4$ is phenyl, m is 1 or 2 and $R^2$ is substituted at the 6 position, $R^5$ cannot be H; and when $R^1$ is $NHNH_2$, $R^8$ cannot be

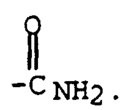

Preferred substituents of compounds of formula I and II include the following:

(a) $R^1$ is $-NH_2$, $-NHNH_2$;
(b) $R^1$ is $-NH_2$;
(c) $R^2$ is $-O(CH_2)_mR^5$ where $R^5$ is -H, $-CO_2H$ or

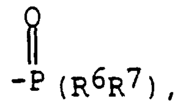

where $R^6$ and $R^7$ are -OH;
(d) $R^2$ is -OH;
(e) $R^2$ is $-O(CH_2)_mR^5$ where $R^5$ is -H, $-CO_2(C_1\text{-}C_4$ alkyl), phenyl or phenyl substituted with $-CO_2H$ or $-CO_2(C_1\text{-}C_4$ alkyl);
(f) $R^2$ is $-O(CH_2)_mR^5$ where $R^5$ is

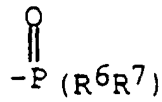

and $R^6$ and $R^7$ are $-O(C_1\text{-}C_4$ alkyl), or when one of $R^6$ and $R^7$ is $-O(C_1\text{-}C_4$ alkyl), the other is -OH;
(g) $R^3$ is -H, $-O(C_1\text{-}C_4$ alkyl) or $-(CH_2)_nR^8$ where
n = 2 and
$R^8$ is H or phenyl;
(h) $R^3$ is H, or $-O(C_1\text{-}C_4$ alkyl);
(i) $R^3$ is $-(CH_2)_nR^8$ where $R^8$ is $-NR^9R^{10}$,

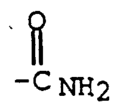

or -CN where $R^9$ and $R^{10}$ are $-(C_1\text{-}C_4)$alkyl ;
(j) $R^4$ is phenyl;
(k) $R^4$ is phenyl substituted at the 2- and 6-position of the phenyl ring with $-(C_1\text{-}C_4)$alkyl, $(C_1\text{-}C_4)$alkoxy, halo or phenyl;
(l) $R^4$ is phenyl substituted at the 2- or 6-position of the phenyl ring with $-(C_1\text{-}C_4)$alkyl, $-(C_1\text{-}C_4)$alkoxy, halo or phenyl;
(m) $R^4$ is phenyl substituted at the 3- or 5-position of the phenyl ring with $-(C_1\text{-}C_4)$alkyl, $-(C_1\text{-}C_4)$alkoxy, halo or phenyl;
(n) $R^4$ is $-(C_6\text{-}C_{14})$alkyl or $-(C_6\text{-}C_{14})$cycloalkyl;
(o) Z is cyclohexenyl;
(p) $R^5$ is H, $-CO_2H$, $-CO_2(C_1\text{-}C_4$ alkyl),

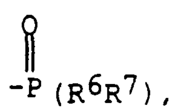

$-NHSO_2(C_1\text{-}C_6)$alkyl, $-CONHSO_2(C_1\text{-}C_6)$alkyl, tetrazolyl, phenyl, or phenyl substituted with $-CO_2H$ or $-CO_2(C_1\text{-}C_4$ alkyl) where $R^6$ and $R^7$ are each independently -OH or $-O(C_1\text{-}C_4$ alkyl) and m is 1-3;
(q) $R^5$ is H, $-CO_2H$, $-CO_2(C_1\text{-}C_4$ alkyl),

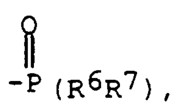

phenyl, or phenyl substituted with $-CO_2H$ or $-CO_2(C_1-C_4$ alkyl) where $R^6$ and $R^7$ are each independently -OH or $-O(C_1-C_4$ alkyl) and m is 1-3;

(r) Z is cyclohexenyl, phenyl, pyridyl wherein the nitrogen is at the 1-, 2- or 3-position or a 6-membered heterocyclic ring having one heteroatom selected from the group consisting of sulfur or oxygen at the 1-, 2- or 3-position and nitrogen at the 1-, 2-, 3- or 4-position wherein one carbon on the heterocyclic ring is optionally substituted with

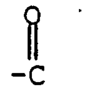

(s) Z is cyclohexenyl or phenyl; or a pharmaceutically acceptable salt, racemate or optical isomer thereof; provided that when $R^3$ is H, $R^4$ is phenyl, m is 1 or 2 and $R^2$ is substituted at the 6 position, $R^5$ cannot be H; and when $R^1$ is $NHNH_2$, $R^8$ cannot be

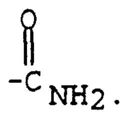

(t) A is phenyl; and

(u) A is pyridyl wherein the nitrogen is at the 5-, 6-, 7- or 8-position.

Further typical examples of compounds of formula I which are useful in the present invention include:

5-hydroxy-7-(5-cyanopentyl)-9-methyl-1,2,3,4-tetrahydrocarbazole-4-carboxamide;
6-(2-carboxyethoxy)-8-methoxy-9-cyclopentylmethyl-1,2,3,4-tetrahydrocarbazole-4-carboxamide;
5-(3-phenylpropoxy)-7-ethoxy-9-butyl-1,2,3,4-tetrahydrocarbazole-4-carboxamide;
6-(2-phosphonoethoxy)-8-phenylhexyl-9-(cyclotetradecyl)methyl-1,2,3,4-tetrahydrocarbazole-4-carboxamide;
5-ethoxycarbonylmethoxy-8-(5-carbamoylpent-1-yl)-9-(3,5-dipropylphenyl)methyl-1,2,3,4-tetrahydrocarbazole-4-carboxamide;
6-(diethoxyphosphonyl)methoxy-9-(4-methoxyphenyl)methyl-1,2,3,4-tetrahydrocarbazole-4-carboxamide;
6-(3-(4-carboxyphenyl)prop-1-yl)oxy-8-heptyl-9-(3-phenylethyl)phenyl)methyl-1,2,3,4-tetrahydrocarbazole-4-carboxamide;
6-(2-propoxycarbonyl)ethoxy-8-(3-(N,N-dimethylamino)prop-1-yl)-9-methyl-1,2,3,4-tetrahydrocarbazole-4-carboxamide;
5-(di-t-butoxyphosphonyl)methoxy-7-nonyl-9-(3-propylthiophenyl)methyl-1,2,3,4-tetrahydrocarbazole-4-carboxamide;
5-(2-(3-methoxycarbonyl)phenyl)ethoxy-7-pentyl-9-methyl-1,2,3,4-tetrahydrocarbazole-4-carboxamide;
6-hydroxy-8-(4-(N,N-diethylamino)but-1-yl)-9-(3-fluorophenyl)methyl-1,2,3,4-tetrahydrocarbazole-4-carboxamide;
6-(2-phenylethoxy)-9-(2-phenylphenyl)methyl-1,2,3,4-tetrahydrocarbazole-4-carboxamide;
(S)-6-((3-carboxy)prop-1-yl)oxy-8-propoxy-9-(7-cyanohept-1-yl)-1,2,3,4-tetrahydrocarbazole-4-carboxamide;
5-(propoxycarbonyl)methoxy-9-cyclopentylmethyl-1,2,3,4-tetrahydrocarbazole-4-carboxamide;
(S)-5-(2-ethoxyphosphonyl)ethoxy-(4-carbamoyl)but-1-yl-9-(3-methylthiophenyl)methyl-1,2,3,4-tetrahydrocarbazole-4-carboxamide;
5-(3-(ethoxycarbonyl)prop-1-yl)oxy-7-propoxy-9-(cyclononyl)methyl-1,2,3,4-tetrahydrocarbazole-4-carboxamide;
5-(3-phosphonoprop-1-yl)oxy-8-heptyl-9-(4-chlorophenyl)methyl-1,2,3,4-tetrahydrocarbazole-4-carboxamide;
6-methoxycarbonylmethoxy-7-(5-cyanopent-1-yl)-9-tridecylmethyl-1,2,3,4-tetrahydrocarbazole-4-carboxamide;
(S)-6-propoxycarbonylmethoxy-9-((3-isopropyl-5-methoxy)phenyl)methyl-1,2,3,4-tetrahydrocarbazole-4-carboxamide;
(S)-6-dimethoxyphosphonoethoxy-8-(6-(N,N-dimethylamino)hex-1-yl-9-(3,5-dimethoxyphenyl)methyl-1,2,3,4-tetrahydrocarbazole-4-carboxamide;
5-hydroxy-7-(5-cyanopentyl)-9-methylcarbazole-4-carboxamide;
6-(2-carboxyethoxy)-8-methoxy-9-cyclopentylmethyl-carbazole-4-carboxamide;
5-(3-phenylprop-1-yl)oxy-7-ethoxy-9-butylcarbazole-4-carboxamide;
6-(2-phosphonoethoxy)-8-phenylhexyl-9-(cyclotetradecyl)methylcarbazole-4-carboxamide;

5-ethoxycarbonylmethoxy-8-(5-carbamoylpent-1-yl) -9-(3, 5-dipropylphenyl)methylcarbazole-4-carboxamide;
6-(diethoxyphosphonyl)methoxy-9-(4-methoxyphenyl)methylcarbazole-4-carboxamide;
6-(3-(4-carboxyphenyl)prop-1-yl)oxy-8-heptyl-9-(3-phenylethyl)phenyl)methylcarbazole-4-carboxamide;
6-(2-propoxycarbonyl)ethoxy-8-(3-(N,N-dimethylamino)prop-1-yl)-9-methylcarbazole-4-carboxamide;
5-((di-t-butoxyphosphonyl)methoxy-7-nonyl-9-(3-propylthiopheny)methylcarbazole-4-carboxamide;
(S)-5-(2-(3-methoxycarbonyl)phenyl)ethoxy-7-pentyl-9-methylcarbazole-4-carboxamide;
(S)-6-hydroxy-8-(4-(N,N-diethylamino)but-1-yl)-9-(3-fluorophenyl)methylcarbazole-4-carboxamide;
(S)-6-(2-phenylethoxy)-9-((2-phenyl)phenyl)methylcarbazole-4-carboxamide;
6-((3-carboxy)prop-1-yl)oxy-8-propoxy-9-(7-cyanohept-1-yl)-carbazole-4-carboxamide;
5-(propoxycarbonyl)methoxy-9-cyclopentylmethylcarbazole-4-carboxamide;
5-(2-ethoxyphosphonyl)ethoxy-(4-carbamoyl)but-1-yl-9-(3-methylthiophenyl)methylcarbazole-4-carboxamide;
5-((3-ethoxycarbonyl)prop-1-yl)oxy-7-propoxy-9-(cyclononyl)methylcarbazole-4-carboxamide;
(S)-5-(3-phosphonoprop-1-yl)oxy-8-heptyl-9-(4-chlorophenyl)methylcarbazole-4-carboxamide;
(S)-6-methoxycarbonylmethoxy-7-(5-cyanopent-1-yl)-9-tridecylmethylcarbazole-4-carboxamide;
6-(propoxycarbonyl)methoxy-9-(3-isopropyl-5-methoxy)phenyl)methylcarbazole-4-carboxamide;
6-dimethoxyphosphonoethoxy-8-(6-(N,N-dimethylamino)hex-1-yl-9-(3,5-dimethoxyphenyl)methyl-carbazole-4-carboxamide;
5-hydroxy-7-(5-cyanopentyl)-9-methyl-1,2,3,4-tetrahydrocarbazole-4-carboxylic acid hydrazide;
6-(2-carboxyethoxy-8-methoxy-9-cyclopentyl)methyl-1,2,3,4-tetrahydrocarbazole-4-carboxylic acid hydrazide;
5-(3-phenylprop-1-yl)oxy-7-ethoxy-9-butyl-1,2,3,4-tetrahydrocarbazole-4-carboxylic acid hydrazide;
6-(2-phosphonoethoxy)-8-phenylhexyl-9-(cyclotetradecyl)methyl-1,2,3,4-tetrahydrocarbazole-4-carboxylic acid hydrazide;
5-ethoxycarbonylmethoxy-8-(5-carbamoylpent-1-yl)-9-(3,5-dipropylphenyl)methyl-1,2,3,4-tetrahydrocarbazole-4-carboxylic acid hydrazide;
(S)-6-(diethoxyphosphonyl)methoxy-9-(4-methoxyphenyl)methyl-1,2,3,4-tetrahydrocarbazole-4-carboxylic acid hydrazide;
6-(3-(4-carboxyphenyl)prop-1-yl)oxy-8-heptyl-9-((3-phenylethyl)phenyl)methyl-1,2,3,4-tetrahydrocarbazole-4-carboxylic acid hydrazide;
(S)-6-(2-propoxycarbonyl)ethoxy-8-(3-(N,N-dimethylamino)prop-1-yl)-9-methyl-1,2,3,4-tetrahydrocarbazole-4-carboxylic acid hydrazide;
5-(di-t-butoxyphosphonyl)methoxy-7-nonyl-9-(3-propylthiopheny)methyl-1,2,3,4-tetrahydrocarbazole-4-carboxylic acid hydrazide;
(S)-5-(2-(3-methoxycarbonyl)phenyl)ethoxy-7-pentyl-9-methyl-1,2,3,4-tetrahydrocarbazole-4-carboxylic acid hydrazide;
6-hydroxy-8-(4-(N,N-diethylamino)but-1-yl)-9-(3-fluorophenyl)methyl-1,2,3,4-tetrahydrocarbazole-4-carboxylic acid hydrazide;
(S)-6-(2-phenylethoxy)-9-(2-phenylphenyl)methyl-1,2,3,4-tetrahydrocarbazole-4-carboxylic acid hydrazide;
6-((3-carboxy)prop-1-yl)oxy-8-propoxy-9-(7-cyanohept-1-yl)methyl-1,2,3,4-tetrahydrocarbazole-4-carboxylic acid hydrazide;
5-(propoxycarbonyl)methoxy-9-cyclopentylmethyl-1,2,3,4-tetrahydrocarbazole-4-carboxylic acid hydrazide;
5-(2-ethoxyphosphonyl)ethoxy-(4-carbamoyl)but-1-yl-9-(3-methylthiophenyl)methyl-1,2,3,4-tetrahydrocarbazole-4-carboxylic acid hydrazide;
5-((3-(ethoxycarbonyl)prop-1-yl)oxy-7-propoxy-9-(cyclononyl)methyl-1,2,3,4-tetrahydrocarbazole-4-carboxylic acid hydrazide;
5-(3-phosphonoprop-1-yl)oxy-8-heptyl-9-(4-chlorophenyl)methyl-1,2,3,4-tetrahydrocarbazole-4-carboxylic acid hydrazide;
6-methoxycarbonylmethoxy-7-(5-cyanopent-1-yl)-9-tridecyl-1,2,3,4-tetrahydrocarbazole-4-carboxylic acid hydrazide;
6-propoxycarbonylmethoxy-9-(3-isopropyl-5-methoxyphenyl)methyl-1,2,3,4-tetrahydrocarbazole-4-carboxylic acid hydrazide;
6-dimethoxyphosphonoethoxy-8-(6-(N,N-dimethylamino)hex-1-yl-9-(3,5-dimethoxyphenyl)methyl-1,2,3,4-tetrahydrocarbazole-4-carboxylic acid hydrazide;
5-hydroxy-7-(5-cyanopentyl)-9-methylcarbazole-4-carboxylic acid hydrazide;
6-(2-carboxyethyloxy)-8-methoxy-9-cyclopentylmethyl-carbazole-4-carboxylic acid hydrazide;
5-(3-phenylprop-1-yl)oxy-7-ethoxy-9-butylcarbazole-4-carboxylic acid hydrazide;
6-(2-phosphonoethoxy-8-phenylhexyl-9-(cyclotetradecyl)methylcarbazole-4-carboxylic acid hydrazide;
5-ethoxycarbonylmethoxy-8-(5-carbamoylpent-1-yl)-9-(3,5-dipropylphenyl)methylcarbazole-4-carboxylic acid hydrazide;

6-(diethoxyphosphonyl)methoxy-9-(4-methoxyphenyl)methylcarbazole-4-carboxylic acid hydrazide;
6-(3-(4-carboxyphenyl)prop-1-yl)oxy-8-heptyl-9-((3-phenylethyl)phenyl)methylcarbazole-4-carboxylic acid hydrazide;
6-(2-propoxycarbonyl)ethoxy-8-(3-(N,N-dimethylamino)prop-1-yl)-9-methylcarbazole-4-carboxylic acid hydrazide;
5-(di-t-butoxyphosphonyl)methoxy-7-nonyl-9-(3-propylthiophenyl)methylcarbazole-4-carboxylic acid hydrazide;
5-(2-(3-methoxycarbonyl)phenyl)ethoxy-7-pentyl-9-methylcarbazole-4-carboxylic acid hydrazide;
6-hydroxy-8-(4-(N,N-diethylamino)but-1-yl)-9-(3-fluorophenyl)methylcarbazole-4-carbazole;
6-(2-phenylethoxy)-9-(2-phenylphenyl)methylcarbazole-4-carboxylic acid hydrazide;
6-((3-carboxy)prop-1-yl)oxy-8-propoxy-9-(7-cyanohept-1-yl)-carbazole-4-carboxylic acid hydrazide;
(S)-5-(propoxycarbonyl)methoxy-9-cyclopentylmethylcarbazole-4-carboxylic acid hydrazide;
5-(2-ethoxyphosphonyl)ethoxy-(4-carbamoyl)but-1-yl-9-(3-methylthiophenyl)methylcarbazole-4-carboxylic acid hydrazide;
(S)-5-(3-(ethoxycarbonyl)prop-1-yl)oxy-7-propoxy-9-(cyclononyl)methylcarbazole-4-carboxylic acid hydrazide;
5-(3-phosphonoprop-1-yl)oxy-8-heptyl-9-(4-chlorophenyl)methylcarbazole-4-carboxylic acid hydrazide;
6-methoxycarbonylmethoxy-7-(5-cyanopent-1-yl)-9-tridecylcarbazole-4-carboxylic acid hydrazide;
6-propoxycarbonylmethoxy-9-((3-isopropyl-5-methoxy)phenyl)methylcarbazole-4-carboxylic acid hydrazide;
(S)-6-dimethoxyphosphonoethoxy-8-(6-(N,N-dimethylamino)hex-1-yl-9-(3,5-dimethoxyphenyl)methyl-carbazole-4-carboxylic acid hydrazide.

Synthesis Methods

The compounds of formula I where Z is cyclohexene are prepared according to the following reaction Schemes I(a) and (c).

Scheme I(a)

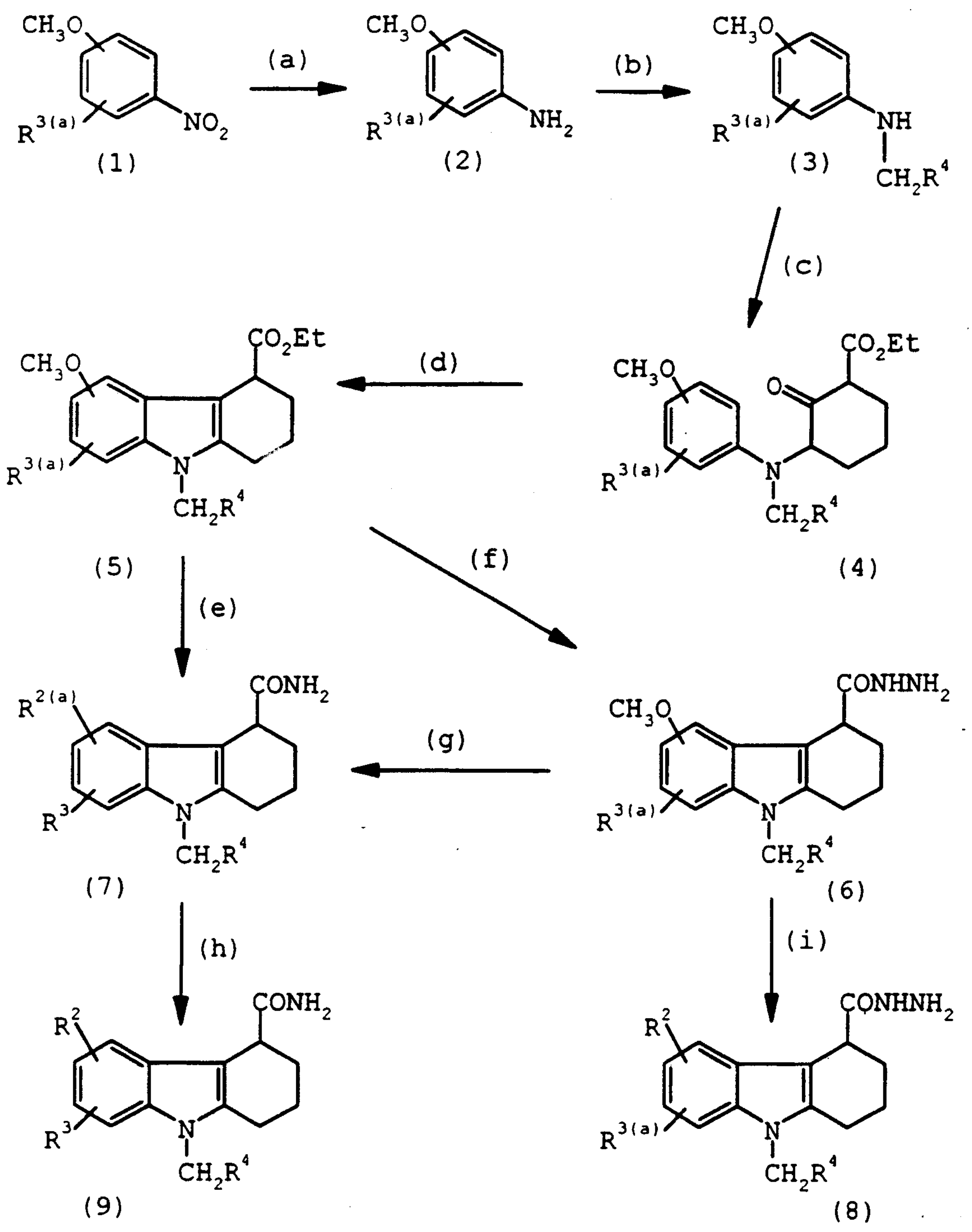


When $R^1$ is $-NH_2$, $R^{3(a)}$ is H, $-O(C_1-C_4$ alkyl) or $-(CH_2)_nR^8$ where $R^8$ is H, $-NR^9R^{10}$, -CN,

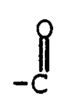 O($C_1$-$C_4$ alkyl) or phenyl,

where $R^9$ and $R^{10}$ are independently $-(C_1-C_4)$alkyl or $-(C_1-C_4)$alkylphenyl and n is 1-8 when $R^1$ is $-NH_2$;

when $R^1$ is $-NHNH_2$, $R^{3(a)}$ is H, $-O(C_1-C_4)$alkyl or $(CH_2)_nR^8$ where $R^8$ is H, $NR^9R^{10}$, -CN or phenyl where $R^9$ and $R^{10}$ are independently $-(C_1-C_4)$alkyl or $-(C_1-C_4)$alkylphenyl and n is 1-8;

$R^{2(a)}$ is $-OCH_3$ or -OH.

An appropriately substituted aldehyde is treated with Wittig reagent to prepare the appropriately substituted nitrobenzene (1) which can be reduced to the aniline (2) by treatment with a reducing agent, such as hydrogen in the presence of Pd/C, preferably at room temperature.

Compound (2) is N-alkylated at temperatures of from about 0 to 20°C using an alkylating agent such as an appropriately substituted aldehyde and sodium cyanoborohydride to form (3). Alternately, an appropriately substituted benzyl halide may be used for the first alkylation step. The resulting intermediate is further N-alkylated by treatment with 2-carbethoxy-6-bromocyclohexanone, preferably at temperatures of about 80°C to yield (4).

The product (4) is cyclized to the tetrahydrocarbazole (5) by refluxing with $ZnCl_2$ in benzene for from about 1 to 2 days, preferably at 80°C. (Ref 1). Compound (5) is converted to the hydrazide (6) by treatment with hydrazine at temperatures of about 100°C, or to the amide (7) by reacting with methylchloroaluminum amide in benzene. (Ref 2) Alternatively, (7) may be produced by treatment of (6) with Raney nickel active catalyst.

It will be readily appreciated that when $R^{3(a)}$ is

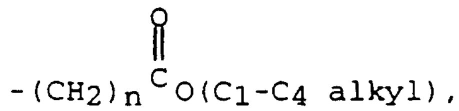

conversion to the amide will also be achieved in this procedure.

Compounds (6) and (7) may be dealkylated, preferably at 0°C to room temperature, with a dealkylating agent, such as boron tribromide or sodium thioethoxide, to give compound (7) where $R^{2(a)}$ is -OH, which may then be further converted to compound (9), by realkylating with a base, such as sodium hydride, and an alkylating agent, such as Br $(CH_2)_mR^5$, where $R^5$ is the carboxylate or phosphonic diester or nitrile as defined above. Conversion of $R^2$ to the carboxylic acid may be accomplished by treatment with an aqueous base. When $R^2$ is nitrile, conversion to the tetrazole may be achieved by reacting with tri-butyl tin azide. When $R^2$ is the phosphonic diester, conversion to the acid may be achieved by reacting with a dealkylating agent such as trimethylsilyl bromide. The monoester may be accomplished by reacting the diester with an aqueous base.

When $R^2$ and $R^3$ are both methoxy, selective demethylation can be achieved by treating with sodium ethanethiolate in dimethylformamide at 100°C.

Ref 1 Julia, M.; Lenzi, J. Preparation d'acides tetrahydro-1,2,3,4-carbazole-1 ou -4. *Bull.Soc.Chim.France*, **1962,** 2262-2263.

Ref 2 Levin, J.I.; Turos, E.; Weinreb, S.M. An alternative procedure for the aluminum-mediated conversion of esters to amides. *Syn.Comm.*, **1982,** *12,* 989-993.

An alternative synthesis of intermediate (5) is shown in Scheme I(b), as follows.

Scheme I(b)

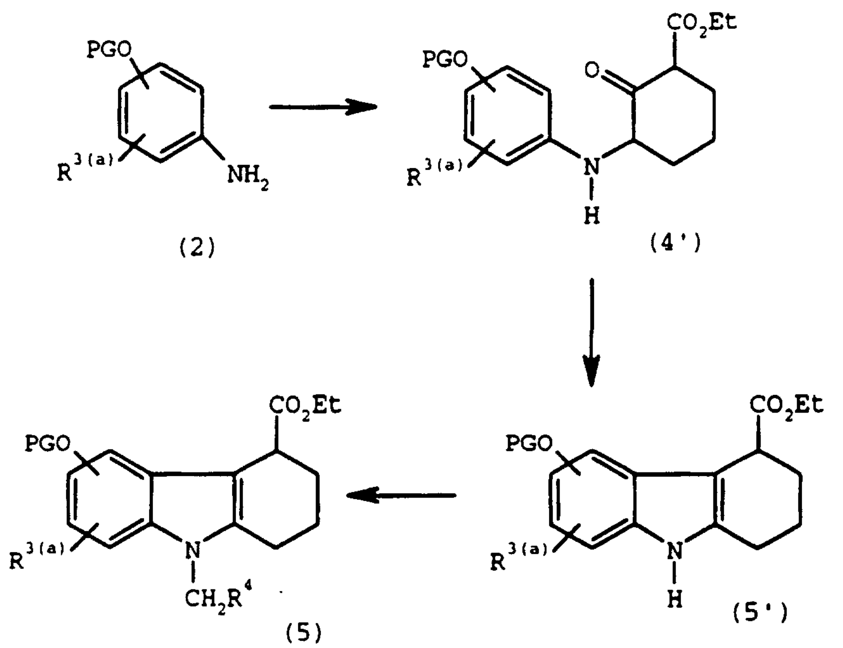

(2) (4') (5') (5)

PG is a protecting group
$R^{3a}$ is as defined in Scheme 1, above.

The aniline (2) is N-alkylated with 2-carbethoxy-6-bromocyclohexanone in dimethy formamide in the presence of sodium bicarbonate for 8-24 hours at 50°C. Preferred protecting groups include methyl, carbonate, and silyl groups, such as t-butyldimethylsilyl. The reaction product (4') is cyclized to (5') using the $ZnCl_2$ in benzene conditions described in Scheme I(a), above. N-alkylation of (5') to yield (5) is accomplished by treatment with sodium hydride and the appropriate alkyl halide in dimethylformamide at room temperature for 4-8 hours.

Scheme I(c)

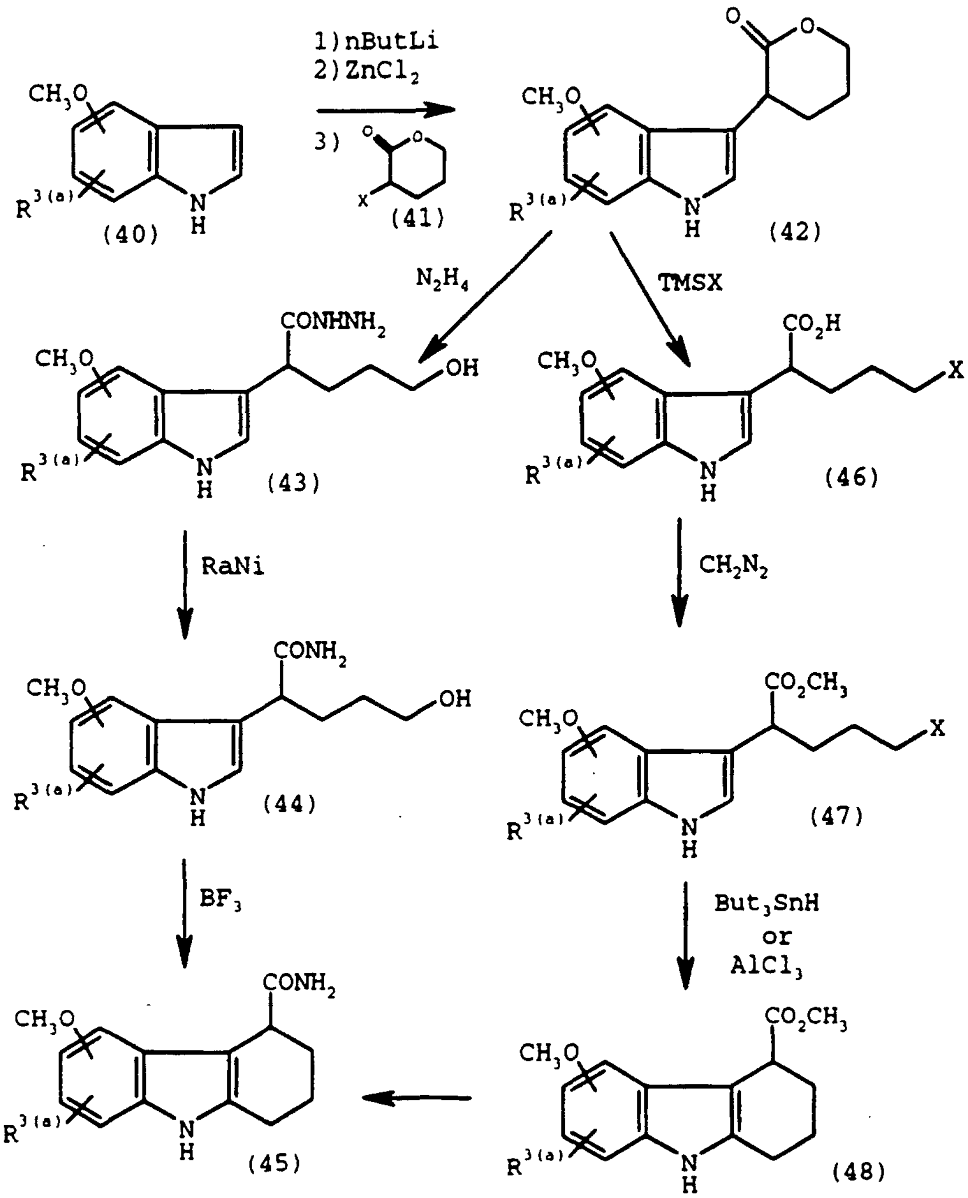


X is halo
$R^{3(a)}$ is as defined in Scheme I(a)

The zinc salt of a-methoxyindole (40) is alkylated by alpha-bromovalerolactone (41) to give (42) which are ring opened to (46) by trimethylsilyl halides to give the omega-haloacids (47) which are esterified and then cyclized to the tetrahydrocarbazoles (48) by Lewis acids (eg. aluminum chloride) or radical initiators (eg. tributyltin hydride).

Alternatively, (42) can be ring opened by hydrazine to give hydrazides (43) which are cleaved to amides (44) by Raney nickel catalyst and cyclized by acids to the tetrahydrocarbazoles (45).

N-alkylation is accomplished by treatment with sodium hydride and the appropriate alkyl halide, $XCH_2R^4$, in diemthylformamide as described above.

Tetrahydro derivatives may be dehydrogenation with palladium on carbon in refluxing carbitol as described below to prepare the desired carbazoles (see Scheme III(a)).

Racemates of the compounds of formula I may be converted to their respective enantiomers as described in Scheme II below.

Scheme II

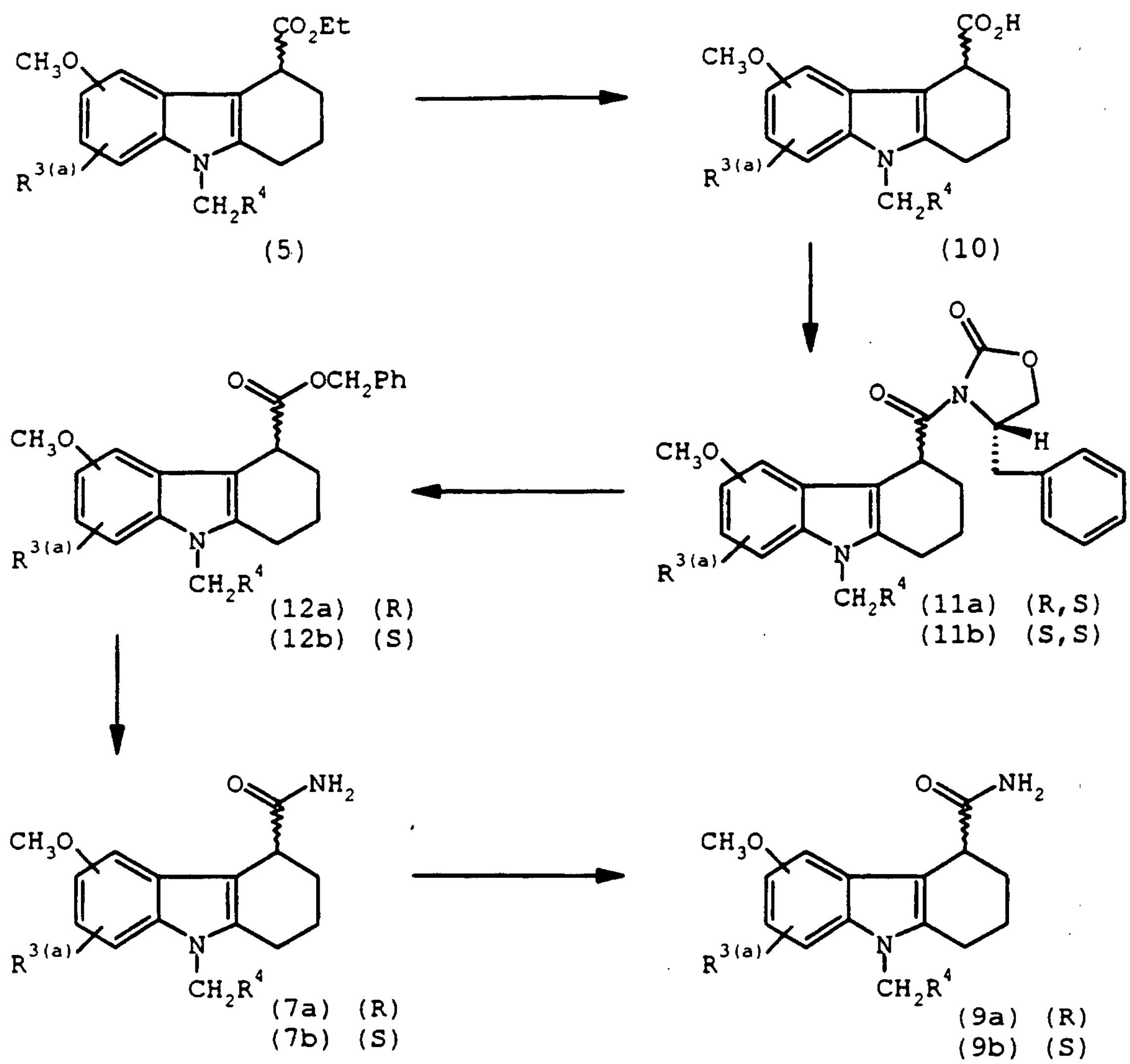

(5)

(10)

(12a) (R)
(12b) (S)

(11a) (R,S)
(11b) (S,S)

(7a) (R)
(7b) (S)

(9a) (R)
(9b) (S)

$R^{3(a)}$ is as defined in Scheme I.

As discussed in Scheme I above, carbazole (5) is hydrolyzed to the carboxylic acid (10) by treatment with an aqueous base, preferably at room temperature to about 100°C. The intermediate is then converted to an acid chloride utilizing, for example, oxalyl chloride and dimethylformamide, and then further reacted with a lithium salt of (S) or (R)-4-alkyl-2-oxazolidine at a temperature of about -75°C, to give (11a) and (11b), which are separable by chromatography.

The diastereomers are converted to the corresponding enantiomeric benzyl esters (12) by brief treatment at temperatures of about 0°C to room temperature with lithium benzyl oxide. (Ref 3) The esters (12) are then converted to (7) preferably by treatment with methylchloroaluminum amide (Ref 2, above) or, alternately, by hydrogenation using, for example, hydrogen and palladium on carbon, as described above, to make the acid and then reacting with an acyl azide, such as diphenylphosphoryl azide followed by treatment with ammonia. Using the procedure described above in Scheme I, compound (9a) or (9b) may be accomplished.

Ref 3 Evans, D.A.; Ennis, M.D.; Mathre, D.J. Asymmetric alkylation reactions of chiral imide enolates. A practical approach to the enantioselective synthesis of *alpha*-substituted carboxylic acid derivatives. *J.Am.Chem.Soc.*,

**1982,** *104*, 1737-1738.

Compounds of formula I where Z is phenyl can be prepared as follows in Schemes III(a) - (d), below.

Scheme III (a)

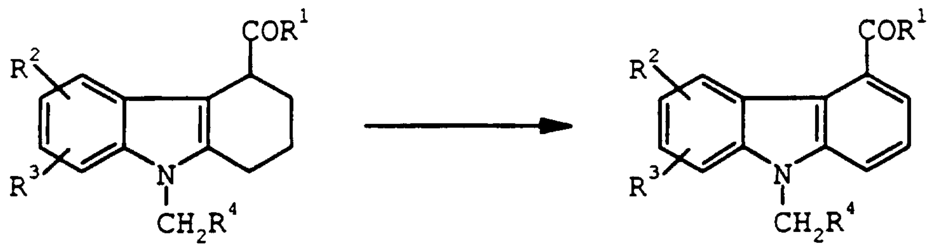

(13) (14)

A 1,2,3,4-tetrahydrocarbazole-4-carboxamide or 4-carboxhydrazide (13) is dehydrogenated by refluxing in a solvent such as carbitol in the presence of Pd/C to produce the carbazole-4-carboxamide.

The intermediates and final products may be isolated and purified by conventional techniques, for example by concentration of the solvents, followed by washing of the residue with water, then purification by conventional techniques, such as chromatography or recrystallization.

It will be readily appreciated by the skilled artisan that the starting materials are either commercially available or can be readily prepared by known techniques from commercially available starting materials. All other reactants used to prepare the compounds in the instant invention are commercially available.

Scheme III(b)

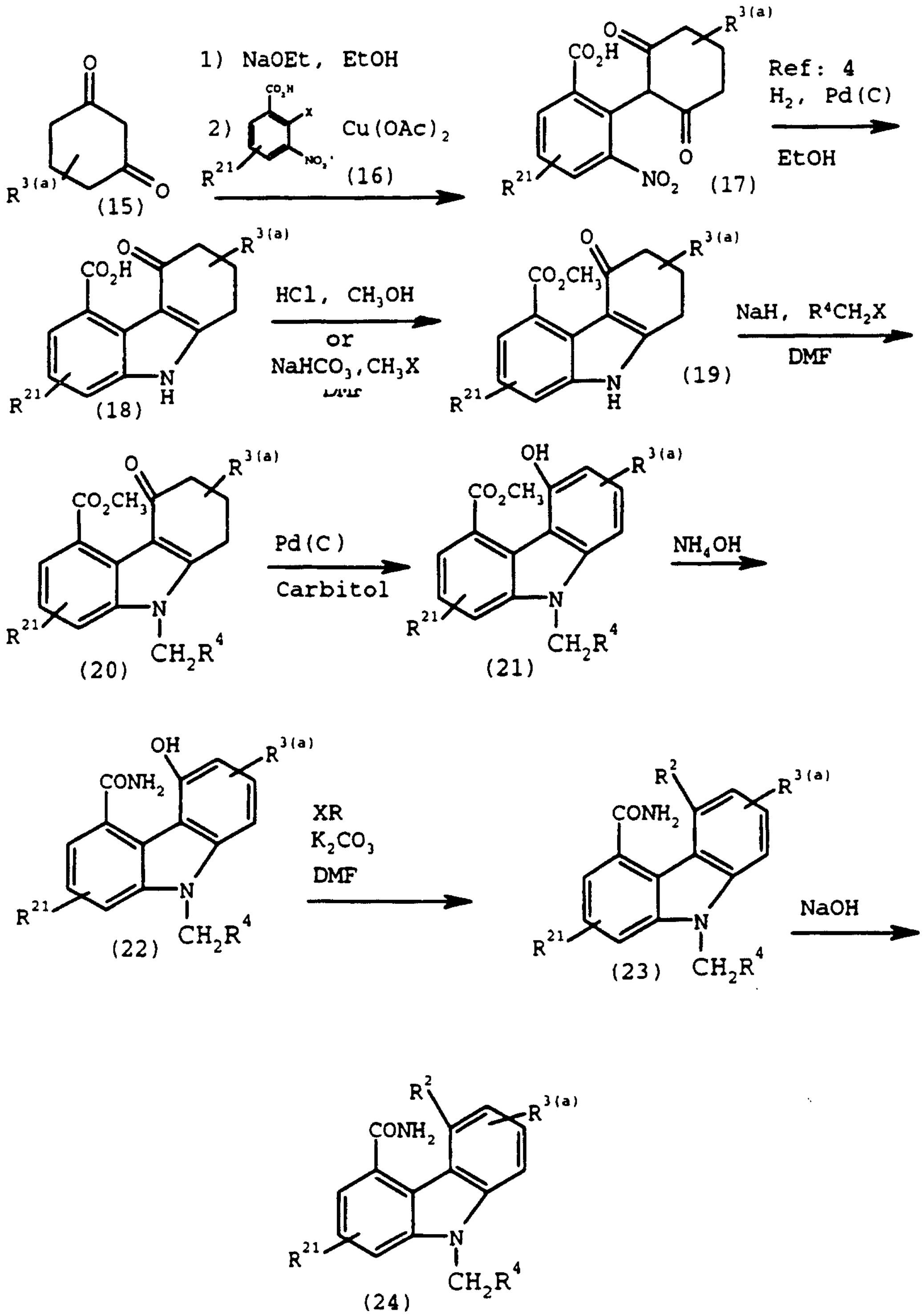


$R^{3(a)}$ is as defined in Scheme I(a), above;
R is $-(CH_2)_mR^5$; and
X is halo.

The Dione (15) and benzoic acid derivative (16) are condensed according to the general procedure of Ames and Ribiero (Ref 1), in an noninterfering solvent, preferably ethanol, in the presence of a base, preferably sodium ethoxide, and a copper salt, preferably copper (II) acetate to afford the coupled product (17). Other solvents, such as

methanol, propanol, isopropanol, t-butanol, and the like can be used. Other bases, such as the potassium, sodium, and lithium salts of the corresponding alcoholic solvents can also be used.

Reduction of the nitro group with a reducing agent, such as hydrogen in the presence of palladium on carbon, in a noninterfering solvent, such as ethanol, at 1 to 60 atmospheres, at a temperature of 0 to 60°C affords the cyclized product (18) (Ref 1). The acid (18) is converted to the methyl ester (19) by treatment with methanol in the presence of an acid, such as hydrochloric acid. Alternately, the acid (18) can be converted to the ester (19) by treatment with methyl iodide in the presence of a base, such as potassium carbonate or sodium carbonate, in an inert solvent, such as dimethylformamide or dimethylsulfoxide.

Compound (19) is N-alkylated with an appropriately substituted halide in the presence of a base, such as sodium hydride or potassium carbonate, in a noninterfering solvent, such as dimethylformamide or dimethylsulfoxide to afford ketone (20).

Ketone (20) is dehydrogenated by treatment with palladium on carbon in a noninterfering solvent, such as carbitol or cymene, to produce the phenolic derivative (21).

Ester (21) is converted to the corresponding amide (22) under standard conditions with ammonia or an ammonium salt, such as ammonium acetate, in an inert solvent, such as water or alcohol, or with $MeClAlNH_2$ in an inert solvent, such as toluene, at a temperature between 0 to 110°C.

Alkylation of the phenolic oxygen of compound (22) with an appropriate haloester, such as methyl bromoacetate, in the presence of a base, such as potassium carbonate or sodium carbonate, in an inert solvent, such as dimethylformamide or dimethylsulfoxide affords the ester-amide (23). Other haloesters, such as ethyl bromoacetate, propyl bromoacetate, butyl bromoacetate, and the like can also be used to prepare the corresponding esters.

Saponification of compound (23), with sodium hydroxide in an inert solvent, such as methanol-water, affords carbazole (24). The intermediate and final products may isolated and purified by conventional techniques such as chromatography or recrystallization. Regioisomeric products and intermediates can be separated by standard methods, such as, recrystallization or chromatography.

Reference:

4) D. E. Ames and O. Ribiero, J. Chem. Soc., Perkin Trans. I, 1073 (1976)

Scheme III(c)

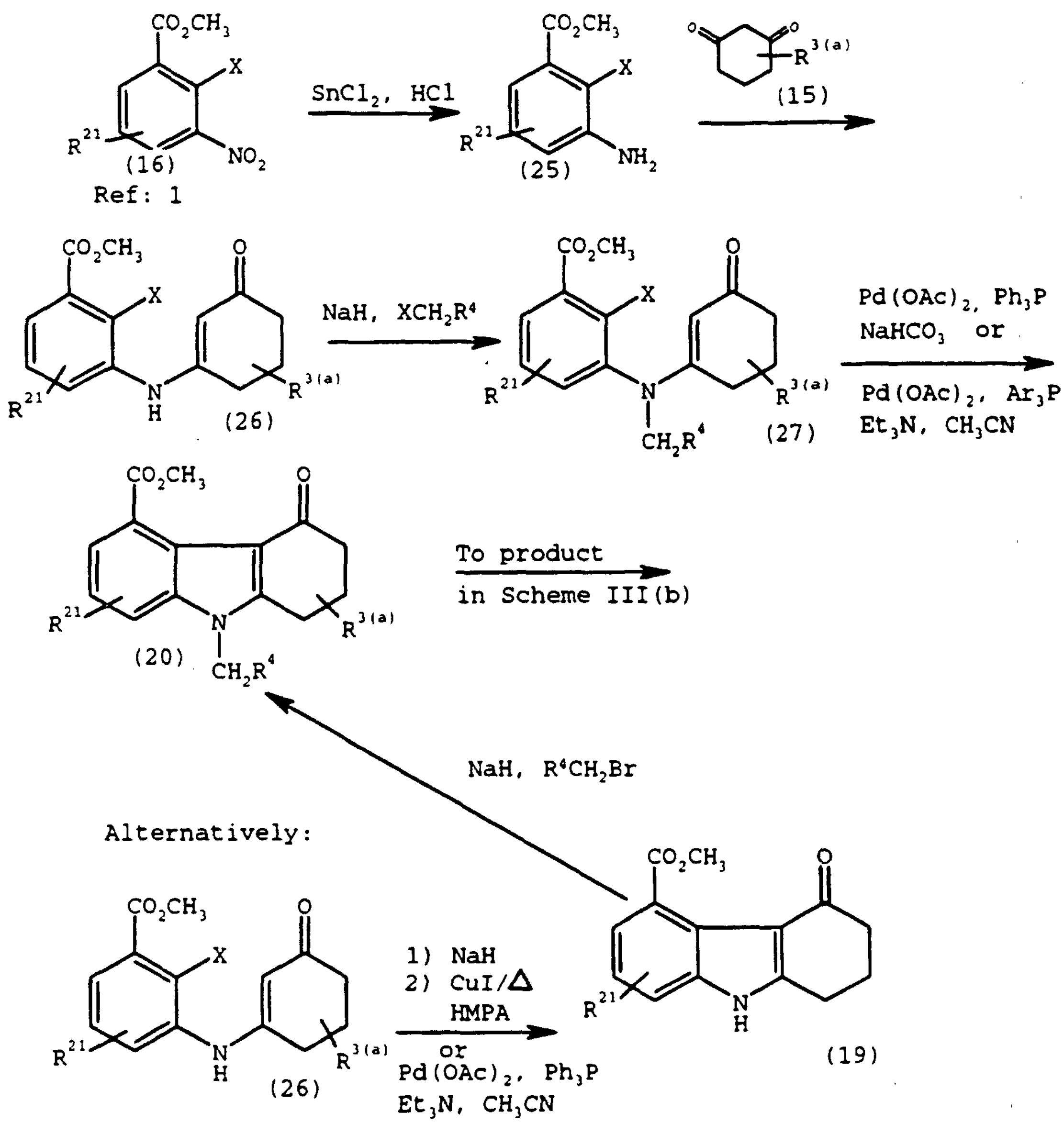


X is halo.

Benzoic acid derivatives (16) (X= Cl, Br, or I), described above, are reduced to the corresponding anilines (25) with a reducing agent, such as $SnCl_2$ in hydrochloric acid in an inert solvent, such as ethanol.

The aniline (25) and dione (15) are condensed under dehydrating conditions, for example, using the general procedure of Iida, et al., (Ref 5), with or without a noninterfering solvent, such as toluene, with or without a catalytic amount of an acid, such as p-toluenesulfonic acid, to afford the coupled product (26).

Compound (26) is N-alkylated with an appropriately substituted alkyl or aryl halide in the presence of a base, such as sodium hydride, in a noninterfering solvent, such as toluene, dimethylformamide, or dimethylsulfoxide to afford ketone (27).

Ketone (27) is cyclized according to the general procedure of Iida et al (Ref 5) or Kashara, et al., (Ref 6) in the presence of a palladium catalyst, such as $Pd(OAc)_2$, a triarylphosphine, such as tri-o-tolylphosphine, a base, such as sodium bicarbonate or triethylamine, in an inert solvent, such as acetonitrile or triethylamine to afford compound (20). Compound (20) can be processed as described in Scheme III(b) above, to the carbazole product (24). Alternatively, intermediate (26) can be cyclized to compound (19) using the palladium catalyzed conditions described for the procedures to convert compound (27) to (20) above.

Alternatively, intermediate (26) can be cyclized to compound (19) using the general procedure of Osuka, et al., (Ref 7) utilizing a base, such as sodium hydride, and a copper salt, such as copper (I) iodide, in an inert solvent, such as, hexamethylphosphoric triamide. Compound (19) can be converted to product carbazole product (24) as described in Scheme III(b), above.

The intermediate and final products may be isolated and purified by conventional techniques such as chromatography or recrystallization. Regioisomeric products and intermediates can be separated by standard methods, such as, recrystallization or chromatography.

References:

5) H. Iida et al., J. Org. Chem., 45, 2938 (1980)
6) A. Kasahara et al., Bull. Chem. Soc. Jpn., 59, 927 (1986)
7) A. Osuka et al., Chemistry Letters, 2031 (1982)

Scheme III(d)

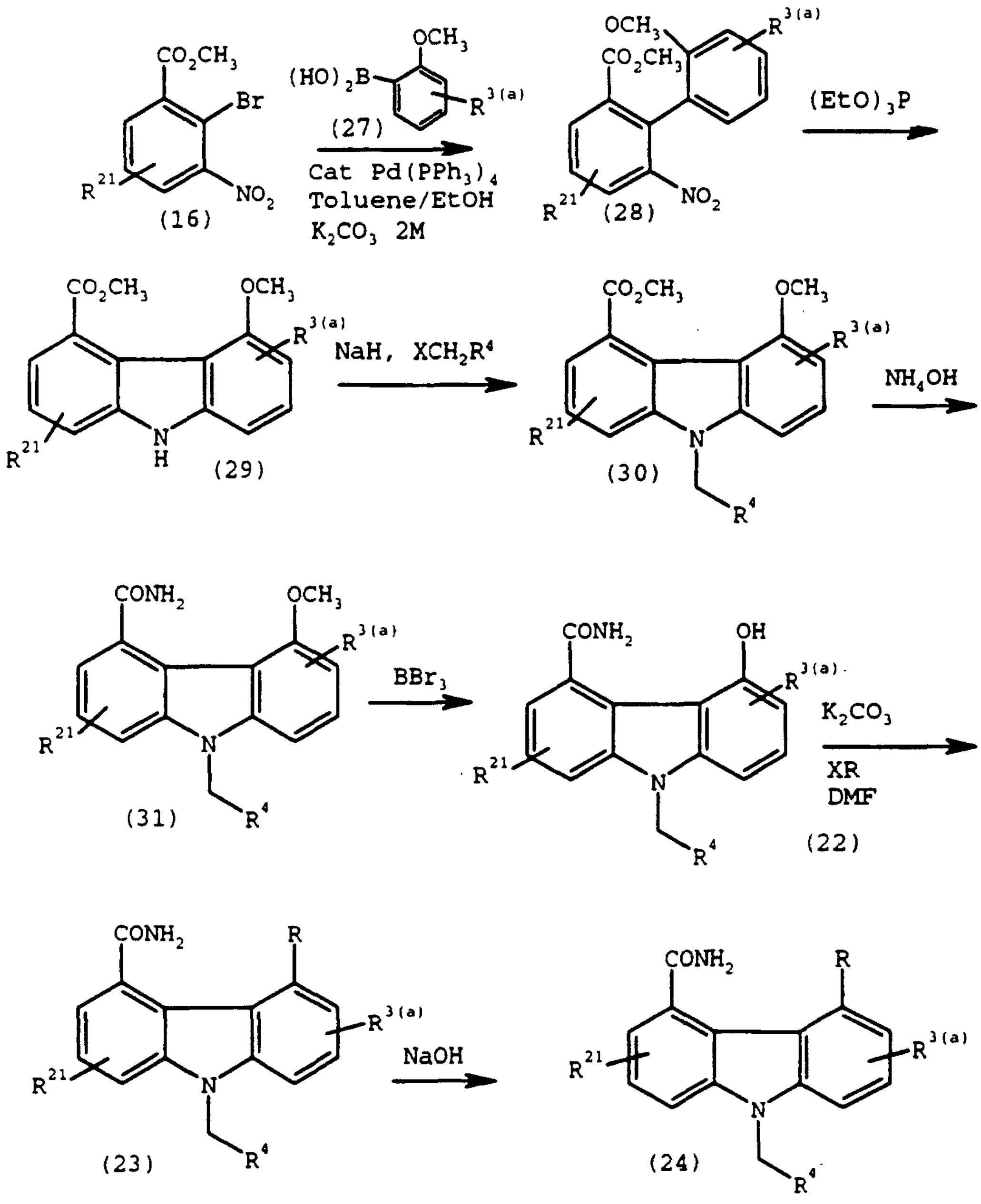


R is as defined in Scheme III(b),
$R^{3(a)}$ is as defined in Scheme I(a), above; and
X is halo.

Benzoic acid derivatives (16) (X= Cl, Br, or I) and boronic acid derivatives (27) (either commercially available or readily prepared by known techniques from commercially available starting materials) are condensed under the general procedure of Miyaura, et al., (Ref 8a) or Trecourt, et al., (Ref 8b) in the presence of a palladium catalyst, such as $Pd(Ph_3P)_4$, a base, such as sodium bicarbonate, in an inert solvent, such as toluene or ethanol, to afford compound (28).

Compound (28) is converted to the carbazole product (29) by treatment with a trialkyl phosphite or phosphine, such as, triethylphosphite or triphenyl phosphine, according to the general procedure of Cadogan, et al. (Ref 6).

Compound (29) is N-alkylated with an appropriately substituted alkyl or aryl halide in the presence of a base, such as sodium hydride or potassium carbonate, in a noninterfering solvent, such as toluene, dimethylformamide, or dimethylsulfoxide to afford carbazole (30).

Compound (30) is converted to the corresponding amide (31) under standard conditions with ammonia or an ammonium salt, such as ammonium acetate, in an inert solvent, such as water or alcohol, or with methylchloroaluminum amide in an inert solvent, such as toluene, at a temperature between 0 to 110°C.

Compound (31) may be dealkylated with a dealkylating agent, such as boron tribromide or sodium thioethoxide, to give compound (22). Compound (22) can be converted to product carbazole product (24) as described in Scheme III(b) above.

Scheme III(d)

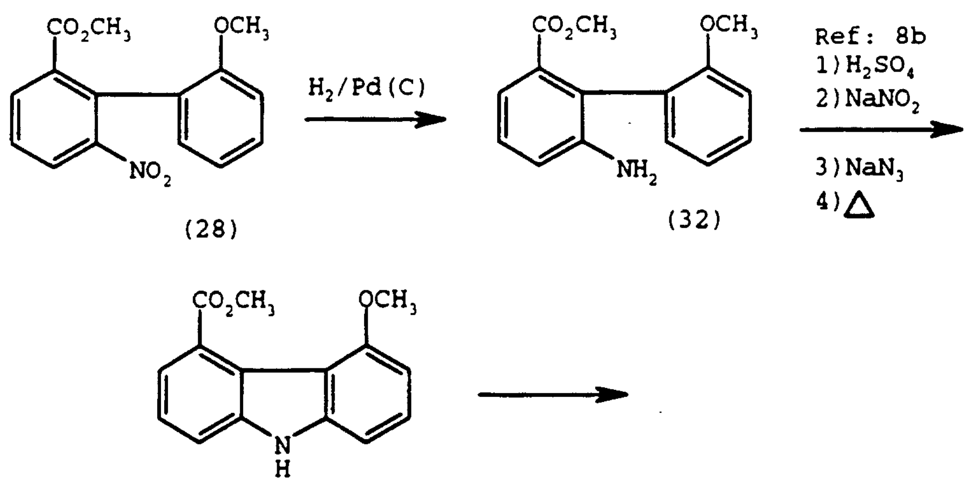


(28) (32) (29)

Alternatively, reduction of the nitro group of compound (28) with a reducing agent, such as hydrogen in the presence of palladium on carbon, in a noninterfering solvent, such as ethanol, at 1 to 60 atmospheres, at a temperature of 0 to 60°C affords the corresponding aniline (32). Compound (32) is converted to the carbazole (29) according to the general procedure described by Trecourt, et al. (Ref 8b). The aniline is treated with sulfuric acid and sodium nitrite, followed by sodium azide to form an intermediate azide which is cyclized to carbazole (29) by heating in an inert sovent, such as toluene. Compound (29) is converted to carbazole product (24) as described previously in Scheme III(b).

References:

8) a. N. Miyaura, et al., Synth. Commun. 11, 513 (1981)

b. F. Trecourt, et al., Tetrahedron, 51, 11743 6) J. Cadogan et al., J. Chem. Soc., 4831 (1965)

Scheme IV(a)

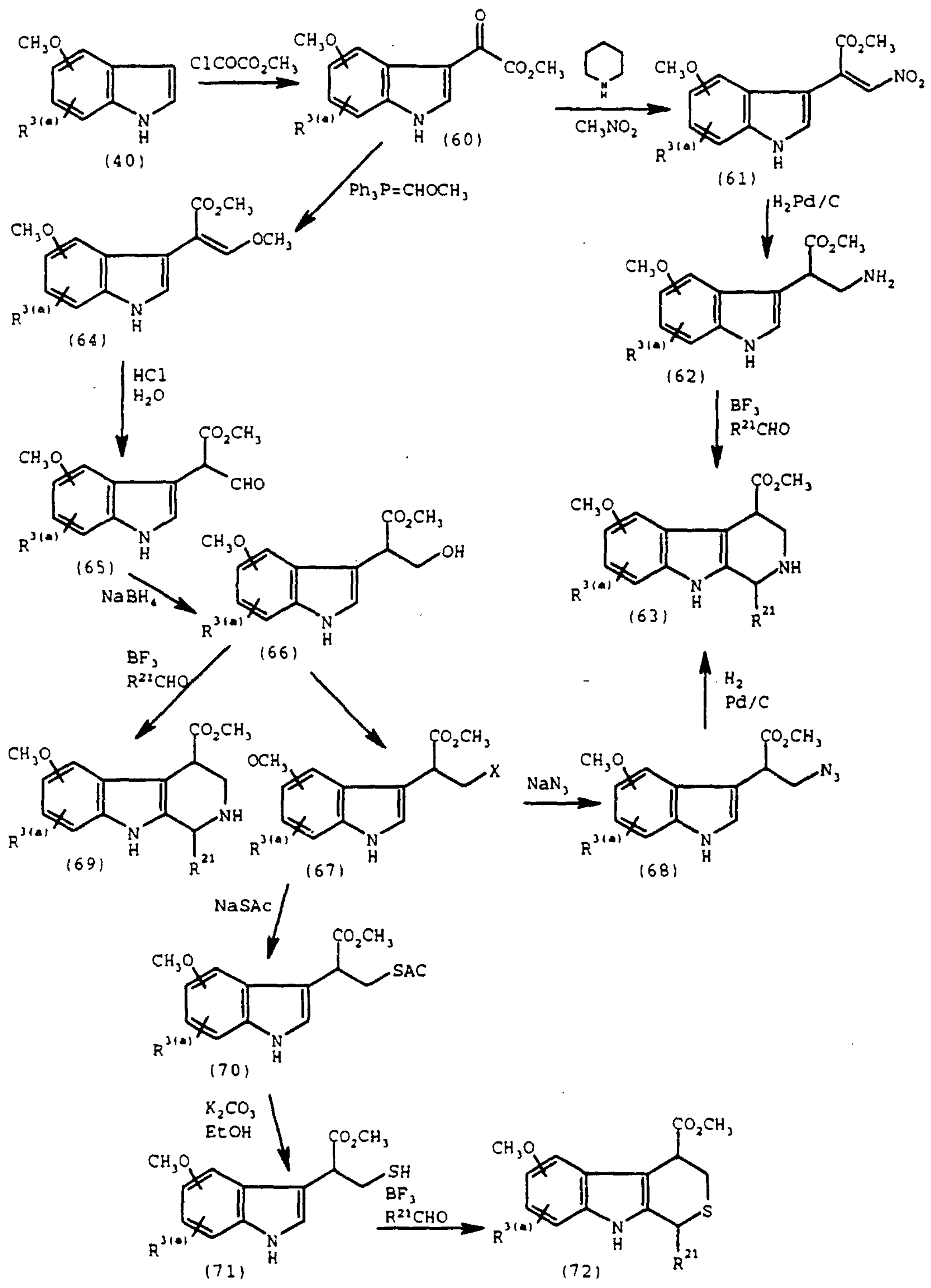


X is halo
$R^{3(a)}$ is as defined in Scheme I(a).

4-Methoxyindole (40) is acylated by methyl oxalyl chloride to give the indole-3-glyoxyl derivative (60). Alternately, treatment with oxalyl chloride and then ammonia may be employed to give the amide which can be used in subsequent steps as well as the ester. Compound (61) is condensed with nitromethane in the presence of a base to

give the nitro olefin (61) which is reduced to the amine (62) by, for example, treatment with hydrogen and palladium or carbon. Amine (62) on reaction with an appropriately substituted aldehyde and acid produces the carbolines (63).

Reaction of (60) with the methoxymethylene Wittig reagent gives the enol ether (64) which is hydrolyzed to the aldehyde (65) and reduced with hydride to the alcohol (66). Reaction of this alcohol with aldehyde and acid produces the pyranoindole (69).

Conversion of the hydroxyl function of (66) to a halide or sulfate functionality by treatment with triphenylphospine and $CH_4X$ (where X is a halogen) to make compounds of formula (67) where X is a halide; or by treatment with triethylamine and methanesulfonyl chloride to make the sulfate (67), followed by displacement with the sodium salt of thiol acetic acid gives (70) which in turn is hydrolyzed by base to the thiol (71) which is reacted with an appropriately substituted aldehyde and acid to produce the thiopyranoindoles (72).

Intermediate (67) may also be reacted with sodium azide to give the azido derivatives (68) which are reduced by hydrogen catalytically to give the amines (63) which are converted to the carbolines (64) as above.

Intermediates (63), (69) and (72) may be N-alkylated as described in Scheme I above, using sodium hydride and an appropriately substituted alkylhalide $XCH_2R^4$.

Scheme IV(b)

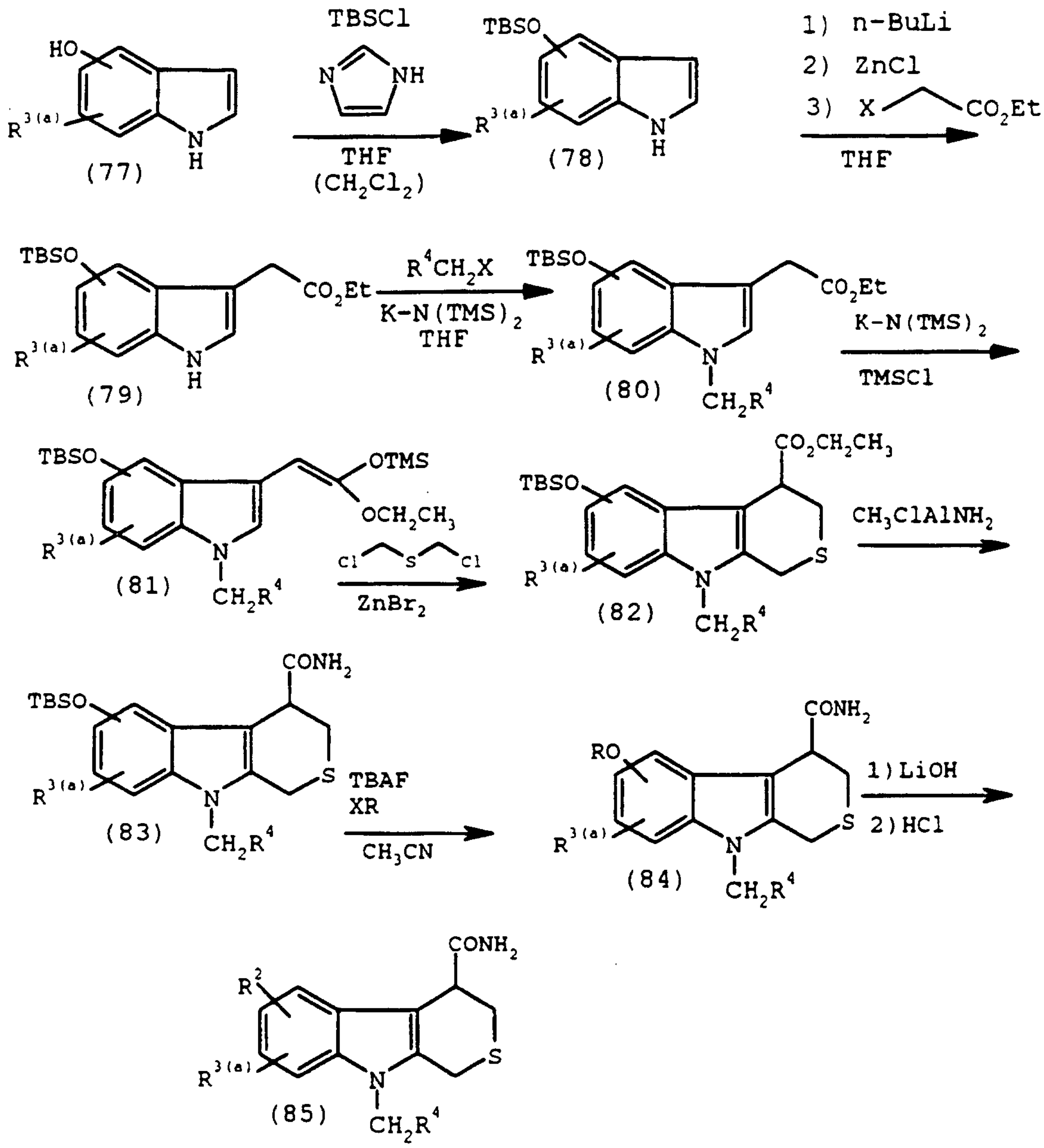


X is halo,

$R^{3(a)}$ is as defined in Scheme I(a); and
R is as defined in Scheme III(b).

Protection of the oxygen by treatment of (77) with tert-butyldimethylsilyl chloride and imidazole in an aprotic polar solvent such as tetrahydrofuran or methylene chloride accomplishes (78).

Alkylation at the 3-position of the indole (78) is achieved by treatment with n-butyllithum then zinc chloride at temperatures starting at about 10°C and warming to room temperature, followed by reaction with an appropriate haloalkyl ester such as ethyl or bromo acetate. The reaction is preferably conducted at room temperature in an appropriate aprotic polar solvent such as tetrahydrofuran.

Alkylation of the indole-nitrogen can then be achieved by reacting (79) with a suitable alkyl halide in the presence of potassium bis(trimethylsilyl)amide to prepare (80).

The ester functionality of (80) is converted to a trimethylsilylketene acetal (81) by treatment with potassium bis(trimethylsilyl)amide and trimethylsilyl chloride. Treatment of the ketene acetal (81) with bis(chloromethyl)sulfide and zinc bromide in methylene chloride affords the cyclized product (82). Conversion to amide (83) can be accomplished by a Weinreb reaction with methylchloroaluminum amide. Removal of the oxygen protecting group with a fluoride source, such as TBAF, and concommitant reaction of the resulting anion with, for example, ethyl bromoacetate yields the ester (84). Deprotection of the ester yields the desired acid (85).

Scheme IV(d)

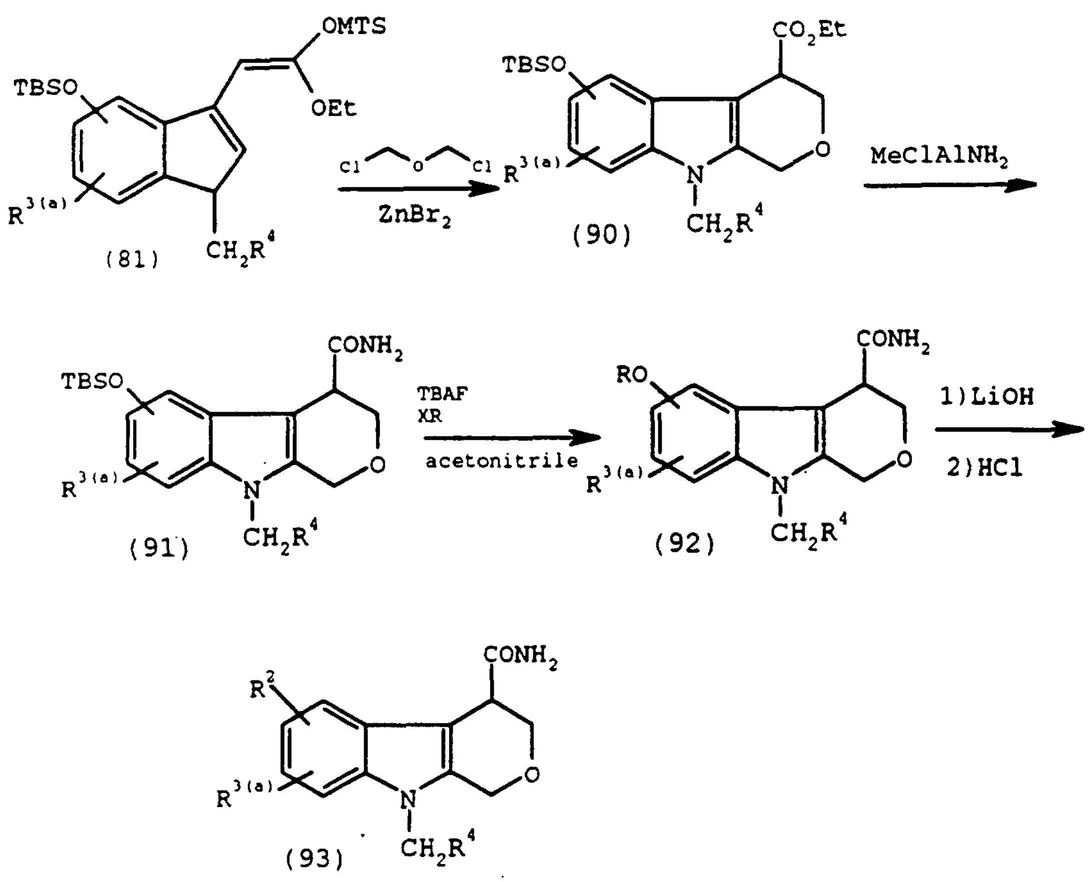


$R^3$(a) is as described in Scheme I(a) and
R is as described in Scheme III(b).

Treatment of the ketene acetal (81) with bis(chloromethyl)ether and zinc bromide in methylene chloride affords the cyclized product (90). Conversion to amide (91) can be accomplished by a Weinreb reaction with methylchloroaluminum amide. Removal of the oxygen protecting group with a fluoride source, such as TBAF, and concommitant reaction of the resulting anion with ethyl bromoacetate yields the ester (92). Deprotection of the ester yields the desired

acid (93).

Compounds where Z is an aromatic or heterocyclic ring containing nitrogen can be prepared as described in Schemes V(a)-(e), below.

Scheme V(a)

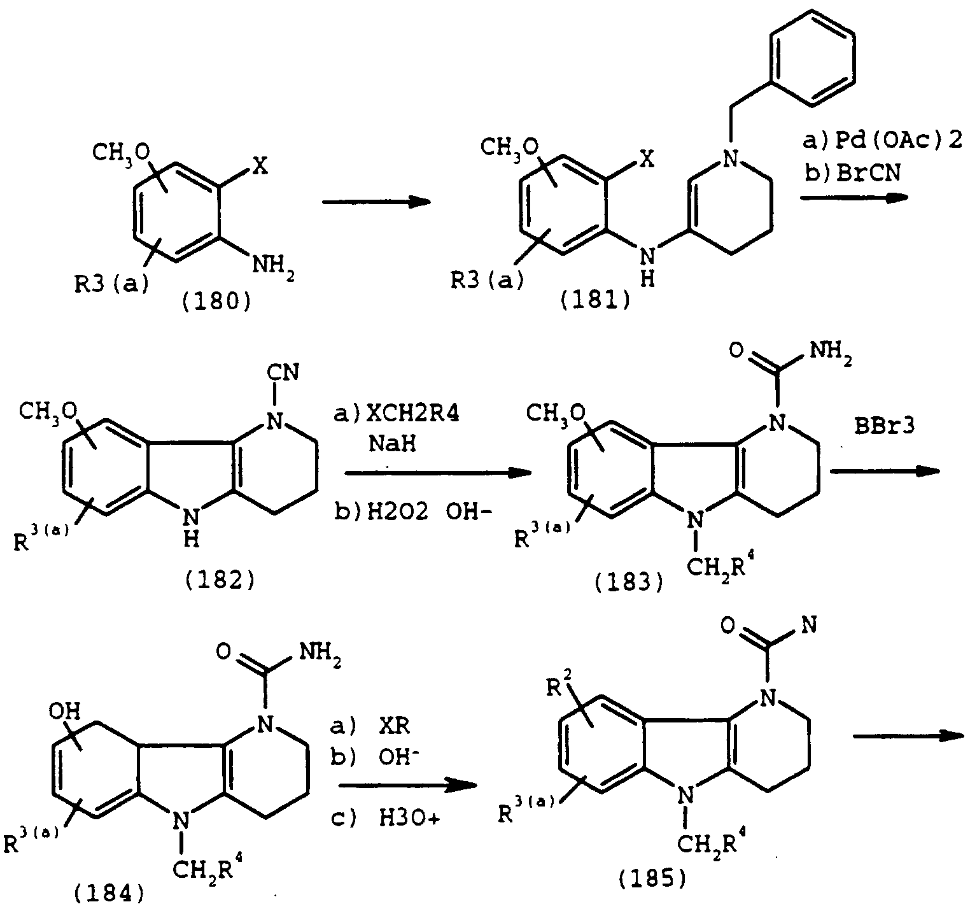


Substituted haloaniline (180) is condensed with N-benzyl-3-piperidone to provide enamine (181). Ring closure is effected by treatment of (181) with palladium (II) acetate and the resultant product is converted to (182) by treatment with cyanogen bromide. Alkylation of (182) is accomplished by treatment with the appropriate alkyl bromide using sodium hydride as base. Hydrolysis of this N-alkylated product with basic hydrogen peroxide under standard conditions provides (183). Demethylation of (183) is carried out by treatment with boron tribromide in methylene chloride. The resulting phenol (184) is converted by the standard sequence of O-alkylation with methyl bromoacetate in the presence of a base, hydrolysis with hydroxide to provide the intermediate salt which is then protonated in aqueous acid to provide desired δ-carboline (185).

Scheme V(b)

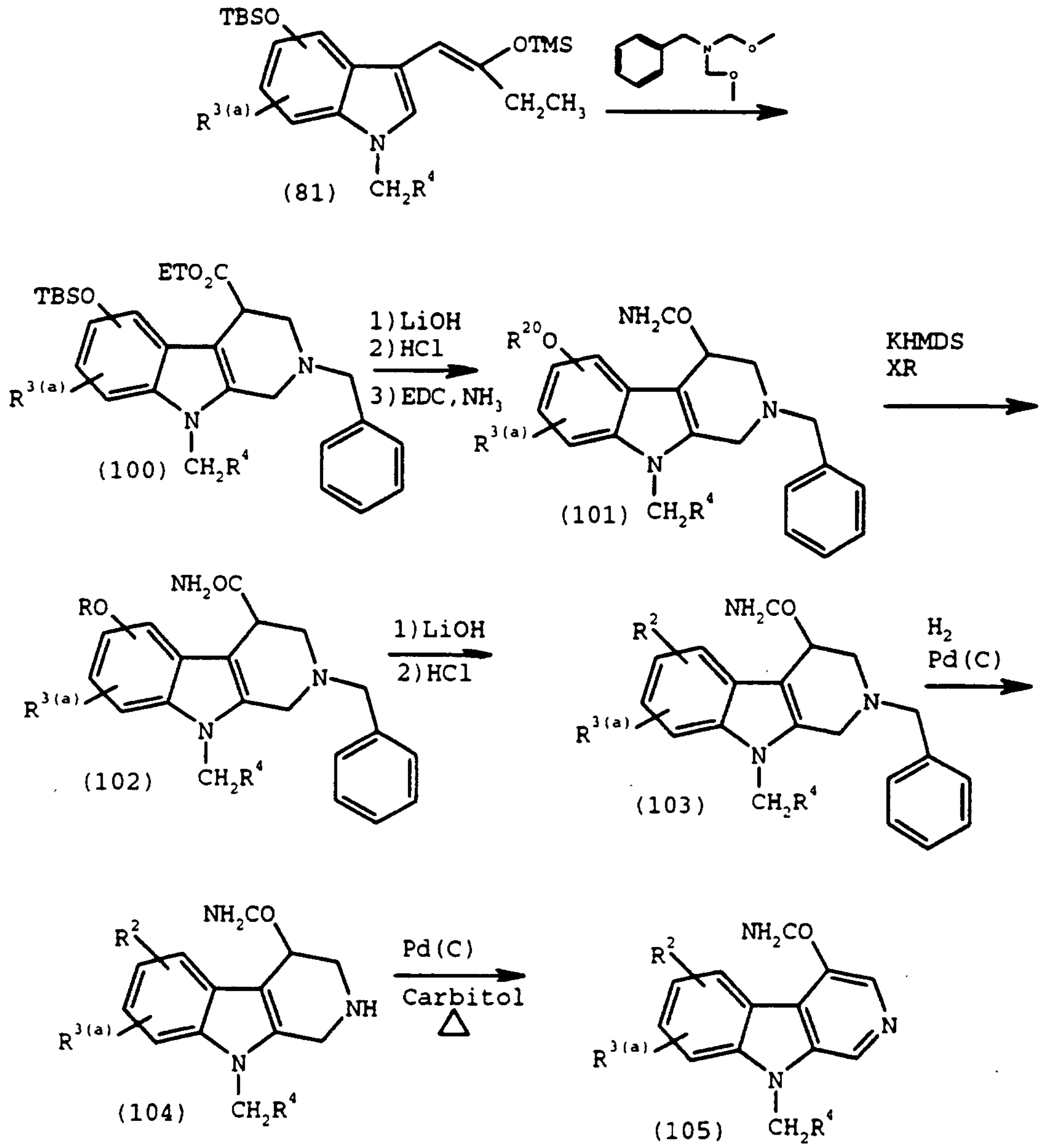


X is halo,
R is as defined in Scheme III(b), and
$R^{3(a)}$ is as defined in Scheme I(a).

Ketene acetal (81), prepared as described in Scheme IV(c), is reacted with benzyl bis(methoxymethyl)amine in the presence of zinc chloride to give the tetrahydro-beta-carboline (100).

Treatment of (100) with lithium hydroxide, neutralization with hydrochloric acid and subsequent treatment with ethylaluminum dichloride and ammonia provides the desilyated amide (101) where $R^{20}$ is hydrogen, which can be alkylated with, for example, ethylbromoacetate to give ester (102).

Alternatively, treatment of (100) with the appropriate Weinreb reagent provides amide (101) ($R^{20}$ is t-butyldimethylsilyl) which is desilylated with tetra-n-butylammonium fluoride and alkylated with, for example, ethyl bromoacetate to give ester (102). Lithium hydroxide-mediated hydrolysis gives acid (103), which may be hydrogenated over an appropriate catalyst to give the tetrahydro-beta-carboline (104). Compound (104) may in turn be aromatized by refluxing in carbitol with palladium on carbon to provide beta-carboline (105).

Scheme V(c)

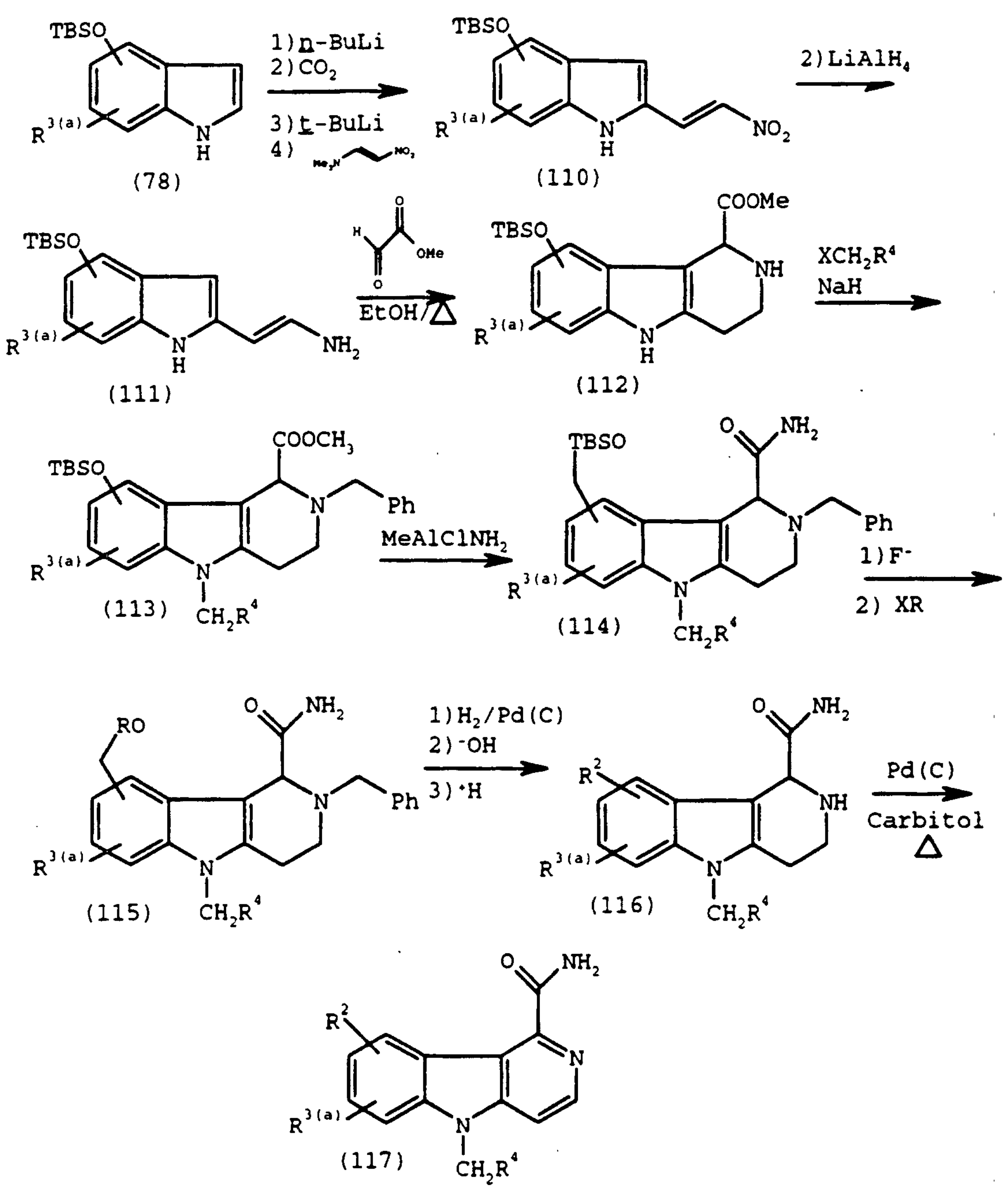


(78) (110) (111) (112) (113) (114) (115) (116) (117)

X is halo,
R is as defined in Scheme III(b); and
$R^3$(a) is as defined in Scheme I(a).

In a one-pot reaction, indole (78) is successively treated with one equivalent n-butyllithium, carbon dioxide gas, one equivalent of t-butyllithium, and 1-dimethylamino-2-nitroethene to give (110). Nitroalkene (110) is reduced with lithium aluminum hydride to amine (111), which is cyclized with methyl glyoxylate (Ref. 9) in refluxing ethanol to give tetrahydrocarboline (112). Alkylation of both nitrogens of (112) leads to intermediate (113), which is treated with the appropriate Weinreb reagent to provide amide (114). Fluoride-assisted desilylation and alkylation with, for example, ethyl iodoacetate gives ester (115), which may be hydrogenated over a suitable catalyst and base-hydrolyzed to give acid (116). Aromatization of (116) to carboline (117) is achieved by refluxing in carbitol in the presence of palladium-on-carbon.

Reference 9:

Kelley, T. R.; Schmidt, T. E.; Haggerty, J. G. A convenient preparation of methyl and ethyl glyoxylate, *Synthesis*, **1972,** 544-5.

Scheme V(d)

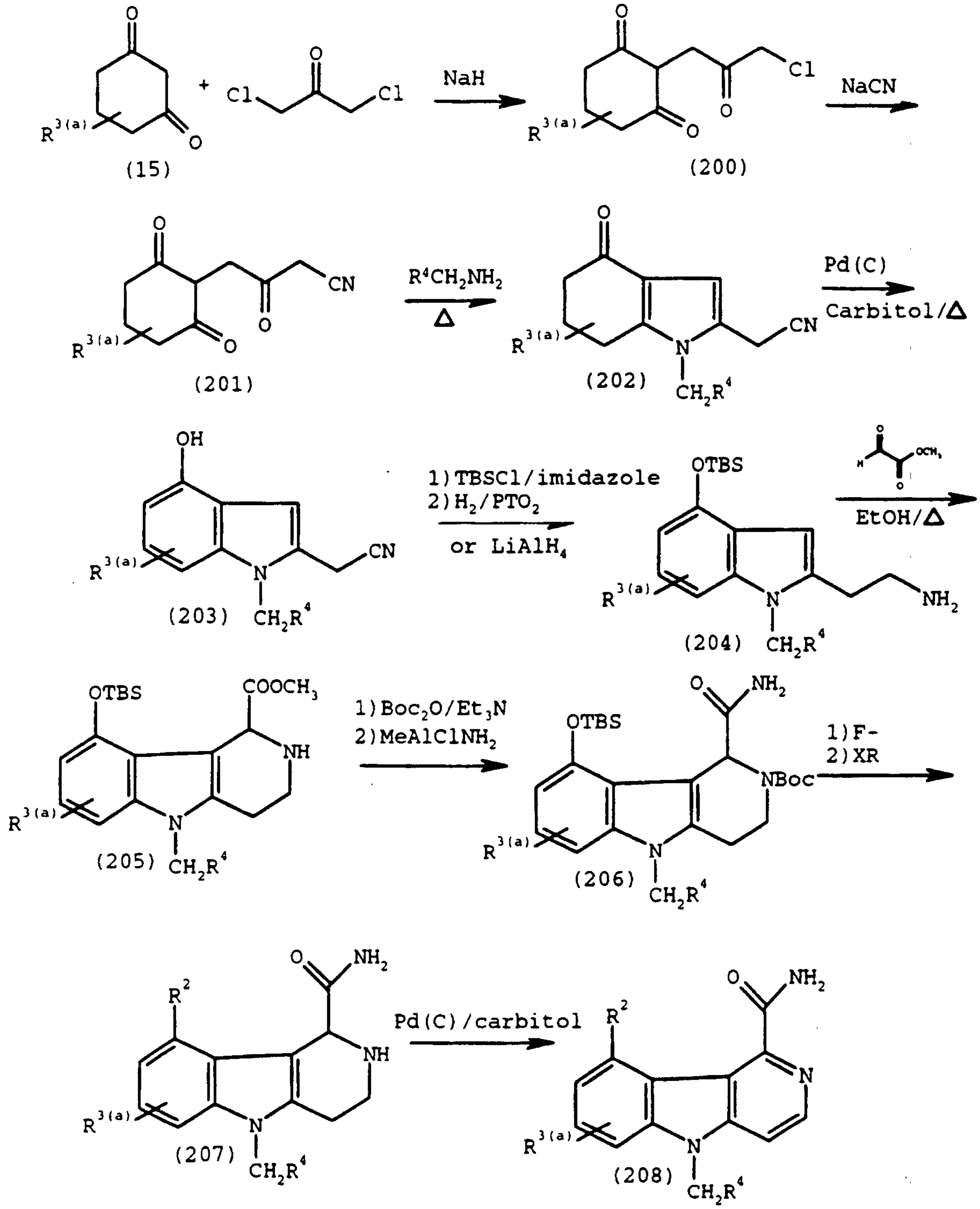


$R^{3(a)}$ is as defined in Scheme I(a),
X is halo, and

R is as defined in Scheme III(b).

1,3-Cyclohexadione (15) is alkylated with 1,3-dichloroacetone to give chloride (200), which is reacted with sodium cyanide to provide nitrile (201). Cyclization with, for example, benzyl amine gives pyrrole (202), which is then aromatized by refluxing in carbitol to produce indole (203). Protection of the phenol with t-butyldimethylsilyl chloride followed by reduction of the acetonitrile side chain using lithium aluminum hydride or hydrogen with platinum oxide provides amine (204). Cyclization with methyl glyoxylate in refluxing ethanol gives tetrahydrocarboline (205), which is treated with di-t-butyl dicarbonate and triethylamine followed by the appropriate Weinreb reagent to provide amide (206). Fluoride-assisted desilylation, alkylation with ethyl iodoacetate, and acid hydrolysis gives acid (207), which may be aromatized by refluxing in carbitol or some other suitable high boiling solvent to provide (208).

Scheme V(e)

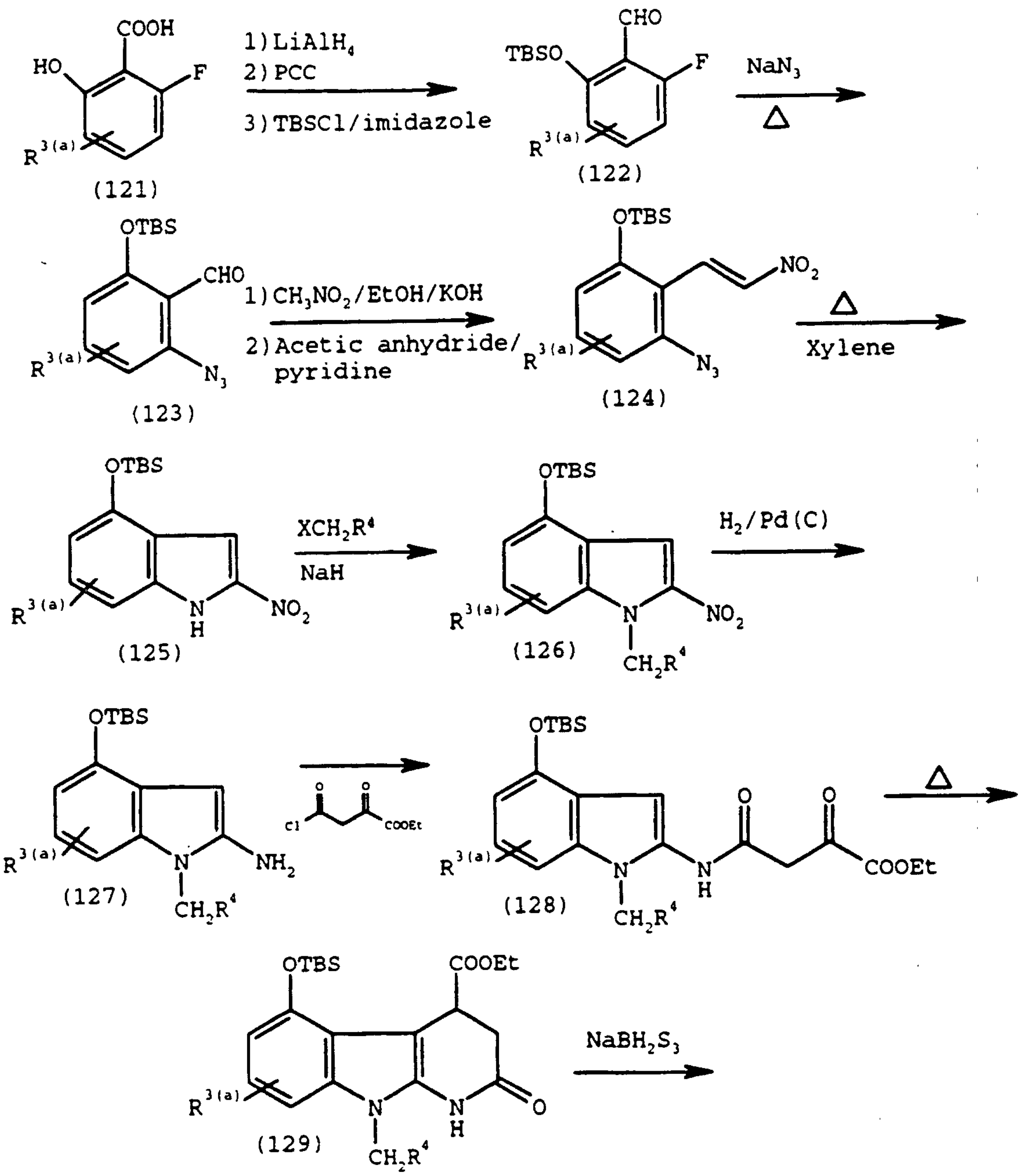


(121) (122) (123) (124) (125) (126) (127) (128) (129)

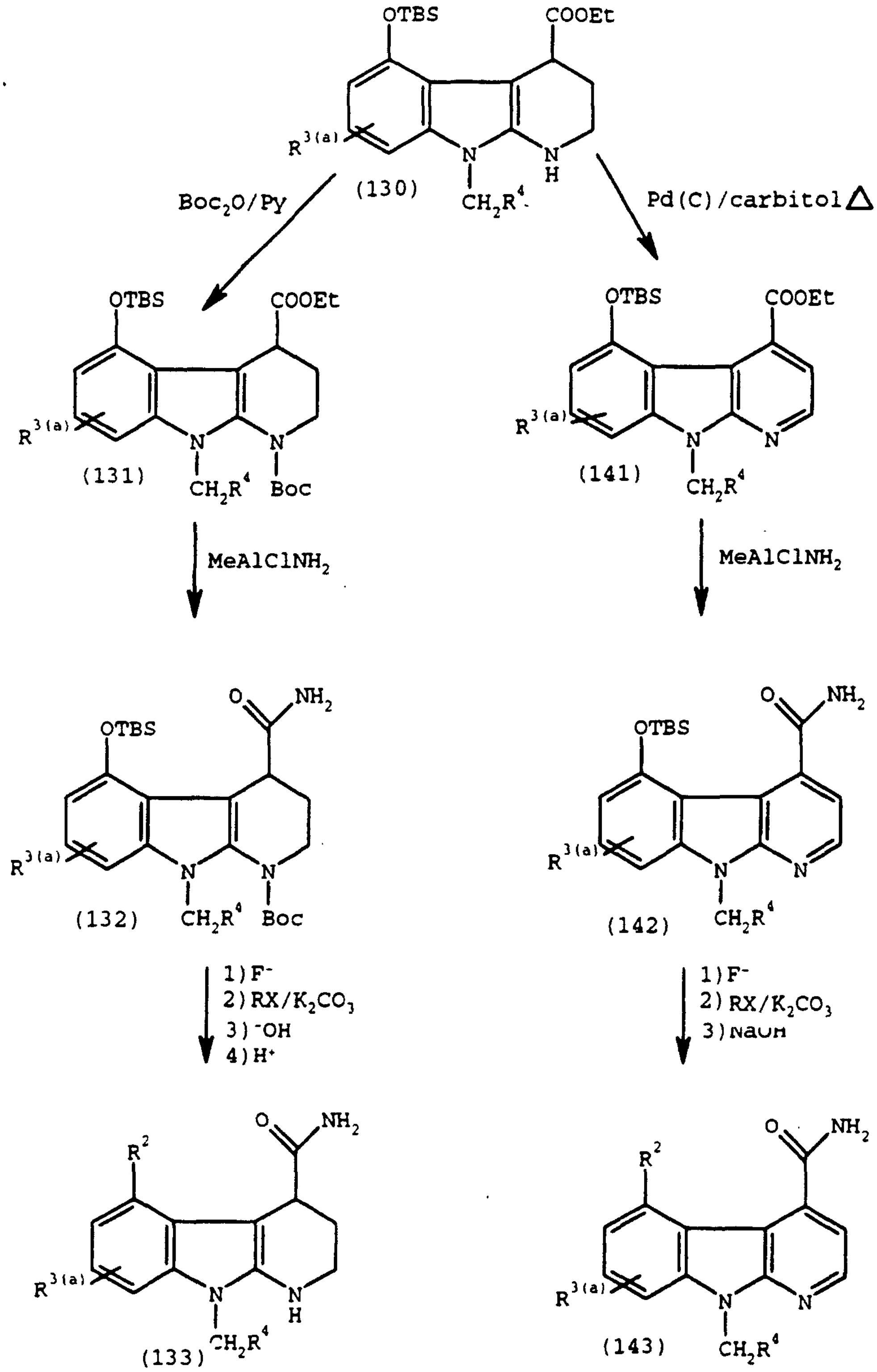


The commercially available acid (121) is reduced with lithium aluminum hydride, oxidized with pyridinium chlorochromate, and silylated with t-butyldimethylsilyl chloride to give (122). Treatment with sodium azide provides azide (123), which is reacted with nitromethane and potassium hydroxide in ethanol, followed by treatment with acetic anhydride and pyridine to give nitroolefin (124). Heating in xylene induces cyclization to produce indole (125). Alkylation with, for example, benzyl iodide and sodium hydride gives (126), which is hydrogenated in the presence of palladium-on-carbon to give amine (127). Acylation with the acid chloride of commercially available oxalacetic acid monoethyl

ester gives (128), which is thermally cyclized to lactam (129). Selective reduction of the lactam carbonyl may be accomplished by treatment with $NaBH_2S_3$ to provide amine (130).

Protection of amine (130) with di-t-butyl dicarbonate and pyridine produces (131), which is converted via the appropriate Weinreb reagent to amide (132). Fluoride-assisted desilylation, alkylation with, for example, ethyl iodoacetate and potassium carbonate, base hydrolysis, and acid hydrolysis produce the tetrahydro-alpha-carboline (133).

Alternatively, amine (130) may be aromatized by refluxing in carbitol or some other suitable high boiling solvent to give alpha-carboline (141), which is converted via the appropriate Weinreb reagent to amide (142). Fluoride-assisted desilylation, alkylation with ethyl iodoacetate and potassium carbonate, and base hydrolysis as described above provides alpha-carboline (143).

Reverse indoles, i.e., compounds where B is carbon and D is nitrogen can be prepared as described in Scheme VI, below.

Scheme VI

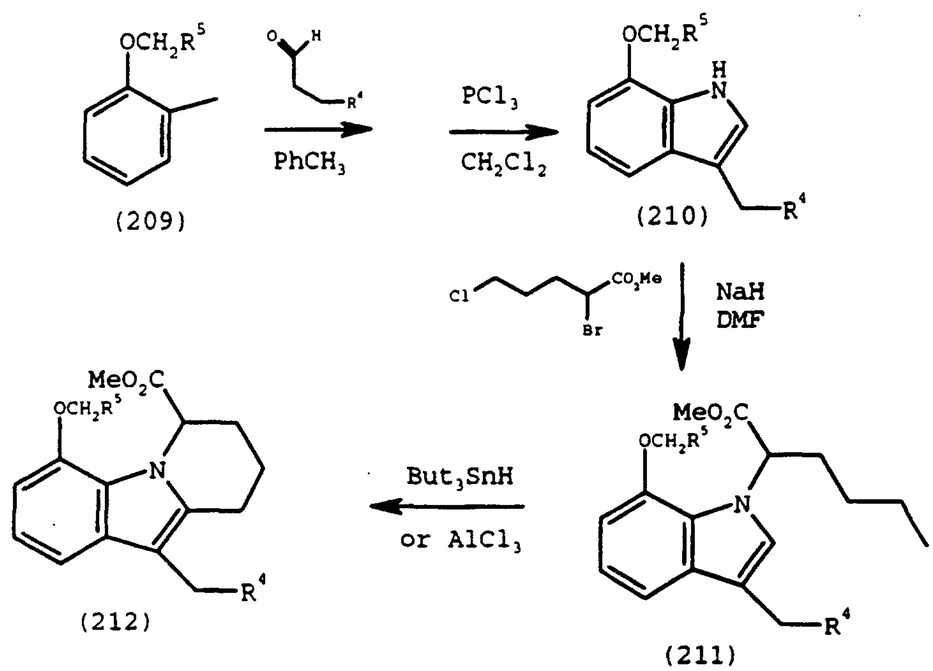

(209) (210) (211) (212)

Aryl hydrazines (209) are condensed with substituted prpionaldehydes to form hydrazones which are cyclized to indoles (210) by treatment with phosphorous trichloride at room temperature (Ref 1). The indoles are N-alkylated on reaction with a base such as sodium hydride and an alph-bromo ester to give indoles (211) which are cyclized to tetrahydrocarbazoles (212) by Lewis acids (e.g., aluminum chloride) or by radical initiators (e.g., tributyltin hydride). Compounds (212) can be converted to carbazoles by the procedures of Scheme III.

Compounds of formula III wherein A is pyridyl can be prepared as described in Scheme VIII, below.

Scheme VIII(a)

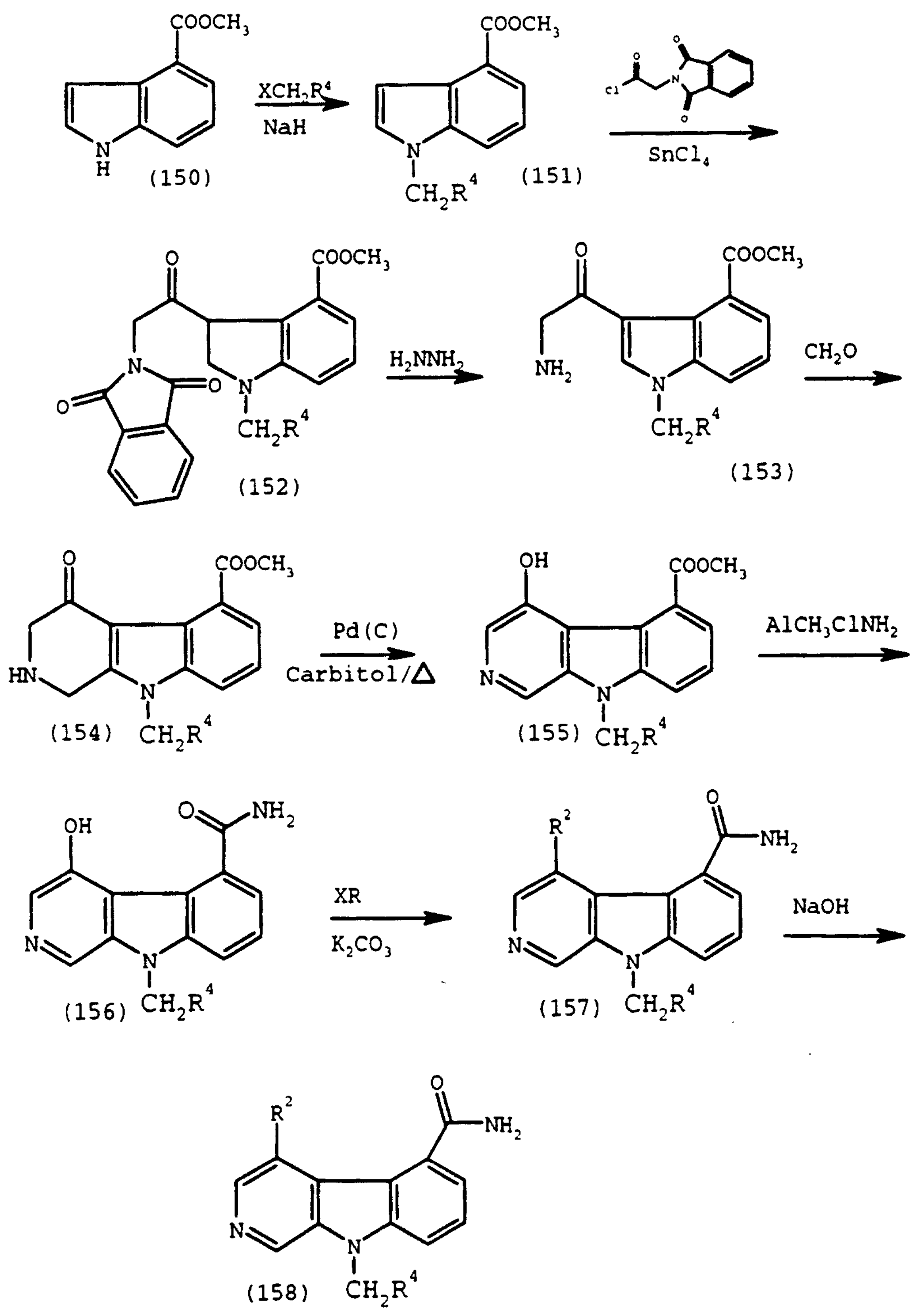


R is as defined in Scheme III(b) and
X is halo.

Commercially available 4-carbomethoxyindole (150) with, for example, benzyl iodide and a base such as sodium hydride to provide indole (151). Acylation with the acyl chloride of N-phthaloylglycine, catalyzed by a Lewis acid such as stannous tetrachloride, provides intermediate (152), which may be deprotected to give amine (153) by

treatment with hydrazine in refluxing ethanol. Pictet-Spengler cyclization of (153) with formaldehyde gives (154), which is then aromatized by refluxing in carbitol or some other suitable high boiling solvent. The resulting beta-carboline (155) is treated with the appropriate Weinreb reagent to provide amide (156), which is alkylated with, for example, ethyl bromoacetate to produce (157) and saponified to give beta-carboline (158).

Scheme VIII(b)

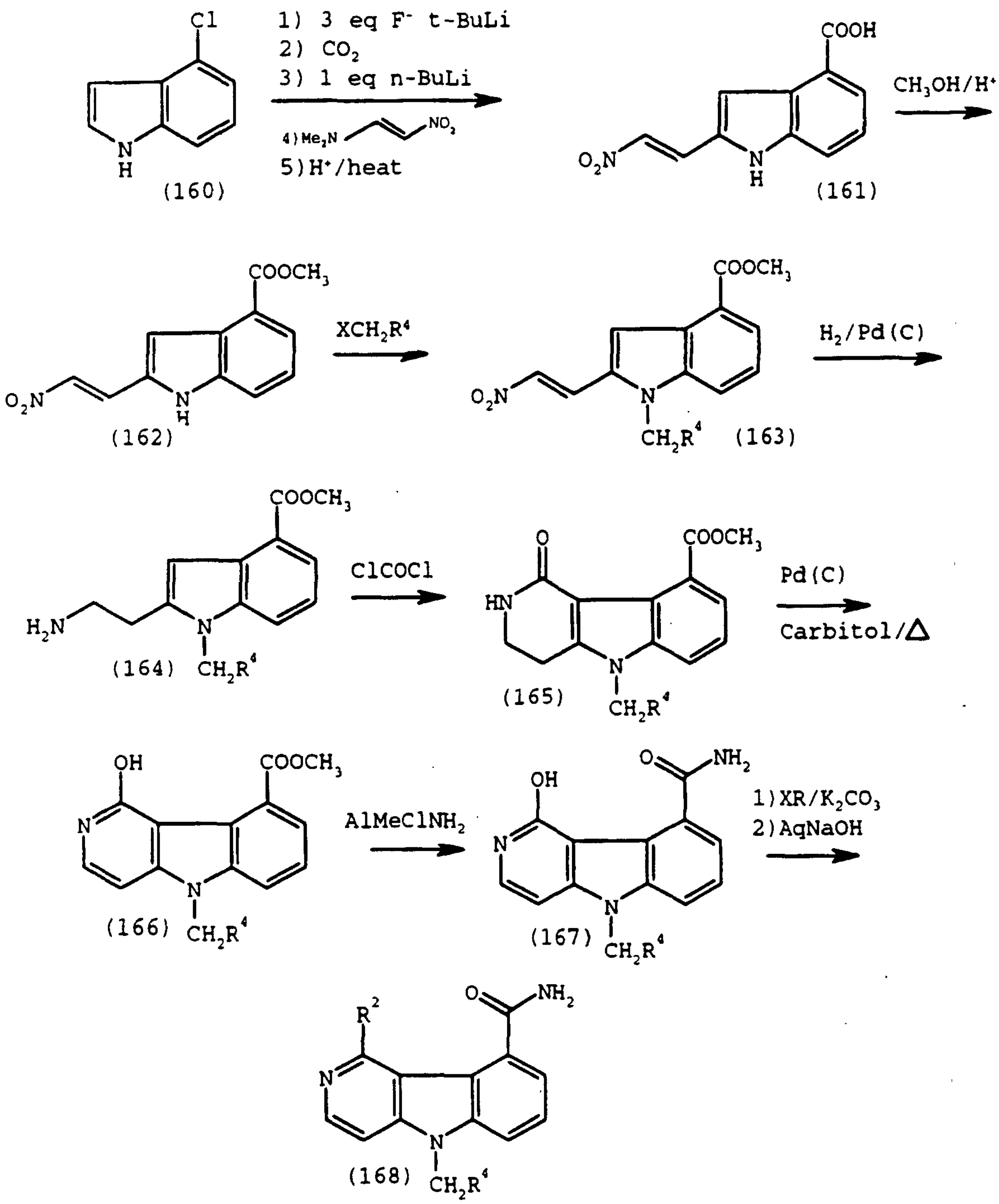


X is halo and
R is as defined in Scheme III(b).

Commercially available 4-chloroindole (160) is treated with 3 equivalents of t-butyllithium followed by carbon

dioxide, 1 equivalent of n-butyllithium, 1-dimethylamino-2-nitroethene, and acid to provide carboxylic acid (161), which may be esterified to give (162). Alkylation at the 1-position followed by hydrogenation provides aminoethyl indole (164). Cyclization with phosgene to (165) followed by aromatization gives carboline (166). Treatment of (166) with the appropriate Weinreb reagent provides amide (167), which may be alkylated with, for example, ethyl bromoacetate and saponified with sodium hydroxide to give the carboline (168).

Scheme VIII(c)

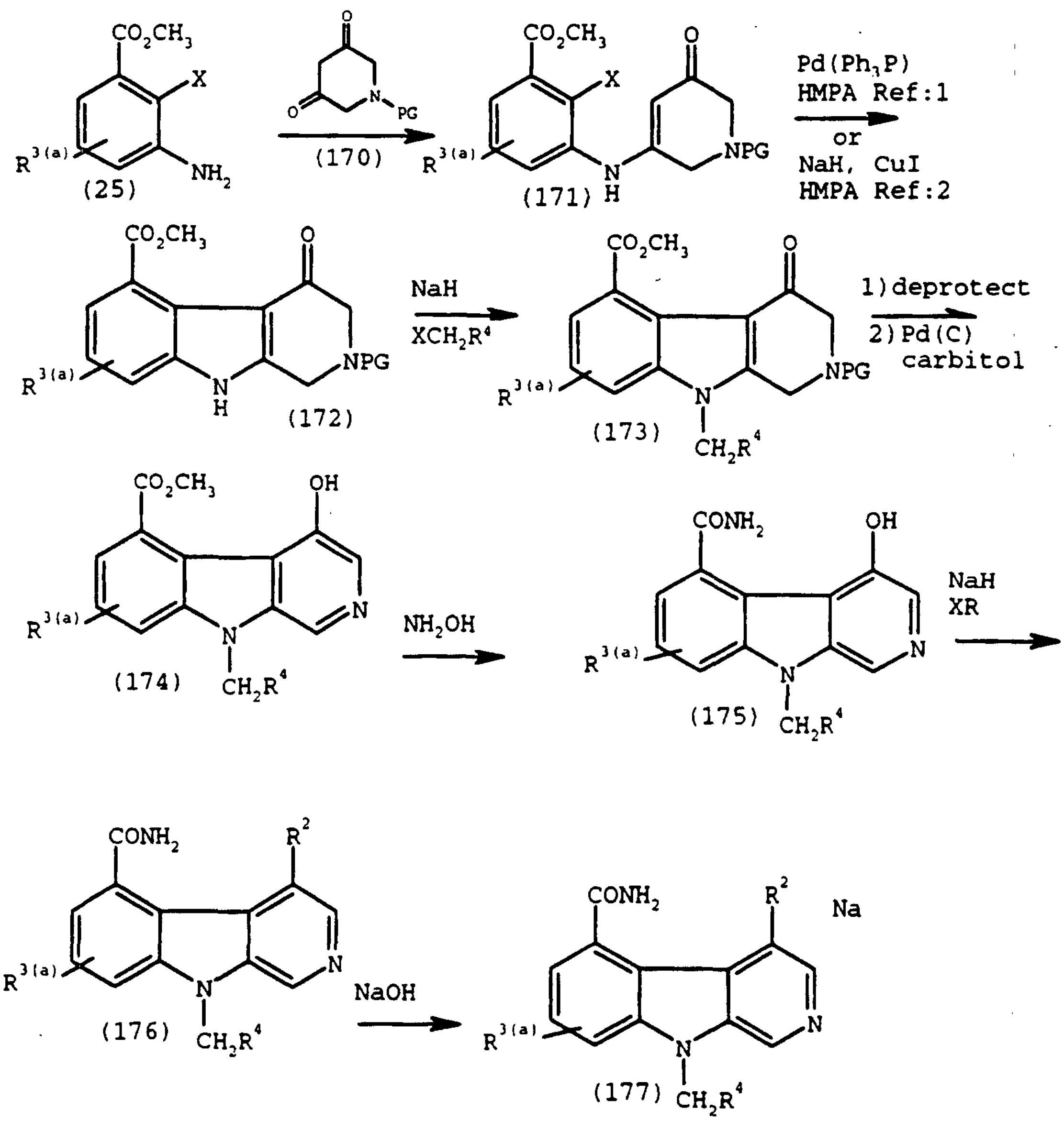


R3(a) is as defined in Scheme I(a),
X is halo, and
R is defined in Scheme III(b).

The 1,3-dione structures (170) are either commercially available or readily prepared by known techniques from commercially available starting materials. Preparation of the aniline derivatives (25) (X= Cl, Br, or I) are described in Scheme III(c) above. The amino group of (170) is protected with an appropriate protecting group, such as the, carboethoxyl, benzyl, CBZ or BOC protecting group, and the like.

The dione (170) and aniline derivative (25) are condensed according to the general procedure of Chen, et al., (Ref 10) or Yang, et al., (Ref 11), with or without a noninterfering solvent, such as methanol, toluene, or methylene

chloride, with or without an acid, such as p-toluenesulfonic acid or trifluoroacetic acid, with or without N-chlorosuccinimide and dimethyl sulfide, to afford the coupled product (172).

Compound (171) is cyclized under basic conditions with a copper (I) salt in an inert solvent according to the general procedure of Yang, et al., (Ref 8). The derivative (171) is treated with a base, such as sodium hydride, in an inert solvent, such as HMPA, at a temperature between 0 and 25°C. A copper (I) salt, such as copper (I) iodide, is added and the resultant mixture stirred at a temperature between 25 and 150°C for 1 to 48 hours to afford compound (173).

Compound (171) may also be cyclized according to the general procedure of Chen, et al., (Ref 10). The derivative (171) is treated with a base, such as sodium bicarbonate, and a palladium catalyst, such as $Pd(PPh_3)_4$, in an inert solvent, such as HMPA, at a temperature between 25 and 150°C to afford compound (172).

Compound (172) is N-alkylated with an appropriately substituted benzyl halide in the presence of a base, such as sodium hydride or potassium carbonate, in a noninterfering solvent, such as dimethylformamide or dimethylsulfoxide to afford ketone (173). The nitrogen protecting group of (172) is removed under standard conditions and the resulting ketone is dehydrogenated by treatment with palladium on carbon in a noninterfering solvent, such as carbitol or cymene, to produce the phenolic derivative (174).

The ester (174) is converted to the corresponding amide (175) under standard conditions with ammonia or an ammonium salt, such as ammonium acetate, in an inert solvent, such as water or alcohol, or with $MeClAlNH_2$ in an inert solvent, such as toluene, at a temperature between 0 to 110°C. Alkylation of the phenolic oxygen of compound 38 with an appropriate haloester, such as methyl bromoacetate, in the presence of a base, such as potassium or sodium carbonate, in an inert solvent, such as dimethylformamide or dimethylsulfoxide affords the ester-amide (176). Other haloesters, such as ethyl bromoacetate, propyl bromoacetate, butyl bromoacetate, and the like can also be used to prepare the corresponding esters.

Saponification of compound (176), with sodium hydroxide in an inert solvent, such as methanol-water, affords (177). The intermediate and final products may isolated and purified by conventional techniques such as chromatography or recrystallization. Regioisomeric products and intermediates can be separated by standard methods, such as, recrystallization or chromatography.

References:

10) L.-C. Chen et al., Synthesis 385 (1995)

11) S.-C. Yang et al., Heterocycles, 32, 2399 (1991)

The following examples further illustrate the preparation of the compounds of this invention. The examples are illustrative only and are not intended to limit the scope of the invention in any way.

Example 1

Preparation of 9-benzyl-6-methoxy-1,2,3,4-tetrahydrocarbazole-4-carboxamide.

A. Preparation of 9-benzyl-6-methoxy-1,2,3,4-tetrahydrocarbazole-4-carboxylic acid hydrazide.

Compound (5) ($R^2$=6-MeO, $R^{3(a)}$=H, $R^4$=phenyl) was prepared using the method given in Julia, M. and Lenzi, J. Bull. Soc. Chim. France, 1962, 2262-2263. Equimolar quantities of N-benzyl-para-anisidine (3) and ethyl 3-bromo-2-cyclohexanonecarboxylate (Sheehan, J. and Mumaw, C.E., J. Am. Chem. Soc., 1950, 72, 2127-2129) were dissolved in dimethylformamide and stirred in the presence of excess sodium bicarbonate for five days to give ethyl 3-N-benzyl-4-methoxyanilino-2-cyclohexanonecarboxylate (4) which was treated with zinc chloride in refluxing benzene to give (5) ($R^2$=6-MeO $R^3$=H).

A solution of 0.5gm compound (5) and 2-3ml of hydrazine hydrate in 30ml of ethanol was refluxed for 66 hours, cooled, and filtered to give sub-titled compound, 405mg, 80%, mp 185-187°C.

| Elemental Analyses for $C_{21}H_{23}N_3O_2$: | | | |
|---|---|---|---|
| Calculated | C 72.18; | H 6.63; | N 12.02 |
| Found | C 71.90; | H 6.68; | N 11.87. |

B. Preparation of 9-benzyl-6-methoxy-1,2,3,4-tetrahydrocarbazole-4-carboxamide.

A mixture of 0.3gm of the compound of part A above, 2-3gm of Raney nickel catalyst, and 100ml of ethanol was refluxed for 2.5 hours, cooled, and the solution was decanted and evaporated *in vacuo*. The residue was chromatographed on silica gel eluting with a gradient methylene chloride/1-3% methanol to give titled compound, 220mg, 80%, mpl54-156°C/diethyl ether.

| Elemental Analyses for $C_{21}H_{22}N_2O_2$ : | | | |
|---|---|---|---|
| Calculated | C 75.42; | H 6.63; | N 8.38 |
| Found | C 75.58; | H 6.62; | N 8.24. |

Example 2

Preparation of 4-[(9-benzyl-4-carbamoyl-1,2,3,4-tetrahydrocarbazol-6-yl)oxy]butyric acid

A solution of 280mg of compound (7) ($R^2$=6-MeO, $R^3$=H, $R^4$=phenyl) in 25ml of dichloromethane was treated with 3ml of 1M boron tribromide in dichloromethane for 2.75 hours, washed with water, washed with brine, dried over sodium sulfate, and evaporated *in vacuo* to give 290mg of crude compound (7) ($R^2$=6-OH, $R^3$=H) which was dissolved in 10ml of tetrahydrofuran and 50ml of dimethylsulfoxide and treated with 40mg of sodium hydride (60% in mineral oil) for 10 minutes and then with 0.15ml of ethyl 4-bromobutyrate for 1.75 hours. The solution was diluted with ethyl acetate and water and the organic phase washed with water, washed with brine, dried over sodium sulfate, and evaporated *in vacuo.* The residue was chromatographed on silica gel eluting with a gradient methylene chloride/0-2% methanol to give 340mg of compound (9) ($R^2$= $-O(CH_2)_4CO_2Et$, $R^3$=H) which was dissolved in 25ml of ethanol containing 2-3ml of 2N sodium hydroxide and stirred 4.25 hours, acidified with hydrochloric acid and extracted with ethyl acetate. The organic phase was washed with brine, dried over sodium sulfate, and concentrated *in vacuo* to give title compound, 300mg, 90%,mp 199-200°C.

| Elemental Analyses for $C_{24}H_{26}N_2O_4$: | | | |
|---|---|---|---|
| Calculated | C 70.92; | H 6.45; | N 6.89 |
| Found | C 70.63; | H 6.49; | N 6.87 |

Example 3

Preparation of 3-[(9-benzyl-4-carbamoyl-1,2,3,4-tetrahydrocarbazol-6-yl)oxy]propylphosphonic acid dihydrate

A. Preparation of dimethyl-3-[(9-benzyl-4-carbamoyl-1,2,3,4-tetrahydrocarbazol-6-yl)oxy]propylphosphonic acid.

A solution of 840mg of compound (7) ($R^{2(a)}$=OH $R^3$=H) (prepared as in example 2) in 20ml of tetrahydrofuran and 70ml of dimethylsulfoxide was treated with 120mg of sodium hydride (60% in mineral oil) for 10 minutes and then with 700mg of dimethyl 3-bromopropylphosphonate for 5 hours. The solution was diluted with water and ethyl acetate. The organic phase was washed with water, washed with brine, dried over sodium sulfate, and evaporated *in vacuo* to give sub-titled compound, 940mg, 75%, amorphous solid.

| Elemental Analyses for $C_{25}H_{31}N_2O_5P$: | | | |
|---|---|---|---|
| Calculated | C 63.82; | H 6.64; | N 5.95 |
| Found | C 63.94; | H 6.58; | N 6.15 |

B. A solution of 450mg of the compound of Part A and 1.5ml of trimethylsilyl bromide in 25ml of dichloromethane was stirred for 16 hours and then evaporated *in vacuo*. The residue was dissolved in 25ml of methanol, stirred for 2.5 hours, evaporated *in vacuo*, and crystallized from a mixture of ethyl acetate and methanol to give title compound, 325mg, 78%, mp 200-201°C.

| Elemental Analyses for $C_{23}H_{27}N_2O_5P \cdot 2H_2O$: | | | |
|---|---|---|---|
| Calculated | C 57.73; | H 6.53; | N 5.86 |
| Found | C 57.51; | H 5.94; | N 6.00 |

Example 4

Preparation of 2-[(9-benzyl-4-carbamoyl-1,2,3,4-tetrahydro-carbazol-6-yl)oxy]methylbenzoic acid hydrate

A. Preparation of methyl 2-[(9-benzyl-4-carbamoyl-1,2,3,4-tetrahydrocarbazol-6-yl)oxy]methylbenzoate.

A solution of 700mg of compound (7) ($R^{2(a)}$=OH, $R^3$=H, $R^4$=phenyl) (prepared as in example 2) in 20ml of tetrahydrofuran and 70ml of dimethylsulfoxide was treated with 100mg of sodium hydride (60% in mineral oil) for 10 minutes and then with 575mg of methyl 2-bromomethylbenzoate for 2.5 hours. The solution was diluted with water and ethyl acetate. The organic phase was washed with water, washed with brine, and evaporated *in vacuo*. The residue was chromatographed on silica gel eluting with a gradient methylene chloride/0-2% methanol to give sub-titled compound, 840mg, 90%, mp 119-120°C/$Et_2O$.

| Elemental Analyses for $C_{29}H_{28}N_2O_4$: | | | |
|---|---|---|---|
| Calculated | C 74.34; | H 6.02; | N 5.98 |
| Found | C 74.22; | H 6.03; | N 5.70 |

B. Preparation of 2-[(9-benzyl-4-carbamoyl-1,2,3,4-tetrahydrocarbazol-6-yl)oxy]methylbenzoic acid hydrate.

A solution of 670mg of the compound of Part A and 5ml of 2N sodium hydroxide in 100ml of ethanol and 15ml of tetrahydrofuran was stirred for 16.5 hours, acidified with hydrochloric acid, and extracted with ethyl acetate. The organic phase was washed with brine, dried over sodium sulfate, and evaporated *in vacuo*. The residue was chromatographed on silica gel eluting with a gradient methylene chloride/2-4% methanol to give title compound, 230mg, 34%, amorphous solid.

| Elemental Analyses for $C_{28}H_{26}N_2O_4 \cdot H_2O$: | | | |
|---|---|---|---|
| Calculated | C 71.17; | H 5.97; | N 5.93 |
| Found | C 71.31; | H 5.68; | N 5.65 |

Example 5

Preparation of 9-benzyl-5,7-dimethoxy-1,2,3,4-tetrahydrocarbazole-4-carboxylic acid hydrazide

A. Preparation of N-benzyl-3,5-dimethoxyaniline.

A solution of 25gm. (0.163 mol) of 3,5-dimethoxyaniline and 18.3ml. (0.18 mol) of benzaldehyde in 300ml. of methanol was cooled in ice-water and treated with 10.3gm. (0.18 mol) of sodium cyanoborohydride in portions. The solution was stirred and cooled for 3 hours, treated with 1-2gm. of sodium borohydride for 30 minutes, diluted with water and extracted with ethyl acetate. The organic phase was washed with brine, dried over sodium sulfate, and evaporated *in vacuo*. The residue was chromatographed on silica gel eluting with a gradient hexane/15-70% ether to give 9-benzyl-3,5-dimethoxyaniline, 28.0gm., 71%, as an oil.

| Elemental Analyses for $C_{15}H_{17}NO_2$: | | | |
|---|---|---|---|
| Calculated | C 74.05; | H 7.04; | N 5.76 |
| Found | C 74.30; | H 7.12; | N 5.70 |

B. Preparation of 9-benzyl-5,7-dimethoxy-1,2,3,4-tetrahydrocarbazole-4-carboxylic acid hydrazide.

A solution of 9.72gm of the compound of part A and 4.98gm of 2-carbethoxy-6-bromocyclohexanone (J.Sheehan and C.E.Mumaw, J.Am.Chem.Soc., 72, 2127-2129, (1950).) in 125ml of benzene was refluxed for 72 hours, cooled, filtered, and evaporated *in vacuo.* The residue (12gm) and 10gm of zinc chloride were refluxed in 250ml of benzene for 6 hours, cooled and evaporated *in vacuo.* The residue was taken up in ethyl acetate, washed with 1N hydrochloric acid, washed with water, dried over sodium sulfate, and evaporated in vacuo. The residue was chromatographed on silica gel eluting with a gradient toluene/0-5% ethyl acetate to give compound (5) ($R^2$=5-MeO $R^3$=7-MeO $R^4$=phenyl), 1.88gm which was dissolved in 100ml of ethanol containing 10ml of hydrazine hydrate and refluxed for 5 days, cooled, the solution decanted, diluted with ethyl acetate, washed with brine, dried over sodium sulfate, and evaporated *in vacuo* to give title compound, 1.1gm, 60%, mp 189-190°C/$CH_2Cl_2$-EtOH.

| Elemental Analyses for $C_{22}H_{25}N_3O_3$ : | | | |
|---|---|---|---|
| Calculated | C 69.64; | H 6.64; | N 11.07 |
| Found | C 69.59; | H 6.74; | N 10.84 |

Example 6

Preparation of 9-benzyl-5,7-dimethoxy-1,2,3,4-tetrahydrocarbazole-4-carboxamide

A mixture of 980mg of the compound of example 5, 2gm of Raney nickel catalyst, 1-2ml of hydrazine hydrate, and 125ml of ethanol was refluxed 1 hour, the solution decanted, diluted with ethyl acetate, washed with water, washed with brine, dried over sodium sulfate, and evaporated *in vacuo*. The residue was chromatographed on silica gel eluting with a gradient methylene chloride/1-3% methanol to give title compound, 820mg, 84%, mp 190-192°C/EtOH.

| Elemental Analyses for $C_{22}H_{24}N_2O_3$ : | | | |
|---|---|---|---|
| Calculated | C 72.51; | H 6.64; | N 7.69 |
| Found | C 71.88; | H 6.89; | N 7.81 |

Example 7

Preparation of [9-benzyl-4-carbamoyl-7-methoxy-1,2,3,4-tetrahydrocarbazol-5-yl]oxyacetic acid sodium salt

A. Preparation of 9-benzyl-5-hydroxy-7-methoxy-1,2,3,4-tetrahydrocarbazole-4-carboxamide.

A solution of 1.75gm. (4.8 mmol) of the compound of example 6 in 50ml. of dimethylformamide was mixed with a solution of sodium thioethoxide (13.5 mmol) in 75ml. of dimethylformamide and then heated at 100°C for 21 hours. The mixture was cooled, diluted with water, acidified with hydrochloric acid, and extracted with ethyl acetate. The organic phase was washed with brine, dried over sodium sulfate, and evaporated *in vacuo.* The residue was chromatographed on silica gel eluting with a gradient methylene chloride/0-4% methanol to give the sub-titled product, 825mg., 50%, mp 225-7°C/ethanol.

| Elemental Analyses for $C_{21}H_{22}N_2O_3$: | | | |
|---|---|---|---|
| Calculated | C 71.98; | H 6.33; | N 7.99 |
| Found | C 71.71; | H 6.37; | N 7.72 |

B. Preparation of [9-benzyl-4-carbamoyl-7-methoxy-1,2,3,4-tetrahydrocarbazol-5-yl]oxyacetic acidethyl ester.

A solution of 700mg. (2.0 mmol) of the product from Part A in 70ml. of dimethylformamide and 15ml. of tetrahydrofuran was treated with 100mg. of sodium hydride (60% in mineral oil; 2.5 mmol) for 10 minutes and then with 0.3ml. (2.7 mmol) of ethyl bromoacetate for 3 hours. The mixture was diluted with ethyl acetate, washed with water, washed with brine, dried over sodium sulfate, and evaporated *in vacuo*. The residue was chromatographed on silica gel eluting with a gradient methylene chloride/1-2% methanol to give sub-titled product, 670mg., 77%, mp 167-169°C/ether.

| Elemental Analyses for $C_{25}H_{28}N_2O_5$: | | | |
|---|---|---|---|
| Calculated | C 68.79; | H 6.47; | N 6.42 |
| Found | C 69.57; | H 6.39; | N 5.77 |

C. Preparation of [9-benzyl-4-carbamoyl-7-methoxy-1,2,3,4-tetrahydrocarbazol-5-yl]oxyacetic acid.

A suspension of 650mg. of the product from Part B in 20ml. of tetrahydrofuran and 70ml. of ethanol was treated with 5ml. of 2N sodium hydroxide and the resulting solution was stirred for 15.5 hours. The solution was diluted with ethyl acetate and water and acidified with. hydrochloric acid. The organic phase was washed with brine, dried over sodium sulfate, concentrated *in vacuo*, and filtered to give title product, 540mg., 87%, mp 251-254°C.

| Elemental Analyses for $C_{23}H_{24}N_2O_5$: | | | |
|---|---|---|---|
| Calculated | C 67.63; | H 5.92; | N 6.86 |
| Found | C 67.73; | H 5.74; | N 6.82 |

D. Preparation of [9-benzyl-4-carbamoyl-7-methoxy-1,2,3,4-tetrahydrocarbazol-5-yl]oxyacetic acid sodium salt.

A suspension of 120mg. of the product from Part C in 20mL of ethanol was treated with 0.15mL of 2.0 N sodium hydroxide and warmed until dissolved. The resulting solution was concentrated *in vacuo,* diluted with ethyl acetate and again concentrated *in vacuo* and left to stand overnight. The precipitate was filtered and air dried to give the title product as an amorphous solid, 80mg, 63%.

| Elemental Analyses for $C_{23}H_{23}NaN_2O_5 \cdot 0.4H_2O$: | | | |
|---|---|---|---|
| Calculated | C 63.18; | H 5.39; | N 6.40 |
| Found | C 63.31; | H 5.48; | N 6.25 |

Example 8

Preparation of 3-[(9-benzyl-4-carbamoyl-7-n-octyl-1,2,3,4-tetrahydrocarbazol-6-yl)oxy]propylphosphonic acid dihydrate

A. Preparation of ethyl 9-benzyl-6-methoxy-7-n-octyl-1,2,3,4-tetrahydrocarbazole-4-carboxylate.

A mixture of 1.3gm of 9-benzyl-4-methoxy-5-n-octylaniline (3) (prepared by the procedures of Example 9, Part A, utilizing n-heptyltriphenylphosphonium bromide), 1.0gm of 2-carbethoxy-6-bromocyclohexanone, 675mg of sodium bicarbonate, and 40ml of dimethylformamide was stirred for 5 days, diluted with ethyl acetate, washed with water, washed with brine, dried over sodium sulfate, and evaporated *in vacuo*. The residue and 10gm of zinc chloride in 250ml of benzene was refluxed for 6 hours, cooled, diluted with ethyl acetate, washed with 1N hydrochloric acid, washed with brine dried over sodium sulfate, and evaporated *in vacuo*. The residue was chromatographed on silica gel eluting with a gradient hexane/10-15% diethyl ether to give compound (5) ($R^2$=6-MeO $R^{3a}$=7-n-octyl $R^4$=phenyl), 930mg, which was dissolved in 30ml of benzene and treated with 15ml of an 0.67M solution of methyl chloroaluminum amide at 50°C for 16 hours, cooled, decomposed with ice and 1N hydrochloric acid, and extracted with ethyl acetate. The organic phase was washed with brine, dried over sodium sulfate, and evaporated *in vacuo* to give sub-titled compound, 795mg, 91%, mp 131-133°C/$Et_2O$.

| Elemental Analyses for $C_{29}H_{38}N_2O_2$: | | | |
|---|---|---|---|
| Calculated | C 77.97; | H 8.58; | N 6.27 |
| Found | C 77.75; | H 8.62; | N 5.99 |

B. Preparation of 9-benzyl-6-hydroxy-7-n-octyl-1,2,3,4-tetrahydrocarbazole-4-carboxamide.

A solution of 770mg of the compound of Part A, in 75ml of dichloromethane was treated with 10ml of 1M boron tribromide in dichloromethane for 1.75 hours and then decomposed with ice and 1N hydrochloric acid. The organic phase was washed with brine, dried over sodium sulfate, and evaporated *in vacuo.* The residue was chromatographed on silica gel eluting with a gradient methylene chloride/1-3% methanol to give sub-titled compound,

360mg, 47%, mp 223-225°C.

| Elemental Analyses for $C_{28}H_{36}N_2O_2$: | | | |
|---|---|---|---|
| Calculated | C 77.74; | H 8.39; | N 6.48 |
| Found | C 77.97; | H 8.45; | N 6.40 |

C. Preparation of 3-[(9-benzyl-4-carbamoyl-7-n-octyl-1,2,3,4-tetrahydrocarbazol-6-yl)oxy]propylphosphonic acid dihydrate.

A solution of 360mg of the compound of Part B in 10ml of tetrahydrofuran and 70ml of dimethylformamide was treated with 40mg of sodium hydride (60% in mineral oil) for 15 minutes and then with 230mg of dimethyl 3-bromopropylphosphonate for 4 hours, diluted with water, and extracted with ethyl acetate. The organic phase was washed with brine, dried over sodium sulfate, and evaporated *in vacuo.* The residue was chromatographed on silica gel eluting with a gradient methylene chloride/1-5% methanol to give compound (9) ($R^2$=6-$(MeO)_2P$=$O(CH_2)_3$, $R^3$=7-n-octyl, $R^4$=phenyl), 290mg, which was dissolved in 30ml of dichloromethane and 1ml of trimethylsilyl bromide, stirred for 20 hours, evaporated *in vacuo*, dissolved in 30ml of methanol, stirred 2.25 hours, evaporated *in vacuo*, and crystallized from a mixture of benzene-methanol-diethyl ether, to give title compound, 185mg, 67%, mp 160-162°C.

| Elemental Analyses for $C_{31}H_{43}N_2O_5P \cdot 2H_2O$: | | | |
|---|---|---|---|
| Calculated | C 63.03; | H 8.02; | N 4.74 |
| Found | C 63.18; | H 7.53; | N 4.93 |

Example 9

Preparation of 4-[(9-benzyl-4-carbamoyl-7-ethyl-1,2,3,4-tetrahydrocabazol-6-yl)oxy]butyric acid

A. Preparation of benzyl 9-benzyl-6-methoxy-7-ethyl-1,2,3,4-tetrahydrocarbazole-4-carboxylate.

A suspension of 22gm (0.13mol) of 5-nitrosalicaldehyde, 10ml (0.16mol) of methyl iodide, 28gm (0.2mol) of potassium carbonate, 75ml of dimethylsulfoxide, and 125ml of 2-butanone was refluxed for 20 hours, cooled, diluted with ethyl acetate, washed with water, washed with brine, dried over sodium sulfate, and evaporated *in vacuo*. The residue was chromatographed on silica gel eluting with a gradient hexane/10-50% ether to give (1) ($R^2$=4-MeO $R^{3(a)}$=3-CHO), 15gm, 67%.

A suspension of 19.6gm (0.055mol) of methyltriphenylphosphonium bromide in 300ml of tetrahydrofuran was cooled at -5°C and treated slowly with 35ml of n-butyl lithium (1.6M in hexane, 0.055mol). The cooling bath was removed and the mixture allowed to warm to room temperature over 45 minutes. A solution of 9.1gm (0.050mol) of of the aldehyde prepared above was slowly added to this yellow solution and stirred 2 hours, diluted with water, and extracted with ethyl acetate. The organic phase was washed with brine, dried over sodium sulfate, and evaporated *in vacuo*. The residue was chromatographed on silica gel eluting with a gradient hexane/15-50% ether to give (1) ($R^2$=4-MeO $R^{3(a)}$=3-$CH_2$=CH), 7.7gm, 86%.

This product and 3gm of 10% Pd/C in 200ml of ethanol was stirred under 1 atmosphere of hydrogen for 6 hours, filtered, and evaporated *in vacuo* to give (2) ($R^2$=4-MeO $R^{3(a)}$=3-$CH_3CH_2$). This crude product and 5ml of benzaldehyde in 150ml of methanol was stirred at 0-5°C, while adding in portions 2.5gm of sodium cyanoborohydride. After an additional 60 minutes at this temperature the mixture was diluted with water and extracted with ethyl acetate. The organic phase was washed with brine, dried over sodium sulfate, and evaporated *in vacuo.* The residue was chromatographed on silica gel eluting with a gradient hexane/5-15% ether to give 9-benzyl-4-methoxy-5-ethylaniline, 6.0gm, 60%.

A mixture of 3.3gm of 9-benzyl-4-methoxy-5-ethylaniline, 3.7gm of 2-carbethoxy-6-bromocyclohexanone, 1.3gm of sodium bicarbonate, and 100ml of dimethylformamide was stirred for 5 days, diluted with ethyl acetate, washed with water, washed with brine, dried over sodium sulfate, and evaporated *in vacuo.* The residue and 10gm of zinc chloride in 250ml of benzene was refluxed for 1.75 hours, cooled, diluted with ethyl acetate, washed with 1N hydrochloric acid, washed with brine, dried over sodium sulfate, and evaporated *in vacuo*. The residue was chromatographed on silica gel eluting with a gradient hexane/10-20% diethyl ether to give sub-titled compound, 2.6gm, 50%, mp 85-86/EtOH.

| Elemental Analyses for $C_{25}H_{29}NO_3$: | | | |
|---|---|---|---|
| Calculated | C 76.70; | H 7.47; | N 3.58 |
| Found | C 77.00; | H 7.56; | N 3.69 |

B. Preparation of 9-benzyl-6-methoxy-7-ethyl-1,2,3,4-tetrahydrocarbazole-4-carboxamide.

A solution of 2.6gm of the compound of Part A in 75ml of benzene and 30ml of 0.67M methyl chloroaluminum amide in benzene/toluene was heated at 50°C for 24 hours, cooled, decomposed with 1N hydrochloric acid, and extracted with ethyl acetate. The organic phase was washed with brine, dried over sodium sulfate, and evaporated *in vacuo* to give sub-titled compound, 2.2gm, 91%, mp 168-169°C/$CH_2Cl_2$-EtOH.

| Elemental Analyses for $C_{23}H_{26}N_2O_2$: | | | |
|---|---|---|---|
| Calculated | C 76.21; | H 7.23; | N 7.73 |
| Found | C 76.55; | H 7.74; | N 6.84 |

C. Preparation of 4-[(9-benzyl-4-carbamoyl-7-ethyl-1,2,3,4-tetrahydrocarbazol-6-yl)oxy]butyric acid.

A solution of 2.1gm of the compound of Part B in 150ml of dichloromethane and 15ml of 1M boron tribromide in dichloromethane was stirred for 2 hours, decomposed with ice and water, and the organic phase washed with brine, dried over sodium sulfate, and evaporated *in vacuo* to give compound (7) ($R^{2(a)}$=6-HO, $R^3$=7-ethyl, $R^4$=phenyl), 1.6gm, 80%, mp 255°C dec./methylene chloride-ethanol. A solution of 750mg of this material in 20ml of tetrahydrofuran and 70 ml of dimethylformamide was treated with 100mg of sodium hydride (60% in mineral oil) for 10 minutes and then with 0.33ml of ethyl 4-bromobutyrate for 4.5 hours, diluted with ethyl acetate, washed with water, washed with brine, dried over sodium sulfate, and evaporated *in vacuo*. The residue was chromatographed on silica gel eluting with a gradient methylene chloride/1-3% methanol to give compound (9) ($R^2$=6-$EtO_2CCH_2CH_2CH_2O$, $R^3$=7-ethyl), 625mg, 64%, mp 134-136/ethylene chloride-ethanol, which was dissolved in 10ml of tetrahydrofuran and 40ml of ethanol containing 3ml of 2N sodium hydroxide and stirred for 22 hours. The solution was acidified with hydrochloric acid and extracted with ethyl acetate. The organic phase was washed with brine, dried over sodium sulfate, and evaporated *in vacuo* to give title compound, 410mg, 73%, mp 208-210°C/$CH_2Cl_2$-EtOH.

| Elemental Analyses for $C_{26}H_{30}N_2O_4 \cdot 0.4H_2O$: | | | |
|---|---|---|---|
| Calculated | C 64.05; | H 7.15; | N 5.54 |
| Found | C 71.76; | H 6.90; | N 6.56 |

Example 10

Preparation of 3-[(9-benzyl-4-carbamoyl-7-ethyl-1,2,3,4-tetrahydrocarbazol-6-yl)oxy]propylphosphonic acid

A. Preparation of dimethyl 3-[(9-benzyl-4-carbamoyl-7-ethyl-1,2,3,4-tetrahydrocarbazol-6-yl)oxy]propylphosphonate.

A solution of 750mg of the compound of example 8 in 20ml of tetrahydrofuran and 75ml of dimethylformamide was treated with 100mg of sodium hydride (60% in mineral oil) for 10 minutes and then with 510mg of dimethyl 3-bromopropylphosphonate for 5.25 hours, diluted with ethyl acetate, washed with water, washed with brine, dried over sodium sulfate, and evaporated *in vacuo.* The residue was chromatographed on silica gel eluting with a gradient methylene chloride/1-5% methanol to give sub-titled, 655mg, 61%, amorphous solid.

| Elemental Analyses for $C_{27}H_{35}N_2O_5P$: | | | |
|---|---|---|---|
| Calculated | C 64.12; | H 7.15; | N 5.54 |
| Found | C 64.27; | H 7.00; | N 5.92 |

B. Preparation of 3-[(9-benzyl-4-carbamoyl-7-ethyl-1,2,3,4-tetrahydrocarbazol-6-yl)oxy]propylphosphonic acid.

A solution of the compound of Part A and 1ml of trimethylsilyl bromide in 30ml of dichloromethane was stirred

18 hours, evaporated *in vacuo,* dissolved in 25ml of methanol, stirred 3.75 hours, and concentrated *in vacuo* to give title compound, 475mg, 80%, mp 235-238°C dec.

| Elemental Analyses for $C_{25}H_{31}N_2O_5P$: | | | |
|---|---|---|---|
| Calculated | C 63.82; | H 6.64; | N 5.95 |
| Found | C 63.56; | H 6.62; | N 6.07 |

Example 11

Preparation of (S)-(+)-4-[(9-benzyl-4-carbamoyl-7-ethyl-1,2,3,4-tetrahydrocarbazol-6-yl)oxy]butyric acid

A. Preparation of (S)-(-)-1-[(S)-(+)-9-benzyl-6-methoxy-7-ethyl-1,2,3,4-tetrahydrocarbazol-4-yl]carbonyl-4-benzyl-2-oxazolidinone.

A solution of 8.9gm. of the compound of example 8, Part A, in 25ml. of tetrahydrofuran, 150ml. of ethanol, and 25ml. of 5N sodium hydroxide was stirred for 21 hours, diluted with water, acidified with 5N hydrochloric acid, and extracted with ethyl acetate. The organic phase was washed with brine, dried over sodium sulfate and evaporated *in vacuo* to give the corresponding acid, 7.6gm., 92%, which was suspended in 200ml of dichloromethane containing 0.5ml. of dimethylformamide and treated with 2.2ml. of oxalyl chloride for 30 minutes and then evaporated *in vacuo.* The residue was dissolved in 100ml. of tetrahydrofuran and added slowly to a solution prepared by adding 25 mmole of n-butyl lithium to 25 mmole of (S)-(-)-4-benzyl-2-oxazolidinone in 150ml. of tetrahydrofuran at -75°C. After stirring for 2 hours at that temperature, the solution was diluted with water and extracted with ethyl acetate. The organic phase was washed with brine, dried over sodium sulfate, and evaporated *in vacuo.* The residue was chromatographed on silica gel eluting with a gradient hexane/15-60% ether to give first the (S,S)-diastereomer, 3.3gm., 28%, followed by 1.2gm., 11%, of mixture, and then 3.3gm., 28%, of the (R,S)-diastereomer.

| Elemental Analyses for $C_{33}H_{34}N_2O_4$: | | | |
|---|---|---|---|
| Calculated | C 75.84; | H 6.56; | N 5.36 |
| Found | C 76.09; | H 6.44; | N 5.42 |

B. Preparation of (S)-(+)-9-benzyl-6-methoxy-7-ethyl-1,2,3,4-tetrahydrocarbazole-4-carboxamide.

A solution of 3.2gm. (6 mmol) of the title compound of Part A in 50ml. of tetrahydrofuran was added to a solution prepared by adding 60 mmole of n-butyl lithium to 60 mmole of benzyl alcohol in 150ml. of tetrahydrofuran at -5°C. The resulting solution was stirred 20 minutes, diluted with water and extracted with ethyl acetate. The organic phase was washed with brine, dried over sodium sulfate and evaporated *in vacuo.* The residue was chromatographed on silica gel eluting with a gradient hexane/10-15% ether to give the benzyl ester of the carbazole-4-carboxylic acid 2.6gm., 93%, which was treated with 4 equivalents of methylchloroaluminum amide in 100ml. of benzene at 50°C for 18 hours, cooled, decomposed with ice-hydrochloric acid, and extracted with ethyl acetate. The organic phase was washed with brine, dried over sodium sulfate, and evaporated in *vacuo* to give the subtitled product, 2.5gm., 100%, mp 184-186°C, rotation at 589 +39.7deg.

| Elemental Analyses for $C_{23}H_{26}N_2O_2$: | | | |
|---|---|---|---|
| Calculated | C 76.21; | H 7.23; | N 7.73 |
| Found | C 76.19; | H 7.29; | N 7.69 |

C. Preparation of (S)-(+)-9-benzyl-6-hydroxy-7-ethyl-1,2,3,4-tetrahydrocarbazole-4-carboxamide.

A solution of 2.4gm. of the above 6-methoxy derivative in 200ml. of dichloromethane was treated with 20ml. of 1M boron tribromide in dichloromethane for 2.5 hours, decomposed with ice-water, washed with brine, dried over sodium sulfate, and evaporated *in vacuo* to give the 6-hydroxytetrahydrocarbazole, 2.2gm., 100%, mp 240-242°C/EtOH-$CH_2Cl_2$.

| Elemental Analyses for $C_{22}H_{24}N_2O_2$: | | | |
|---|---|---|---|
| Calculated | C 75.84; | H 6.94; | N 8.04 |

(continued)

| Elemental Analyses for $C_{22}H_{24}N_2O_2$: | | | |
|---|---|---|---|
| Found | C 76.01; | H 7.18; | N 8.13 |

D. Preparation of (S)-(+)-ethyl 4-[(9-benzyl-4-carbamoyl-7-ethyl-1,2,3,4-tetrahydrocarbazol-6-yl)oxy]butyrate.

A solution of 2.1gm. (6 mmol) of the above 6-hydroxycarbazole in 25ml. of tetrahydrofuran and 125ml. of dimethylformamide was treated with 280mg. of sodium hydride (60% in mineral oil; 7 mmol) for 5 minutes and then with 1.0ml. (7 mmol) of ethyl 4-bromobutyrate for 1.5 hours, diluted with water and extracted with ethyl acetate. The organic phase was washed with brine, dried over sodium sulfate, and evaporated *in vacuo.* The residue was chromatographed on silica gel eluting with a gradient dichloromethane/0-5% methanol to give sub-titled product, 760mg., 29%, mp 188-1900C/MeOH, rotation at 589 +32.2deg.

| Elemental Analyses for $C_{28}H_{34}N_2O_4$: | | | |
|---|---|---|---|
| Calculated | C 72.70; | H 7.41; | N 6.06 |
| Found | C 72.94; | H 7.34; | N 6.20 |

E. Preparation of (S)-(+)-4-[(9-benzyl-4-carbamoyl-7-ethyl-1,2,3,4-tetrahydrocarbazol-6-yl)oxy]butyric acid.

A solution of 635mg. of the above ester and 250mg. of lithium hydroxide in 15ml. of water and 50ml. of tetrahydrofuran was stirred 5 hours, washed with ether, acidified with 1N hydrochloric acid, and extracted with ethyl acetate. The organic phase was washed with brine, dried over sodium sulfate, and evaporated *in vacuo* to give title product, 260mg., 44%, mp 188-190°C, rotation at 589 +22.8deg.

| Elemental Analyses for $C_{26}H_{30}N_2O_4$: | | | |
|---|---|---|---|
| Calculated | C 71.87; | H 6.96; | N 6.45 |
| Found | C 72.08; | H 7.11; | N 6.32 |

Example 12

Preparation of 4-[9-benzyl-4-carbamoyl-7-(2-cyanoethyl)-1,2,3,4-tetrahydrocarbazol-6-yl]oxybutyric acid

A. Preparation of methyl 2-methoxy-5-nitrocinnamate.

A solution of 11.0gm. (0.06 mol) of 2-methoxy-5-nitrobenzaldehyde and 23.1gm. (0.07 mol) of methyl triphenylphosphoranylidene acetate in 250ml. of tetrahydrofuran was refluxed for 1.5 hours, cooled, diluted with water and extracted with ethyl acetate. The organic phase was washed with brine, dried over sodium sulfate, and evaporated *in vacuo.* The residue was chromatographed on silica gel eluting with dichloromethane to give sub-titled product, 14.0gm., 97%, mp 156-158°C.

| Elemental Analyses for $C_{11}H_{11}NO_5$: | | | |
|---|---|---|---|
| Calculated | C 55.70; | H 4.67; | N 5.90 |
| Found | C 55.92; | H 4.64; | N 6.01 |

B. Preparation of ethyl 9-benzyl-6-methoxy-7-(3-amino-3-oxopropyl)-1,2,3,4-tetrahydrocarbazole-4-carboxylate.

A mixture of 14gm. of the above cinnamate, 5gm. of 10% Pd/C, 100ml. of tetrahydrofuran, and 150ml. of ethanol was stirred under 1 atmosphere of hydrogen for 14 hours, filtered and evaporated *in vacuo* to give methyl 3-(2-methoxy-5-aminophenyl)propionate,(2), 11.5gm., 94%, which was dissolved in 200ml. of methanol containing 7ml. of benzaldehyde and cooled to 0-5°C. After portionwise addition of 5gm. of sodium cyanoborohydride, the solution was stirred for 3 hours at that temperature, decomposed with water, and extracted with ethyl acetate. The organic phase was washed with brine, dried over sodium sulfate, and evaporated in vacuo. The residue was chromatographed on silica gel eluting with a gradient hexane/10-25% ether to give methyl 3-(9-benzyl-2-methoxy-5-aminophenyl)propionate, 11.4gm., 70%. A mixture of 4.5gm. (15 mmol) of this material, 5gm. (20 mmol) of 2-bromo-6-ethoxycarbonylcyclohexanone, 2.5gm. (30 mmol) of sodium bicarbonate, and 75ml. of dimethylformamide

was stirred for four days, diluted with water, and extracted with ethyl acetate. The organic phase was washed with brine, dried over sodium sulfate, and evaporated *in vacuo.* The residue and 15gm. of zinc chloride in 300ml. of benzene was refluxed with a water separator for 6 hours, cooled, diluted with ethyl acetate, and stirred well with 1N hydrochloric acid. The organic phase was washed with brine, dried over sodium sulfate, and evaporated *in vacuo.* The residue was chromatographed on silica gel eluting with a gradient hexane/0-25% ether to give ethyl 9-benzyl-6-methoxy-7-(2-methoxycarbonylethyl)-1,2,3,4-tetrahydrocarbazole-4-carboxylate, 3.0gm., 40%, which was dissolved in 90ml. of benzene and treated with 4 equivalents of methylchloroaluminum amide at 50°C for 3 hours, cooled, decomposed with ice-hydrochloric acid, and extracted with ethyl acetate. The organic phase was washed with brine, dried over sodium sulfate, and evaporated *in vacuo* to give ethyl 9-benzyl-6-methoxy-7-(3-amino-3-oxopropyl)-1,2,3,4-tetrahydrocarbazole-4-carboxylate, 1.97gm., 73%, mp 125-130°C.

| Elemental Analyses for $C_{24}H_{27}N_3O_3$: | | | |
|---|---|---|---|
| Calculated | C 71.87; | H 6.96; | N 6.45 |
| Found | C 71.62; | H 7.02; | N 6.51 |

C. Preparation of 9-benzyl-6-methoxy-7-(2-cyanoethyl)-1,2,3,4-tetrahydrocarbazole-4-carboxamide and 9-benzyl-6-methoxy-7-(3-amino-3-oxypropyl)-1,2,3,4-tetrahydrocarbazole-4-carboxamide.

A suspension of 2.0gm. of the above ester in 100ml. of benzene was heated at 50°C with 4 equivalents of methylchloroaluminum amide for 66 hours, cooled, decomposed with ice-hydrochloric acid, shaken with ethyl acetate, and filtered to give 9-benzyl-6-methoxy(3-amino-3-oxopropyl)-1,2,3,4-tetrahydrocarbazole-4-carboxamide, 380mg., 21%, mp 253-255°C.

| Elemental Analyses for $C_{24}H_{27}N_3O_3$: | | | |
|---|---|---|---|
| Calculated | C 71.09; | H 6.71; | N 10.36 |
| Found | C 70.85; | H 6.83; | N 10.20 |

The filtrate was extracted with ethyl acetate and the organic phase washed with brine, dried over sodium sulfate, and evaporated *in vacuo.* The residue was chromatographed on silica gel eluting with a gradient dichloromethane/2-7% methanol to give the bis amide above, 395mg., 22%, and subtitled product, 560mg., 33%, mp 177-180°C/ether.

| Elemental Analyses for $C_{24}H_{25}N_3O_2$: | | | |
|---|---|---|---|
| Calculated | C 74.39; | H 6.50; | N 10.85 |
| Found | C 72.55; | H 6.50; | N 10.09 |

D. Preparation of 9-benzyl-6-hydroxy-7-(2-cyanoethyl)-1,2,3,4-tetrahydrocarbazole-4-carboxamide.

A solution of 500mg. of the product from Part C and 10ml. of 1M boron tribromide in dichloromethane and 75ml. of dichloromethane was stirred for 45 hours, decomposed with ice-water, washed with brine, dried over sodium sulfate, and evaporated *in vacuo.* The residue was chromatographed on silica gel eluting with a gradient dichloromethane/2-5% methanol to give sub-titled product, 225mg., 47%, mp 238-240°C.

| Elemental Analyses for $C_{23}H_{23}N_3O_2$: | | | |
|---|---|---|---|
| Calculated | C 73.97; | H 6.21; | N 11.25 |
| Found | C 73.74; | H 6.29; | N 11.48 |

E. Preparation of ethyl 4-[9-benzyl-4-carboxamido-7-(2-cyanoethyl)-1,2,3,4-tetrahydrocarbazol-6-yl]oxybutyrate.

A solution of 210mg. (0.6 mmol) of the above 6-hydroxytetrahydrocarbazole in 5ml. of tetrahydrofuran and 20ml. of dimethylformamide was treated with 40mg. of sodium hydride (60% in mineral oil; 0.8 mmol) for 5 minutes and then with 0.1ml. (0.9 mmol) of ethyl 4-bromobutyrate for 2 hours, diluted with water and extracted with ethyl acetate. The organic phase was washed with brine, dried over sodium sulfate, and evaporated *in vacuo.* The residue was chromatographed on silica gel eluting with a gradient dichloromethane/ 1-3% methanol to give sub-titled product, 190mg., 70%, mp 122-124°C/ether.

| Elemental Analyses for $C_{29}H_{33}N_3O_4$: | | | |
|---|---|---|---|
| Calculated | C 71.44; | H 6.82; | N 8.62 |
| Found | C 71.51; | H 6.77; | N 8.38 |

F. Preparation of [9-benzyl-4-carboxamido-7-(2-cyanoethyl)-1,2,3,4-tetrahydrocarbazol-6-yl]oxybutyric acid.

A solution of 175mg. of the above ester and 2ml. of 2N sodium hydroxide in 20ml. of ethanol was stirred for 15 hours, diluted with 1N hydrochloric acid, and extracted in ethyl acetate. The organic phase was washed with brine, dried over sodium sulfate, and evaporated *in vacuo* to give title product, 125mg., 76%, mp 184-192°C.

| Elemental Analyses for $C_{27}H_{29}N_3O_4$: | | | |
|---|---|---|---|
| Calculated | C 70.57; | H 6.36; | N 9.14 |
| Found | C 70.78; | H 6.52; | N 9.38 |

Example 13

Preparation of 4-[9-benzyl-4-carboxamido-7-(2-phenylethyl)-1,2,3,4-tetrahydrocarbazol-6-yl]oxybutyric acid

A. Preparation of 3-(2-phenylethenyl)-4-methoxy nitrobenzene.

A suspension of 14.3gm. (33 mmol) of benzyltriphenylphosphonium bromide in 200ml. of tetrahydrofuran was cooled to -5°C and treated slowly with 21ml. of n-butyl lithium (1.6M in hexane; 34 mmol). The cooling bath was removed and the mixture stirred and allowed to warm to room temperature over 60 minutes. A solution of 5.4gm. (30 mmol) of 2-methoxy-5-nitrosalicaldehyde in 50ml. of tetrahydrofuran was added rapidly dropwise and then stirred for 2 hours, diluted with water, acidified with hydrochloric acid, and extracted with ethyl acetate. The organic phase was washed with brine, dried over sodium sulfate, and evaporated in vacuo. The residue was chromatographed on silica gel eluting with a gradient hexane/15-25% ether to give sub-titled product, 7.8gm., 93%, mp 109-111°C/ether-hexane.

| Elemental Analyses for $C_{15}H_{13}NO_3$: | | | |
|---|---|---|---|
| Calculated | C 70.58; | H 5.13; | N 5.49 |
| Found | C 70.82; | H 5.17; | N 5.60 |

B. Preparation of N-benzyl-3-(2-phenylethyl)-4-methoxyaniline.

A mixture of 7.8gm. of the product from Part A, 4gm. of 10% Pd/C, 25ml. of tetrahydrofuran, and 175ml. of ethanol was stirred under 1 atmosphere of hydrogen for 6.5 hours, filtered, and evaporated *in vacuo*. The residue was dissolved in 200ml. of methanol containing 3ml. of benzaldehyde and cooled to -5°C. Sodium cyanoborohydride (6gm.) was added in portions and the solution was stirred and cooled for 3 hours, diluted with water, and extracted with ethyl acetate. The organic phase was washed with brine, dried over sodium sulfate, and evaporated *in vacuo*. The residue was chromatographed on silica gel eluting with a gradient hexane/5-10% ether to give sub-titled product, 4.8gm., 50% (two steps), as an oil.

| Elemental Analyses for $C_{22}H_{23}NO$: | | | |
|---|---|---|---|
| Calculated | C 83.24; | H 7.30; | N 4.41 |
| Found | C 83.53; | H 7.30; | N 4.69 |

C. Preparation of N-benzyl-6-methoxy-7-(2-phenylethyl)-1,2,3,4-tetrahydrocarbazole-4-carboxamide.

A mixture of 1.6gm. (5.0 mmol) of the product from Part B, 1.5gm, (6 mmol) of ethyl 3-bromo-2-cyclohexanone carboxylate, 1.0gm. (12 mmol) of sodium bicarbonate, and 40ml. of dimethylformamide was stirred for 6 days, diluted with ethyl acetate, washed with water, washed with brine, dried over sodium sulfate, and evaporated in vacuo. The residue and 3.5gm. (30 mmol) of zinc chloride in 250ml. of benzene was refluxed with a water trap for 6 hours, cooled, diluted with ethyl acetate, and stirred well with 1N hydrochloric acid. The organic phase was washed with brine, dried over sodium sulfate, and evaporated *in vacuo.* The residue was chromatographed on

silica gel eluting with a gradient hexane/10-20% ether to give ethyl 9-benzyl-6-methoxy-7-(2-phenylethyl)-1,2,3,4-tetrahydrocarbazole-4-carboxylate as an oil, 910mg., 39%, which was dissolved in 60ml. of benzene, mixed with 10-15ml. of an 0.67M solution of methylchloroaluminum amide in 2:1 benzene:toluene and heated at 50°C for 16 hours The mixture was cooled, decomposed with ice-water, and extracted with 3:1 dichloromethane:isopropanol. The organic phase was washed with brine, dried over sodium sulfate, and evaporated *in vacuo*. The residue was chromatographed on silica gel eluting with a gradient dichloromethane/1-2% methanol to give sub-titled product, 630mg., 76%, mp 165-166°C/ethanol.

| Elemental Analyses for $C_{29}H_{30}N_2O_2$: | | | |
|---|---|---|---|
| Calculated | C 79.42; | H 6.90; | N 6.39 |
| Found | C 79.64; | H 6.90; | N 6.32 |

D. Preparation of 9-benzyl-6-hydroxy-7-(2-phenylethyl)-1,2,3,4-tetrahydrocarbazole-4-carboxamide.

A solution of 610mg. of the product from Part C in 100ml. of dichloromethane was treated with 5ml. of 1M boron tribromide in dichloromethane for 4hr., decomposed with ice-water, and extracted with 3:1 dichloromethane: isopropanol. The organic phase was washed with brine, dried over sodium sulfate, and evaporated *in vacuo* to give sub-titled product, 580mg., 100%, mp 209-211°C/ethanol.

| Elemental Analyses for $C_{28}H_{28}N_2O_2$: | | | |
|---|---|---|---|
| Calculated | C 78.84; | H 7.09; | N 6.57 |
| Found | C 78.54; | H 6.79; | N 6.33 |

E. Preparation of ethyl 4-[9-benzyl-4-carboxamido-7-(2-phenylethyl)-1,2,3,4-tetrahydrocarbazol-6-yl]oxybutyric acid.

A solution of 560mg. (1.3 mmol) of the product of Part D in 50ml. of dimethylformamide and 10ml. of tetrahydrofuran was stirred with 60mg. of sodium hydride (60% in mineral oil; 1.5 mmol) for 5 minutes and then with 0.2ml. (1.4 mmol) of ethyl 4-bromobutyrate for 3 hours, diluted with ethyl acetate, washed with water, washed with brine, dried over sodium sulfate, and evaporated *in vacuo.* The residue was chromatographed on silica gel eluting with a gradient dichloromethane/1-2% methanol to give sub-titled product, 535mg., 75%, mp 144-146°C/ethanol.

| Elemental Analyses for $C_{34}H_{38}N_2O_4$: | | | |
|---|---|---|---|
| Calculated | C 75.81; | H 7.11; | N 5.20 |
| Found | C 75.71; | H 7.10; | N 5.12 |

F. Preparation of 4-[9-benzyl-4-carboxamido-7-(2-phenylethyl)-1,2,3,4-tetrahydrocarbazol-6-yl]oxybutyric acid.

A solution of 520mg. of the product from Part E and 4ml. of 2N sodium hydroxide in 25ml. of tetrahydrofuran and 25ml. of ethanol was stirred for 16 hours, diluted with ethyl acetate, acidified with hydrochloric acid, washed with brine, dried over sodium sulfate, and concentrated *in vacuo*, and filtered to give title product, 230mg., 44%, mp 202-205°C.

| Elemental Analyses for $C_{32}H_{34}N_2O_4$: | | | |
|---|---|---|---|
| Calculated | C 75.27; | H 6.71; | N 5.49 |
| Found | C 75.04; | H 6.69; | N 5.41 |

Example 14

Preparation of 4-[9-benzyl-4-carboxamidocarbazol-6-yl]oxybutyric acid

A. Preparation of 9-benzyl-6-methoxycarbazole-4-carboxamide.

A mixture of 2.5gm. of of the tetrahydrocarbazole from Example 1, Part A, and 5gm. of 5% Pd/C in 130ml. of carbitol was refluxed for 7 hours, cooled, and evaporated *in vacuo*. The residue was crystallized from dichloromethane-ethanol to give sub-titled product, 1.1gm., 44%, mp 226-226°C.

| Elemental Analyses for $C_{21}H_{18}N_2O_2$: | | | |
|---|---|---|---|
| Calculated | C 76.34; | H 5.49; | N 8.48 |
| Found | C 76.61; | H 5.75; | N 8.41 |

B. Preparation of 9-benzyl-6-hydroxycarbazole-4-carboxamide.

A suspension of 1.0gm. of the 6-methoxycarbazole from Part A, above, and 10ml. of 1M boron tribromide in 250ml. of dichloromethane was stirred for 2 hours, decomposed with ice-water, and extracted with 3:1 dichloromethane:isopropanol. The organic phase was washed with brine, dried over sodium sulfate, and evaporated *in vacuo* to give sub-titled product, 1.0gm., 100%, mp 246-248°C.

| Elemental Analyses for $C_{20}H_{16}N_2O_2{\cdot}0.3H_2O$: | | | |
|---|---|---|---|
| Calculated | C 74.66; | H 5.20; | N 8.71 |
| Found | C 74.46; | H 5.19; | N 8.45 |

C. Preparation of 4-[9-benzyl-4-carboxamidocarbazol-6-yl]oxybutyric acid.

A solution of 700mg.(2.2mmol) of the above 6-hydroxycarbazole in 25ml. of tetrahydrofuran and 75ml. of dimethylformamide was treated with 100mg. of sodium hydride (60% in mineral oil, 2.5mmol) for 5 minutes and then 0.35ml. (2.5mmol) of ethyl 4-bromobutyrate for 3 hours, diluted with water, and extracted with ethyl acetate. The organic phase was washed with brine, dried over sodium sulfate, and evaporated in *vacuo.* The residue was chromatographed on silica gel eluting with a gradient dichloromethane/0-3% methanol to give the O-alkylated derivative, 700mg., 74%, mp 150-152°C, which was dissolved in 75ml. of ethanol and 5ml. of 2N sodium hydroxide, stirred 16 hours, acidified with 1N hydrochloric acid, and extracted with ethyl acetate. The organic phase was washed with brine, dried over sodium sulfate, and evaporated *in vacuo* to give title product, 475mg., 72%, mp 252-254°C.

| Elemental Analyses for $C_{24}H_{22}N_2O_4$: | | | |
|---|---|---|---|
| Calculated | C 71.63; | H 5.51; | N 6.96 |
| Found | C 71.39; | H 5.66; | N 6.72 |

Example 15

Preparation of [9-benzyl-4-carbamoyl-7-methoxycarbazol-5-yl]oxyacetic acid.

A mixture of 430mg. of the product from Example 7 Part D, 2.0gm. of 5% Pd/C, and 20mL of carbitol was heated to reflux and refluxed for 21 hours, cooled, and filtered. The filtrate was diluted with water, acidified with hydrochloric acid, and extracted well with ethyl acetate. The organic phase was washed with brine, dried over sodium sulfate and evaporated *in vacuo.* The residue was triturated with dichloromethane and filtered to remove solid tetrahydrocarbazole. The filtrate was evaporated *in vacuo* to give the title product, 125mg, 31%.

| Elemental Analyses for $C_{23}H_{20}N_2O_5{\cdot}0.4H_2O$: | | | |
|---|---|---|---|
| Calculated | C 67.11; | H 5.09; | N 6.81 |
| Found | C 67.25; | H 5.19; | N 6.75 |

Example 16

Preparation of methyl [9-benzyl-4-carbamoyl-7-methoxycarbazol-5-yl]oxyacetic acid

A. Preparation of 9-benzyl-4-carbamoyl-5,7-dimethoxycarbazole

A mixture of 2.0gm. of the product from Example 6, 2gm. of 5% Pd/C, and 100mL of carbitol was refluxed for 17 hours, filtered while still hot, and the solid washed well with ethyl acetate. The combined filtrates were washed with water, washed with brine, dried over sodium sulfate, and evaporated in vacuo to give subtitled product, 1.4gm.,

70%, mp 240-243°C.

| Elemental Analyses for $C_{22}H_{20}N_2O_3$: | | | |
|---|---|---|---|
| Calculated | C 73.32; | H 5.59; | N 7.77 |
| Found | C 74.26; | H 5.73; | N 8.04 |

B. Preparation of 9-benzyl-4-carbamoyl-5-hydroxy-7-methoxycarbazole.

A solution of 1.2gm. (3.3 mmol) of the product from Part A and 10 mmol of sodium ethanethiolate in 100mL of dimethylformamide was heated at 100°C for 42 hours, cooled, diluted with water, and the pH adjusted to 5-6 with hydrochloric acid. The mixture was extracted with ethyl acetate, the organic phase was washed with water, washed with brine, dried over sodium sulfate, and evaporated in vacuo. The residue was chromatographed on silica gel eluting with ethyl acetate to give product, 550mg., 48%, mp 234-236°C dec.

| Elemental Analyses for $C_{21}H_{18}N_2O_3$: | | | |
|---|---|---|---|
| Calculated | C 72.82; | H 5.24; | N 8.09 |
| Found | C 72.54; | H 5.19; | N 8.04 |

C. Preparation of methyl [9-benzyl-4-carbamoyl-7-methoxycarbazol-5-yl]oxyacetic acid.

A solution of 430mg. (1.2 mmol) of the product from Part B in 40mL of dimethylformamide and a few mLs of tetrahydrofuran was treated with 60mg. of sodium hydride (60% in mineral oil; 1.5 mmol) for 15 minutes and then with 0.13mL (1.4 mmol) of methylbromoacetate for 16 hours, diluted with ethyl acetate, washed with water, washed with brine, dried over sodium sulfate, and evaporated in vacuo. The residue was chromatographed on silica gel eluting with a gradient dichloromethane/ 1-3% methanol to give title compound, 320mg., 62%, mp 170-172°C.

| Elemental Analyses for $C_{24}H_{22}N_2O_5$: | | | |
|---|---|---|---|
| Calculated | C 68.89; | H 5.30; | N 6.69 |
| Found | C 68.64; | H 5.41; | N 6.57 |

D. Preparation of [9-benzyl-4-carbamoyl-7-methoxycarbazol-5-yl]oxyacetic acid sodium salt

To a suspension of 60mg (0.15mmol) of the product from part C in 30mL of ethanol was added 0.075mL of 2.0 N sodium hydroxide. The mixture was heated until solution, cooled, concentrated *in vacuo*, diluted with ethyl acetate, concentrated *in vacuo*, cooled, and filtered to give product, amorphous sold, 50mg., 80%.

MS (FAB+) 427.2 : MS (ion spray) +Q1 405.5, -Q1 403.5

Example 17

Preparation of 9-benzyl-7-methoxy-5-cyanomethyloxy-1,2,3,4-tetrahydrocarbazole-4-carboxamide.

A solution of 1.47gram (4.19 mmol) of the product from Example 7, Part A in 146ml. of dimethylformamide and 31ml. tetrahydrofuran was treated with 210mg. of sodium hydride (60% in mineral oil; 5.24mmol) for 10 minutes and then with 0.39ml. (0.66 mmol) of bromoacetonitrile for 3.5 hours. The mixture was diluted with ethyl acetate, washed with water, washed with brine, dried over sodium sulfate, and evaporated *in vacuo*. The residue was chromatographed on silica gel eluting with a gradient of 0 to 4% methanol in methylene chloride to give the titled product, 1.34gram, 82%.

| Elemental analysis for $C_{23}H_{23}N_3O_3$: | | | |
|---|---|---|---|
| Calculated | C 70.93; | H 5.95; | N 10.79 |
| Theory | C 70.67; | H 6.06; | N 10.83 |

Example 18

Preparation of 9-benzyl-7-methoxy-5-(1H-tetrazol-5-yl-methyl)oxy)-1,2,3,4-tetrahydrocarbazole-4-carboxamide.

A portion of the compound of Example 17, 0.45gram (1.16mmol) was heated with 5ml. tri-n-butyl in hydride at 95°C for 1 hour. The reaction was then added to a mixture of 125 ml. acetonitrile, 25ml. tetrahydrofuran, and 50ml.

acetic acid and stirred for 2 hours. The mixture was extracted 4 times with hexane and the residue evaporated *in vacuo*. Crystallization from acetone and hexane afforded the titled compound, 0.30gram, 60%.

| Elemental analysis for $C_{23}H_{24}N_6O_3$: | | | |
|---|---|---|---|
| Calculated | C 63.88; | H 5.59; | N 19.43 |
| Theory | C 64.06; | H 5.64; | N 19.28 |

Example 19

Preparation of 2-[(2,9 bis-benzyl-4-carbamoyl-1,2,3,4-tetrahydro-beta-carbol-5-yl)oxy]acetic acid

A. Preparation of 4-(tert-butyldimethylsilyl)oxyindole.

Imidazole (15.3 g, 225 mmol) was added to a solution of 4-hydroxyindole (20 g, 150 mmol) in 300 mL of anhydrous CH2Cl2 at ambient temperature. The resulting mixture was treated with tert-butyldimethylsilyl chloride (25 g, 165 mmol). After stirring overnight at ambient temperature, the reaction mixture was poured into 300 mL of water. The layers were separated, and the aqueous phase was extracted with CH2Cl2 (2 X 100 mL). The combined organic layers were dried over sodium sulfate, filtered and concentrated in vacuo to a black oil. The crude residue was purified on the Prep 500 (silica gel; 0% to 5% ethyl acetate/hexanes) to give the title compound as a light purple waxy solid in quantitative yield.

MS (ion spray, $NH_4OAc$) m/e $[M+1]^+$ 248, $[M-1]^-$ 246.

| Elemental Analysis for $C_{14}H_{21}NOSi$: | | | |
|---|---|---|---|
| Calculated | C 67.96; | H 8.55; | N 5.66 |
| Found | C 69.10; | H 8.79; | N 5.70 |

B. Preparation of Ethyl [4-(tert-butyldimethylsilyl)oxyindole]-3-acetic acid

A solution of indole (78) (247 mg, 1.00 mmol) in dry tetrahydrofuran (2 mL) under a nitrogen atmosphere was cooled to -10°C then n-butyllithium (0.625 mL, 1.00 mmol), 1.6 M in hexanes, was added dropwise over 30 sec by syringe. The resultant solution was stirred 15 minutes the zinc chloride (1.0 mL, 1.0 mmol), 1 M in ether, was added all at once. The solution was stirred 2 hours while warming to ambient temperature. To this solution was added ethyl iodoacetate (0.118 mL, 1.00 mmol) all at once. The reaction mixture darkened but remained clear. The mixture was stirred 3 hours at ambient temperature the concentrated in vacuo. The residue was purified directly on silica gel (30 X 35 mm column) eluting with methylene chloride. Concentration of appropriate fractions yielded 192 mg (57.8%) of the titled product as a white solid.

MS (ion spray, $NH_4OAc$) m/e $[M+1]^+$ 334, $[M-1]^-$ 332.

| Elemental Analyses for $C_{18}H_{27}NO_3Si$: | | | |
|---|---|---|---|
| Calculated | C 64.86; | H 8.11; | N 4.20 |
| Found | C 65.11; | H 8.02; | N 4.24 |

C. Preparation of Ethyl [2,9-bis-benzyl-5-(tert-butyldimethylsilyl)oxy-1,2,3,4-tetrahydro-beta-carboline]-4-acetic acid

A solution of the ester (79) (5.08 g, 15.2 mmol) in dry tetrahydrofuran (100 mL) was cooled to -78°C then treated dropwise with 0.5 M potassium bis(trimethylsilyl)amide in toluene (32 mL, 16 mmol). The resultant solution was stirred 10 min then benzyl iodide (3.32g, 15.2 mmol) was added all at once. The cooling bath was removed, the mixture warmed quickly to 0°C, then slowly to ambient temperature. After stirring 75 minutes at ambient temperature the mixture was concentrated in vacuo. The residue was taken up in ether and washed successively with 10% aqueous citric acid, water and saturated sodium bicarbonate solution. The ethereal solution was dried over magnesium sulfate and concentrated in vacuo. The residue was purified on silica gel (70 X 130 mm column) eluting with 500 mL 1:1 methylene chloride/hexanes then 500 mL methylene chloride. The appropriate fractions were combined and concentrated in vacuo to yield 5.90 g (91%) of ethyl [1-benzyl-4-(tert-butyldimethylsilyl)oxyindole]-3-acetic acid as a brown oil.

Benzyl amine (2.14 g, 20.0 mmol) and paraformaldehyde (1.80 g, 120 mmol) were combined and warmed to

reflux in anhydrous methanol (10 mL) for 2 hours. The mixture was concentrated in vacuo and dried under vacuum for 30 minutes to yield crude benzyl bis(methoxymethyl)amine as a water white oil. This material was used immediately without purification.

To a cooled solution of ethyl [1-benzyl-4-(tert-butyldimethylsilyl)oxyindole]-3-acetic acid (190 mg, 0.45 mmol) in dry tetrahydrofuran (2 mL) was added potassium bis(trimethylsilyl)amide (0.98 mL, 0.49 mmol), 0.5 M in toluene, dropwise by syringe. After stirring the mixture 10 minutes, trimethylsilylchloride (0.057 mL, 0.45 mmol) was added all at once. The mixture was allowed to warm to ambient temperature then concentrated in vacuo. The residue was dried 30 minutes under vacuum to yield the trimethylsilylketene acetal (81). The residual ketene acetal (81) was immediately dissolved in methylene chloride (30 mL) to which was added freshly prepared benzyl bis(methoxymethyl)amine (175 mg, 0.90 mmol). The mixture was cooled to -78°C and treated with 1 M zinc chloride in ether(0.9 mL, 0.9 mmol). The mixture was allowed to warm to ambient temperature and stirred for an additional 45 minutes. The mixture was washed with saturated sodium bicarbonate solution then passed through a silica gel plug eluting with 1:4 ethyl acetate/hexane. The desired fractions were combined and concentrated in vacuo then further purified on an SCX cartridge (lg, Varian) with methanol and ammonia. Desired fractions were combined, concentrated and finally purified on silica gel eluting with methylene chloride to yield 34 mg (14%) of the titled tricyclic indole.

MS (ion spray, $NH_4OAc$) m/e $[M+1]^+$ 555.

| Elemental Analyses for $C_{34}H_{42}N_2O_3Si$: | | | |
|---|---|---|---|
| Calculated | C 73.64; | H 7.58; | N 5.05 |
| Found | C 73.42; | H 7.61; | N 5.15 |

D. Preparation of ethyl 2-[(2,9-bis-benzyl-4-carbamoyl-1,2,3,4-tetrahydro-beta-carbol-5-yl)oxy]acetic acid

A solution of 565 mg (1.02 mmol) of the compound of Part C in 10 mL 1:1 methanol/tetrahydrofuran was treated with 5 mL (5 mmol) 1 N lithium hydroxide under an atmosphere of nitrogen. The mixture was warmed briefly, allowed to stir at ambient temperature for 2 hours then concentrated in vacuo to about 5 mL. The pH of the solution was adjusted to ~5 to 6 with 1 N hydrochloric acid. The resultant precipitate was collected and dried to yield 430 mg (102%) of hydroxy acid.

This product was suspended with hydroxybenzotriazole (160 mg, 1.19mmol) and 1-(3-dimethylaminopropyl)-3-ethylcarbodiimide hydrochloride (940 mg, 2.30 mmol) in 30 mL of 1:1 tetrahydrofuran/methylene chloride. The mixture was stirred vigorously for 10 minutes, saturated with ammonia gas, stirred vigorously for 1 hour, then concentrated in vacuo. The residue was partitioned between ethyl acetate and saturated sodium bicarbonate solution. The ethyl acetate solution was dried over anhydrous magnesium sulfate, filtered and concentrated in vacuo. The residue was passed through a plug of silica gel with ethyl acetate. The eluant was evaporated to yield 175 mg (43%) of the carboxamide.

This compound was dissolved in 3 mL dry tetrahydrofuran, cooled to -70°C and treated with 0.5 M potassium bis(trimethylsilyl)amide in toluene (0.85 mL, 0.425 mmol). The solution was stirred 10 min then ethyl bromoacetate was added all at once. The reaction was stirred 6 hours while warming to ambient temperature. The mixture was concentrated in vacuo and the residue purified on silica gel eluting with ethyl acetate to yield 86 mg (41%) of the title compound.

MS (ion spray, $NH_4OAc$) m/e $[M+1]^+$ 498.

| Elemental Analyses for $C_{30}H_{31}N_3O_4$: | | | |
|---|---|---|---|
| Calculated | C 72.43; | H 6.24; | N 8.45 |
| Found | C 72.54; | H 6.36; | N 8.64 |

E. Preparation of 2-[(2,9-bis-benzyl-4-carbamoyl-1,2,3,4-tetrahydro-beta-carbol-5-yl)oxy]acetic acid

A solution of the compound from Part D (78 mg, 0.16 mmol) in 2 mL 1:1 tetrahydrofuran/methanol was stirred with 1 M lithium hydroxide (0.63 mL, 0.63 mmol) for 3 hours. The mixture was concentrated in vacuo to give a white solid. The solid was suspended in 2 mL water and the pH adjusted to ~ 5 to 6 with 1 N hydrochloric acid forming a somewhat different white solid. The new solid was collected by filtration and dried under vacuum to yield 68 mg (93%) of the title compound.

MS (ion spray, $NH_4OAc$) m/e $[M+1]^+$ 470.

| Elemental Analyses for $C_{28}H_{27}N_3O_4 \cdot 0.8\ H_2O$: | | | |
|---|---|---|---|
| Calculated | C 69.49; | H 5.96; | N 8.68 |
| Found | C 69.50; | H 5.64; | N 8.54 |

Therapeutic Use of Tricvclic Compounds

The compounds described herein are believed to achieve their beneficial therapeutic action principally by direct inhibition of human $sPLA_2$, and not by acting as antagonists for arachidonic acid, nor other active agents below arachidonic acid in the arachidonic acid cascade, such as 5-lipoxygenases, cyclooxygenases, etc.

The method of the invention for inhibiting $sPLA_2$ mediated release of fatty acids comprises contacting $sPLA_2$ with an therapeutically effective amount of the compound of Formula (III) or its salt.

The compounds of the invention may be used in a method of treating a mammal (e.g., a human) to alleviate the pathological effects of septic shock, adult respiratory distress syndrome, pancreatitus, trauma, bronchial asthma, allergic rhinitis, and rheumatoid arthritis; wherein the method comprises administering to the mammal a compound of formula (III) in a therapeutically effective amount. A "therapeutically effective" amount is an amount sufficient to inhibit $sPLA_2$ mediated release of fatty acid and to thereby inhibit or prevent the arachidonic acid cascade and its deleterious products. The therapeutic amount of compound of the invention needed to inhibit $sPLA_2$ may be readily determined by taking a sample of body fluid and assaying it for $sPLA_2$ content by conventional methods.

Pharmaceutical Formulations of the Invention

As previously noted the compounds of this invention are useful for inhibiting $sPLA_2$ mediated release of fatty acids such as arachidonic acid. By the term, "inhibiting" is meant the prevention or therapeutically significant reduction in release of $sPLA_2$ initiated fatty acids by the compounds of the invention. By "pharmaceutically acceptable" it is meant the carrier, diluent or excipient must be compatible with the other ingredients of the formulation and not deleterious to the recipient thereof.

In general, the compounds of the invention are most desirably administered at a dose that will generally afford effective results without causing any serious side effects and can be administered either as a single unit dose, or if desired, the dosage may be divided into convenient subunits administered at suitable times throughout the day.

The specific dose of a compound administered according to this invention to obtain therapeutic or prophylactic effects will, of course, be determined by the particular circumstances surrounding the case, including, for example, the route of administration, the age, weight and response of the individual patient, the condition being treated and the severity of the patient's symptoms. Typical daily doses will contain a non-toxic dosage level of from about 0.01 mg/kg to about 50 mg/kg of body weight of an active compound of this invention.

Preferably the pharmaceutical formulation is in unit dosage form. The unit dosage form can be a capsule or tablet itself, or the appropriate number of any of these. The quantity of active ingredient in a unit dose of composition may be varied or adjusted from about 0.1 to about 1000 milligrams or more according to the particular treatment involved. It may be appreciated that it may be necessary to make routine variations to the dosage depending on the age and condition of the patient. The dosage will also depend on the route of administration.

A "chronic" condition means a deteriorating condition of slow progress and long continuance. As such, it is treated when it is diagnosed and continued throughout the course of the disease. An "acute" condition is an exacerbation of short course followed by a period of remission. In an acute event, compound is administered at the onset of symptoms and discontinued when the symptoms disappear.

Pancreatitis, trauma-induced shock, bronchial asthma, allergic rhinitis and rheumatoid arthritis may occur as an acute event or a chronic event. Thus, the treatment of these conditions contemplates both acute and chronic forms. Septic shock and adult respiratory distress, on the other hand, are acute conditions treated when diagnosed.

The compound can be administered by a variety of routes including oral, aerosol, rectal, transdermal, subcutaneous, intravenous, intramuscular, and intranasal.

Pharmaceutical formulations of the invention are prepared by combining (e.g., mixing) a therapeutically effective amount of the compounds of the invention together with a pharmaceutically acceptable carrier or diluent therefor. The present pharmaceutical formulations are prepared by known procedures using well known and readily available ingredients.

In making the compositions of the present invention, the active ingredient will usually be admixed with a carrier, or diluted by a carrier, or enclosed within a carrier which may be in the form of a capsule, sachet, paper or other container. When the carrier serves as a diluent, it may be a solid, semi-solid or liquid material which acts as a vehicle,

or can be in the form of tablets, pills, powders, lozenges, elixirs, suspensions, emulsions, solutions, syrups, aerosols (as a solid or in a liquid medium), or ointment, containing, for example, up to 10% by weight of the active compound. The compounds of the present invention are preferably formulated prior to administration.

For the pharmaceutical formulations any suitable carrier known in the art can be used. In such a formulation, the carrier may be a solid, liquid, or mixture of a solid and a liquid. Solid form formulations include powders, tablets and capsules. A solid carrier can be one or more substances which may also act as flavoring agents, lubricants, solubilisers, suspending agents, binders, tablet disintegrating agents and encapsulating material.

Tablets for oral administration may contain suitable excipients such as calcium carbonate, sodium carbonate, lactose, calcium phosphate, together with disintegrating agents, such as maize, starch, or alginic acid, and/or binding agents, for example, gelatin or acacia, and lubricating agents such as magnesium stearate, stearic acid, or talc.

In powders the carrier is a finely divided solid which is in admixture with the finely divided active ingredient. In tablets the active ingredient is mixed with a carrier having the necessary binding properties in suitable proportions and compacted in the shape and size desired. The powders and tablets preferably contain from about 1 to about 99 weight percent of the active ingredient which is the novel compound of this invention. Suitable solid carriers are magnesium carbonate, magnesium stearate, talc, sugar lactose, pectin, dextrin, starch, gelatin, tragacanth, methyl cellulose, sodium carboxymethyl cellulose, low melting waxes, and cocoa butter.

Sterile liquid form formulations include suspensions, emulsions, syrups and elixirs.

The active ingredient can be dissolved or suspended in a pharmaceutically acceptable carrier, such as sterile water, sterile organic solvent or a mixture of both. The active ingredient can often be dissolved in a suitable organic solvent, for instance aqueous propylene glycol. Other compositions can be made by dispersing the finely divided active ingredient in aqueous starch or sodium carboxymethyl cellulose solution or in a suitable oil.

The following pharmaceutical formulations 1 through 8 are illustrative only and are not intended to limit the scope of the invention in any way. "Active ingredient", refers to a compound according to Formula (III) or a pharmaceutically acceptable salt, solvate, or prodrug thereof.

Formulation 1

Hard gelatin capsules are prepared using the following ingredients:

| | Quantity (mg/capsule) |
|---|---|
| 5-hydroxy-7-(5-cyanopentyl)-9-methyl-1,2,3,4-tetrahydrocarbazole-4-carboxamide | 250 |
| Starch, dried | 200 |
| Magnesium stearate | 10 |
| Total | 460 mg |

Formulation 2

A tablet is prepared using the ingredients below:

| | Quantity (mg/tablet) |
|---|---|
| 6-(3-(4-carboxyphenyl)prop-1-yl)oxy-8-heptyl-9-(5-ethylphenyl)phenyl)methylcarbazole-4-carboxamide | 250 |
| Cellulose, microcrystalline | 400 |
| Silicon dioxide, fumed | 10 |
| Stearic acid | 5 |
| Total | 665 mg |

Formulation 3

An aerosol solution is prepared containing the following components:

| | Weight |
|---|---|
| 5-(di-t-butoxyphosphonyl)methoxy-7-decyl-9-(5-propylthiopheny)methyl-1,2,3,4-tetrahydrocarbazole-4-carboxylic acid hydrazide | 0.25 |

(continued)

| | Weight |
|---|---|
| Ethanol | 25.75 |
| Propellant 22 (Chlorodifluoromethane) | 74.00 |
| Total | 100.00 |

The active compound is mixed with ethanol and the mixture added to a portion of the propellant 22, cooled to -30°C and transferred to a filling device. The required amount is then fed to a stainless steel container and diluted with the remainder of the propellant. The valve units are then fitted to the container.

Formulation 4

Tablets, each containing 60 mg of active ingredient, are made as follows:

| | |
|---|---|
| 5-(di-t-butoxyphosphonyl)methoxy-7-decyl-9-(5-propylthiopheny)methyl-1,2,3,4-tetrahydrocarbazole-4-carboxylic acid hydrazide | 60 mg |
| Starch | 45 mg |
| Microcrystalline cellulose | 35 mg |
| Polyvinylpyrrolidone (as 10% solution in water) | 4 mg |
| Sodium carboxymethyl starch | 4.5 mg |
| Magnesium stearate | 0.5 mg |
| Talc | 1 mg |
| Total | 150 mg |

The active ingredient, starch and cellulose are passed through a No. 45 mesh U.S. sieve and mixed thoroughly. The aqueous solution containing polyvinylpyrrolidone is mixed with the resultant powder, and the mixture then is passed through a No. 14 mesh U.S. sieve. The granules so produced are dried at 50°C and passed through a No. 18 mesh U.S. sieve. The sodium carboxymethyl starch, magnesium stearate and talc, previously passed through a No. 60 mesh U.S. sieve, are then added to the granules which, after mixing, are compressed on a tablet machine to yield tablets each weighing 150 mg.

Formulation 5

Capsules, each containing 80 mg of active ingredient, are made as follows:

| | |
|---|---|
| 5-ethoxycarbonylmethoxy-8-(5-carbamoylpent-1-yl)-9-(3,5-dipropylphenyl)methyl-1,2,3,4-tetrahydrocarbazole-4-carboxamide | 80 mg |
| Starch | 59 mg |
| Microcrystalline cellulose | 59 mg |
| Magnesium stearate | 2 mg |
| Total | 200 mg |

The active ingredient, cellulose, starch, and magnesium stearate are blended, passed through a No. 45 mesh U.S. sieve, and filled into hard gelatin capsules in 200 mg quantities.

Formulation 6

Suppositories, each containing 225 mg of active ingredient, are made as follows:

| | |
|---|---|
| 6-hydroxy-8-(4-(N,N-diethylamino)but-1-yl)-9-(5-fluorophenyl)methyl-4-carboxamide | 225 mg |
| Saturated fatty acid glycerides | 2,000 mg |
| Total | 2,225 mg |

The active ingredient is passed through a No. 60 mesh U.S. sieve and suspended in the saturated fatty acid

glycerides previously melted using the minimum heat necessary. The mixture is then poured into a suppository mold of nominal 2 g capacity and allowed to cool.

Formulation 7

Suspensions, each containing 50 mg of active ingredient per 5 ml dose, are made as follows:

| | |
|---|---|
| 5-(propoxycarbonyl)methoxy-9-cyclopentylmethyl-1,2,3,4-tetrahydrocarbazole-4-carboxamide | 50 mg |
| Sodium carboxymethyl cellulose | 50 mg |
| Syrup | 1.25 ml |
| Benzoic acid solution | 0.10 ml |
| Flavor | q.v. |
| Color | q.v. |
| Purified water to total | 5 ml |

The active ingredient is passed through a No. 45 mesh U.S. sieve and mixed with the sodium carboxymethyl cellulose and syrup to form a smooth paste. The benzoic acid solution, flavor and color are diluted with a portion of the water and added, with stirring. Sufficient water is then added to produce the required volume.

Formulation 8

An intravenous formulation may be prepared as follows:

| | |
|---|---|
| 5-(di-t-butoxyphosphonyl)methoxy-7-decyl-9-(5-propylthiopheny)methyl-1,2,3,4-tetrahydrocarbazole-4-carboxylic acid hydrazide | 100 mg |
| Isotonic saline | 1,000 ml |

The solution of the above ingredients generally is administered intravenously to a subject at a rate of 1 ml per minute.

Assay Experiments

Assay Example 1

The following chromogenic assay procedure was used to identify and evaluate inhibitors of recombinant human secreted phospholipase $A_2$. The assay described herein has been adapted for high volume screening using 96 well microtiter plates. A general description of this assay method is found in the article, "Analysis of Human Synovial Fluid Phospholipase $A_2$ on Short Chain Phosphatidylcholine-Mixed Micelles: Development of a Spectrophotometric Assay Suitable for a Microtiterplate Reader", by Laure J. Reynolds, Lori L. Hughes, and Edward A Dennis, Analytical Biochemistry, 204, pp. 190-197, 1992 (the disclosure of which is incorporated herein by reference):
Reagents:

REACTION BUFFER -

$CaCl_2.2H_2O$ (1.47 g/L)
KCl (7.455 g/L)
Bovine Serum Albumin (fatty acid free) (1 g/L) (Sigma A-7030, product of Sigma Chemical Co. St. Louis MO, USA)
TRIS HCl (3.94 g/L)
pH 7.5 (adjust with NaOH)

ENZYME BUFFER -

0.05 $NaOAc.3H_2O$, pH 4.5
0.2 NaCl
Adjust pH to 4.5 with acetic acid

DTNB - 5,5'-dithiobis-2-nitrobenzoic acid
RACEMIC DIHEPTANOYL THIO - PC

racemic 1,2-bis(heptanoylthio)-1,2-dideoxy-*sn*-glycero-3-phosphorylcholine
TRITON X-100™ prepare at 6.249 mg/ml in reaction buffer to equal 10uM
TRITON X-100™ is a polyoxy ethylene non-ionic detergent supplied by Pierce Chemical Company, 3747 N. Meridian Road, Rockford, Illinois 61101.

REACTION MIXTURE -

A measured volume of racemic dipheptanoyl thio PC supplied in chloroform at a concentration of 100 mg/ml is taken to dryness and redissolved in 10 millimolar TRITON X-100™ nonionic detergent aqueous solution. Reaction Buffer is added to the solution, then DTNB to give the Reaction Mixture.
The reaction mixture thus obtained contains 1mM diheptanoly thio-PC substrate, 0.29 mm Triton X-100™ detergent, and 0.12 mm DTMB in a buffered aqueous solution at pH 7.5.

Assay Procedure:

1. Add 0.2 ml reaction mixture to all wells;
2. Add 10 ul test compound (or solvent blank) to appropriate wells, mix 20 seconds;
3. Add 50 nanograms of $sPLA_2$ (10 microliters) to appropriate wells;
4. Incubate plate at 40°C for 30 minutes;
5. Read absorbance of wells at 405 nanometers with an automatic plate reader.

All compounds were tested in triplicate. Typically, compounds were tested at a final concentration of 5 ug/ml. Compounds were considered active when they exhibited 40% inhibition or greater compared to uninhibited control reactions when measured at 405 nanometers. Lack of color development at 405 nanometers evidenced inhibition. Compounds initially found to be active were reassayed to confirm their activity and, if sufficiently active, $IC_{50}$ values were determined. Typically, the $IC_{50}$ values (see, Table I, below) were determined by diluting test compound serially two-fold such that the final concentration in the reaction ranged from 45 ug/mL to 0.35 ug/ml. More potent inhibitors required significantly greater dilution. In all cases, % inhibition measured at 405 nanometers generated by enzyme reactions containing inhibitors relative to the uninhibited control reactions was determined. Each sample was titrated in triplicate and result values were averaged for plotting and calculation of $IC_{50}$ values. $IC_{50}$ were determined by plotting log concentration versus inhibition values in the range from 10-90% inhibition.
Compounds of the instant invention (Examples 1-19) were tested in Assay Example 1 and were found to be effective at concentrations of less than 100μM.

Assay Example 2

Method:

Male Hartley strain guinea pigs (500-700g) were killed by cervical dislocation and their heart and lungs removed intact and placed in aerated (95% $O_2$:5% $CO_2$) Krebs buffer. Dorsal pleural strips (4x1x25mm) were dissected from intact parenchymal segments (8x4x25mm) cut parallel to the outer edge of the lower lung lobes. Two adjacent pleural strips, obtained from a single lobe and representing a single tissue sample, were tied at either end and independently attached to a metal support rod. One rod was attached to a Grass force-displacement transducer Model FTO3C, product of Grass Medical Instruments Co., Quincy, MA, USA). Changes in isometric tension were displayed on a monitor and thermal recorder (product of Modular Instruments, Malvern, PA). All tissues were placed in 10 ml jacketed tissue baths maintained at 37°C. The tissue baths were continuously aerated and contained a modified Krebs solution of the following composition (millimolar) NaCl, 118.2; KCl, 4.6; $CaCl_2 \cdot 2H_2O$, 2.5; $MgSO_4 \cdot 7H_2O$, 1.2; $NaHCO_3$, 24.8; $KH_2PO_4$, 1.0; and dextrose, 10.0. Pleural strips from the opposite lobes of the lung were used for paired experiments. Preliminary data generated from tension/response curves demonstrated that resting tension of 800mg was optimal. The tissues were allowed to equilibrate for 45 min. as the bath fluid was changed periodically.

Cumulative concentration-response curves:

Initially tissues were challenged 3 times with KCl (40 mM) to test tissue viability and to obtain a consistent response. After recording the maximal response to KCl, the tissues were washed and allowed to return to baseline before the next challenge. Cumulative concentration-response curves were obtained from pleural strips by increasing the agonist concentration ($sPLA_2$) in the tissue bath by half-log10 increments while the previous concentration remained in contact with the tissues (Ref.1, supra.). Agonist concentration was increased after reaching the plateau of the contraction elicited by the preceding concentration. One concentration-response curve was obtained from each tissue. To minimize variability between tissues obtained from different animals, contractile responses were expressed as a percentage of the maximal response obtained with the final KCl challenge. When studying the effects of various drugs on the contractile effects of $sPLA_2$, the compounds and their respective vehicles were added to the tissues 30 minutes prior to starting the $sPLA_2$ concentration-response curves.

Statistical analysis:

Data from different experiments were pooled and presented as a percentage of the maximal KCl responses (mean ± S.E.). To estimate the drug induced rightward shifts in the concentration response curves, the curves were analyzed simultaneously using statistical nonlinear modeling methods similar to those described by Waud (1976), Equation 26, p. 163, (Ref.2). The model includes four parameters: the maximum tissue response which was assumed the same for each curve, the $ED_{50}$ for the control curve, the steepness of the curves, and the $pA_2$, the concentration of antagonist that requires a two-fold increase in agonist to achieve an equivalent response. The Schild slope was determined to be 1, using statistical nonlinear modeling methods similar to those described by Waud (1976), Equation 27, p. 164 (Ref. 2). The Schild slope equal to 1 indicates the model is consistent with the assumptions of a competitive antagonist; therefore, the pA2 may be interpreted as the apparent $K_{B,}$ the dissociation constant of the inhibitor.

To estimate the drug-induced suppression of the maximal responses, $sPLA_2$ responses (10 ug/ml) were determined in the absence and presence of drug, and percent suppression was calculated for each pair of tissues. Representative examples of inhibitory activities are presented in Table 2, below.

Ref. 1 - Van, J.M.: Cumulative dose-response curves. II. Technique for the making of dose-response curves in isolated organs and the evaluation of drug parameters. Arch. Int. Pharmacodyn. Ther., 143: 299-330, 1963.

Ref. 2 - Waud, D.: Analysis of dose-response relationships. in Advances in General and Cellular Pharmacology eds Narahashi, Bianchi 1:145-178, 1976.

Compounds of the instant invention (Examples 1-19) were tested in Assay Example 2 and were found to be effective at concentrations below 20μM.

Assay Example 3

$sPLA_2$ Transgenic Mice Assay

Materials & Methods

The mice utilized in these studies were mature, 6-8 month old, $ZnSO_4$-stimulated, hemizygous line 2608[a] transgenic mice (Fox et. al. 1996). Transgenic mice from this line express human $sPLA_2$ in the liver and other tissues and typically achieve levels of human $sPLA_2$ in their circulation of approximately 173 ± 10 ng/ml when maximally stimulated with $ZnSO_4$ (Fox, *et al*. 1996). The mice were housed under constant humidity and temperature and received food and water ad libitum. Animal room lighting was maintained on a 12-hour light/dark cycle and all experiments were performed at the same time of the day during the early morning light period.

For intravenous testing, compounds or vehicle were administered as an IV bolus via the tail vein in a volume of 0.15 ml. Vehicle consisted of 1-5% dimethylsulfoxide, 1-5% ethanol and 10-30% polyethylene glycol 300 in $H_2O$; the concentrations of these ingredients were adjusted according to the solubility of the compound. Mice were bled retro-orbitally prior to drug or vehicle administration and 30 minutes, 2 and 4 hours thereafter. Three to six mice were used for each dose. $PLA_2$ catalytic activity in the serum was assayed with a modified phosphatidylcholine/deoxycholine mixed micelle assay (Fox, *et al*. 1996, Schadlich, *et al.*, 1987) utilizing 3 mM sodium deoxycholate and 1 mM 1-palmitoyl-2-oleoyl-sn-glycero-3-phosphocholine.

For oral testing, compounds were dissolved in 1-5% ethanol/10-30% polyethylene glycol 300 in $H_2O$ or were suspended in 5% dextrose in $H_2O$ and administered by oral gavage. Serum was prepared from retro-orbital blood and assayed for $PLA_2$ catalytic activity as above.

References

Fox, N., M. Song, J. Schrementi, J. D. Sharp, D. L. White, D. W. Snyder, L. W. Hartley, D. G. Carlson, N. J. Bach, R. D. Dillard, S. E. Draheim, J. L. Bobbitt, L. Fisher and E. D. Mihelich. 1996.
Eur. J. Pharmacol. 308: 195. Schadlich, H.R., M. Buchler, and H. G. Beger, 1987, J. Clin. Chem. Clin. Biochem. 25, 505.

Compounds of the instant invention were tested in Assay Example 3 and were found to be effective.

While the present invention has been illustrated above by certain specific embodiments, it is not intended that these specific examples should limit the scope of the invention as described in the appended claims.

## Claims

**1.** A compound of the formula (III)

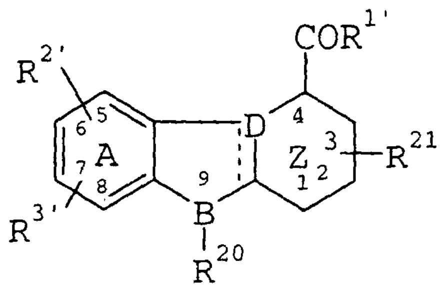

(III)

wherein ;

A is phenyl or pyridyl wherein the nitrogen is at the 5-, 6-, 7- or 8-position;
one of B or D is nitrogen and the other is carbon;
Z is cyclohexenyl, phenyl, pyridyl, wherein the nitrogen is at the 1-, 2- or 3-position, or a 6-membered heterocyclic ring having one heteroatom selected from the group consisting of sulfur or oxygen at the 1-, 2- or 3-position, and nitrogen at the 1-, 2-, 3- or 4-position;
---- is a double or single bond;
$R^{20}$ is selected from groups (a), (b) and (c) where;

(a) is $-(C_5-C_{20})$alkyl, $-(C_5-C_{20})$alkenyl, $-(C_5-C_{20})$alkynyl, carbocyclic radicals derived from a saturated or unsaturated, substituted or unsubstituted 5 to 14 membered organic nucleus whose ring forming atoms (other than hydrogen) are solely carbon atoms, or heterocyclic radicals derived from monocyclic or polycylic, saturated or unsaturated, substituted or unsubstituted heterocylic nuclei having 5 to 14 ring atoms and containing from 1 to 3 hetero atoms selected from the group consisting of nitrogen, oxygen or sulfur, or (b) is a member of (a) substituted with one or more substituents independently selected from hydrogen, $-(C_1-C_{12})$alkyl, $-(C_2-C_6)$alkenyl, $-(C_2-C_6)$alkynyl, $-(C_7-C_{12})$aralkyl,$-(C_7-C_{12})$alkaryl, $-(C_3-C_8)$cycloalkyl, $-(C_3-C_8)$cycloalkenyl, phenyl, tolulyl, xylenyl, biphenyl, $-(C_1-C_6)$alkoxy, $-(C_2-C_6)$alkenyloxy, $-(C_2-C_6)$alkynyloxy, $-(C_1-C_{12})$alkoxyalkyl, $-(C_1-C_{12})$alkoxyalkyloxy, $-(C_1-C_{12})$alkylcarbonyl, $-(C_1-C_{12})$alkylcarbonylamino, $-(C_1-C_{12})$alkoxyamino, $-(C_1-C_{12})$alkoxyaminocarbonyl, - $(C_1-C_{12})$alkylamino, $-(C_1-C_6)$alkylthio, - $(C_1-C_{12})$alkylthiocarbonyl, $-(C_1-C_6)$alkylsulfinyl, - $(C_1-C_6)$alkylsulfonyl, $-(C_1-C_6)$haloalkoxy, $-(C_1-C_6)$ haloalkylsulfonyl, $-(C_1-C_6)$haloalkyl, $-(C_1-C_6)$hydroxyalkyl, $-(CH_2)_nCN$, $-(CH_2)_nNR^9R^{10}$, $-C(O)O(C_1-C_6$ alkyl), $-(CH_2)_nO(C_1-C_6$ alkyl), benzyloxy, phenoxy, phenylthio, $-(CONHSO_2R)$, -CHO, amino, amidino, halo, carbamyl, carboxyl, carbalkoxy, $-(CH_2)_nCO_2H$, cyano, cyanoguanidinyl, guanidino, hydrazide, hydrazino, hydrazido, hydroxy, hydroxyamino, nitro, phosphono, $-SO_3H$, thioacetal, thiocarbonyl, and $(C_1-C_6)$ alkylcarbonyl; where n is from 1 to 8 and $R^9$ and $R^{10}$ are independently $-(C_1-C_4)$alkyl or phenyl$(C_1-C_4)$ alkyl; or

(c) is the group -(L)-$R^{80}$; where, -(L)- is a divalent linking group of 1 to 12 atoms selected from carbon, hydrogen, oxygen, nitrogen, and sulfur; wherein the combination of atoms in -(L)- are selected from the group consisting of (i) carbon and hydrogen only, (ii) one sulfur only, (iii) one oxygen only, (iv) one or two nitrogen and hydrogen only, (v) carbon, hydrogen, and one sulfur only, and (vi) and carbon, hydrogen, and oxygen only; and where $R^{80}$ is a group selected from (a) or (b);

$R^{21}$ is selected from hydrogen, -($C_1$-$C_{12}$)alkyl, -($C_2$-$C_6$)alkenyl, -($C_2$-$C_6$)alkynyl, -($C_7$-$C_{12}$)aralkyl,-($C_7$-$C_{12}$) alkaryl, -($C_3$-$C_8$)cycloalkyl, -($C_3$-$C_8$)cycloalkenyl, phenyl, tolulyl, xylenyl, biphenyl, -($C_1$-$C_6$)alkoxy, -($C_2$-$C_6$) alkenyloxy, -($C_2$-$C_6$)alkynyloxy, -($C_1$-$C_{12}$)alkoxyalkyl, -($C_1$-$C_{12}$)alkoxyalkyloxy, -($C_1$-$C_{12}$)alkylcarbonyl, -($C_1$-$C_{12}$)alkylcarbonylamino, -($C_1$-$C_{12}$)alkoxyamino, -($C_1$-$C_{12}$)alkoxyaminocarbonyl, -($C_1$-$C_{12}$)alkylamino, -($C_1$-$C_6$)alkylthio, -($C_1$-$C_{12}$)alkylthiocarbonyl,-($C_1$-$C_6$)alkylsulfinyl, -($C_1$-$C_6$)alkylsulfonyl, -($C_1$-$C_6$)haloalkoxy, -($C_1$-$C_6$)haloalkylsulfonyl, -($C_1$-$C_6$)haloalkyl, -($C_1$-$C_6$)hydroxyalkyl, -$(CH_2)_n$CN, -$(CH_2)_n$N$R^9R^{10}$, -C(O)O($C_1$-$C_6$ alkyl), -$(CH_2)_n$O($C_1$-$C_6$ alkyl), benzyloxy, phenoxy, phenylthio, -(CONH$SO_2$R), -CHO, amino, amidino, halo, carbamyl, carboxyl, carbalkoxy, -$(CH_2)_nCO_2H$, cyano, cyanoguanidinyl, guanidino, hydrazide, hydrazino, hydrazido, hydroxy, hydroxyamino, nitro, phosphono, -$SO_3H$, thioacetal, thiocarbonyl, and ($C_1$-$C_6$)alkylcarbonyl; where n is from 1 to 8 and $R^9$ and $R^{10}$ are independently-($C_1$-$C_4$)alkyl or phenyl($C_1$-$C_4$)alkyl;
$R^{1'}$ is -$NHNH_2$ or -$NH_2$;
$R^{2'}$ is selected from the group -OH, -O$(CH_2)_t$$R^5$ where $R^5$ is CN or phenyl, or -($L_a$)-(acidic group); wherein the acidic group is an organic group which when attached to a tricylic nucleus, through suitable linking atoms, acts as a proton donor capable of hydrogen bonding and -($L_a$)- is a divalent linking group having a linker length of 1 to 7 and t is 1-5;
$R^{3'}$ is selected from hydrogen, -($C_1$-$C_{12}$)alkyl, -($C_2$-$C_6$)alkenyl, -($C_2$-$C_6$)alkynyl, -($C_7$-$C_{12}$)aralkyl, -($C_7$-$C_{12}$) alkaryl, -($C_3$-$C_8$)cycloalkyl, -($C_3$-$C_8$)cycloalkenyl, phenyl, tolulyl, xylenyl, biphenyl, -($C_1$-$C_6$)alkoxy, -($C_2$-$C_6$) alkenyloxy, -($C_2$-$C_6$)alkynyloxy,-($C_1$-$C_{12}$)alkoxyalkyl, -($C_1$-$C_{12}$)alkoxyalkyloxy, -($C_1$-$C_{12}$)alkylcarbonyl, -($C_1$-$C_{12}$)alkylcarbonylamino, -($C_1$-$C_{12}$)alkoxyamino, -($C_1$-$C_{12}$)alkoxyaminocarbonyl,-($C_1$-$C_{12}$)alkylamino, -($C_1$-$C_6$)alkylthio, -($C_1$-$C_{12}$)alkylthiocarbonyl, -($C_1$-$C_6$)alkylsulfinyl, -($C_1$-$C_6$)alkylsulfonyl, -($C_1$-$C_6$)haloalkoxy, -($C_1$-$C_6$)haloalkylsulfonyl, -($C_1$-$C_6$)haloalkyl, -($C_1$-$C_6$)hydroxyalkyl, -$(CH_2)_n$CN, -$(CH_2)_n$N$R^9R^{10}$, -C(O)O($C_1$-$C_6$ alkyl), -$(CH_2)_n$O($C_1$-$C_6$ alkyl), benzyloxy, phenoxy, phenylthio, -(CONH$SO_2$R), -CHO, amino, amidino, halo, carbamyl, carboxyl, carbalkoxy, -$(CH_2)_nCO_2H$, cyano, cyanoguanidinyl, guanidino, hydrazide, hydrazino, hydrazido, hydroxy, hydroxyamino, nitro, phosphono, -$SO_3H$, thioacetal, thiocarbonyl, and ($C_1$-$C_6$)alkylcarbonyl; where n is from 1 to 8 and $R^9$ and $R^{10}$ are independently -($C_1$-$C_4$)alkyl or phenyl($C_1$-$C_4$)alkyl; carbocyclic radicals derived from a saturated or unsaturated 5 to 14 membered organic nucleus whose ring forming atoms (other than hydrogen) are solely carbon atoms; or heterocylic radicals derived from monocyclic or polycylic, saturated or unsaturated heterocylic nuclei having 5 to 14 ring atoms and containing from 1 to 3 hetero atoms selected from the group consisting of nitrogen, oxygen or sulfur; and carbocyclic radicals derived from a saturated or unsaturated 5 to 14 membered organic nucleus whose ring forming atoms (other than hydrogen) are solely carbon atoms or heterocyclic radicals derived from monocyclic or polycylic, saturated or unsaturated, heterocylic nuclei having 5 to 14 ring atoms and containing from 1 to 3 hetero atoms selected from the group consisting of nitrogen, oxygen or sulfur substituted with groups selected from hydrogen, -($C_1$-$C_{12}$)alkyl, -($C_2$-$C_6$)alkenyl,-($C_2$-$C_6$)alkynyl, -($C_7$-$C_{12}$)aralkyl, -($C_7$-$C_{12}$)alkaryl, -($C_3$-$C_8$)cycloalkyl, -($C_3$-$C_8$)cycloalkenyl, phenyl, tolulyl, xylenyl, biphenyl, -($C_1$-$C_6$)alkoxy, -($C_2$-$C_6$)alkenyloxy, -($C_2$-$C_6$)alkynyloxy, -($C_1$-$C_{12}$)alkoxyalkyl, -($C_1$-$C_{12}$)alkoxyalkyloxy, -($C_1$-$C_{12}$)alkylcarbonyl, -($C_1$-$C_{12}$)alkylcarbonylamino, -($C_1$-$C_{12}$)alkoxyamino, -($C_1$-$C_{12}$)alkoxyaminocarbonyl, -($C_1$-$C_{12}$)alkylamino, -($C_1$-$C_6$)alkylthio, -($C_1$-$C_{12}$)alkylthiocarbonyl, -($C_1$-$C_6$) alkylsulfinyl, -($C_1$-$C_6$)alkylsulfonyl, -($C_1$-$C_6$)haloalkoxy, -($C_1$-$C_6$)haloalkylsulfonyl, -($C_1$-$C_6$)haloalkyl, ($C_1$-$C_6$) hydroxyalkyl, -$(CH_2)_n$CN, -$(CH_2)_n$N$R^9R^{10}$, -C(O)O($C_1$-$C_6$ alkyl), -$(CH_2)_n$O($C_1$-$C_6$ alkyl), benzyloxy, phenoxy, phenylthio, -(CONH$SO_2$R), -CHO, amino, amidino, halo, carbamyl, carboxyl, carbalkoxy, -$(CH_2)_nCO_2H$, cyano, cyanoguanidinyl, guanidino, hydrazide, hydrazino, hydrazido, hydroxy, hydroxyamino, nitro, phosphono, -$SO_3H$, thioacetal, thiocarbonyl, and ($C_1$-$C_6$)alkylcarbonyl; where n is from 1 to 8 and $R^9$ and $R^{10}$ are independently -($C_1$-$C_4$)alkyl or phenyl($C_1$-$C_4$)alkyl ;
or a pharmaceutically acceptable salt, racemate, solvate, tautomer, or optical isomer thereof;
provided that when D is nitrogen, the heteroatom of Z is selected from the group consisting of sulfur or oxygen at the 1-, 2- or 3-position and nitrogen at the 1-, 2-, 3-or 4-position.

**2.** A compound of the formula (I)

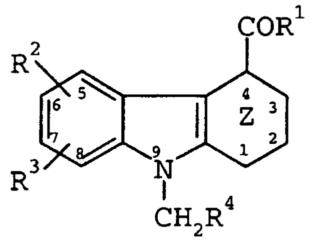

(I)

wherein:

$R^1$ is $-NHNH_2$, or $-NH_2$;
$R^2$ is -OH, or $-O(CH_2)_mR^5$ where
$R^5$ is H, $-CO_2H$, $-CO_2(C_1\text{-}C_4$ alkyl),

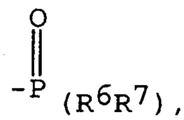

phenyl, or phenyl substituted with $-CO_2H$ or $-CO_2(C_1\text{-}C_4$ alkyl) where $R^6$ and $R^7$ are each independently -OH or $-O(C_1\text{-}C_4$ alkyl) and m is 1-3;
$R^3$ is H, $-O(C_1\text{-}C_4$ alkyl), or $-(CH_2)_nR^8$ where
$R^8$ is H, $-NR^9R^{10}$,

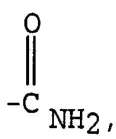

-CN, or phenyl where $R^9$ and $R^{10}$ are independently $-(C_1\text{-}C_4)$alkyl, or phenyl$(C_1\text{-}C_4)$alkyl and n is 1-8;
$R^4$ is H, $-(C_5\text{-}C_{14})$alkyl, $-(C_3\text{-}C_{14})$cycloalkyl, phenyl, or phenyl substituted with 1 or 2 substituents selected from the group consisting of $-(C_1\text{-}C_4)$alkyl, $(C_1\text{-}C_4)$alkoxy,
phenyl$(C_1\text{-}C_4)$alkyl, $(C_1\text{-}C_4)$alkylthio, halo or phenyl; and
Z is cyclohexenyl or phenyl;
or a pharmaceutically acceptable salt, racemate, optical isomer, solvate, or tautomer thereof;
provided that when $R^3$ is H, $R^4$ is phenyl, m is 1 or 2 and $R^2$ is substituted at the 6 position, $R^5$ cannot be H; and

when $R^1$ is $NHNH_2$, $R^8$ cannot be

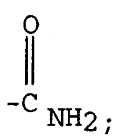

and when $R^1$ is $NH_2$, $R^2$ is OMe substituted at the 6 position and $R^3$ is H, $R^4$ cannot be H.

**3.** A compound of formula I as claimed in Claim 2 wherein

$R^1$ is $-NHNH_2$ or $-NH_2$;
$R^2$ is $-O(CH_2)_mR^5$ where $R^5$ is $-CO_2H$ or

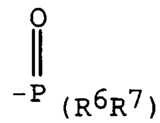

where $R^6$ and $R^7$ are -OH;
$R^3$ is H, -O($C_1$-$C_4$ alkyl) or -$(CH_2)_nR^8$ where $R^8$ is H or phenyl;
$R^4$ is phenyl; and
Z is cyclohexene.

4. A compound of Claim 3 which is 4-[(9-benzyl-4-carbamoyl-1,2,3,4-tetrahydrocarbazol-6-yl)oxy]butyric acid; 3-[(9-benzyl-4-carbamoyl-1,2,3,4-tetrahydrocarbazol-6-yl)oxy]propylphosphonic acid; 2-[(9-benzyl-4-carbamoyl-1,2,3,4-tetrahydrocarbazol-6-yl)oxy]methylbenzoic acid; 3-[(9-benzyl-4-carbamoyl-7-n-octyl-1,2,3,4-tetrahydrocarbazol-6-yl)oxy]propylphosphonic acid; 4-[(9-benzyl-4-carbamoyl-7-ethyl-1,2,3,4-tetrahydrocarbazol-6-yl)oxy] butyric acid; 3-[(9-benzyl-4-carbamoyl-7-ethyl-1,2,3,4-tetrahydrocarbazol-6-yl)oxy]propylphosphonic acid; 3-[(9-benzyl-4-carbamoyl-7-ethyl-1,2,3,4-tetrahydrocarbazol-6-yl)oxy]propylphosphonic acid; (S)-(+)-4-[(9-benzyl-4-carbamoyl-7-ethyl-1,2,3,4-tetrahydrocarbazol-6-yl)oxy]butyric acid; 4-[9-benzyl-4-carbamoyl-6-(2-cyanoethyl)-1,2,3,4-tetrahydrocarbazol-6-yl]oxybutyric acid; 4-[9-benzyl-4-carboxamido-7-(2-phenylethyl)-1,2,3,4-tetrahydrocarbazol-6-yl]oxybutyric acid; 4-[9-benzyl-4-carboxamidocarbazol-6-yl]oxybutyric acid; methyl 2-[(9-benzyl-4-carbamoyl-1,2,3,4-tetrahydrocarbazol-6-yl)oxy]methylbenzoate; 9-benzyl-5,7-dimethoxy-1,2,3,4-tetrahydrocarbazole-4-carboxylic acid hydrazide; 9-benzyl-5,7-dimethoxy-1,2,3,4-tetrahydrocarbazole-4-carboxamide; [9-benzyl-4-carbamoyl-7-methoxy-1,2,3,4-tetrahydrocarbazol-5-yl]oxyacetic acid sodium salt; 4-[9-benzyl-4-carbamoyl-7-(2-cyanoethyl)-1,2,3,4-tetrahydrocarbazol-6-yl]oxybutyric acid; [9-benzyl-4-carbamoyl-7-methoxycarbazol-5-yl]oxyacetic acid; methyl [9-benzyl-4-carbamoyl-7-methoxycarbazol-5-yl]oxyacetic acid; 9-benzyl-7-methoxy-5-cyanomethyloxy-1,2,3,4-tetrahydrocarbazole-4-carboxamide; 9-benzyl-7-methoxy-5-(1H-tetrazol-5-yl-methyl)oxy)-1,2,3,4-tetrahydrocarbazole-4-carboxamide; [9-benzyl-4-carbamoyl-8-methyl-1,2,3,4-tetrahydrocarbazole-5-yl]oxyacetic acid; [9-benzyl-4-carbamoyl-8-methyl-carbazole-5-yl]oxyacetic acid; [9-benzyl-4-carbamoyl-1,2,3,4-tetrahydrocarbazol-5-yl]oxyacetic acid; and [9-benzyl-4-carbamoyl-carbazole-5-yl]oxyacetic acid or a pharmaceutically acceptable salt, racemate, solvate, tautomer, or optical isomer thereof.

5. A pharmaceutical formulation comprising a compound of formula III as claimed in Claim 1 together with a pharmaceutically acceptable carrier or diluent therefor.

6. A pharmaceutical formulation comprising a compound of formula I as claimed in Claim 2 together with a pharmaceutically acceptable carrier or diluent therefor.

7. Use for the manufacture of a medicament for selectively inhibiting $sPLA_2$ in a mammal in need of such treatment of a compound of formula (III)

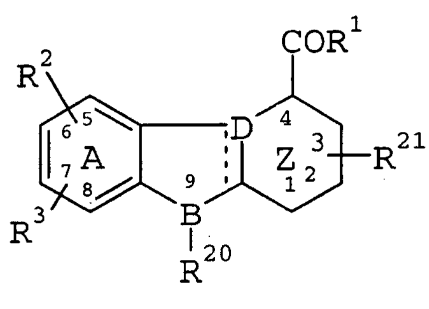

(III)

wherein ;

A is phenyl or pyridyl;
B and D are each independently nitrogen or carbon;
Z is cyclohexenyl, phenyl, pyridyl, wherein the nitrogen is at the 1-, 2- or 3-position or a 6-membered heterocyclic ring having one to three heteroatoms which are the same or different and which are selected from the group consisting of sulfur or oxygen at the 1-, 2- or 3-position, and nitrogen at the 1-, 2-, 3- or 4-position;

---- is a double or single bond;
$R^{20}$ is selected from groups (a), (b) and (c) where;

(a) is $C_5$-$C_{20}$ alkyl, $C_5$-$C_{20}$ alkenyl, $C_5$-$C_{20}$ alkynyl, carbocyclic radicals derived from a saturated or unsaturated, substituted or unsubstituted 5 to 14 membered organic nucleus whose ring forming atoms (other than hydrogen) are solely carbon atoms, or heterocyclic radicals derived from monocyclic or polycylic, saturated or unsaturated, substituted or unsubstituted heterocylic nuclei having 5 to 14 ring atoms and containing from 1 to 3 hetero atoms selected from the group consisting of nitrogen, oxygen or sulfur, or
(b) is a member of (a) substituted with one or more substituents independently selected from hydrogen, -($C_1$-$C_{12}$)alkyl, -($C_2$-$C_6$)alkenyl, -($C_2$-$C_6$)alkynyl, -($C_7$-$C_{12}$)aralkyl, -($C_7$-$C_{12}$)alkaryl, -($C_3$-$C_8$)cycloalkyl, -($C_3$-$C_8$)cycloalkenyl, phenyl, tolulyl, xylenyl, biphenyl, -($C_1$-$C_6$)alkoxy, -($C_2$-$C_6$)alkenyloxy, -($C_2$-$C_6$)alkynyloxy, -($C_1$-$C_{12}$)alkoxyalkyl, -($C_1$-$C_{12}$)alkoxyalkyloxy, - ($C_1$-$C_{12}$)alkylcarbonyl, -($C_1$-$C_{12}$)alkylcarbonylamino, - ($C_1$-$C_{12}$)alkoxyamino, -($C_1$-$C_{12}$)alkoxyaminocarbonyl, - ($C_1$-$C_{12}$)alkylamino, -($C_1$-$C_6$)alkylthio, -($C_1$-$C_{12}$)alkylthiocarbonyl, -($C_1$-$C_6$)alkylsulfinyl, - ($C_1$-$C_6$)alkylsulfonyl, -($C_1$-$C_6$)haloalkoxy, -($C_1$-$C_6$) haloalkylsulfonyl, -($C_1$-$C_6$)haloalkyl, -($C_1$-$C_6$)hydroxyalkyl, -$(CH_2)_n$CN, -$(CH_2)_nNR^9R^{10}$, -C(O)O($C_1$-$C_6$ alkyl), -$(CH_2)_n$O($C_1$-$C_6$ alkyl), benzyloxy, phenoxy, phenylthio, -($CONHSO_2R$), -CHO, amino, amidino, halo, carbamyl, carboxyl, carbalkoxy, -$(CH_2)_nCO_2H$, cyano, cyanoguanidinyl, guanidino, hydrazide, hydrazino, hydrazido, hydroxy, hydroxyamino, nitro, phosphono, -$SO_3H$, thioacetal, thiocarbonyl, and ($C_1$-$C_6$) alkylcarbonyl; where n is from 1 to 8 and $R^9$ and $R^{10}$ are independently -($C_1$-$C_4$)alkyl or phenyl($C_1$-$C_4$) alkyl; or
(c) is the group -(L)-$R^{80}$; where, -(L)- is a divalent linking group of 1 to 12 atoms selected from carbon, hydrogen, oxygen, nitrogen, and sulfur; wherein the combination of atoms in -(L)- are selected from the group consisting of (i) carbon and hydrogen only, (ii) one sulfur only, (iii) one oxygen only, (iv) one or two nitrogen and hydrogen only, (v) carbon, hydrogen, and one sulfur only, and (vi) and carbon, hydrogen, and oxygen only; and where $R^{80}$ is a group selected from (a) or (b);

$R^{21}$ is selected from hydrogen, -($C_1$-$C_{12}$)alkyl, -($C_2$-$C_6$)alkenyl, -($C_2$-$C_6$)alkynyl, -($C_7$-$C_{12}$)aralkyl, -($C_7$-$C_{12}$) alkaryl, -($C_3$-$C_8$)cycloalkyl, -($C_3$-$C_8$)cycloalkenyl, phenyl, tolulyl, xylenyl, biphenyl, -($C_1$-$C_6$)alkoxy, -($C_2$-$C_6$) alkenyloxy, -($C_2$-$C_6$)alkynyloxy, -($C_1$-$C_{12}$)alkoxyalkyl, -($C_1$-$C_{12}$)alkoxyalkyloxy, -($C_1$-$C_{12}$)alkylcarbonyl, -($C_1$-$C_{12}$)alkylcarbonylamino, -($C_1$-$C_{12}$)alkoxyamino, -($C_1$-$C_{12}$)alkoxyaminocarbonyl, -($C_1$-$C_{12}$)alkylamino, -($C_1$-$C_6$)alkylthio, -($C_1$-$C_{12}$)alkylthiocarbonyl, -($C_1$-$C_6$)alkylsulfinyl, -($C_1$-$C_6$)alkylsulfonyl,-($C_1$-$C_6$)haloalkoxy, -($C_1$-$C_6$)haloalkylsulfonyl, -($C_1$-$C_6$)haloalkyl, -($C_1$-$C_6$)hydroxyalkyl, -$(CH_2)_n$CN, -$(CH_2)_nNR^9R^{10}$, -C(O)O($C_1$-$C_6$ alkyl), -$(CH_2)_n$O($C_1$-$C_6$ alkyl), benzyloxy, phenoxy, phenylthio, -($CONHSO_2R$), -CHO, amino, amidino, halo, carbamyl, carboxyl, carbalkoxy, -$(CH_2)_nCO_2H$, cyano, cyanoguanidinyl, guanidino, hydrazide, hydrazino, hydrazido, hydroxy, hydroxyamino, nitro, phosphono, -$SO_3H$, thioacetal, thiocarbonyl, and ($C_1$-$C_6$)alkylcarbonyl; where n is from 1 to 8 and $R^9$ and $R^{10}$ are independently -($C_1$-$C_4$)alkyl or phenyl($C_1$-$C_4$)alkyl;
$R^{1'}$ is -$NHNH_2$ or -$NH_2$;
$R^{2'}$ is selected from the group -OH, -$O(CH_2)_tR^5$ where $R^5$ is CN or phenyl, or -($L_a$)-(acidic group); wherein the acidic group is an organic group which when attached to a tricylic nucleus, through suitable linking atoms, acts as a proton donor capable of hydrogen bonding and -($L_a$)- is a divalent linking group having a linker length of 1 to 7 and t is 1-5;
$R^{3'}$ is selected from hydrogen, -($C_1$-$C_{12}$)alkyl, -($C_2$-$C_6$)alkenyl,-($C_2$-$C_6$)alkynyl, -($C_7$-$C_{12}$)aralkyl, -($C_7$-$C_{12}$) alkaryl, -($C_3$-$C_8$)cycloalkyl, -($C_3$-$C_8$)cycloalkenyl, phenyl, tolulyl, xylenyl, biphenyl, -($C_1$-$C_6$)alkoxy, -($C_2$-$C_6$) alkenyloxy, -($C_2$-$C_6$)alkynyloxy, -($C_1$-$C_{12}$)alkoxyalkyl, -($C_1$-$C_{12}$)alkoxyalkyloxy, -($C_1$-$C_{12}$)alkylcarbonyl, -($C_1$-$C_{12}$)alkylcarbonylamino, -($C_1$-$C_{12}$)alkoxyamino, -($C_1$-$C_{12}$)alkoxyaminocarbonyl, -($C_1$-$C_{12}$)alkylamino, -($C_1$-$C_6$)alkylthio, -($C_1$-$C_{12}$)alkylthiocarbonyl, -($C_1$-C6)alkylsulfinyl, -($C_1$-$C_6$)alkylsulfonyl, -($C_1$-$C_6$)haloalkoxy, -($C_1$-$C_6$)haloalkylsulfonyl, -($C_1$-$C_6$)haloalkyl, -($C_1$-$C_6$)hydroxyalkyl, -$(CH_2)_n$CN, -$(CH_2)_nNR^9R^{10}$, -C(O)O($C_1$-$C_6$ alkyl), -$(CH_2)_n$O($C_1$-$C_6$ alkyl), benzyloxy, phenoxy, phenylthio, -($CONHSO_2R$), -CHO, amino, amidino, halo, carbamyl, carboxyl, carbalkoxy, -$(CH_2)_nCO_2H$, cyano, cyanoguanidinyl, guanidino, hydrazide, hydrazino, hydrazido, hydroxy, hydroxyamino, nitro, phosphono, -$SO_3H$, thioacetal, thiocarbonyl, and ($C_1$-$C_6$)alkylcarbonyl; where n is from 1 to 8 and $R^9$ and $R^{10}$ are independently -($C_1$-$C_4$)alkyl or phenyl($C_1$-$C_4$)alkyl; carbocyclic radicals derived from a saturated or unsaturated 5 to 14 membered organic nucleus whose ring forming atoms (other than hydrogen) are solely carbon atoms; or heterocyclic radicals derived from monocyclic or polycyclic, saturated or unsaturated, heterocylic nuclei having 5 to 14 ring atoms and containing from 1 to 3 hetero atoms selected from the group consisting of nitrogen, oxygen or sulfur; and carbocyclic radicals derived from a saturated or unsaturated 5 to 14 membered organic nucleus whose ring forming atoms (other than hydrogen) are solely carbon atoms or heterocyclic radicals derived from monocyclic or polycylic, saturated or unsaturated heterocylic nuclei having 5 to 14 ring atoms and containing from 1 to 3 hetero atoms selected from the group

consisting of nitrogen, oxygen or sulfur substituted with groups selected from hydrogen, $-(C_1-C_{12})$alkyl, $-(C_2-C_6)$alkenyl, $-(C_2-C_6)$alkynyl, $-(C_7-C_{12})$aralkyl, $-(C_7-C_{12})$alkaryl, $-(C_3-C_8)$cycloalkyl, $-(C_3-C_8)$cycloalkenyl, phenyl, tolulyl, xylenyl, biphenyl, $-(C_1-C_6)$alkoxy, $-(C_2-C_6)$alkenyloxy,$-(C_2-C_6)$alkynyloxy,$-(C_1-C_{12})$alkoxyalkyl, $-(C_1-C_{12})$alkoxyalkyloxy, $-(C_1-C_{12})$alkylcarbonyl, $-(C_1-C_{12})$alkylcarbonylamino, $-(C_1-C_{12})$alkoxyamino, $-(C_1-C_{12})$alkoxyaminocarbonyl, $-(C_1-C_{12})$alkylamino, $-(C_1-C_6)$alkylthio, $-(C_1-C_{12})$alkylthiocarbonyl, $-(C_1-C_6)$alkylsulfinyl, $-(C_1-C_6)$alkylsulfonyl, $-(C_1-C_6)$haloalkoxy, $-(C_1-C_6)$haloalkylsulfonyl, $-(C_1-C_6)$haloalkyl, $-(C_1-C_6)$hydroxyalkyl, $-(CH_2)_nCN$, $-(CH_2)_nNR^9R^{10}$, $-C(O)O(C_1-C_6$ alkyl), $-(CH_2)_nO(C_1-C_6$ alkyl), benzyloxy, phenoxy, phenylthio, $-(CONHSO_2R)$, -CHO, amino, amidino, halo, carbamyl, carboxyl, carbalkoxy, $-(CH_2)_nCO_2H$, cyano, cyanoguanidinyl, guanidino, hydrazide, hydrazino, hydrazido, hydroxy, hydroxyamino, nitro, phosphono, $-SO_3H$, thioacetal, thiocarbonyl, and $(C_1-C_6)$alkylcarbonyl; where n is from 1 to 8 and $R^9$ and $R^{10}$ are independently $-(C_1-C_4)$alkyl or phenyl$(C_1-C_4)$alkyl;
or a pharmaceutically acceptable salt, racemate, solvate, tautomer, or optical isomer thereof.

**8.** Use for the manufacture of a medicament for selectively inhibiting $sPLA_2$ in a mammal in need of such treatment of a compound of formula (II)

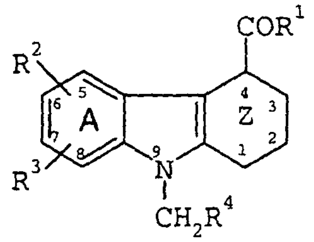

(II)

wherein:

$R^1$ is $-NHNH_2$, or $-NH_2$;
$R^2$ is -OH, or $-O(CH_2)_mR^5$ where
$R^5$ is H, $-CO_2H$, $-CO_2(C_1-C_4$ alkyl),

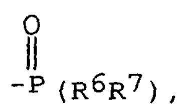

$-NHSO_2(C_1-C_6)$alkyl, $-CONHSO_2(C_1-C_6)$alkyl, -CN, tetrazolyl, phenyl, or phenyl substituted with $-CO_2H$ or $-CO_2(C_1-C_4$ alkyl) where $R^6$ and $R^7$ are each independently -OH or $-O(C_1-C_4$ alkyl) and m is 1-3;
$R^3$ is H, $-O(C_1-C_4$ alkyl), or $-(CH_2)_nR^8$ where
$R^8$ is H, $-NR^9R^{10}$,

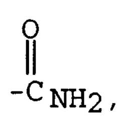

-CN, or phenyl where $R^9$ and $R^{10}$ are independently $-(C_1-C_4)$alkyl, or -phenyl$(C_1-C_4)$alkyl and n is 1-8;
$R^4$ is H, $-(C_5-C_{14})$alkyl, $-(C_3-C_{14})$cycloalkyl, phenyl, or phenyl substituted with 1 or 2 substituents selected from the group consisting of $-(C_1-C_4)$alkyl,$-(C_1-C_4)$alkoxy, -phenyl$(C_1-C_4)$alkyl, $-(C_1-C_4)$alkylthio, halo or phenyl; and
Z is cyclohexenyl, phenyl, pyridyl wherein the nitrogen is at the 1-, 2-, 3- or 4-position or a 6-membered heterocyclic ring having one heteroatom selected from the group consisting of sulfur or oxygen at the 1-, 2- or 3-position and nitrogen at the 1-, 2-, 3- or 4-position, or wherein one carbon on the heterocyclic ring is optionally substituted with =O;

A is phenyl or pyridyl wherein the nitrogen is at the 5-, 6-, 7- or 8-position;
or a pharmaceutically acceptable salt, racemate, optical isomer, tautomer, or solvate thereof;
provided that when $R^3$ is H, $R^4$ is phenyl, m is 1 or 2 and $R^2$ is substituted at the 6 position, $R^5$ cannot be H; and when $R^1$ is $NHNH_2$, $R^8$ cannot be

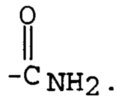

**9.** The use of Claim 7 wherein the mammal is a human.

**10.** The use of Claim 8 wherein the mammal is a human.

**11.** The use of Claim 7 wherein the medicament is for alleviating the pathological effects of septic shock, adult respiratory distress syndrome, pancreatitis, trauma-induced shock, bronchial asthma, allergic rhinitis, rheumatoid arthritis, cystic fibrosis, stroke, acute bronchitis, chronic bronchitis, acute bronchiolitis, chronic bronchiolitis, osteoarthritis, gout, spondylarthropathris, ankylosing spondylitis, Reiter's syndrome, psoriatic arthropathy, enterapathric spondylitis, Juvenile arthropathy or juvenile ankylosing spondylitis, Reactive arthropathy, infectious or post-infectious arthritis, gonoccocal arthritis, Tuberculous arthritis, viral arthritis, fungal arthritis, syphilitic arthritis, Lyme disease, arthritis associated with "vasculitic syndromes", polyarteritis nodosa, hypersensitivity vasculitis, Luegenec's granulomatosis, polymyalgin rheumatica, joint cell arteritis, calcium crystal deposition arthropathris, pseudo gout, non-articular rheumatism, bursitis, tenosynomitis, epicondylitis (tennis elbow), carpal tunnel syndrome, repetitive use injury (typing), miscellaneous forms of arthritis, neuropathic joint disease (charco and joint), hemarthrosis (hemarthrosic), Henoch-Schonlein Purpura, hypertrophic osteoarthropathy, multicentric reticulohistiocytosis, arthritis associated with certain diseases, surcoilosis, hemochromatosis, sickle cell disease and other hemoglobinopathries, hyperlipoproteineimia, hypogammaglobulinemia, hyperparathyroidism, acromegaly, familial Mediterranean fever, Behat's Disease, systemic lupus erythrematosis, or relapsing polychondritis; and related diseases

**12.** The use of Claim 8 wherein the medicament is for alleviating the pathological effects of septic shock, adult respiratory distress syndrome, pancreatitis, trauma-induced shock, bronchial asthma, allergic rhinitis, rheumatoid arthritis, cystic fibrosis, stroke, acute bronchitis, chronic bronchitis, acute bronchiolitis, chronic bronchiolitis, osteoarthritis, gout, spondylarthropathris, ankylosing spondylitis, Reiter's syndrome, psoriatic arthropathy, enterapathric spondylitis, Juvenile arthropathy or juvenile ankylosing spondylitis, Reactive arthropathy, infectious or post-infectious arthritis, gonoccocal arthritis, Tuberculous arthritis, viral arthritis, fungal arthritis, syphilitic arthritis, Lyme disease, arthritis associated with "vasculitic syndromes", polyarteritis nodosa, hypersensitivity vasculitis, Luegenec's granulomatosis, polymyalgin rheumatica, joint cell arteritis, calcium crystal deposition arthropathris, pseudo gout, non-articular rheumatism, bursitis, tenosynomitis, epicondylitis (tennis elbow), carpal tunnel syndrome, repetitive use injury (typing), miscellaneous forms of arthritis, neuropathic joint disease (charco and joint), hemarthrosis (hemarthrosic), Henoch-Schonlein Purpura, hypertrophic osteoarthropathy, multicentric reticulohistiocytosis, arthritis associated with certain diseases, surcoilosis, hemochromatosis, sickle cell disease and other hemoglobinopathries, hyperlipoproteineimia, hypogammaglobulinemia, hyperparathyroidism, acromegaly, familial Mediterranean fever, Behat's Disease, systemic lupus erythrematosis, or relapsing polychondritis; and related diseases.

**13.** The use of Claim 11 wherein the mammal is a human.

**14.** The use of Claim 12 wherein the mammal is a human.

**15.** A process of preparing compounds of formula

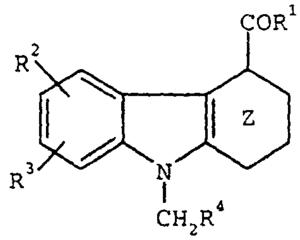

wherein

$R^1$ is $-NHNH_2$, or $-NH_2$;
$R^2$ is -OH, or $-O(CH_2)_mR^5$ where
$R^5$ is H, $-CO_2H$, $-CO_2(C_1-C_4$ alkyl),

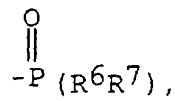

phenyl, or phenyl substituted with $-CO_2H$ or $-CO_2(C_1-C_4$ alkyl) where $R^6$ and $R^7$ are each independently -OH or $-O(C_1-C_4$ alkyl) and m is 1-3;
$R^3$ is H, $-O(C_1-C_4$ alkyl), or $-(CH_2)_nR^8$ where
$R^8$ is H, $-NR^9R^{10}$,

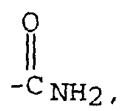

-CN, or phenyl where $R^9$ and $R^{10}$ are independently $-(C_1-C_4)$alkyl, or -phenyl$(C_1-C_4)$alkyl and n is 1-8;
$R^4$ is H, $-(C_5-C_{14})$alkyl, $-(C_3-C_{14})$cycloalkyl, phenyl, or phenyl substituted with 1 or 2 substituents selected from the group consisting of $-(C_1-C_4)$alkyl,$-(C_1-C_4)$alkoxy, -phenyl$(C_1-C_4)$alkyl, $-(C_1-C_4)$alkylthio, halo or phenyl; and
Z is phenyl comprising dehydrogenating a compound of the formula

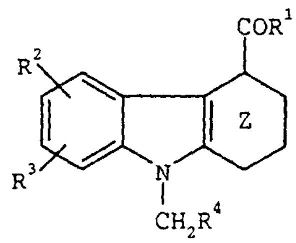

wherein

$R^1$ is $-NHNH_2$, or $-NH_2$;
$R^2$ is -OH, or $-O(CH_2)_mR^5$ where
$R^5$ is H, $-CO_2H$, $-CO_2(C_1-C_4$ alkyl),

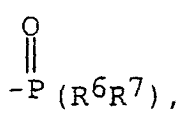

phenyl, or phenyl substituted with $-CO_2H$ or $-CO_2(C_1-C_4$ alkyl) where $R^6$ and $R^7$ are each independently -OH

or $-O(C_1-C_4$ alkyl) and m is 1-3;
$R^3$ is H, $-O(C_1-C_4$ alkyl), or $-(CH_2)_nR^8$ where
$R^8$ is H, $-NR^9R^{10}$,

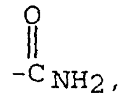

-CN, or phenyl where $R^9$ and $R^{10}$ are independently $-(C_1-C_4)$alkyl, or -phenyl$(C_1-C_4)$alkyl and n is 1-8;
$R^4$ is H, $-(C_5-C_{14})$alkyl, $-(C_3-C_{14})$cycloalkyl, phenyl, or phenyl substituted with 1 or 2 substituents selected from the group consisting of $-(C_1-C_4)$alkyl,$-(C_1-C_4)$alkoxy, -phenyl$(C_1-C_4)$alkyl, $-(C_1-C_4)$alkylthio, halo or phenyl; and
Z is cyclohexenyl.

**16.** A compound of Formula III as claimed in Claim 1 for treating pathological effects of septic shock, adult respiratory distress syndrome, pancreatitis, trauma-induced shock, bronchial asthma, allergic rhinitis, rheumatoid arthritis, cystic fibrosis, stroke, acute bronchitis, chronic bronchitis, acute bronchiolitis, chronic bronchiolitis, osteoarthritis, gout, spondylarthropathris, ankylosing spondylitis, Reiter's syndrome, psoriatic arthropathy, enterapathric spondylitis, Juvenile arthropathy or juvenile ankylosing spondylitis, Reactive arthropathy, infectious or post-infectious arthritis, gonoccocal arthritis, Tuberculous arthritis, viral arthritis, fungal arthritis, syphilitic arthritis, Lyme disease, arthritis associated with "vasculitic syndromes", polyarteritis nodosa, hypersensitivity vasculitis, Luegenec's granulomatosis, polymyalgin rheumatica, joint cell arteritis, calcium crystal deposition arthropathris, pseudo gout, non-articular rheumatism, bursitis, tenosynomitis, epicondylitis (tennis elbow), carpal tunnel syndrome, repetitive use injury (typing), miscellaneous forms of arthritis, neuropathic joint disease (charco and joint), hemarthrosis (hemarthrosic), Henoch-Schonlein Purpura, hypertrophic osteoarthropathy, multicentric reticulohistiocytosis, arthritis associated with certain diseases, surcoilosis, hemochromatosis, sickle cell disease and other hemoglobinopathries, hyperlipoproteineimia, hypogammaglobulinemia, hyperparathyroidism, acromegaly, familial Mediterranean fever, Behat's Disease, systemic lupus erythrematosis, or relapsing polychondritis; and related diseases.

**17.** A compound of Formula I as claimed in any one of Claims 2 to 4 for treating pathological effects of septic shock, adult respiratory distress syndrome, pancreatitis, trauma-induced shock, bronchial asthma, allergic rhinitis, rheumatoid arthritis, cystic fibrosis, stroke, acute bronchitis, chronic bronchitis, acute bronchiolitis, chronic bronchiolitis, osteoarthritis, gout, spondylarthropathris, ankylosing spondylitis, Reiter's syndrome, psoriatic arthropathy, enterapathric spondylitis, Juvenile arthropathy or juvenile ankylosing spondylitis, Reactive arthropathy, infectious or post-infectious arthritis, gonoccocal arthritis, Tuberculous arthritis, viral arthritis, fungal arthritis, syphilitic arthritis, Lyme disease, arthritis associated with "vasculitic syndromes", polyarteritis nodosa, hypersensitivity vasculitis, Luegenec's granulomatosis, polymyalgin rheumatica, joint cell arteritis, calcium crystal deposition arthropathris, pseudo gout, non-articular rheumatism, bursitis, tenosynomitis, epicondylitis (tennis elbow), carpal tunnel syndrome, repetitive use injury (typing), miscellaneous forms of arthritis, neuropathic joint disease (charco and joint), hemarthrosis (hemarthrosic), Henoch-Schonlein Purpura, hypertrophic osteoarthropathy, multicentric reticulohistiocytosis, arthritis associated with certain diseases, surcoilosis, hemochromatosis, sickle cell disease and other hemoglobinopathries, hyperlipoproteineimia, hypogammaglobulinemia, hyperparathyroidism, acromegaly, familial Mediterranean fever, Behat's Disease, systemic lupus erythrematosis, or relapsing polychondritis; and related diseases.

**18.** The use of a compound of Formula III as claimed in Claim 1 for the manufacture of 'a medicament for treating pathological effects of septic shock, adult respiratory distress syndrome, pancreatitis, trauma-induced shock, bronchial asthma, allergic rhinitis, rheumatoid arthritis, cystic fibrosis, stroke, acute bronchitis, chronic bronchitis, acute bronchiolitis, chronic bronchiolitis, osteoarthritis, gout, spondylarthropathris, ankylosing spondylitis, Reiter's syndrome, psoriatic arthropathy, enterapathric spondylitis, Juvenile arthropathy or juvenile ankylosing spondylitis, Reactive arthropathy, infectious or post-infectious arthritis, gonoccocal arthritis, Tuberculous arthritis, viral arthritis, fungal arthritis, syphilitic arthritis, Lyme disease, arthritis associated with "vasculitic syndromes", polyarteritis nodosa, hypersensitivity vasculitis, Luegenec's granulomatosis, polymyalgin rheumatica, joint cell arteritis, calcium crystal deposition arthropathris, pseudo gout, non-articular rheumatism, bursitis, tenosynomitis, epicondylitis (tennis elbow), carpal tunnel syndrome, repetitive use injury (typing), miscellaneous forms of arthritis, neuropathic joint disease (charco and joint), hemarthrosis (hemarthrosic), Henoch-Schonlein Purpura, hypertrophic osteoarthropathy, multicentric reticulohistiocytosis, arthritis associated with certain diseases, surcoilosis, hemochromatosis, sickle cell disease and other hemoglobinopathries, hyperlipoproteineimia, hypogammaglobulinemia, hyperparathyroidism, acromegaly, familial Mediterranean fever, Behat's Disease, systemic lupus erythrematosis, or relapsing polychondritis; and related diseases.

**19.** The use of a compound of Formula I as claimed in any one of Claims 2 to 4 for the manufacture of a medicament for treating pathological effects of septic shock, adult respiratory distress syndrome, pancreatitis, trauma-induced shock, bronchial asthma, allergic rhinitis, rheumatoid arthritis, cystic fibrosis, stroke, acute bronchitis, chronic bronchitis, acute bronchiolitis, chronic bronchiolitis, osteoarthritis, gout, spondylarthropathris, ankylosing spondylitis, Reiter's syndrome, psoriatic arthropathy, enterapathric spondylitis, Juvenile arthropathy or juvenile ankylosing spondylitis, Reactive arthropathy, infectious or post-infectious arthritis, gonoccocal arthritis, Tuberculous arthritis, viral arthritis, fungal arthritis, syphilitic arthritis, Lyme disease, arthritis associated with "vasculitic syndromes", polyarteritis nodosa, hypersensitivity vasculitis, Luegenec's granulomatosis, polymyalgin rheumatica, joint cell arteritis, calcium crystal deposition arthropathris, pseudo gout, non-articular rheumatism, bursitis, tenosynomitis, epicondylitis (tennis elbow), carpal tunnel syndrome, repetitive use injury (typing), miscellaneous forms of arthritis, neuropathic joint disease (charco and joint), hemarthrosis (hemarthrosic), Henoch-Schonlein Purpura, hypertrophic osteoarthropathy, multicentric reticulohistiocytosis, arthritis associated with certain diseases, surcoilosis, hemochromatosis, sickle cell disease and other hemoglobinopathries, hyperlipoproteineimia, hypogammaglobulinemia, hyperparathyroidism, acromegaly, familial Mediterranean fever, Behat's Disease, systemic lupus erythrematosis, or relapsing polychondritis; and related diseases.

**20.** A pharmaceutical formulation adapted for the treatment of pathological effects of septic shock, adult respiratory distress syndrome, pancreatitis, trauma-induced shock, bronchial asthma, allergic rhinitis, rheumatoid arthritis, cystic fibrosis, stroke, acute bronchitis, chronic bronchitis, acute bronchiolitis, chronic bronchiolitis, osteoarthritis, gout, spondylarthropathris, ankylosing spondylitis, Reiter's syndrome, psoriatic arthropathy, enterapathric spondylitis, Juvenile arthropathy or juvenile ankylosing spondylitis, Reactive arthropathy, infectious or post-infectious arthritis, gonoccocal arthritis, Tuberculous arthritis, viral arthritis, fungal arthritis, syphilitic arthritis, Lyme disease, arthritis associated with "vasculitic syndromes", polyarteritis nodosa, hypersensitivity vasculitis, Luegenec's granulomatosis, polymyalgin rheumatica, joint cell arteritis, calcium crystal deposition arthropathris, pseudo gout, non-articular rheumatism, bursitis, tenosynomitis, epicondylitis (tennis elbow), carpal tunnel syndrome, repetitive use injury (typing), miscellaneous forms of arthritis, neuropathic joint disease (charco and joint), hemarthrosis (hemarthrosic), Henoch-Schonlein Purpura, hypertrophic osteoarthropathy, multicentric reticulohistiocytosis, arthritis associated with certain diseases, surcoilosis, hemochromatosis, sickle cell disease and other hemoglobinopathries, hyperlipoproteineimia, hypogammaglobulinemia, hyperparathyroidism, acromegaly, familial Mediterranean fever, Behat's Disease, systemic lupus erythrematosis, or relapsing polychondritis; and related diseases containing a compound of Formula III as claimed in Claim 1.

**21.** The use as in any one of Claims 7 to 10 wherein the medicament is for alleviating the pathological effects of septic shock, adult respiratory distress syndrome, pancreatitis, trauma-induced shock, bronchial asthma, allergic rhinitis, rheumatoid arthritis, cystic fibrosis, stroke, acute bronchitis, chronic bronchitis, acute bronchiolitis, chronic bronchiolitis, osteoarthritis, gout, spondylarthropathris, ankylosing spondylitis, Reiter's syndrome, psoriatic arthropathy, enterapathric spondylitis, Juvenile arthropathy or juvenile ankylosing spondylitis, Reactive arthropathy, infectious or post-infectious arthritis, gonoccocal arthritis, Tuberculous arthritis, viral arthritis, fungal arthritis, syphilitic arthritis, Lyme disease, arthritis associated with "vasculitic syndromes", polyarteritis nodosa, hypersensitivity vasculitis, Luegenec's granulomatosis, polymyalgin rheumatica, joint cell arteritis, calcium crystal deposition arthropathris, pseudo gout, non-articular rheumatism, bursitis, tenosynomitis, epicondylitis (tennis elbow), carpal tunnel syndrome, repetitive use injury (typing), miscellaneous forms of arthritis, neuropathic joint disease (charco and joint), hemarthrosis (hemarthrosic), Henoch-Schonlein Purpura, hypertrophic osteoarthropathy, multicentric reticulohistiocytosis, arthritis associated with certain diseases, surcoilosis, hemochromatosis, sickle cell disease and other hemoglobinopathries, hyperlipoproteineimia, hypogammaglobulinemia, hyperparathyroidism, acromegaly, familial Mediterranean fever, Behat's Disease, systemic lupus erythrematosis, or relapsing polychondritis; and related diseases.

## Patentansprüche

**1.** Verbindung der Formel (III)

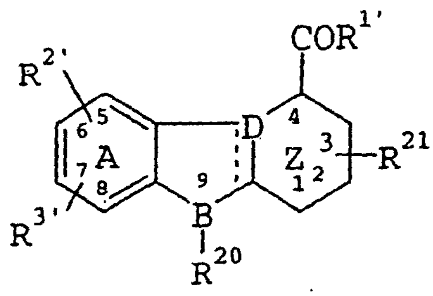

(III)

worin

A für Phenyl oder Pyridyl steht, worin der Stickstoff an der Position 5, 6, 7 oder 8 ist,
eines von B oder D für Stickstoff und das andere für Kohlenstoff steht,
Z für Cyclohexenyl, Phenyl, Pyridyl, worin der Stickstoff an der Position 1, 2 oder 3 ist, oder einen sechsgliedrigen heterocyclischen Ring mit einem Heteroatom steht, ausgewählt aus der Gruppe, die aus Schwefel oder Sauerstoff an der Position 1, 2 oder 3 und Stickstoff an der Position 1, 2, 3 oder 4 besteht,
----- für eine Doppel- oder Einfachbindung steht,
$R^{20}$ aus den Gruppen (a), (b) und (c) ausgewählt ist, worin

(a) besteht aus -($C_5$-$C_{20}$) Alkyl, -($C_5$-$C_{20}$) Alkenyl, -($C_5$-$C_{20}$) Alkinyl, carbocyclischen Resten, die von einer gesättigten oder ungesättigten, substituierten oder unsubstituierten fünf- bis vierzehngliedrigen organischen Kernstruktur abgeleitet sind, deren ringbildende Atome (die nicht Wasserstoff sind) nur Kohlenstoffatome sind oder heterocyclische Reste, die von monocyclischen oder polycyclischen, gesättigten oder ungesättigten, substituierten oder unsubstituierten heterocyclischen Kernstrukturen mit 5 bis 14 Ringatomen abgeleitet sind und die 1 bis 3 Heteroatome enthalten, ausgewählt aus der Gruppe, die besteht aus Stickstoff, Sauerstoff oder Schwefel, oder
(b) ein Vertreter von (a) ist, der mit einem oder mehreren Substituenten substituiert ist, unabhängig ausgewählt aus Wasserstoff, -($C_1$-$C_{12}$) Alkyl, -($C_2$-$C_6$) Alkenyl, -($C_2$-$C_6$) Alkinyl, -($C_7$-$C_{12}$) Aralkyl, -($C_7$-$C_{12}$) Alkaryl, -($C_3$-$C_8$) Cycloalkyl, -($C_3$-$C_8$) Cycloalkenyl, Phenyl, Tolulyl, Xylenyl, Biphenyl, -($C_1$-$C_6$) Alkoxy, -($C_2$-$C_6$) Alkenyloxy, -($C_2$-$C_6$) Alkinyloxy, -($C_1$-$C_{12}$) Alkoxyalkyl, -($C_1$-$C_{12}$) Alkoxyalkyloxy, -($C_1$-$C_{12}$) Alkylcarbonyl, -($C_1$-$C_{12}$) Alkylcarbonylamino, -($C_1$-$C_{12}$) Alkoxyamino, -($C_1$-$C_{12}$) Alkoxyaminocarbonyl, -($C_1$-$C_{12}$) Alkylamino, -($C_1$-$C_6$) Alkylthio, -($C_1$-$C_{12}$) Alkylthiocarbonyl, -($C_1$-$C_6$) Alkylsulfinyl, -($C_1$-$C_6$) Alkylsulfonyl, -($C_1$-$C_6$) Halogenalkoxy, -($C_1$-$C_6$) Halogenalkylsulfonyl, -($C_1$-$C_6$) Halogenalkyl, -($C_1$-$C_6$) Hydroxyalkyl, -$(CH_2)_n$CN, -$(CH_2)_n NR^9R^{10}$, -C(O)O($C_1$-$C_6$ Alkyl), -$(CH_2)_n$O($C_1$-$C_6$ Alkyl), Benzyloxy, Phenoxy, Phenylthio, -($CONHSO_2R$), -CHO, Amino, Amidino, Halogen, Carbamyl, Carboxyl, Carbalkoxy, -$(CH_2)_nCO_2H$, Cyano, Cyanoguanidinyl, Guanidino, Hydrazid, Hydrazino, Hydrazido, Hydroxy, Hydroxyamino, Nitro, Phosphono, -$SO_3H$, Thioacetal, Thiocarbonyl und ($C_1$-$C_6$) Alkylcarbonyl, worin n für 1 bis 8 steht und $R^9$ und $R^{10}$ unabhängig für -($C_1$-$C_4$) Alkyl oder Phenyl-($C_1$-$C_4$)-alkyl stehen, oder
(c) die Gruppe -(L)-$R^{80}$ ist, worin -(L)- für eine divalente Verbindungsgruppe von 1 bis 12 Atomen steht, ausgewählt aus Kohlenstoff, Wasserstoff, Sauerstoff, Stickstoff und Schwefel, worin die Kombinationen an Atomen in -(L)- ausgewählt sind aus der Gruppe, die besteht aus (i) nur Kohlenstoff und Wasserstoff, (ii) nur einem Schwefel, (iii) nur einem Sauerstoff, (iv) nur ein oder zwei Stickstoffe und Wasserstoff, (v) nur Kohlenstoff, Wasserstoff und ein Schwefel und (vi) nur Kohlenstoff, Wasserstoff und Sauerstoff, und worin $R^{80}$ für eine Gruppe steht, die ausgewählt ist aus (a) oder (b),

$R^{21}$ ausgewählt ist aus Wasserstoff, -($C_1$-$C_{12}$) Alkyl, -($C_2$-$C_6$) Alkenyl, -($C_2$-$C_6$) Alkinyl, -($C_7$-$C_{12}$) Aralkyl, -($C_7$-$C_{12}$) Alkaryl, -($C_3$-$C_8$) Cycloalkyl, -($C_3$-$C_8$) Cycloalkenyl, Phenyl, Tolulyl, Xylenyl, Biphenyl, -($C_1$-$C_6$) Alkoxy, -($C_2$-$C_6$) Alkenyloxy, -($C_2$-$C_6$) Alkinyloxy, -($C_1$-$C_{12}$) Alkoxyalkyl, -($C_1$-$C_{12}$) Alkoxyalkyloxy, -($C_1$-$C_{12}$) Alkylcarbonyl, -($C_1$-$C_{12}$) Alkylcarbonylamino, -($C_1$-$C_{12}$) Alkoxyamino, -($C_1$-$C_{12}$) Alkoxyaminocarbonyl, -($C_1$-$C_{12}$) Alkylamino, -($C_1$-$C_6$) Alkylthio, -($C_1$-$C_{12}$) Alkylthiocarbonyl, -($C_1$-$C_6$) Alkylsulfinyl, -($C_1$-$C_6$) Alkylsulfonyl, -($C_1$-$C_6$) Halogenalkoxy, -($C_1$-$C_6$) Halogenalkylsulfonyl, -($C_1$-$C_6$) Halogenalkyl, -($C_1$-$C_6$) Hydroxyalkyl, -$(CH_2)_n$CN, -$(CH_2)_n NR^9R^{10}$, -C(O)O($C_1$-$C_6$ Alkyl), -$(CH_2)_n$O($C_1$-$C_6$ Alkyl), Benzyloxy, Phenoxy, Phenylthio, -($CONHSO_2R$), -CHO, Amino, Amidino, Halogen, Carbamyl, Carboxyl, Carbalkoxy, -$(CH_2)_nCO_2H$, Cyano, Cyanoguanidinyl, Guanidino, Hydrazid, Hydrazino, Hydrazido, Hydroxy, Hydroxyamino, Nitro, Phosphono, -$SO_3H$, Thioacetal, Thiocarbonyl und ($C_1$-$C_6$) Alkylcarbonyl, worin n für 1 bis 8 steht und $R^9$ und $R^{10}$ unab-

hängig für $-(C_1-C_4)$ Alkyl oder Phenyl-$(C_1-C_4)$-alkyl stehen,

$R^{1'}$ für $-NHNH_2$ oder $-NH_2$ steht,

$R^{2'}$ ausgewählt ist aus der Gruppe -OH, $-O(CH_2)_tR^5$, worin $R^5$ für CN oder Phenyl steht, oder $-(L_a)$-(saure Gruppe), worin die saure Gruppe eine organische Gruppe ist, die bei einer Bindung an eine tricyclische Kernstruktur durch geeignete Bindeatome als Protonendonor wirkt, der zur Wasserstoffbindung fähig ist und $-(L_a)$- für eine divalente Bindegruppe mit einem Linker mit einer Länge von 1 bis 7 steht und t für 1 bis 5 steht,

$R^{3'}$ ausgewählt ist aus Wasserstoff, $-(C_1-C_{12})$ Alkyl, $-(C_2-C_6)$ Alkenyl, $-(C_2-C_6)$ Alkinyl, $-(C_7-C_{12})$ Aralkyl, $-(C_7-C_{12})$ Alkaryl, $-(C_3-C_8)$ Cycloalkyl, $-(C_3-C_8)$ Cycloalkenyl, Phenyl, Tolulyl, Xylenyl, Biphenyl, $-(C_1-C_6)$ Alkoxy, $-(C_2-C_6)$ Alkenyloxy, $-(C_2-C_6)$ Alkinyloxy, $-(C_1-C_{12})$ Alkoxyalkyl, $-(C_1-C_{12})$ Alkoxyalkyloxy, $-(C_1-C_{12})$ Alkylcarbonyl, $-(C_1-C_{12})$ Alkylcarbonylamino, $-(C_1-C_{12})$ Alkoxyamino, $-(C_1-C_{12})$ Alkoxyaminocarbonyl, $-(C_1-C_{12})$ Alkylamino, $-(C_1-C_6)$ Alkylthio, $-(C_1-C_{12})$ Alkylthiocarbonyl, $-(C_1-C_6)$ Alkylsulfinyl, $-(C_1-C_6)$ Alkylsulfonyl, $-(C_1-C_6)$ Halogenalkoxy, $-(C_1-C_6)$ Halogenalkylsulfonyl, $-(C_1-C_6)$ Halogenalkyl, $-(C_1-C_6)$ Hydroxyalkyl, $-(CH_2)_nCN$, $-(CH_2)_nNR^9R^{10}$, $-C(O)O(C_1-C_6$ Alkyl), $-(CH_2)_nO(C_1-C_6$ Alkyl), Benzyloxy, Phenoxy, Phenylthio, $-(CONHSO_2R)$, -CHO, Amino, Amidino, Halogen, Carbamyl, Carboxyl, Carbalkoxy, $-(CH_2)_nCO_2H$, Cyano, Cyanoguanidinyl, Guanidino, Hydrazid, Hydrazino, Hydrazido, Hydroxy, Hydroxyamino, Nitro, Phosphono, $-SO_3H$, Thioacetal, Thiocarbonyl und $(C_1-C_6)$ Alkylcarbonyl, worin n für 1 bis 8 steht und $R^9$ und $R^{10}$ unabhängig für $-(C_1-C_4)$ Alkyl oder Phenyl-$(C_1-C_4)$-alkyl stehen, carbocyclischen Resten, die von einer gesättigten oder ungesättigten fünf- bis vierzehngliedrigen organischen Kernstruktur abgeleitet sind, deren ringbildende Atome (die nicht Wasserstoff sind) nur Kohlenstoffatome sind oder heterocyclische Reste, die von monocyclischen oder polycyclischen, gesättigten oder ungesättigten, heterocyclischen Kernstrukturen mit 5 bis 14 Ringatomen abgeleitet sind und die 1 bis 3 Heteroatome enthalten, ausgewählt aus der Gruppe, die besteht aus Stickstoff, Sauerstoff oder Schwefel, und carbocyclischen Resten, die von einer gesättigten oder ungesättigten, fünf- bis vierzehngliedrigen organischen Kernstruktur abgeleitet sind, deren ringbildende Atome (die nicht Wasserstoff sind) nur Kohlenstoffatome sind oder heterocyclische Reste, die von monocyclischen oder polycyclischen, gesättigten oder ungesättigten heterocyclischen Kernstrukturen mit 5 bis 14 Ringatomen abgeleitet sind und die 1 bis 3 Heteroatome enthalten, ausgewählt aus der Gruppe, die besteht aus Stickstoff, Sauerstoff oder Schwefel, welche mit Gruppen substituiert sind, ausgewählt aus Wasserstoff, $-(C_1-C_{12})$ Alkyl, $-(C_2-C_6)$ Alkenyl, $-(C_2-C_6)$ Alkinyl, $-(C_7-C_{12})$ Aralkyl, $-(C_7-C_{12})$ Alkaryl, $-(C_3-C_8)$ Cycloalkyl, $-(C_3-C_8)$ Cycloalkenyl, Phenyl, Tolulyl, Xylenyl, Biphenyl, $-(C_1-C_6)$ Alkoxy, $-(C_2-C_6)$ Alkenyloxy, $-(C_2-C_6)$ Alkinyloxy, $-(C_1-C_{12})$ Alkoxyalkyl, $-(C_1-C_{12})$ Alkoxyalkyloxy, $-(C_1-C_{12})$ Alkylcarbonyl, $-(C_1-C_{12})$ Alkylcarbonylamino, $-(C_1-C_{12})$ Alkoxyamino, $-(C_1-C_{12})$ Alkoxyaminocarbonyl, $-(C_1-C_{12})$ Alkylamino, $-(C_1-C_6)$ Alkylthio, $-(C_1-C_{12})$ Alkylthiocarbonyl, $-(C_1-C_6)$ Alkylsulfinyl, $-(C_1-C_6)$ Alkylsulfonyl, $-(C_1-C_6)$ Halogenalkoxy, $-(C_1-C_6)$ Halogenalkylsulfonyl, $-(C_1-C_6)$ Halogenalkyl, $-(C_1-C_6)$ Hydroxyalkyl, $-(CH_2)_nCN$, $-(CH_2)_nNR^9R^{10}$, $-C(O)O(C_1-C_6$ Alkyl), $-(CH_2)_n$ $(C_1-C_6$ Alkyl), Benzyloxy, Phenoxy, Phenylthio, $-(CONHSO_2R)$, -CHO, Amino, Amidino, Halogen, Carbamyl, Carboxyl, Carbalkoxy, $-(CH_2)_nCO_2H$, Cyano, Cyanoguanidinyl, Guanidino, Hydrazid, Hydrazino, Hydrazido, Hydroxy, Hydroxyamino, Nitro, Phosphono, $-SO_3H$, Thioacetal, Thiocarbonyl und $(C_1-C_6)$ Alkylcarbonyl, worin n für 1 bis 8 steht und $R^9$ und $R^{10}$ unabhängig für $-(C_1-C_4)$ Alkyl oder Phenyl-$(C_1-C_4)$-alkyl stehen,

oder ein pharmazeutisch annehmbares Salz, Razemat, Solvat, Tautomer oder optisches Isomer hiervon,

mit der Maßgabe, daß wenn D für Stickstoff steht, das Heteroatom von Z dann aus der Gruppe ausgewählt ist, die besteht aus Schwefel oder Sauerstoff an Position 1, 2 oder 3 und Stickstoff an der Position 1, 2, 3 oder 4.

**2.** Verbindung der Formel (I)

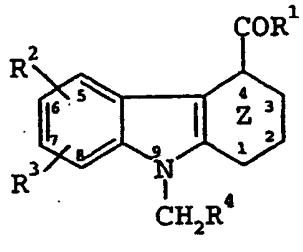

(I)

worin

$R^1$ für $-NHNH_2$ oder $-NH_2$ steht,

$R^2$ für -OH oder $-O(CH_2)_mR^5$ steht, worin $R^5$ für H, $-CO_2H$, $-CO_2(C_1-C_4$ Alkyl),

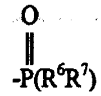

Phenyl oder Phenyl steht, das substituiert ist mit $-CO_2H$ oder $-CO_2(C_1-C_4$ Alkyl), worin $R^6$ und $R^7$ jeweils unabhängig für -OH oder $-O(C_1-C_4$ Alkyl) stehen und m für 1 bis 3 steht,
$R^3$ für H, $-O(C_1-C_4$ Alkyl) oder $-(CH_2)_nR^8$ steht, worin $R^8$ für H, $-NR^9R^{10}$,

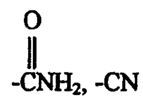

oder Phenyl steht, worin $R^9$ und $R^{10}$ unabhängig für $-(C_1-C_4)$ Alkyl oder Phenyl-$(C_1-C_4)$-alkyl stehen und n für 1 bis 8 steht,
$R^4$ für H, $-(C_5-C_{14})$ Alkyl, $-(C_3-C_{14})$ Cycloalkyl, Phenyl oder Phenyl steht, das substituiert ist mit 1 oder 2 Substituenten, ausgewählt aus der Gruppe, die besteht aus $-(C_1-C_4)$ Alkyl, $-(C_1-C_4)$ Alkoxy, Phenyl-$(C_1-C_4)$-alkyl, $-(C_1-C_4)$ Alkylthio, Halogen oder Phenyl, und
2 für Cyclohexenyl oder Phenyl steht,
oder ein pharmazeutisch annehmbares Salz, Razemat, optisches Isomer, Solvat oder Tautomer hiervon,
mit der Maßgabe, daß wenn $R^3$ für H steht, $R^4$ für Phenyl steht, m für 1 oder 2 steht und $R^2$ an der Position 6 substituiert ist, $R^5$ dann nicht für H stehen kann, und wenn $R^1$ für $NHNH_2$ steht, $R^8$ nicht für

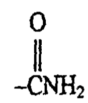

stehen kann und wenn $R^1$ für $NH_2$ steht, $R^2$ an der Position 6 OMe substituiert ist und $R^3$ für H steht, $R^4$ dann nicht für H stehen kann.

**3.** Verbindung der Formel I nach Anspruch 2, worin

$R^1$ für $-NHNH_2$ oder $NH_2$ steht,
$R^2$ für $-O(CH_2)_mR^5$ steht, worin $R^5$ für $-CO_2H$ oder

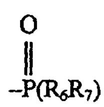

steht, worin $R^6$ und $R^7$ für -OH stehen,
$R^3$ für H, $-O(C_1-C_4$ Alkyl) oder $-(CH_2)_nR^8$ steht, worin $R^8$ für H oder Phenyl steht,
$R^4$ für Phenyl steht, und
Z für Cyclohexen steht.

**4.** Verbindung nach Anspruch 3, die folgende ist 4-[(9-Benzyl-4-carbamoyl-1,2,3,4-tetrahydrocarbazol-6-yl]oxy]buttersäure, 3-[(9-Benzyl-4-carbamoyl-1,2,3,4-tetrahydrocarbazol-6-yl]oxy]propylphosphonsäure, 2-[(9-Benzyl-4-carbamoyl-1,2,3,4-tetrahydrocarbazol-6-yl]oxy]methylbenzoesäure, 3-[(9-Benzyl-4-carbamoyl-7-n-octyl-1,2,3,4-tetrahydrocarbazol-6-yl]oxy]propylphosphonsäure, 4-[(9-Benzyl-4-carbamoyl-7-ethyl-1,2,3,4-tetrahydrocarbazol-6-yl]oxy]buttersäure, 3-[(9-Benzyl-4-carbamoyl-7-ethyl-1,2,3,4-tetrahydrocarbazol-6-yl]oxy]propylphosphonsäure, 3-[(9-Benzyl-4-carbamoyl-7-ethyl-1,2,3,4-tetrahydrocarbazol-6-yl]oxy]propylphosphonsäure, (S)-(+)-4-[(9-Benzyl-4-carbamoyl-7-ethyl-1,2,3,4-tetrahydrocarbazol-6-yl]oxy]buttersäure, 4-[9-Benzyl-4-carbamoyl-6-(2-cyanoethyl)-1,2,3,4-tetrahydrocarbazol-6-yl]oxybuttersäure, 4-[9-Benzyl-4-carboxamido-7-(2-phenylethyl)-1,2,3,4-tetrahydrocarbazol-6-yl]oxybuttersäure, 4-[9-Benzyl-4-carboxamidocarbazol-6-yl]oxybuttersäure, Methyl-2-[(9-Benzyl-4-carbamoyl-1,2,3,4-tetrahydrocarbazol-6-yl]oxy]methylbenzoat, 9-Benzyl-5,7-dimethoxy-1,2,3,4-tetrahydrocarbazol-4-carbonsäurehydrazid, 9-Benzyl-5,7-dimethoxy-1,2,3,4-tetrahydrocarbazol-4-carboxamid, [9-Benzyl-4-carbamoyl-7-methoxy-1,2,3,4-tetrahydrocarbazol-5-yl]oxyessigsäure-Natriumsalz, 4-[9-Benzyl-4-carbamoyl-7-(2-cyanoethyl)-1,2,3,4-tetrahydrocarbazol-6-yl]oxybuttersäure, [9-Benzyl-4-carbamoyl-7-methoxycarbazol-5-yl]oxyessigsäure, Methyl-[9-Benzyl-4-carbamoyl-7-methoxycarbazol-5-yl]oxyessigsäure, 9-Benzyl-7-methoxy-5-cyanomethyloxy-1,2,3,4-tetrahydrocarbazol-4-carboxamid, 9-Benzyl-7-methoxy-5-(1H-tetrazol-5-yl-methyl)oxy)-1,2,3,4-tetrahydrocarbazol-4-carboxamid, [9-Benzyl-4-carbamoyl-8-methyl-1,2,3,4-tetrahydrocarbazol-5-yl]oxyessigsäure, [9-Benzyl-4-carbamoyl-8-methylcarbazol-5-yl]oxyessigsäure, [9-Benzyl-4-carbamoyl-1,2,3,4-tetrahydrocarbazol-5-yl]oxyessigsäure, und [9-Benzyl-4-carbamoylcarbazol-5-yl] oxyessigsäure, oder ein pharmazeutisch annehmbares Salz, Razemat, Solvat, Tautomer oder optisches Isomer hiervon.

5. Pharmazeutische Formulierung, die eine Verbindung der Formel III nach Anspruch 1 zusammen mit einem pharmazeutisch annehmbaren Träger oder Verdünnungsmittel hierfür enthält.

6. Pharmazeutische Formulierung, die eine Verbindung der Formel I nach Anspruch 2 zusammen mit einem pharmazeutisch annehmbaren Träger oder Verdünnungsmittel hierfür enthält.

7. Verwendung zur Herstellung eines Arzneimittels zur selektiven Hemmung der $sPLA_2$ bei einem Säuger, der einer solchen Behandlung mit einer Verbindung der Formel (III) bedarf

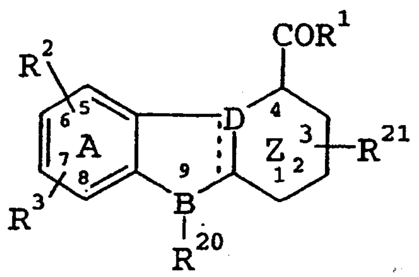

(III)

worin

A für Phenyl oder Pyridyl steht,
B und D jeweils unabhängig für Stickstoff oder Kohlenstoff stehen,
Z für Cyclohexenyl, Phenyl, Pyridyl, worin der Stickstoff an der Position 1, 2 oder 3 ist, oder einen sechsgliedrigen heterocyclischen Ring mit einem Heteroatom steht, ausgewählt aus der Gruppe, die aus Schwefel oder Sauerstoff an der Position 1, 2 oder 3 und Stickstoff an der Position 1, 2, 3 oder 4 besteht,
----- für eine Doppel- oder Einfachbindung steht,
$R^{20}$ aus den Gruppen (a), (b) und (c) ausgewählt ist, worin

(a) besteht aus $-(C_5-C_{20})$ Alkyl, $-(C_5-C_{20})$ Alkenyl, $-(C_5-C_{20})$ Alkinyl, carbocyclischen Resten, die von einer gesättigten oder ungesättigten, substituierten oder unsubstituierten fünf- bis vierzehngliedrigen organischen Kernstruktur abgeleitet sind, deren ringbildende Atome (die nicht Wasserstoff sind) nur Kohlenstoffatome sind oder heterocyclische Reste, die von monocyclischen oder polycyclischen, gesättigten oder ungesättigten, substituierten oder unsubstituierten heterocyclischen Kernstrukturen mit 5 bis 14 Ringatomen abgeleitet sind und die 1 bis 3 Heteroatome enthalten, ausgewählt aus der Gruppe, die besteht aus Stickstoff, Sauerstoff oder Schwefel, oder
(b) ein Vertreter von (a) ist, der mit einem oder mehreren Substituenten substituiert ist, unabhängig ausgewählt aus Wasserstoff, $-(C_1-C_{12})$ Alkyl, $-(C_2-C_6)$ Alkenyl, $-(C_2-C_6)$ Alkinyl, $-(C_7-C_{12})$ Aralkyl, $-(C_7-C_{12})$ Alkaryl, $-(C_3-C_8)$ Cycloalkyl, $-(C_3-C_8)$ Cycloalkenyl, Phenyl, Tolulyl, Xylenyl, Biphenyl, $-(C_1-C_6)$ Alkoxy, $-(C_2-C_6)$ Alkenyloxy, $-(C_2-C_6)$ Alkinyloxy, $-(C_1-C_{12})$ Alkoxyalkyl, $-(C_1-C_{12})$ Alkoxyalkyloxy, $-(C_1-C_{12})$ Alkylcarbonyl, $-(C_1-C_{12})$ Alkylcarbonylamino, $-(C_1-C_{12})$ Alkoxyamino, $-(C_1-C_{12})$ Alkoxyaminocarbonyl, $-(C_1-C_{12})$ Alkylamino, $-(C_1-C_6)$ Alkylthio, $-(C_1-C_{12})$ Alkylthiocarbonyl, $-(C_1-C_6)$ Alkylsulfinyl, $-(C_1-C_6)$ Alkylsulfonyl, $-(C_1-C_6)$ Halogenalkoxy, $-(C_1-C_6)$ Halogenalkylsulfonyl, $-(C_1-C_6)$ Halogenalkyl, $-(C_1-C_6)$ Hydroxyalkyl, $-(CH_2)_nCN$, $-(CH_2)_nNR^9R^{10}$, $-C(O)O(C_1-C_6$ Alkyl), $-(CH_2)_nO(C_1-C_6$ Alkyl), Benzyloxy, Phenoxy,

Phenylthio, -($CONHSO_2R$), -CHO, Amino, Amidino, Halogen, Carbamyl, Carboxyl, Carbalkoxy, -$(CH_2)_nCO_2H$, Cyano, Cyanoguanidinyl, Guanidino, Hydrazid, Hydrazino, Hydrazido, Hydroxy, Hydroxyamino, Nitro, Phosphono, -$SO_3H$, Thioacetal, Thiocarbonyl und ($C_1$-$C_6$) Alkylcarbonyl, worin n für 1 bis 8 steht und $R^9$ und $R^{10}$ unabhängig für -($C_1$-$C_4$) Alkyl oder Phenyl-($C_1$-$C_4$)-alkyl stehen, oder
(c) die Gruppe -(L)-$R^{80}$ ist, worin -(L)- für eine divalente Verbindungsgruppe von 1 bis 12 Atomen steht, ausgewählt aus Kohlenstoff, Wasserstoff, Sauerstoff, Stickstoff und Schwefel, worin die Kombinationen an Atomen in -(L)- ausgewählt sind aus der Gruppe, die besteht aus (i) nur Kohlenstoff und Wasserstoff, (ii) nur einem Schwefel, (iii) nur einem Sauerstoff, (iv) nur ein oder zwei Stickstoffe und Wasserstoff, (v) nur Kohlenstoff, Wasserstoff und ein Schwefel und (vi) nur Kohlenstoff, Wasserstoff und Sauerstoff, worin $R^{80}$ für eine Gruppe steht, die ausgewählt ist aus (a) oder (b),

$R^{21}$ ausgewählt ist aus Wasserstoff, -($C_1$-$C_{12}$) Alkyl, -($C_2$-$C_6$) Alkenyl, -($C_2$-$C_6$) Alkinyl, -($C_7$-$C_{12}$) Aralkyl, -($C_7$-$C_{12}$) Alkaryl, -($C_3$-$C_8$) Cycloalkyl, -($C_3$-$C_8$) Cycloalkenyl, Phenyl, Tolulyl, Xylenyl, Biphenyl, -($C_1$-$C_6$) Alkoxy, -($C_2$-$C_6$) Alkenyloxy, -($C_2$-$C_6$) Alkinyloxy, -($C_1$-$C_{12}$) Alkoxyalkyl, -($C_1$-$C_{12}$) Alkoxyalkyloxy, -($C_1$-$C_{12}$) Alkylcarbonyl, -($C_1$-$C_{12}$) Alkylcarbonylamino, -($C_1$-$C_{12}$) Alkoxyamino, -($C_1$-$C_{12}$) Alkoxyaminocarbonyl, -($C_1$-$C_{12}$) Alkylamino, -($C_1$-$C_6$) Alkylthio, -($C_1$-$C_{12}$) Alkylthiocarbonyl, -($C_1$-$C_6$) Alkylsulfinyl, -($C_1$-$C_6$) Alkylsulfonyl, -($C_1$-$C_6$) Halogenalkoxy, -($C_1$-$C_6$) Halogenalkylsulfonyl, -($C_1$-$C_6$) Halogenalkyl, -($C_1$-$C_6$) Hydroxyalkyl, -$(CH_2)_nCN$, -$(CH_2)_nNR^9R^{10}$, -C(O)O($C_1$-$C_6$ Alkyl), -$(CH_2)_nO$($C_1$-$C_6$ Alkyl), Benzyloxy, Phenoxy, Phenylthio, -($CONHSO_2R$), -CHO, Amino, Amidino, Halogen, Carbamyl, Carboxyl, Carbalkoxy, -$(CH_2)_nCO_2H$, Cyano, Cyanoguanidinyl, Guanidino, Hydrazid, Hydrazino, Hydrazido, Hydroxy, Hydroxyamino, Nitro, Phosphono, -$SO_3H$, Thioacetal, Thiocarbonyl und ($C_1$-$C_6$) Alkylcarbonyl, worin n für 1 bis 8 steht und $R^9$ und $R^{10}$ unabhängig für -($C_1$-$C_4$) Alkyl oder Phenyl-($C_1$-$C_4$)-alkyl stehen,
$R^{1'}$ für -$NHNH_2$ oder -$NH_2$ steht,
$R^{2'}$ ausgewählt ist aus der Gruppe -OH, -$O(CH_2)_t(R^5$, worin $R^5$ für CN oder Phenyl steht, oder -($L_a$)-(saure Gruppe), worin die saure Gruppe eine organische Gruppe ist, die bei einer Bindung an eine tricyclische Kernstruktur durch geeignete Bindeatome als Protonendonor wirkt, der zur Wasserstoffbindung fähig ist und -($L_a$)- für eine divalente Bindegruppe mit einem Linker mit einer Länge von 1 bis 7 steht und t für 1 bis 5 steht,
$R^{3'}$ ausgewählt ist aus Wasserstoff, -($C_1$-$C_{12}$) Alkyl, -($C_2$-$C_6$) Alkenyl, -($C_2$-$C_6$) Alkinyl, -($C_7$-$C_{12}$) Aralkyl, -($C_7$-$C_{12}$) Alkaryl, -($C_3$-$C_8$) Cycloalkyl, -($C_3$-$C_8$) Cycloalkenyl, Phenyl, Tolulyl, Xylenyl, Biphenyl, -($C_1$-$C_6$) Alkoxy, -($C_2$-$C_6$) Alkenyloxy, -($C_2$-$C_6$) Alkinyloxy, -($C_1$-$C_{12}$) Alkoxyalkyl, -($C_1$-$C_{12}$) Alkoxyalkyloxy, -($C_1$-$C_{12}$) Alkylcarbonyl, -($C_1$-$C_{12}$) Alkylcarbonylamino, -($C_1$-$C_{12}$) Alkoxyamino, -($C_1$-$C_{12}$) Alkoxyaminocarbonyl, -($C_1$-$C_{12}$) Alkylamino, -($C_1$-$C_6$) Alkylthio, -($C_1$-$C_{12}$) Alkylthiocarbonyl, -($C_1$-$C_6$) Alkylsulfinyl, -($C_1$-$C_6$) Alkylsulfonyl, -($C_1$-$C_6$) Halogenalkoxy, -($C_1$-$C_6$) Halogenalkylsulfonyl, -($C_1$-$C_6$) Halogenalkyl, -($C_1$-$C_6$) Hydroxyalkyl, -$(CH_2)_nCN$, -$(CH_2)_nNR^9R^{10}$, -C(O)O($C_1$-$C_6$ Alkyl), -$(CH_2)_nO$($C_1$-$C_6$ Alkyl), Benzyloxy, Phenoxy, Phenylthio, -($CONHSO_2R$), -CHO, Amino, Amidino, Halogen, Carbamyl, Carboxyl, Carbalkoxy, -$(CH_2)_nCO_2H$, Cyano, Cyanoguanidinyl, Guanidino, Hydrazid, Hydrazino, Hydrazido, Hydroxy, Hydroxyamino, Nitro, Phosphono, -$SO_3H$, Thioacetal, Thiocarbonyl und ($C_1$-$C_6$) Alkylcarbonyl, worin n für 1 bis 8 steht und $R^9$ und $R^{10}$ unabhängig für -($C_1$-$C_4$) Alkyl oder Phenyl-($C_1$-$C_4$)-alkyl stehen, carbocyclischen Resten, die von einer gesättigten oder ungesättigten fünf- bis vierzehngliedrigen organischen Kernstruktur abgeleitet sind, deren ringbildende Atome (die nicht Wasserstoff sind) nur Kohlenstoffatome sind oder heterocyclische Reste, die von monocyclischen oder polycyclischen, gesättigten oder ungesättigten, heterocyclischen Kernstrukturen mit 5 bis 14 Ringatomen abgeleitet sind und die 1 bis 3 Heteroatome enthalten, ausgewählt aus der Gruppe, die besteht aus Stickstoff, Sauerstoff oder Schwefel, und carbocyclischen Resten, die von einer gesättigten oder ungesättigten, fünf- bis vierzehngliedrigen organischen Kernstruktur abgeleitet sind, deren ringbildende Atome (die nicht Wasserstoff sind) nur Kohlenstoffatome sind oder heterocyclische Reste, die von monocyclischen oder polycyclischen, gesättigten oder ungesättigten heterocyclischen Kernstrukturen mit 5 bis 14 Ringatomen abgeleitet sind und die 1 bis 3 Heteroatome enthalten, ausgewählt aus der Gruppe, die besteht aus Stickstoff, Sauerstoff oder Schwefel, welche mit Gruppen substituiert sind, ausgewählt aus Wasserstoff, -($C_1$-$C_{12}$) Alkyl, -($C_2$-$C_6$) Alkenyl, -($C_2$-$C_6$) Alkinyl, -($C_7$-$C_{12}$) Aralkyl, -($C_7$-$C_{12}$) Alkaryl, -($C_3$-$C_8$) Cycloalkyl, -($C_3$-$C_8$) Cycloalkenyl, Phenyl, Tolulyl, Xylenyl, Biphenyl, -($C_1$-$C_6$) Alkoxy, -($C_2$-$C_6$) Alkenyloxy, -($C_2$-$C_6$) Alkinyloxy, -($C_1$-$C_{12}$) Alkoxyalkyl, -($C_1$-$C_{12}$) Alkoxyalkyloxy, -($C_1$-$C_{12}$) Alkylcarbonyl, -($C_1$-$C_{12}$) Alkylcarbonylamino, -($C_1$-$C_{12}$) Alkoxyamino, -($C_1$-$C_{12}$) Alkoxyaminocarbonyl, -($C_1$-$C_{12}$) Alkylamino, -($C_1$-$C_6$) Alkylthio, -($C_1$-$C_{12}$) Alkylthiocarbonyl, -($C_1$-$C_6$) Alkylsulfinyl, -($C_1$-$C_6$) Alkylsulfonyl, -($C_1$-$C_6$) Halogenalkoxy, -($C_1$-$C_6$) Halogenalkylsulfonyl, -($C_1$-$C_6$) Halogenalkyl, -($C_1$-$C_6$) Hydroxyalkyl, -$(CH_2)_nCN$, -$(CH_2)_nNR^9R^{10}$, -C(O)O($C_1$-$C_6$ Alkyl), -$(CH_2)_nO$ ($C_1$-$C_6$ Alkyl), Benzyloxy, Phenoxy, Phenylthio, -($CONHSO_2R$), -CHO, Amino, Amidino, Halogen, Carbamyl, Carboxyl, Carbalkoxy, -$(CH_2)_nCO_2H$, Cyano, Cyanoguanidinyl, Guanidino, Hydrazid, Hydrazino, Hydrazido, Hydroxy, Hydroxyamino, Nitro, Phosphono, -$SO_3H$, Thioacetal, Thiocarbonyl und ($C_1$-$C_6$) Alkylcarbonyl, worin n für 1 bis 8 steht und $R^9$ und $R^{10}$ unabhängig für -($C_1$-$C_4$) Alkyl oder Phenyl-($C_1$-$C_4$)-alkyl stehen,

oder ein pharmazeutisch annehmbares Salz, Razemat, Solvat, Tautomer oder optisches Isomer hiervon.

**8.** Verwendung zur Herstellung eines Arzneimittels zur selektiven Hemmung der $sPLA_2$ bei einem Säuger, der einer solchen Behandlung mit einer Verbindung der Formel (II) bedarf

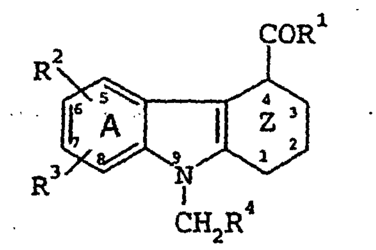

(II)

worin

$R^1$ für $-NHNH_2$ oder $-NH_2$ steht,
$R^2$ für -OH oder $-O(CH_2)_mR^5$ steht, worin $R^5$ für H, $-CO_2H$, $-CO_2(C_1-C_4$ Alkyl),

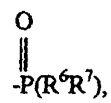

$-NHSO_2(C_1-C_6)$ Alkyl, $-CONHSO_2(C_1-C_6$ Alkyl), -CN, Tetrazolyl, Phenyl oder Phenyl steht, das substituiert ist mit $-CO_2H$ oder $-CO_2(C_1-C_4$ Alkyl), worin $R^6$ und $R^7$ jeweils unabhängig für -OH oder $-O(C_1-C_4$ Alkyl) stehen und m für 1 bis 3 steht,
$R^3$ für H, $-O(C_1-C_4$ Alkyl) oder $-(CH_2)_nR^8$ steht, worin $R^8$ für H, $-NR^9R^{10}$,

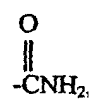

-CN oder Phenyl steht, worin $R^9$ und $R^{10}$ unabhängig für $-(C_1-C_4)$ Alkyl oder Phenyl-$(C_1-C_4)$-alkyl stehen und n für 1 bis 8 steht,
$R^4$ für H, $-(C_5-C_{14})$ Alkyl, $-(C_3-C_{14})$ Cycloalkyl, Phenyl oder Phenyl steht, das substituiert ist mit 1 oder 2 Substituenten, ausgewählt aus der Gruppe, die besteht aus $-(C_1-C_4)$ Alkyl, $-(C_1-C_4)$ Alkoxy, Phenyl-$(C_1-C_4)$-alkyl, $-(C_1-C_4)$ Alkylthio, Halogen oder Phenyl, und
Z für Cyclohexenyl, Phenyl, Pyridyl, worin der Stickstoff an der Position 1, 2, 3 oder 4 ist, oder einen sechsgliedrigen, heterocyclischen Ring mit einem Heteroatom steht, ausgewählt aus Schefel oder Sauerstoff an der Position 1, 2 oder 3 und Sticktsoff an der Position 1, 2, 3 oder 4, oder worin ein Kohlenstoff am heterocyclischen Ring wahlweise mit =O substituiert ist,
A für Phenyl oder Pyridyl steht, worin der Stickstoff an der Position 5, 6, 7 oder 8 ist,
oder ein pharmazeutisch annehmbares Salz, Razemat, optisches Isomer, Solvat oder Tautomer hiervon,
mit der Maßgabe, daß wenn $R^3$ für H steht, $R^4$ für Phenyl steht, m für 1 oder 2 steht und $R^2$ an der Position 6 substituiert ist, $R^5$ dann nicht für H stehen kann, und
wenn $R^1$ für $NHNH_2$ steht, $R^8$ nicht für

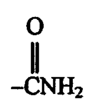

stehen kann.

**9.** Verwendung nach Anspruch 7, worin der Säuger ein Mensch ist.

**10.** Verwendung nach Anspruch 8, worin der Säuger ein Mensch ist.

**11.** Verwendung nach Anspruch 7, worin das Arzneimittel zur Linderung der pathologischen Effekte dient von septischem Schock, Atemstreßsyndrom beim Erwachsenen, Pankreatitis, Trauma-induziertern Schock, Bronchialasthma, allergischer Rhinitis, rheumatoider Arthritis, cystischer Fibrose, Schlaganfall, akuter Bronchitis, chronischer Bronchitis, akuter Broncheolitis, chronischer Broncheolitis, Osteoarthritis, Gicht, Spondylarthropathie, Spondylitis ankylosans, Reiter Syndrom, psoriatischer Arthropathie, enterapathischer Spondylitis, juveniler Arthropathie oder juveniler Spondylitis ankylosans, reaktiver Arthropathie, infektiöser oder postinfektiöser Arthritis, Gonokokkenarthritis, Tuberkulosearthritis, viraler Arthritis, pilzbedingter Arthritis, Syphilis-bedingter Arthritis, Lyme-Erkrankung, Arthritis, die mit "vaskulitischen Syndromen" assoziiert ist, Polyarteriitis nodosa, hyperempfindlicher Vaskulitis, Luegenec Granulomatose, Polymyalgia rheumatica, Gelenkszellarteriitis, Arthropathie durch Calciumkristallablagerung, Pseudogicht, nicht-artikuläres Rheuma, Bursitis, Tenosynovitis, Epicondylitis (Tennisellenbogen), Carpaltunnelsyndrom, Verletzung durch wiederkehrende Tätigkeit (Tippen), verschiedene Formen der Arthritis, neuropathischer Gelenkserkrankung (Charcotgelenk und Gelenke), Hämarthrose (hämarthrotisch), Purpura Schönlein-Hennoch, hypertrophe Osteoarthropathie, multizentrische Reticulohistiocytose, Arthritis, die mit bestimmten Erkrankungen assoziiert ist, Surcoilose, Hämochromatose, Sichelzellerkrankung und andere Hämoglobinopathien, Hyperlipoproteinämie, Hypogammaglobulinämie, Hyperparathyreoidismus, Akromegalie, familiäres Mittelmeerfieber, Behat Erkrankung, systemischer Lupus erythrematosis oder Polychondritisrückfall und verwandter Krankheiten.

**12.** Verwendung nach Anspruch 8, worin das Arzneimittel zur Linderung der pathologischen Effekte dient von septischem Schock, Atemstreßsyndrom beim Erwachsenen, Pankreatitis, Trauma-induziertem Schock, Bronchialasthma, allergischer Rhinitis, rheumatoider Arthritis, cystischer Fibrose, Schlaganfall, akuter Bronchitis, chronischer Bronchitis, akuter Broncheolitis, chronischer Broncheolitis, Osteoarthritis, Gicht, Spondylarthropathie, Spondylitis ankylosans, Reiter Syndrom, psoriatischer Arthropathie, enterapathischer Spondylitis, juveniler Arthropathie oder juveniler Spondylitis ankylosans, reaktiver Arthropathie, infektiöser oder postinfektiöser Arthritis, Gonokokkenarthritis, Tuberkulosearthritis, viraler Arthritis, pilzbedingter Arthritis, Syphilis-bedingter Ar-thritis, Lyme-Erkrankung, Arthritis, die mit "vaskulitischen Syndromen" assoziiert ist, Polyarteriitis nodosa, hyperempfindlicher Vaskulitis, Luegenec Granulomatose, Polymyalgia rheumatica, Gelenkszellarteriitis, Arthropathie durch Calciumkristallablagerung, Pseudogicht, nicht-artikuläres Rheuma, Bursitis, Tenosynovitis, Epicondylitis (Tennisellenbogen), Carpaltunnelsyndrom, Verletzung durch wiederkehrende Tätigkeit (Tippen), verschiedene Formen der Arthritis, neuropathischer Gelenkserkrankung (Charcotgelenk und Gelenke), Hämarthrose (hämarthrotisch), Purpura Schönlein-Hennoch, hypertrophe Osteoarthropathie, multizentrische Reticulohistiocytose, Arthritis, die mit bestimmten Erkrankungen assoziiert ist, Surcoilose, Hämochromatose, Sichelzellerkrankung und andere Hämoglobinopathien, Hyperlipoproteinämie, Hypogammaglobulinämie, Hyperparathyreoidismus, Akromegalie, familiäres Mittelmeerfieber, Behat Erkrankung, systemischer Lupus erythrematosis oder Polychondritisrückfall und verwandter Krankheiten.

**13.** Verwendung nach Anspruch 11, worin der Säuger ein Mensch ist.

**14.** Verwendung nach Anspruch 12, worin der Säuger ein Mensch ist.

**15.** Verfahren zur Herstellung von Verbindungen der Formel

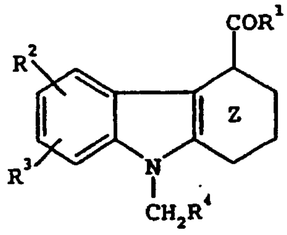

worin

$R^1$ für $-NHNH_2$ oder $-NH_2$ steht,

$R^2$ für -OH oder $-O(CH_2)_mR^5$ steht, worin $R^5$ für H, $-CO_2H$, $-CO_2(C_1-C_4$ Alkyl),

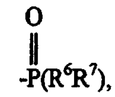

Phenyl oder Phenyl steht, das substituiert ist mit $-CO_2H$ oder $-CO_2(C_1-C_4$ Alkyl), worin $R^6$ und $R^7$ jeweils unabhängig für -OH oder $-O(C_1-C_4$ Alkyl) stehen und m für 1 bis 3 steht,
$R^3$ für H, $-O(C_1-C_4$ Alkyl) oder $-(CH_2)_nR^8$ steht, worin $R^8$ für H, $-NR^9R^{10}$,

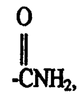

-CN oder Phenyl steht, worin $R^9$ und $R^{10}$ unabhängig für $-(C_1-C_4)$ Alkyl oder Phenyl-$(C_1-C_4)$-alkyl stehen und n für 1 bis 8 steht,
$R^4$ für H, $-(C_5-C_{14})$ Alkyl, $-(C_3-C_{14})$ Cycloalkyl, Phenyl oder Phenyl steht, das substituiert ist mit 1 oder 2 Substituenten, ausgewählt aus der Gruppe, die besteht aus $-(C_1-C_4)$ Alkyl, $-(C_1-C_4)$ Alkoxy, Phenyl-$(C_1-C_4)$-alkyl, $-(C_1-C_4)$ Alkylthio, Halogen oder Phenyl, und
Z für Phenyl steht,

**gekennzeichnet durch** Dehydrierung einer Verbindung der Formel

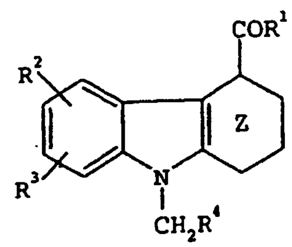

worin

$R^1$ für $-NHNH_2$ oder $-NH_2$ steht,
$R^2$ für -OH oder $-O(CH_2)_mR^5$ steht, worin $R^5$ für H, $-CO_2H$, $-CO_2(C_1-C_4$ Alkyl),

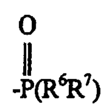

Phenyl oder Phenyl steht, das substituiert ist mit $-CO_2H$ oder $-CO_2(C_1-C_4$ Alkyl, worin $R^6$ und $R^7$ jeweils unabhängig für -OH oder $-O(C_1-C_4$ Alkyl) stehen und m für 1 bis 3 steht,
$R^3$ für H, $-O(C_1-C_4$ Alkyl) oder $-(CH_2)_nR^8$ steht, worin $R^8$ für H, $-NR^9R^{10}$,

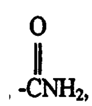

-CN oder Phenyl steht, worin $R^9$ und $R^{10}$ unabhängig für $-(C_1-C_4)$ Alkyl oder Phenyl-$(C_1-C_4)$-alkyl stehen und n für 1 bis 8 steht,
$R^4$ für H, $-(C_5-C_{14})$ Alkyl, $-(C_3-C_{14})$ Cycloalkyl, Phenyl oder Phenyl steht, das substituiert ist mit 1 oder 2 Substituenten, ausgewählt aus der Gruppe, die besteht aus $-(C_1-C_4)$ Alkyl, $-(C_1-C_4)$ Alkoxy, Phenyl-$(C_1-C_4)$-

alkyl, -($C_1$-$C_4$) Alkylthio, Halogen oder Phenyl, und
Z für Cyclohexenyl steht.

**16.** Verbindung der Formel III nach Anspruch 1 zur Behandlung der pathologischen Effekte von septischem Schock, Atemstreßsyndrom beim Erwachsenen, Pankreatitis, Trauma-induziertem Schock, Bronchialasthma, allergischer Rhinitis, rheumatoider Arthritis, cystischer Fibrose, Schlaganfall, akuter Bronchitis, chronischer Bronchitis, akuter Broncheolitis, chronischer Broncheolitis, Osteoarthritis, Gicht, Spondylarthropathie, Spondylitis ankylosans, Reiter Syndrom, psoriatischer Arthropathie, enterapathischer Spondylitis, juveniler Arthropathie oder juveniler Spondylitis ankylosans, reaktiver Arthropathie, infektiöser oder postinfektiöser Arthritis, Gonokokkenarthritis, Tuberkulosearthritis, viraler Arthritis, pilzbedingter Arthritis, Syphilis-bedingter Arthritis, Lyme-Erkrankung, Arthritis, die mit "vaskulitischen Syndromen" assoziiert ist, Polyarteriitis nodosa, hyperempfindlicher Vaskulitis, Luegenec Granulomatose, Polymyalgia rheumatica, Gelenkszellarteriitis, Arthropathie durch Calciumkristallablagerung, Pseudogicht, nicht-artikuläres Rheuma, Bursitis, Tenosynovitis, Epicondylitis (Tennisellenbogen), Carpaltunnelsyndrom, Verletzung durch wiederkehrende Tätigkeit (Tippen), verschiedene Formen der Arthritis, neuropathischer Gelenkserkrankung (Charcotgelenk und Gelenke), Hämarthrose (hämarthrotisch), Purpura Schönlein-Hennoch, hypertrophe Osteoarthropathie, multizentrische Reticulohistiocytose, Arthritis, die mit bestimmten Erkrankungen assoziiert ist, Surcoilose, Hämochromatose, Sichelzellerkrankung und andere Hämoglobinopathien, Hyperlipoproteinämie, Hypogammaglobulinämie, Hyperparathyreoidismus, Akromegalie, familiäres Mittelmeerfieber, Behat Erkrankung, systemischer Lupus erythrematosis oder Polychondritisrückfall und verwandter Krankheiten.

**17.** Verbindung der Formel I nach einem der Ansprüche 2 bis 4 zur Behandlung der pathologischen Effekte von septischem Schock, Atemstreßsyndrom beim Erwachsenen, Pankreatitis, Trauma-induziertem Schock, Bronchialasthma, allergischer Rhinitis, rheumatoider Arthritis, cystischer Fibrose, Schlaganfall, akuter Bronchitis, chronischer Bronchitis, akuter Broncheolitis, chronischer Broncheolitis, Osteoarthritis, Gicht, Spondylarthropathie, Spondylitis ankylosans, Reiter Syndrom, psoriatischer Arthropathie, enterapathischer Spondylitis, juveniler Arthropathie oder juveniler Spondylitis ankylosans, reaktiver Arthropathie, infektiöser oder postinfektiöser Arthritis, Gonokokkenarthritis, Tuberkulosearthritis, viraler Arthritis, pilzbedingter Arthritis, Syphilis-bedingter Arthritis, Lyme-Erkrankung, Arthritis, die mit "vaskulitischen Syndromen" assoziiert ist, Polyarteriitis nodosa, hyperempfindlicher Vaskulitis, Luegenec Granulomatose, Polymyalgia rheumatica, Gelenkszellarteriitis, Arthropathie durch Calciumkristallablagerung, Pseudogicht, nicht-artikuläres Rheuma, Bursitis, Tenosynovitis, Epicondylitis (Tennisellenbogen), Carpaltunnelsyndrom, Verletzung durch wiederkehrende Tätigkeit (Tippen), verschiedene Formen der Arthritis, neuropathischer Gelenkserkrankung (Charcotgelenk und Gelenke), Hämarthrose (hämarthrotisch), Purpura Schönlein-Hennoch, hypertrophe Osteoarthropathie, multizentrische Reticulohistiocytose, Arthritis, die mit bestimmten Erkrankungen assoziiert ist, Surcoilose, Hämochromatose, Sichelzellerkrankung und andere Hämoglobinopathien, Hyperlipoproteinämie, Hypogammaglobulinämie, Hyperparathyreoidismus, Akromegalie, familiäres Mittelmeerfieber, Behat Erkrankung, systemischer Lupus erythrematosis oder Polychondritisrückfall und verwandter Krankheiten.

**18.** Verwendung einer Verbindung der Formel III nach Anspruch 1 zur Herstellung eines Arzneimittels zur Behandlung der pathologischen Effekte von septischem Schock, Atemstreßsyndrom beim Erwachsenen, Pankreatitis, Trauma-induziertem Schock, Bronchialasthma, allergischer Rhinitis, rheumatoider Arthritis, cystischer Fibrose, Schlaganfall, akuter Bronchitis, chronischer Bronchitis, akuter Broncheolitis, chronischer Broncheolitis, Osteoarthritis, Gicht, Spondylarthropathie, Spondylitis ankylosans, Reiter Syndrom, psoriatischer Arthropathie, enterapathischer Spondylitis, juveniler Arthropathie oder juveniler Spondylitis ankylosans, reaktiver Arthropathie, infektiöser oder postinfektiöser Arthritis, Gonokokkenarthritis, Tuberkulosearthritis, viraler Arthritis, pilzbedingter Arthritis, Syphilis-bedingter Arthritis, Lyme-Erkrankung, Arthritis, die mit "vaskulitischen Syndromen" assoziiert ist, Polyarteriitis nodosa, hyperempfindlicher Vaskulitis, Luegenec Granulomatose, Polymyalgia rheumatica, Gelenkszellarteriitis, Arthropathie durch Calciumkristallablagerung, Pseudogicht, nicht-artikuläres Rheuma, Bursitis, Tenosynovitis, Epicondylitis (Tennisellenbogen), Carpaltunnelsyndrom, Verletzung durch wiederkehrende Tätigkeit (Tippen), verschiedene Formen der Arthritis, neuropathischer Gelenkserkrankung (Charcotgelenk und Gelenke), Hämarthrose (hämarthrotisch), Purpura Schönlein-Hennoch, hypertrophe Osteoarthropathie, multizentrische Reticulohistiocytose, Arthritis, die mit bestimmten Erkrankungen assoziiert ist, Surcoilose, Hämochromatose, Sichelzellerkrankung und andere Hämoglobinopathien, Hyperlipoproteinämie, Hypogammaglobulinämie, Hyperparathyreoidismus, Akromegalie, familiäres Mittelmeerfieber, Behat Erkrankung, systemischer Lupus erythrematosis oder Polychondritisrückfall und verwandter Krankheiten.

**19.** Verwendung einer Verbindung der Formel I nach einem der Ansprüche 2 bis 4 zur Herstellung eines Arzneimittels zur Behandlung der pathologischen Effekte von septischem Schock, Atemstreßsyndrom beim Erwachsenen, Pankreatitis, Trauma-induziertem Schock, Bronchialasthma, allergischer Rhinitis, rheumatoider Arthritis, cystischer Fibrose, Schlaganfall, akuter Bronchitis, chronischer Bronchitis, akuter Broncheolitis, chronischer Broncheolitis, Osteoarthritis, Gicht, Spondylarthropathie, Spondylitis ankylosans, Reiter Syndrom, psoriatischer Arthropathie, enterapathischer Spondylitis, juveniler Arthropathie oder juveniler Spondylitis ankylosans, reaktiver Arthropathie, infektiöser oder postinfektiöser Arthritis, Gonokokkenarthritis, Tuberkulosearthritis, viraler Arthritis, pilzbedingter Arthritis, Syphilis-bedingter Arthritis, Lyme-Erkrankung, Arthritis, die mit "vaskulitischen Syndromen" assoziiert ist, Polyarteriitis nodosa, hyperempfindlicher Vaskulitis, Luegenec Granulomatose, Polymyalgia rheumatica, Gelenkszellarteriitis, Arthropathie durch Calciumkristallablagerung, Pseudogicht, nicht-artikuläres Rheuma, Bursitis, Tenosynovitis, Epicondylitis (Tennisellenbogen), Carpaltunnelsyndrom, Verletzung durch wiederkehrende Tätigkeit (Tippen), verschiedene Formen der Arthritis, neuropathischer Gelenkserkrankung (Charcotgelenk und Gelenke), Hämarthrose (hämarthrotisch), Purpura Schönlein-Hennoch, hypertrophe Osteoarthropathie, multizentrische Reticulohistiocytose, Arthritis, die mit bestimmten Erkrankungen assoziiert ist, Surcoilose, Hämochromatose, Sichelzellerkrankung und andere Hämoglobinopathien, Hyperlipoproteinämie, Hypogammaglobulinämie, Hyperparathyreoidismus, Akromegalie, familiäres Mittelmeerfieber, Behat Erkrankung, systemischer Lupus erythrematosis oder Polychondritisrückfall und verwandter Krankheiten.

**20.** Pharmazeutische Formulierung, die angepaßt ist zur Behandlung der pathologischen Effekte von septischem Schock, Atemstreßsyndrom beim Erwachsenen, Pankreatitis, Trauma-induziertem Schock, Bronchialasthma, allergischer Rhinitis, rheumatoider Arthritis, cystischer Fibrose, Schlaganfall, akuter Bronchitis, chronischerBronchitis, akuter Broncheolitis, chronischer Broncheolitis, Osteoarthritis, Gicht, Spondylarthropathie, Spondylitis ankylosans, Reiter Syndrom, psoriatischer Arthropathie, enterapathischer Spondylitis, juveniler Arthropathie oder juveniler Spondylitis ankylosans, reaktiver Arthropathie, infektiöser oder postinfektiöser Arthritis, Gonokokkenarthritis, Tuberkulosearthritis, viraler Arthritis, pilzbedingter Arthritis, Syphilis-bedingter Arthritis, Lyme-Erkrankung, Arthritis, die mit "vaskulitischen Syndromen" assoziiert ist, Polyarteriitis nodosa, hyperempfindlicher Vaskulitis, Luegenec Granulomatose, Polymyalgia rheumatica, Gelenkszellarteriitis, Arthropathie durch Calciumkristallablagerung, Pseudogicht, nicht-artikuläres Rheuma, Bursitis, Tenosynovitis, Epicondylitis (Tennisellenbogen), Carpaltunnelsyndrom, Verletzung durch wiederkehrende Tätigkeit (Tippen), verschiedene Formen der Arthritis, neuropathischer Gelenkserkrankung (Charcotgelenk und Gelenke), Hämarthrose (hämarthrotisch), Purpura Schönlein-Hennoch, hypertrophe Osteoarthropathie, multizentrische Reticulohistiocytose, Arthritis, die mit bestimmten Erkrankungen assoziiert ist, Surcoilose, Hämochromatose, Sichelzellerkrankung und andere Hämoglobinopathien, Hyperlipoproteinämie, Hypogammaglobulinämie, Hyperparathyreoidismus, Akromegalie, familiäres Mittelmeerfieber, Behat Erkrankung, systemischer Lupus erythrematosis oder Polychondritisrückfall und verwandter Krankheiten, die eine Verbindung der Formel III nach Anspruch 1 enthält.

**21.** Verwendung nach einem der Ansprüche 7 bis 10, worin das Arzneimittel dient zur Linderung der pathologischen Effekte von septischem Schock, Atemstreßsyndrom beim Erwachsenen, Pankreatitis, Trauma-induziertem Schock, Bronchialasthma, allergischer Rhinitis, rheumatoider Arthritis, cystischer Fibrose, Schlaganfall, akuter Bronchitis, chronischer Bronchitis, akuter Broncheolitis, chronischer Broncheolitis, Osteoarthritis, Gicht, Spondylarthropathie, Spondylitis ankylosans, Reiter Syndrom, psoriatischer Arthropathie, enterapathischer Spondylitis, juveniler Arthropathie oder juveniler Spondylitis ankylosans, reaktiver Arthropathie, infektiöser oder postinfektiöser Arthritis, Gonokokkenarthritis, Tuberkulosearthritis, viraler Arthritis, pilzbedingter Arthritis, Syphilis-bedingter Arthritis, Lyme-Erkrankung, Arthritis, die mit "vaskulitischen Syndromen" assoziiert ist, Polyarteriitis nodosa, hyperempfindlicher Vaskulitis, Luegenec Granulomatose, Polymyalgia rheumatica, Gelenkszellarteriitis, Arthropathie durch Calciumkristallablagerung, Pseudogicht, nicht-artikuläres Rheuma, Bursitis, Tenosynovitis, Epicondylitis (Tennisellenbogen), Carpaltunnelsyndrom, Verletzung durch wiederkehrende Tätigkeit (Tippen), verschiedene Formen der Arthritis, neuropathischer Gelenkserkrankung (Charcotgelenk und Gelenke), Hämarthrose (hämarthrotisch), Purpura Schönlein-Hennoch, hypertrophe Osteoarthropathie, multizentrische Reticulohistiocytose, Arthritis, die mit bestimmten Erkrankungen assoziiert ist, Surcoilose, Hämochromatose, Sichelzellerkrankung und andere Hämoglobinopathien, Hyperlipoproteinämie, Hypogammaglobulinämie, Hyperparathyreoidismus, Akromegalie, familiäres Mittelmeerfieber, Behat Erkrankung, systemischer Lupus erythrematosis oder Polychondritisrückfall und verwandter Krankheiten.

## Revendications

**1.** Composé de formule (III)

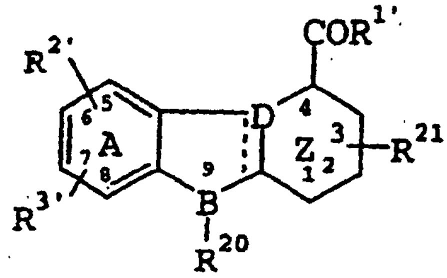

(III)

dans laquelle ;

A est un groupe phényle ou pyridyle dans lequel l'azote est à la position 5, 6, 7 ou 8 ;
un parmi B ou D est un azote et l'autre est un carbone ;
Z est un groupe cyclohexényle, phényle, pyridyle, dans lequel l'azote est à la position 1, 2 ou 3, ou un noyau hétérocyclique à 6 chaînons ayant un hétéroatome choisi dans le groupe constitué d'un soufre ou d'un oxygène à la position 1, 2 ou 3, et d'un azote à la position 1, 2, 3 ou 4 ;
--- est une liaison double ou simple ;
$R^{20}$ est choisi parmi les groupes (a), (b) et (c) où ;

(a) est un groupe alkyle en $C_5$-$C_{20}$, alcényle en $C_5$-$C_{20}$, alcynyle en $C_5$-$C_{20}$, des radicaux carbocycliques dérivés d'un noyau organique ayant 5 à 14 chaînons saturé ou insaturé, substitué ou non substitué, dont les atomes formant le cycle (autres que l'hydrogène) sont seulement des atomes de carbone, ou des radicaux hétérocycliques dérivés de noyaux hétérocycliques monocycliques ou polycycliques, saturés ou insaturés, substitués ou non substitués, ayant 5 à 14 atomes de cycle et contenant 1 à 3 hétéroatomes choisis dans le groupe constitué de l'azote, de l'oxygène ou du soufre, ou
(b) est un membre de (a) substitué par un ou plusieurs substituants indépendamment choisis parmi un hydrogène, un groupe alkyle en $C_1$-$C_{12}$, alcényle en $C_2$-$C_6$, alcynyle en $C_2$-$C_6$, aralkyle en $C_7$-$C_{12}$, alkaryle en $C_7$-$C_{12}$, cycloalkyle en $C_3$-$C_8$, cycloalcényle en $C_3$-$C_8$, phényle, tolulyle, xylényle, biphényle, alcoxy en $C_1$-$C_6$, alcényloxy en $C_2$-$C_6$, alcynyloxy en $C_2$-$C_6$, alcoxyalkyle en $C_1$-$C_{12}$, alcoxyalkyloxy en $C_1$-$C_{12}$, alkylcarbonyle en $C_1$-$C_{12}$, alkylcarbonylamino en $C_1$-$C_{12}$, alcoxyamino en $C_1$-$C_{12}$, alcoxyaminocarbonyle en $C_1$-$C_{12}$, alkylamino en $C_1$-$C_{12}$, alkylthio en $C_1$-$C_6$, alkylthiocarbonyle en $C_1$-$C_{12}$, alkylsulfinyle en $C_1$-$C_6$, alkylsulfonyle en $C_1$-$C_6$, halogénoalcoxy en $C_1$-$C_6$, halogénoalkylsulfonyle en $C_1$-$C_6$, halogénoalkyle en $C_1$-$C_6$, hydroxyalkyle en $C_1$-$C_6$, $-(CH_2)_nCN$, $-(CH_2)_nNR^9R^{10}$, -C(O)O(alkyle en $C_1$-$C_6$), $-(CH_2)_nO$(alkyle en $C_1$-$C_6$), benzyloxy, phénoxy, phénylthio, $-(CONHSO_2R)$, -CHO, amino, amidino, halogéno, carbamyle, carboxyle, carbalcoxy, $-(CH_2)_nCO_2H$, cyano, cyanoguanidinyle, guanidino, hydrazide, hydrazino, hydrazido, hydroxy, hydroxyamino, nitro, phosphono, $-SO_3H$, thioacétal, thiocarbonyle, et (alkyle en $C_1$-$C_6$) carbonyle ; où n vaut de 1 à 8 et $R^9$ et $R^{10}$ sont indépendamment un groupe alkyle en $C_1$-$C_4$ ou phényl (alkyle en $C_1$-$C_4$) ; ou
(c) est le groupe -(L)-$R^{80}$ ; où -(L)- est un groupe de liaison divalent de 1 à 12 atomes choisis parmi carbone, hydrogène, oxygène, azote, et soufre ; dans lequel la combinaison d'atomes dans -(L)- est choisie dans le groupe constitué de (i) carbone et hydrogène seulement, (ii) un soufre seulement, (iii) un oxygène seulement, (iv) un ou deux azote et hydrogène seulement, (v) carbone, hydrogène, et un soufre seulement, et (vi) carbone, hydrogène, et oxygène seulement ; et où $R^{80}$ est un groupe choisi parmi (a) ou (b) ;

$R^{21}$ est choisi parmi un hydrogène, un groupe alkyle en $C_1$-$C_{12}$, alcényle en $C_2$-$C_6$, alcynyle en $C_2$-$C_6$, aralkyle en $C_7$-$C_{12}$, alkaryle en $C_7$-$C_{12}$, cycloalkyle en $C_3$-$C_8$, cycloalcényle en $C_3$-$C_8$, phényle, tolulyle, xylényle, biphényle, alcoxy en $C_1$-$C_6$, alcényloxy en $C_2$-$C_6$, alcynyloxy en $C_2$-$C_6$, alcoxyalkyle en $C_1$-$C_{12}$, alcoxyalkyloxy en $C_1$-$C_{12}$, alkylcarbonyle en $C_1$-$C_{12}$, alkylcarbonylamino en $C_1$-$C_{12}$, alcoxyamino en $C_1$-$C_{12}$, alcoxyaminocarbonyle en $C_1$-$C_{12}$, alkylamino en $C_1$-$C_{12}$, alkylthio en $C_1$-$C_6$, alkylthiocarbonyle en $C_1$-$C_{12}$, alkylsulfinyle en $C_1$-$C_6$, alkylsulfonyle en $C_1$-$C_6$, halogénoalcoxy en $C_1$-$C_6$, halogénoalkylsulfonyle en $C_1$-$C_6$, halogénoalkyle en $C_1$-$C_6$, hydroxyalkyle en $C_1$-$C_6$, $-(CH_2)_nCN$, $-(CH_2)_nNR^9R^{10}$, -C(O)O(alkyle en $C_1$-$C_6$), $-(CH_2)_nO$(alkyle en $C_1$-$C_6$), benzyloxy, phénoxy, phénylthio, $-(CONHSO_2R)$, -CHO, amino, amidino, halogéno, carbamyle, carboxyle, carbalcoxy, $-(CH_2)_nCO_2H$, cyano, cyanoguanidinyle, guanidino, hydrazide, hydrazino, hydrazido, hydroxy, hydroxyamino, nitro, phosphono, $-SO_3H$, thioacétal, thiocarbonyle, et (alkyle en $C_1$-$C_6$)carbonyle ; où

n vaut de 1 à 8 et $R^9$ et $R^{10}$ sont indépendamment un groupe alkyle en $C_1$-$C_4$ ou phényl(alkyle en $C_1$-$C_4$) ;
$R^{1'}$ est -$NHNH_2$ ou -$NH_2$ ;
$R^{2'}$ est choisi dans le groupe -OH, -$O(CH_2)_tR^5$ où $R^5$ est un groupe CN ou un groupe phényle, ou -($L_a$)-(groupe acide) ; dans lequel le groupe acide est un groupe organique qui une fois attaché à un noyau tricyclique, par des atomes de liaison appropriés, agit comme un donneur de protons capable de liaison hydrogène et -($L_a$)- est un groupe de liaison divalent ayant une longueur de lien de 1 à 7 et t vaut 1 à 5 ;
$R^{3'}$ est choisi parmi un hydrogène, un groupe alkyle en $C_1$-$C_{12}$, alcényle en $C_2$-$C_6$, alcynyle en $C_2$-$C_6$, aralkyle en $C_7$-$C_{12}$, alkaryle en $C_7$-$C_{12}$, cycloalkyle en $C_3$-$C_8$, cycloalcényle en $C_3$-$C_8$, phényle, tolulyle, xylényle, biphényle, alcoxy en $C_1$-$C_6$, alcényloxy en $C_2$-$C_6$, alcynyloxy en $C_2$-$C_6$, alcoxyalkyle en $C_1$-$C_{12}$, alcoxyalkyloxy en $C_1$-$C_{12}$, alkylcarbonyle en $C_1$-$C_{12}$, alkylcarbonylamino en $C_1$-$C_{12}$, alcoxyamino en $C_1$-$C_{12}$, alcoxyaminocarbonyle en $C_1$-$C_{12}$, alkylamino en $C_1$-$C_{12}$, alkylthio en $C_1$-$C_6$, alkylthiocarbonyle en $C_1$-$C_{12}$, alkylsulfinyle en $C_1$-$C_6$, alkylsulfonyle en $C_1$-$C_6$, halogénoalcoxy en $C_1$-$C_6$, halogénoalkylsulfonyle en $C_1$-$C_6$, halogénoalkyle en $C_1$-$C_6$, hydroxyalkyle en $C_1$-$C_6$, -$(CH_2)_nCN$, -$(CH_2)_nNR^9R^{10}$, -C(O)O(alkyle en $C_1$-$C_6$), -$(CH_2)_n$O(alkyle en $C_1$-$C_6$), benzyloxy, phénoxy, phénylthio, -($CONHSO_2R$), -CHO, amino, amidino, halogéno, carbamyle, carboxyle, carbalcoxy, -$(CH_2)_nCO_2H$, cyano, cyanoguanidinyle, guanidino, hydrazide, hydrazino, hydrazido, hydroxy, hydroxyamino, nitro, phosphono, -$SO_3H$, thioacétal, thiocarbonyle, et (alkyle en $C_1$-$C_6$)carbonyle ; où n vaut de 1 à 8 et $R^9$ et $R^{10}$ sont indépendamment un groupe alkyle en $C_1$-$C_4$ ou phényl(alkyle en $C_1$-$C_4$); des radicaux carbocycliques dérivés d'un noyau organique ayant 5 à 14 chaînons saturé ou insaturé dont les atomes formant le cycle (autres que l'hydrogène) sont seulement des atomes de carbone ; ou des radicaux hétérocycliques dérivés de noyaux hétérocycliques monocycliques ou polycycliques, saturés ou insaturés, ayant 5 à 14 atomes de cycle et contenant 1 à 3 hétéroatomes choisis dans le groupe constitué de l'azote, de l'oxygène ou du soufre ; et des radicaux carbocycliques dérivés d'un noyau organique ayant 5 à 14 chaînons saturé ou insaturé dont les atomes formant le cycle (autres que l'hydrogène) sont seulement des atomes de carbone ou des radicaux hétérocycliques dérivés de noyaux hétérocycliques monocycliques ou polycycliques, saturés ou insaturés, ayant 5 à 14 atomes de cycle et contenant 1 à 3 hétéroatomes choisis dans le groupe constitué de l'azote, de l'oxygène ou du soufre, substitués par des groupes choisis parmi un hydrogène, un groupe alkyle en $C_1$-$C_{12}$, alcényle en $C_2$-$C_6$, alcynyle en $C_2$-$C_6$, aralkyle en $C_7$-$C_{12}$, alkaryle en $C_7$-$C_{12}$, cycloalkyle en $C_3$-$C_8$, cycloalcényle en $C_3$-$C_8$, phényle, tolulyle, xylényle, biphényle, alcoxy en $C_1$-$C_6$, alcényloxy en $C_2$-$C_6$, alcynyloxy en $C_2$-$C_6$, alcoxyalkyle en $C_1$-$C_{12}$, alcoxyalkyloxy en $C_1$-$C_{12}$, alkylcarbonyle en $C_1$-$C_{12}$, alkylcarbonylamino en $C_1$-$C_{12}$, alcoxyamino en $C_1$-$C_{12}$, alcoxyaminocarbonyle en $C_1$-$C_{12}$, alkylamino en $C_1$-$C_{12}$, alkylthio en $C_1$-$C_6$, alkylthiocarbonyle en $C_1$-$C_{12}$, alkylsulfinyle en $C_1$-$C_6$, alkylsulfonyle en $C_1$-$C_6$, halogénoalcoxy en $C_1$-$C_6$, halogénoalkylsulfonyle en $C_1$-$C_6$, halogénoalkyle en $C_1$-$C_6$, hydroxyalkyle en $C_1$-$C_6$, -$(CH_2)_nCN$, -$(CH_2)_nNR^9R^{10}$, -C(O)O(alkyle en $C_1$-$C_6$), -$(CH_2)_n$O(alkyle en $C_1$-$C_6$), benzyloxy, phénoxy, phénylthio, -($CONHSO_2R$), -CHO, amino, amidino, halogéno, carbamyle, carboxyle, carbalcoxy, -$(CH_2)_nCO_2H$, cyano, cyanoguanidinyle, guanidino, hydrazide, hydrazino, hydrazido, hydroxy, hydroxyamino, nitro, phosphono, -$SO_3H$, thioacétal, thiocarbonyle, et (alkyle en $C_1$-$C_6$)carbonyle ; où n vaut de 1 à 8 et $R^9$ et $R^{10}$ sont indépendamment un groupe alkyle en $C_1$-$C_4$ ou phényl(alkyle en $C_1$-$C_4$) ;
ou un de ses sels, racémates, solvates, tautomères, ou isomères optiques, pharmaceutiquement acceptables ;
pour autant que lorsque D est un azote, l'hétéroatome de Z est choisi dans le groupe constitué d'un soufre ou d'un oxygène à la position 1, 2, ou 3 et d'un azote à la position 1, 2, 3 ou 4.

**2.** Composé de formule (I)

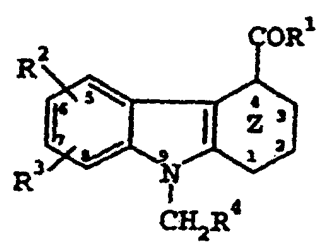

(I)

dans laquelle :

$R^1$ est -$NHNH_2$, ou -$NH_2$ ;

$R^2$ est -OH, ou -O$(CH_2)_m$$R^5$ où

$R^5$ est H, $-CO_2H$, $-CO_2$(alkyle en $C_1$-$C_4$),

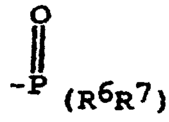
,

un groupe phényle, ou phényle substitué par $-CO_2H$ ou $-CO_2$(alkyle en $C_1$-$C_4$) où $R^6$ et $R^7$ sont chacun indépendamment -OH ou -O(alkyle en $C_1$-$C_4$) et m vaut 1 à 3 ;

$R^3$ est H, -O(alkyle en $C_1$-$C_4$), ou $-(CH_2)_n$$R^8$ où

$R^8$ est H, $-NR^9R^{10}$,

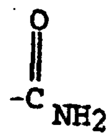
,

-CN, ou un groupe phényle où $R^9$ et $R^{10}$ sont indépendamment un groupe alkyle en $C_1$-$C_4$, ou phényl(alkyle en $C_1$-$C_4$) et n vaut 1 à 8 ;

$R^4$ est H, un groupe alkyle en $C_5$-$C_{14}$, cycloalkyle en $C_3$-$C_{14}$, phényle, ou phényle substitué par 1 ou 2 substituants choisis dans le groupe constitué d'un groupe alkyle en $C_1$-$C_4$, alcoxy en $C_1$-$C_4$, phényl(alkyle en $C_1$-$C_4$), (alkyle en $C_1$-$C_4$)thio, un halogène ou un groupe phényle ; et

Z est un groupe cyclohexényle ou phényle ;

ou un de ses sels, racémates, isomères optiques, solvates ou tautomères, pharmaceutiquement acceptables ;

pour autant que lorsque $R^3$ est H, $R^4$ est un groupe phényle, m vaut 1 ou 2 et $R^2$ est substitué en position 6, $R^5$ ne peut pas être H ; et

lorsque $R^1$ est $NHNH_2$, $R^8$ ne peut pas être

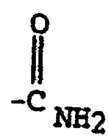
;

et lorsque $R^1$ est $NH_2$, $R^2$ est OMe substitué en position 6 et $R^3$ est H, $R^4$ ne peut pas être H.

3. Composé de formule I selon la revendication 2 dans lequel

$R^1$ est $-NHNH_2$, ou $-NH_2$ ;

$R^2$ est -O$(CH_2)_m$$R^5$ où $R^5$ est $-CO_2H$ ou

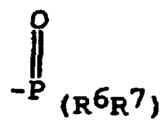

où $R^6$ et $R^7$ sont -OH ;

$R^3$ est H, -O(alkyle en $C_1$-$C_4$), ou $-(CH_2)_n$$R^8$ où $R^8$ est H ou un groupe phényle ;

$R^4$ est un groupe phényle ; et

Z est un cyclohexène.

4. Composé selon la revendication 3 qui est l'acide 4-[(9-benzyl-4-carbamoyl-1,2,3,4-tétrahydrocarbazol-6-yl)oxy]butyrique ; l'acide 3-[(9-benzyl-4-carbamoyl-1,2,3,4-tétrahydrocarbazol-6-yl)oxy]propylphosphonique ; l'acide 2-[(9-benzyl-4-carbamoyl-1,2,3,4-tétrahydrocarbazol-6-yl)oxy]méthyl-benzoïque ; l'acide 3-[(9-benzyl-4-carbamoyl-7-n-octyl-1,2,3,4-tétrahydrocarbazol-6-yl)oxy]propylphosphonique; l'acide 4-[(9-benzyl-4-carbamoyl-7-éthyl-1,2,3,4-tétrahydrocarbazol-6-yl)oxy]butyrique ; l'acide 3-[(9-benzyl-4-carbamoyl-7-éthyl-1,2,3,4-tétrahydrocarbazol-6-yl)oxy]propyl-phosphonique ; l'acide 3-[(9-benzyl-4-carbamoyl-7-éthyl-1,2,3,4-tétrahydrocarbazol-6-yl)oxy]propylphosphonique ; l'acide (S)-(+)-4-[(9-benzyl-4-carbamoyl-7-éthyl-1,2,3,4-tétrahydrocarbazol-6-yl) oxy]butyrique ; l'acide 4-[9-benzyl-4-carbamoyl-6-(2-cyanoéthyl)-1,2,3,4-tétrahydrocarbazol-6-yl]oxybutyrique ; l'acide 4-[9-benzyl-4-carboxamido-7-(2-phényléthyl)-1,2,3,4-tétrahydrocarbazol-6-yl]oxybutyrique ; l'acide 4-[9-benzyl-4-carboxamido-carbazol-6-yl]oxybutyrique ; le 2-[(9-benzyl-4-carbamoyl-1,2,3,4-tétrahydrocarbazol-6-yl)oxy]méthylbenzoate de méthyle; l'hydrazide d'acide 9-benzyl-5,7-diméthoxy-1,2,3,4-tétrahydrocarbazole-4-carboxylique ; le 9-benzyl-5,7-diméthoxy-1,2,3,4-tétrahydrocarbazole-4-carboxamide ; le sel de sodium de l'acide [9-benzyl-4-carbamoyl-7-méthoxy-1,2,3,4-tétrahydrocarbazol-5-yl]oxyacétique ; l'acide 4-[9-benzyl-4-carbamoyl-7-(2-cyanoéthyl)-1,2,3,4-tétrahydrocarbazol-6-yl]oxybutyrique ; l'acide méthyl-[9-benzyl-4-carbamoyl-7-méthoxycarbazol-5-yl]oxyacétique ; l'acide [9-benzyl-4-carbamoyl-7-méthoxycarbazol-5-yl]oxyacétique ; le 9-benzyl-7-méthoxy-5-cyanométhyloxy-1,2,3,4-tétrahydrocarbazole-4-carboxamide ; le 9-benzyl-7-méthoxy-5-(1H-tétrazol-5-yl-méthyl)oxy)-1,2,3,4-tétrahydrocarbazole-4-carboxamide ; l'acide [9-benzyl-4-carbamoyl-8-méthyl-1,2,3,4-tétrahydrocarbazole-5-yl]oxyacétique ; l'acide [9-benzyl-4-carbamoyl-8-méthyl-carbazole-5-yl] oxyacétique ; l'acide [9-benzyl-4-carbamoyl-1,2,3,4-tétrahydrocarbazol-5-yl]oxyacétique ; et l'acide [9-benzyl-4-carbamoyl-carbazole-5-yl]oxyacétique ou un de ses sels, racémates, solvates, tautomères, ou isomères optiques, pharmaceutiquement acceptables.

**5.** Formulation pharmaceutique comprenant un composé de formule III selon la revendication 1 conjointement avec un support ou diluant pour celui-ci pharmaceutiquement acceptable.

**6.** Formulation pharmaceutique comprenant un composé de formule I selon la revendication 2 conjointement avec un support ou diluant pour celui-ci pharmaceutiquement acceptable.

**7.** Utilisation, pour la fabrication d'un médicament destiné à inhiber sélectivement $sPLA_2$ chez un mammifère ayant besoin d'un tel traitement, d'un composé de formule (III)

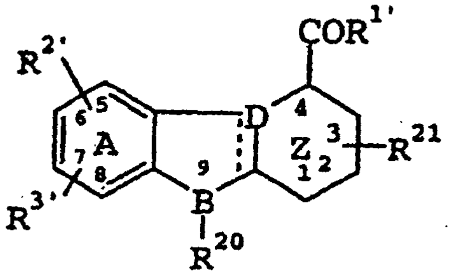

(III)

dans laquelle :

A est un groupe phényle ou pyridyle ;
B et D sont chacun indépendamment un azote ou un carbone ;
Z est un groupe cyclohexényle, phényle, pyridyle, dans lequel l'azote est à la position 1, 2 ou 3, ou un noyau hétérocyclique à 6 chaînons ayant un à trois hétéroatomes qui sont identiques ou différents et qui sont choisis dans le groupe constitué d'un soufre ou d'un oxygène à la position 1, 2 ou 3, et d'un azote à la position 1, 2, 3 ou 4 ;
---- est une liaison double ou simple ;
$R^{20}$ est choisi parmi les groupes (a), (b) et (c) où ;

(a) est un groupe alkyle en $C_5$-$C_{20}$, alcényle en $C_5$-$C_{20}$, alcynyle en $C_5$-$C_{20}$, des radicaux carbocycliques dérivés d'un noyau organique ayant 5 à 14 chaînons saturé ou insaturé, substitué ou non substitué, dont les atomes formant le cycle (autres que l'hydrogène) sont seulement des atomes de carbone, ou des radicaux hétérocycliques dérivés de noyaux hétérocycliques monocycliques ou polycycliques, saturés ou insaturés, substitués ou non substitués, ayant 5 à 14 atomes de cycle et contenant 1 à 3 hétéroatomes

choisis dans le groupe constitué de l'azote, de l'oxygène ou du soufre, ou

(b) est un membre de (a) substitué par un ou plusieurs substituants indépendamment choisis parmi un hydrogène, un groupe alkyle en $C_1$-$C_{12}$, alcényle en $C_2$-$C_6$, alcynyle en $C_2$-$C_6$, aralkyle en $C_7$-$C_{12}$, alkaryle en $C_7$-$C_{12}$, cycloalkyle en $C_3$-$C_8$, cycloalcényle en $C_3$-$C_8$, phényle, tolulyle, xylényle, biphényle, alcoxy en $C_1$-$C_6$, alcényloxy en $C_2$-$C_6$, alcynyloxy en $C_2$-$C_6$, alcoxyalkyle en $C_1$-$C_{12}$, alcoxyalkyloxy en $C_1$-$C_{12}$, alkylcarbonyle en $C_1$-$C_{12}$, alkylcarbonylamino en $C_1$-$C_{12}$, alcoxyamino en $C_1$-$C_{12}$, alcoxyaminocarbonyle en $C_1$-$C_{12}$, alkylamino en $C_1$-$C_{12}$, alkylthio en $C_1$-$C_6$, alkylthiocarbonyle en $C_1$-$C_{12}$, alkylsulfinyle en $C_1$-$C_6$, alkylsulfonyle en $C_1$-$C_6$, halogénoalcoxy en $C_1$-$C_6$, halogénoalkylsulfonyle en $C_1$-$C_6$, halogénoalkyle en $C_1$-$C_6$, hydroxyalkyle en $C_1$-$C_6$, $-(CH_2)_nCN$, $-(CH_2)_nNR^9R^{10}$, -C(O)O(alkyle en $C_1$-$C_6$), $-(CH_2)_nO$(alkyle en $C_1$-$C_6$), benzyloxy, phénoxy, phénylthio, $-(CONHSO_2R)$, -CHO, amino, amidino, halogéno, carbamyle, carboxyle, carbalcoxy, $-(CH_2)_nCO_2H$, cyano, cyanoguanidinyle, guanidino, hydrazide, hydrazino, hydrazido, hydroxy, hydroxyamino, nitro, phosphono, $-SO_3H$, thioacétal, thiocarbonyle, et (alkyle en $C_1$-$C_6$) carbonyle ; où n vaut de 1 à 8 et $R^9$ et $R^{10}$ sont indépendamment un groupe alkyle en $C_1$-$C_4$ ou phényl (alkyle en $C_1$-$C_4$) ; ou

(c) est le groupe -(L)-$R^{80}$ ; où -(L)- est un groupe de liaison divalent de 1 à 12 atomes choisis parmi carbone, hydrogène, oxygène, azote, et soufre ; dans lequel la combinaison d'atomes dans -(L)- est choisie dans le groupe constitué de (i) carbone et hydrogène seulement, (ii) un soufre seulement, (iii) un oxygène seulement, (iv) un ou deux azote et hydrogène seulement, (v) carbone, hydrogène, et un soufre seulement, et (vi) et carbone, hydrogène, et oxygène seulement ; et où $R^{80}$ est un groupe choisi parmi (a) ou (b);

$R^{21}$ est choisi parmi un hydrogène, un groupe alkyle en $C_1$-$C_{12}$, alcényle en $C_2$-$C_6$, alcynyle en $C_2$-$C_6$, aralkyle en $C_7$-$C_{12}$, alkaryle en $C_7$-$C_{12}$, cycloalkyle en $C_3$-$C_8$, cycloalcényle en $C_3$-$C_8$, phényle, tolulyle, xylényle, biphényle, alcoxy en $C_1$-$C_6$, alcényloxy en $C_2$-$C_6$, alcynyloxy en $C_2$-$C_6$, alcoxyalkyle en $C_1$-$C_{12}$, alcoxyalkyloxy en $C_1$-$C_{12}$, alkylcarbonyle en $C_1$-$C_{12}$, alkylcarbonylamino en $C_1$-$C_{12}$, alcoxyamino en $C_1$-$C_{12}$, alcoxyaminocarbonyle en $C_1$-$C_{12}$, alkylamino en $C_1$-$C_{12}$, alkylthio en $C_1$-$C_6$, alkylthiocarbonyle en $C_1$-$C_{12}$, alkylsulfinyle en $C_1$-$C_6$, alkylsulfonyle en $C_1$-$C_6$, halogénoalcoxy en $C_1$-$C_6$, halogénoalkylsulfonyle en $C_1$-$C_6$, halogénoalkyle en $C_1$-$C_6$, hydroxyalkyle en $C_1$-$C_6$, $-(CH_2)_nCN$, $-(CH_2)_nNR^9R^{10}$, -C(O)O(alkyle en $C_1$-$C_6$), $-(CH_2)_nO$(alkyle en $C_1$-$C_6$), benzyloxy, phénoxy, phénylthio, $-(CONHSO_2R)$, -CHO, amino, amidino, halogéno, carbamyle, carboxyle, carbalcoxy, $-(CH_2)_nCO_2H$, cyano, cyanoguanidinyle, guanidino, hydrazide, hydrazino, hydrazido, hydroxy, hydroxyamino, nitro, phosphono, $-SO_3H$, thioacétal, thiocarbonyle, et (alkyle en $C_1$-$C_6$)carbonyle ; où n vaut de 1 à 8 et $R^9$ et $R^{10}$ sont indépendamment un groupe alkyle en $C_1$-$C_4$ ou phényl(alkyle en $C_1$-$C_4$) ;

$R^{1'}$ est $-NHNH_2$ ou $-NH_2$ ;

$R^{2'}$ est choisi dans le groupe -OH, $-O(CH_2)_tR^5$ où $R^5$ est CN ou un groupe phényle, ou $-(L_a)$-(groupe acide) ; dans lequel le groupe acide est un groupe organique qui une fois attaché à un noyau tricyclique, par des atomes de liaison appropriés, agit comme un donneur de protons capable de liaison hydrogène et $-(L_a)$- est un groupe de liaison divalent ayant une longueur de lien de 1 à 7 et t vaut 1 à 5 ;

$R^{3'}$ est choisi parmi un hydrogène, un groupe alkyle en $C_1$-$C_{12}$, alcényle en $C_2$-$C_6$, alcynyle en $C_2$-$C_6$, aralkyle en $C_7$-$C_{12}$, alkaryle en $C_7$-$C_{12}$, cycloalkyle en $C_3$-$C_8$, cycloalcényle en $C_3$-$C_8$, phényle, tolulyle, xylényle, biphényle, alcoxy en $C_1$-$C_6$, alcényloxy en $C_2$-$C_6$, alcynyloxy en $C_2$-$C_6$, alcoxyalkyle en $C_1$-$C_{12}$, alcoxyalkyloxy en $C_1$-$C_{12}$, alkylcarbonyle en $C_1$-$C_{12}$, alkylcarbonylamino en $C_1$-$C_{12}$, alcoxyamino en $C_1$-$C_{12}$, alcoxyaminocarbonyle en $C_1$-$C_{12}$, alkylamino en $C_1$-$C_{12}$, alkylthio en $C_1$-$C_6$, alkylthiocarbonyle en $C_1$-$C_{12}$, alkylsulfinyle en $C_1$-$C_6$, alkylsulfonyle en $C_1$-$C_6$, halogénoalcoxy en $C_1$-$C_6$, halogénoalkylsulfonyle en $C_1$-$C_6$, halogénoalkyle en $C_1$-$C_6$, hydroxyalkyle en $C_1$-$C_6$, $-(CH_2)_nCN$, $-(CH_2)_nNR^9R^{10}$, -C(O)O(alkyle en $C_1$-$C_6$), $-(CH_2)_nO$(alkyle en $C_1$-$C_6$), benzyloxy, phénoxy, phénylthio, $-(CONHSO_2R)$, -CHO, amino, amidino, halogéno, carbamyle, carboxyle, carbalcoxy, $-(CH_2)_nCO_2H$, cyano, cyanoguanidinyle, guanidino, hydrazide, hydrazino, hydrazido, hydroxy, hydroxyamino, nitro, phosphono, $-SO_3H$, thioacétal, thiocarbonyle, et (alkyle en $C_1$-$C_6$)carbonyle ; où n vaut de 1 à 8 et $R^9$ et $R^{10}$ sont indépendamment un groupe alkyle en $C_1$-$C_4$ ou phényl(alkyle en $C_1$-$C_4$) ; des radicaux carbocycliques dérivés d'un noyau organique ayant 5 à 14 chaînons saturé ou insaturé dont les atomes formant le cycle (autres que l'hydrogène) sont seulement des atomes de carbone ; ou des radicaux hétérocycliques dérivés de noyaux hétérocycliques monocycliques ou polycycliques, saturés ou insaturés, ayant 5 à 14 atomes de cycle et contenant 1 à 3 hétéroatomes choisis dans le groupe constitué de l'azote, de l'oxygène ou du soufre ; et des radicaux carbocycliques dérivés d'un noyau organique ayant 5 à 14 chaînons saturé ou insaturé dont les atomes formant le cycle (autres que l'hydrogène) sont seulement des atomes de carbone ou des radicaux hétérocycliques dérivés de noyaux hétérocycliques monocycliques ou polycycliques, saturés ou insaturés, ayant 5 à 14 atomes de cycle et contenant 1 à 3 hétéroatomes choisis dans le groupe constitué de l'azote, de l'oxygène ou du soufre, substitués par des groupes choisis parmi un hydrogène, un groupe alkyle en $C_1$-$C_{12}$, alcényle en $C_2$-$C_6$, alcynyle en $C_2$-$C_6$, aralkyle en $C_7$-$C_{12}$, alkaryle en $C_7$-$C_{12}$, cy-

cloalkyle en $C_3$-$C_8$, cycloalcényle en $C_3$-$C_8$, phényle, tolulyle, xylényle, biphényle, alcoxy en $C_1$-$C_6$, alcényloxy en $C_2$-$C_6$, alcynyloxy en $C_2$-$C_6$, alcoxyalkyle en $C_1$-$C_{12}$, alcoxyalkyloxy en $C_1$-$C_{12}$, alkylcarbonyle en $C_1$-$C_{12}$, alkylcarbonylamino en $C_1$-$C_{12}$, alcoxyamino en $C_1$-$C_{12}$, alcoxyaminocarbonyle en $C_1$-$C_{12}$, alkylamino en $C_1$-$C_{12}$, alkylthio en $C_1$-$C_6$, alkylthiocarbonyle en $C_1$-$C_{12}$, alkylsulfinyle en $C_1$-$C_6$, alkylsulfonyle en $C_1$-$C_6$, halogénoalcoxy en $C_1$-$C_6$, halogénoalkylsulfonyle en $C_1$-$C_6$, halogénoalkyle en $C_1$-$C_6$, hydroxyalkyle en $C_1$-$C_6$, $-(CH_2)_nCN$, $-(CH_2)_nNR^9R^{10}$, -C(O)O(alkyle en $C_1$-$C_6$), $-(CH_2)_nO$(alkyle en $C_1$-$C_6$), benzyloxy, phénoxy, phénylthio, $-(CONHSO_2R)$, -CHO, amino, amidino, halogéno, carbamyle, carboxyle, carbalcoxy, $-(CH_2)_nCO_2H$, cyano, cyanoguanidinyle, guanidino, hydrazide, hydrazino, hydrazido, hydroxy, hydroxyamino, nitro, phosphono, $-SO_3H$, thioacétal, thiocarbonyle, et (alkyle en $C_1$-$C_6$)carbonyle ; où n vaut de 1 à 8 et $R^9$ et $R^{10}$ sont indépendamment un groupe alkyle en $C_1$-$C_4$ ou phényl(alkyle en $C_1$-$C_4$) ;
ou un de ses sels, racémates, solvates, tautomères, ou isomères optiques, pharmaceutiquement acceptables.

**8.** Utilisation, pour la fabrication d'un médicament destiné à inhiber sélectivement $sPLA_2$ chez un mammifère ayant besoin d'un tel traitement, d'un composé de formule (II)

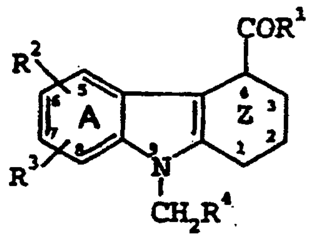

(II)

dans laquelle :

$R^1$ est $-NHNH_2$, ou $-NH_2$ ;
$R^2$ est -OH, ou $-O(CH_2)_mR^5$ où
$R^5$ est H, $-CO_2H$, $-CO_2$(alkyle en $C_1$-$C_4$),

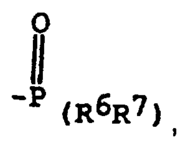

$-NHSO_2$(alkyle en $C_1$-$C_6$), $-CONHSO_2$(alkyle en $C_1$-$C_6$), -CN, un groupe tétrazolyle, phényle, ou phényle substitué par $-CO_2H$ ou $-CO_2$(alkyle en $C_1$-$C_4$) où $R^6$ et $R^7$ sont chacun indépendamment -OH ou -O(alkyle en $C_1$-$C_4$) et m vaut 1 à 3 ;
$R^3$ est H, -O(alkyle en $C_1$-$C_4$), ou $-(CH_2)_nR^8$ où
$R^8$ est H, $-NR^9R^{10}$,

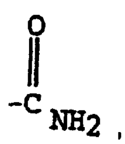

-CN, ou un groupe phényle où $R^9$ et $R^{10}$ sont indépendamment un groupe alkyle en $C_1$-$C_4$, ou phényl(alkyle en $C_1$-$C_4$) et n vaut 1 à 8 ;
$R^4$ est H, un groupe alkyle en $C_5$-$C_{14}$, cycloalkyle en $C_3$-$C_{14}$, phényle, ou phényle substitué par 1 ou 2 substituants choisis dans le groupe constitué d'un groupe alkyle en $C_1$-$C_4$, alcoxy en $C_1$-$C_4$, phényl(alkyle en $C_1$-$C_4$), (alkyle en $C_1$-$C_4$)thio, un halogène ou un groupe phényle ; et
Z est un groupe cyclohexényle, phényle, pyridyle dans lequel l'azote est à la position 1, 2, 3 ou 4 ou un noyau

hétérocyclique à 6 chaînons ayant un hétéroatome choisi dans le groupe constitué d'un soufre ou d'un oxygène à la position 1, 2 ou 3 et d'un azote à la position 1, 2, 3 ou 4, ou dans lequel un carbone sur le noyau hétérocyclique est éventuellement substitué par =O ;
A est un groupe phényle ou pyridyle dans lequel l'azote est à la position 5, 6, 7 ou 8 ;
ou un de ses sels, racémates, isomères optiques, tautomères ou solvates, pharmaceutiquement acceptables ;
pour autant que lorsque $R^3$ est H, $R^4$ est un groupe phényle, m vaut 1 ou 2 et $R^2$ est substitué en position 6, $R^5$ ne peut pas être H ; et
lorsque $R^1$ est $NHNH_2$, $R^8$ ne peut pas être

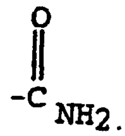

**9.** Utilisation selon la revendication 7 dans laquelle le mammifère est un humain.

**10.** Utilisation selon la revendication 8 dans laquelle le mammifère est un humain.

**11.** Utilisation selon la revendication 7 dans laquelle le médicament est destiné à soulager les effets pathologiques de la sepsie, le choc septique, le syndrome de la détresse respiratoire chez l'adulte, la pancréatite, le choc induit par un traumatisme, l'asthme, la polyarthrite rhumatoïde, l'ostéoarthrite, la bronchite aiguë, la bronchite chronique, l'infection inflammatoire du tube digestif, l'apoptose, l'ictus, la maladie fibro-kystique, la rhinite allergique, la bronchite aiguë, la bronchiolite chronique, la goutte, la spondylarthropathie, la spondylarthrite ankylosante, le syndrome de Reiter, l'arthropathie psoriasique, la spondylite entéropathique, l'arthropathie juvénile ou spondylarthrite ankylosante juvénile, l'arthropathie réactive, l'arthrite infectieuse ou post-infectieuse, l'arthrite gonococcique, l'arthrite tuberculeuse, l'arthrite virale, l'arthrite fongique, l'arthrite syphilitique, la maladie de Lyme,, l'arthrite associée à des "syndromes vasculitiques", la polyartérite noueuse, la vasculite d'hypersensibilité, la granulomatose de Luegenec, la polymyalgie rhumatismale, l'artérite à cellules articulaires, l'arthropathie à dépôt de cristaux calciques, la pseudo-goutte, le rhumatisme non articulaire, la bursite, la ténosynovite, l'épicondylite (coude du tennisman), le syndrome du tunnel carpien, la lésion due à une sur-utilisation répétitive (dactylographie), diverses formes d'arthrite, la maladie articulaire neuropathique (articulation et celle de Charcot), l'hémarthrose (hémarthrosique), le purpura de Henoch-Schonlein, l'ostéoarthropathie hypertrophique, la réticulo-histiocytose multicentrique, l'arthrite associée à certaines maladies, la surcolose, l'hémochromatose, la drépanocytose et autres hémoglobinopathies, l'hyperlipoproteinémie, l'hypogammaglobulinémie, l'hyperparathyroidisme, l'acromégalie, la fièvre méditerranéenne familiale, la maladie de Behat, le lupus érythémateux aigu disséminé, ou la polychondrite récurrente et maladies apparentées.

**12.** Utilisation de la revendication 8 dans laquelle le médicament est destiné à soulager les effets pathologiques suivants : la sepsie, le choc septique, le syndrome de la détresse respiratoire chez l'adulte, la pancréatite, le choc induit par un traumatisme, l'asthme, la polyarthrite rhumatoïde, l'ostéoarthrite, la bronchite aiguë, la bronchite chronique, l'infection inflammatoire du tube digestif, l'apoptose, l'ictus, la maladie fibro-kystique, la rhinite allergique, la bronchite aiguë, la bronchiolite chronique, la goutte, la spondylarthropathie, la spondylarthrite ankylosante, le syndrome de Reiter, l'arthropathie psoriasique, la spondylite entéropathique, l'arthropathie juvénile ou spondylarthrite ankylosante juvénile, l'arthropathie réactive, l'arthrite infectieuse ou post-infectieuse, l'arthrite gonococcique, l'arthrite tuberculeuse, l'arthrite virale, l'arthrite fongique, l'arthrite syphilitique, la maladie de Lyme,, l'arthrite associée à des "syndromes vasculitiques", la polyartérite noueuse, la vasculite d'hypersensibilité, la granulomatose de Luegenec, la polymyalgie rhumatismale, l'artérite à cellules articulaires, l'arthropathie à dépôt de cristaux calciques, la pseudo-goutte, le rhumatisme non articulaire, la bursite, la ténosynovite, l'épicondylite (coude du tennisman), le syndrome du tunnel carpien, la lésion due à une sur-utilisation répétitive (dactylographie), diverses formes d'arthrite, la maladie articulaire neuropathique (articulation et celle de Charcot), l'hémarthrose (hémarthrosique), le purpura de Henoch-Schonlein, l'ostéoarthropathie hypertrophique, la réticulo-histiocytose multicentrique, l'arthrite associée à certaines maladies, la surcolose, l'hémochromatose, la drépanocytose et autres hémoglobinopathies, l'hyperlipoproteinémie, l'hypogammaglobulinémie, l'hyperparathyroidisme, l'acromégalie, la fièvre méditerranéenne familiale, la maladie de Behat, le lupus érythémateux aigu disséminé, ou la polychondrite récurrente et maladies apparentées.

**13.** Utilisation selon la revendication 11 dans laquelle le mammifère est un humain.

**14.** Utilisation selon la revendication 12 dans laquelle le mammifère est un humain.

**15.** Procédé de préparation des composés de formule

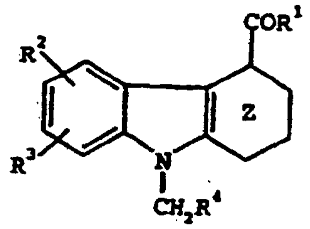

dans laquelle

$R^1$ est $-NHNH_2$, ou $-NH_2$ ;
$R^2$ est -OH, ou $-O(CH_2)_mR^5$ où
$R^5$ est H, $-CO_2H$, $-CO_2$(alkyle en $C_1$-$C_4$),

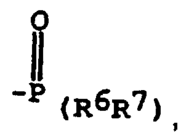

un groupe phényle, ou phényle substitué par $-CO_2H$ ou $-CO_2$(alkyle en $C_1$-$C_4$) où $R^6$ et $R^7$ sont chacun indépendamment -OH ou -O(alkyle en $C_1$-$C_4$) et m vaut 1 à 3 ;
$R^3$ est H, -O(alkyle en $C_1$-$C_4$), ou $-(CH_2)_nR^8$ où
$R^8$ est H, $-NR^9R^{10}$,

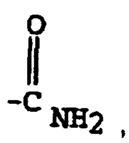

-CN, ou un groupe phényle où $R^9$ et $R^{10}$ sont indépendamment un groupe alkyle en $C_1$-$C_4$, ou phényl(alkyle en $C_1$-$C_4$) et n vaut 1 à 8 ;
$R_4$ est H, un groupe alkyle en $C_5$-$C_{14}$, cycloalkyle en $C_3$-$C_{14}$, phényle, ou phényle substitué par 1 ou 2 substituants choisis dans le groupe constitué d'un groupe alkyle en $C_1$-$C_4$, alcoxy en $C_1$-$C_4$, phényl(alkyle en $C_1$-$C_4$), (alkyle en $C_1$-$C_4$)thio, un halogène ou un groupe phényle ; et
Z est un groupe phényle, comprenant la déshydrogénation, un composé de formule

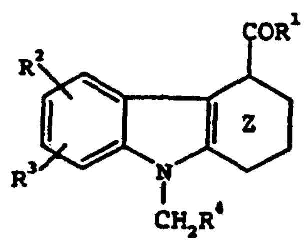

dans laquelle

$R^1$ est $-NHNH_2$, ou $-NN_2$ ;
$R^2$ est -OH, ou $-O(CH_2)_mR^5$ où
$R^5$ est H, $-CO_2H$, $-CO_2$(alkyle en $C_1$-$C_4$),

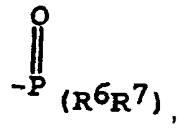

un groupe phényle, ou phényle substitué par $-CO_2H$ ou $-CO_2$(alkyle en $C_1$-$C_4$) où $R^6$ et $R^7$ sont chacun indépendamment -OH ou -O(alkyle en $C_1$-$C_4$) et m vaut 1 à 3 ;
$R^3$ est H, -O(alkyle en $C_1$-$C_4$), ou $-(CH_2)_nR^8$ où
$R^8$ est H, $-NR^9R^{10}$,

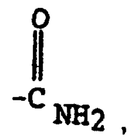

-CN, ou un groupe phényle où $R^9$ et $R^{10}$ sont indépendamment un groupe alkyle en $C_1$-$C_4$, ou phényl(alkyle en $C_1$-$C_4$)et n vaut 1 à 8 ;
$R^4$ est H, un groupe alkyle en $C_5$-$C_{14}$, cycloalkyle en $C_3$-$C_{14}$, phényle, ou phényle substitué par 1 ou 2 substituants choisis dans le groupe constitué d'un groupe alkyle en $C_1$-$C_4$, alcoxy en $C_1$-$C_4$, phényl(alkyle en $C_1$-$C_4$), (alkyle en $C_1$-$C_4$)thio, un halogène ou un groupe phényle ; et
Z est un groupe cyclohexényle.

**16.** Composé de formule III selon la revendication 1 pour le traitement des effets pathologiques suivants : la sepsie, le choc septique, le syndrome de la détresse respiratoire chez l'adulte, la pancréatite, le choc induit par un traumatisme, l'asthme, la polyarthrite rhumatoïde, l'ostéoarthrite, la bronchite aiguë, la bronchite chronique, l'infection inflammatoire du tube digestif, l'apoptose, l'ictus, la maladie fibro-kystique, la rhinite allergique, la bronchite aiguë, la bronchiolite chronique, la goutte, la spondylarthropathie, la spondylarthrite ankylosante, le syndrome de Reiter, l'arthropathie psoriasique, la spondylite entéropathique, l'arthropathie juvénile ou spondylarthrite ankylosante juvénile, l'arthropathie réactive, l'arthrite infectieuse ou post-infectieuse, l'arthrite gonococcique, l'arthrite tuberculeuse, l'arthrite virale, l'arthrite fongique, l'arthrite syphilitique, la maladie de Lyme,, l'arthrite associée à des "syndromes vasculitiques", la polyartérite noueuse, la vasculite d'hypersensibilité, la granulomatose de Luegenec, la polymyalgie rhumatismale, l'artérite à cellules articulaires, l'arthropathie à dépôt de cristaux calciques, la pseudo-goutte, le rhumatisme non articulaire, la bursite, la ténosynovite, l'épicondylite (coude du tennisman), le syndrome du tunnel carpien, la lésion due à une sur-utilisation répétitive (dactylographie), diverses formes d'arthrite, la maladie articulaire neuropathique (articulation et celle de Charcot), l'hémarthrose (hémarthrosique), le purpura de Henoch-Schonlein, l'ostéoarthropathie hypertrophique, la réticulo-histiocytose multicentrique, l'arthrite associée à certaines maladies, la surcolose, l'hémochromatose, la drépanocytose et autres hémoglobinopathies, l'hyperlipoproteinémie, l'hypogammaglobulinémie, l'hyperparathyroidisme, l'acromégalie, la fièvre méditerranéenne familiale, la maladie de Behat, le lupus érythémateux aigu disséminé, ou la polychondrite récurrente et maladies apparentées.

**17.** Composé de formule I selon l'une quelconque des revendications 2 à 4 pour le traitement des effets pathologiques suivants : la sepsie, le choc septique, le syndrome de la détresse respiratoire chez l'adulte, la pancréatite, le choc induit par un traumatisme, l'asthme, la polyarthrite rhumatoïde, l'ostéoarthrite, la bronchite aiguë, la bronchite chronique, l'infection inflammatoire du tube digestif, l'apoptose, l'ictus, la maladie fibro-kystique, la rhinite allergique, la bronchite aiguë, la bronchiolite chronique, la goutte, la spondylarthropathie, la spondylarthrite ankylosante, le syndrome de Reiter, l'arthropathie psoriasique, la spondylite entéropathique, l'arthropathie juvénile ou spondylarthrite ankylosante juvénile, l'arthropathie réactive, l'arthrite infectieuse ou post-infectieuse, l'arthrite gonococcique, l'arthrite tuberculeuse, l'arthrite virale, l'arthrite fongique, l'arthrite syphilitique, la maladie de Lyme,, l'arthrite associée à des "syndromes vasculitiques", la polyartérite noueuse, la vasculite d'hypersensibilité, la granulomatose de Luegenec, la polymyalgie rhumatismale, l'artérite à cellules articulaires, l'arthropathie à dépôt de cristaux

calciques, la pseudo-goutte, le rhumatisme non articulaire, la bursite, la ténosynovite, l'épicondylite (coude du tennisman), le syndrome du tunnel carpien, la lésion due à une sur-utilisation répétitive (dactylographie), diverses formes d'arthrite, la maladie articulaire neuropathique (articulation et celle de Charcot), l'hémarthrose (hémarthrosique), le purpura de Henoch-Schonlein, l'ostéoarthropathie hypertrophique, la réticulo-histiocytose multicentrique, l'arthrite associée à certaines maladies, la surcolose, l'hémochromatose, la drépanocytose et autres hémoglobinopathies, l'hyperlipoproteinémie, l'hypogammaglobulinémie, l'hyperparathyroidisme, l'acromégalie, la fièvre méditerranéenne familiale, la maladie de Behat, le lupus érythémateux aigu disséminé, ou la polychondrite récurrente et maladies apparentées.

**18.** Utilisation d'un composé de formule III selon la revendication 1 pour la fabrication d'un médicament destiné au traitement des effets pathologiques suivants : la sepsie, le choc septique, le syndrome de la détresse respiratoire chez l'adulte, la pancréatite, le choc induit par un traumatisme, l'asthme, la polyarthrite rhumatoïde, l'ostéoarthrite, la bronchite aiguë, la bronchite chronique, l'infection inflammatoire du tube digestif, l'apoptose, l'ictus, la maladie fibro-kystique, la rhinite allergique, la bronchite aiguë, la bronchiolite chronique, la goutte, la spondylarthropathie, la spondylarthrite ankylosante, le syndrome de Reiter, l'arthropathie psoriasique, la spondylite entéropathique, l'arthropathie juvénile ou spondylarthrite ankylosante juvénile, l'arthropathie réactive, l'arthrite infectieuse ou post-infectieuse, l'arthrite gonococcique, l'arthrite tuberculeuse, l'arthrite virale, l'arthrite fongique, l'arthrite syphilitique, la maladie de Lyme,, l'arthrite associée à des "syndromes vasculitiques", la polyartérite noueuse, la vasculite d'hypersensibilité, la granulomatose de Luegenec, la polymyalgie rhumatismale, l'artérite à cellules articulaires, l'arthropathie à dépôt de cristaux calciques, la pseudo-goutte, le rhumatisme non articulaire, la bursite, la ténosynovite, l'épicondylite (coude du tennisman), le syndrome du tunnel carpien, la lésion due à une sur-utilisation répétitive (dactylographie), diverses formes d'arthrite, la maladie articulaire neuropathique (articulation et celle de Charcot), l'hémarthrose (hémarthrosique), le purpura de Henoch-Schonlein, l'ostéoarthropathie hypertrophique, la réticulo-histiocytose multicentrique, l'arthrite associée à certaines maladies, la surcolose, l'hémochromatose, la drépanocytose et autres hémoglobinopathies, l'hyperlipoproteinémie, l'hypogammaglobulinémie, l'hyperparathyroidisme, l'acromégalie, la fièvre méditerranéenne familiale, la maladie de Behat, le lupus érythémateux aigu disséminé, ou la polychondrite récurrente et maladies apparentées.

**19.** Utilisation d'un composé de formule I selon l'une quelconque des revendications 2 à 4 pour la fabrication d'un médicament destiné au traitement des effets pathologiques suivants : la sepsie, le choc septique, le syndrome de la détresse respiratoire chez l'adulte, la pancréatite, le choc induit par un traumatisme, l'asthme, la polyarthrite rhumatoïde, l'ostéoarthrite, la bronchite aiguë, la bronchite chronique, l'infection inflammatoire du tube digestif, l'apoptose, l'ictus, la maladie fibro-kystique, la rhinite allergique, la bronchite aiguë, la bronchiolite chronique, la goutte, la spondylarthropathie, la spondylarthrite ankylosante, le syndrome de Reiter, l'arthropathie psoriasique, la spondylite entéropathique, l'arthropathie juvénile ou spondylarthrite ankylosante juvénile, l'arthropathie réactive, l'arthrite infectieuse ou post-infectieuse, l'arthrite gonococcique, l'arthrite tuberculeuse, l'arthrite virale, l'arthrite fongique, l'arthrite syphilitique, la maladie de Lyme,, l'arthrite associée à des "syndromes vasculitiques", la polyartérite noueuse, la vasculite d'hypersensibilité, la granulomatose de Luegenec, la polymyalgie rhumatismale, l'artérite à cellules articulaires, l'arthropathie à dépôt de cristaux calciques, la pseudo-goutte, le rhumatisme non articulaire, la bursite, la ténosynovite, l'épicondylite (coude du tennisman), le syndrome du tunnel carpien, la lésion due à une sur-utilisation répétitive (dactylographie), diverses formes d'arthrite, la maladie articulaire neuropathique (articulation et celle de Charcot), l'hémarthrose (hémarthrosique), le purpura de Henoch-Schonlein, l'ostéoarthropathie hypertrophique, la réticulo-histiocytose multicentrique, l'arthrite associée à certaines maladies, la surcolose, l'hémochromatose, la drépanocytose et autres hémoglobinopathies, l'hyperlipoproteinémie, l'hypogammaglobulinémie, l'hyperparathyroidisme, l'acromégalie, la fièvre méditerranéenne familiale, la maladie de Behat, le lupus érythémateux aigu disséminé, ou la polychondrite récurrente et maladies apparentées.

**20.** Formulation pharmaceutique adaptée au traitement des effets pathologiques suivants: la sepsie, le choc septique, le syndrome de la détresse respiratoire chez l'adulte, la pancréatite, le choc induit par un traumatisme, l'asthme, la polyarthrite rhumatoïde, l'ostéoarthrite, la bronchite aiguë, la bronchite chronique, l'infection inflammatoire du tube digestif, l'apoptose, l'ictus, la maladie fibro-kystique, la rhinite allergique, la bronchite aiguë, la bronchiolite chronique, la goutte, la spondylarthropathie, la spondylarthrite ankylosante, le syndrome de Reiter, l'arthropathie psoriasique, la spondylite entéropathique, l'arthropathie juvénile ou spondylarthrite ankylosante juvénile, l'arthropathie réactive, l'arthrite infectieuse ou post-infectieuse, l'arthrite gonococcique, l'arthrite tuberculeuse, l'arthrite virale, l'arthrite fongique, l'arthrite syphilitique, la maladie de Lyme,, l'arthrite associée à des "syndromes vasculitiques", la polyartérite noueuse, la vasculite d'hypersensibilité, la granulomatose de Luegenec, la polymyalgie rhumatismale, l'artérite à cellules articulaires, l'arthropathie à dépôt de cristaux calciques, la pseudo-goutte, le rhumatisme non articulaire, la bursite, la ténosynovite, l'épicondylite (coude du tennisman), le syndrome du tunnel

carpien, la lésion due à une sur-utilisation répétitive (dactylographie), diverses formes d'arthrite, la maladie articulaire neuropathique (articulation et celle de Charcot), l'hémarthrose (hémarthrosique), le purpura de Henoch-Schonlein, l'ostéoarthropathie hypertrophique, la réticulo-histiocytose multicentrique, l'arthrite associée à certaines maladies, la surcolose, l'hémochromatose, la drépanocytose et autres hémoglobinopathies, l'hyperlipoproteinémie, l'hypogammaglobulinémie, l'hyperparathyroidisme, l'acromégalie, la fièvre méditerranéenne familiale, la maladie de Behat, le lupus érythémateux aigu disséminé, ou la polychondrite récurrente et maladies apparentées, contenant un composé de formule III selon la revendication 1.

**21.** Utilisation comme dans l'une quelconque des revendications 7 à 10 dans laquelle le médicament est destiné à soulager les effets pathologiques suivants: la sepsie, le choc septique, le syndrome de la détresse respiratoire chez l'adulte, la pancréatite, le choc induit par un traumatisme, l'asthme, la polyarthrite rhumatoïde, l'ostéoarthrite, la bronchite aiguë, la bronchite chronique, l'infection inflammatoire du tube digestif, l'apoptose, l'ictus, la maladie fibro-kystique, la rhinite allergique, la bronchite aiguë, la bronchiolite chronique, la goutte, la spondylarthropathie, la spondylarthrite ankylosante, le syndrome de Reiter, l'arthropathie psoriasique, la spondylite entéropathique, l'arthropathie juvénile ou spondylarthrite ankylosante juvénile, l'arthropathie réactive, l'arthrite infectieuse ou post-infectieuse, l'arthrite gonococcique, l'arthrite tuberculeuse, l'arthrite virale, l'arthrite fongique, l'arthrite syphilitique, la maladie de Lyme,, l'arthrite associée à des "syndromes vasculitiques", la polyartérite noueuse, la vasculite d'hypersensibilité, la granulomatose de Luegenec, la polymyalgie rhumatismale, l'artérite à cellules articulaires, l'arthropathie à dépôt de cristaux calciques, la pseudo-goutte, le rhumatisme non articulaire, la bursite, la ténosynovite, l'épicondylite (coude du tennisman), le syndrome du tunnel carpien, la lésion due à une sur-utilisation répétitive (dactylographie), diverses formes d'arthrite, la maladie articulaire neuropathique (articulation et celle de Charcot), l'hémarthrose (hémarthrosique), le purpura de Henoch-Schonlein, l'ostéoarthropathie hypertrophique, la réticulo-histiocytose multicentrique, l'arthrite associée à certaines maladies, la surcolose, l'hémochromatose, la drépanocytose et autres hémoglobinopathies, l'hyperlipoproteinémie, l'hypogammaglobulinémie, l'hyperparathyroidisme, l'acromégalie, la fièvre méditerranéenne familiale, la maladie de Behat, le lupus érythémateux aigu disséminé, ou la polychondrite récurrente et maladies apparentées.